# EUROPEAN PATENT APPLICATION

(11) **EP 4 509 132 A2**
(43) Date of publication of application: **19.02.2025**
(21) Application number: 24208063.8
(22) Date of filing: 25.05.2018
(51) Int. Cl.: A61K 38/17

(54) **MULTIFUNCTIONAL ANTIBODY-LIGAND TRAPS TO MODULATE IMMUNE TOLERENCE**

(30) Priority: 26.05.2017 US 201762511911 P; 29.11.2017 US 201762592341 P
(62) Divisional of application: 18805109.8
(71) Applicant: The Johns Hopkins University, Baltimore, MD 21218 (US)
(72) Inventor: BEDI, Atul, Baltimore 21218 (US); RAVI, Rajani, Baltimore 21218 (US)
(74) Representative: Murgitroyd & Company

(57) **Abstract**

The invention provides multifunctional antibody-ligand traps and methods of using them to counteract immune tolerance and/or immune dysfunction. The multifunctional antibody-ligand traps and fusion proteins of the invention can counteract immune dysfunction in order to restore and unleash antitumor or pathogen-directed immune responses. Provided here are promising immunotherapeutic agents for treatment and prevention of cancers, infectious diseases, and immuno-inflammatory disorders.

## Description

### CROSS-REFERENCE TO RELATED APPLICATIONS

This application claims the benefit of priority under 35 U.S.C. § 119(e) of U.S. Applications Serial Nos 62/511,911, filed May 26, 2017, and 62/592,341, filed November 29, 2017. The disclosure of the prior applications is considered part of and is incorporated by reference in the disclosure of this application in its entirety.

### INCORPORATION OF SEQUENCE LISTING

The material in the accompanying sequence listing is hereby incorporated by reference into this application. The accompanying sequence listing text file, JHU4100_2WO_Sequence_Listing.txt was created May 25, 2018 and is 1,145 kb. The file can be assessed using Microsoft Word on a computer that uses Windows OS.

### BACKGROUND OF THE INVENTION

### FIELD OF THE INVENTION

The present invention relates generally to the field of targeted immunomodulatory molecules and fusion proteins and more specifically to compositions and methods for targeted immunostimulatory or immunosuppressive molecules and fusion proteins to counteract or induce immune tolerance in cells.

### BACKGROUND INFORMATION

The immune system provides the human body with a means to recognize and defend itself against microorganisms and substances recognized as foreign or potentially harmful. While passive immunotherapy of cancer with monoclonal antibodies and passive transfer of T cells to attack tumor cells have demonstrated clinical efficacy, the goal of active immunotherapy to induce these immune effectors and establish immunological memory against tumor cells has remained challenging. Several tumor-specific and tumor-associated antigens have been identified, yet these antigens are generally weakly immunogenic and tumors employ diverse mechanisms to create a tolerogenic environment that allows them to evade immunologic attack. Strategies to overcome such immune tolerance and activating robust levels of antibody and/or T cell responses hold the key to effective cancer immunotherapy.

### SUMMARY OF THE INVENTION

The present invention is based on the seminal discovery that targeted immunomodulatory antibodies and fusion proteins can counteract or reverse immune tolerance, abnormal T cell differentiation, and T cell dysfunction or exhaustion in cancers, inflammatory disorders, and chronic infectious diseases.

In one embodiment the present invention provides molecules comprising a targeting moiety and one or more immunomodulatory moiety/moieties, wherein the targeting moiety comprises a polypeptide which specifically binds a component of a tumor cell, tumor microenvironment, tumor associated growth factor or receptor, tumor associated cytokine or receptor, tumor associated T lymphocyte, T cell co-stimulatory or inhibitory molecule, immune cell, pathogen, or pathogen-associated cell, and the immunomodulatory moiety comprises the extracellular domain (ECD) sequence or ligand binding fragment thereof of a T lymphocyte immunoreceptor, T cell inhibitory receptor (TCIR), T-cell co-inhibitory molecule, T cell co-stimulatory molecule, B lymphocyte receptor, DC receptor, NK cell receptor, cytokine receptor, growth factor receptor, chemokine receptor, or tumor cell receptor.

In one aspect, the targeting moiety polypeptide comprises an antigen-binding domain of an immunoglobulin, antibody, bispecific or multispecific antibody, antibody fragment, single chain variable fragment (scFv), bivalent or multivalent scFv, or Fc-containing polypeptide. In another aspect, the targeting moiety comprises a ligand-binding sequence from the extracellular domain (ECD) of a receptor.

In certain aspects, the targeting moiety is an antibody that specifically binds a T cell inhibitory receptor (TCIR), a T cell inhibitory receptor ligand (TCIR ligand), a T-cell co-inhibitory molecule, or a T cell co-stimulatory molecule. In an additional aspect, the antibody is an antagonist of a TCIR, TCIR ligand, or co-inhibitory molecule. In another aspect, the antibody is an agonist of a T cell co-stimulatory molecule. In an additional aspect, the targeting moiety polypeptide specifically binds one or more of the following molecules: Cytotoxic T lymphocyte associated antigen-4 (CTLA-4, CD 152), Programmed Death-1 protein (PD-1), Programmed death ligand-1 (PD-L1), Programmed death ligand (PD-L2), B7-H3 (CD276), T-cell immunoglobulin and mucin-domain containing-3 (TIM-3), Carcinoembryonic antigen-related cell adhesion molecule 1 (CEACAM-1), Carcinoembryonic Antigen (CEA), V domain Ig suppressor of T cell activation (VISTA), V-set and immunoglobulin domain containing 8 (VSIG8), B and T lymphocyte attenuator (BTLA), Herpesvirus Entry Mediator (HVEM), CD160, T cell Ig and ITM domain (TIGIT), CD226, CD96, Lymphocyte activation gene-3 (LAG-3), transforming growth factor β (TGF-β), transforming growth factor β receptor (TGFβR), Receptor Activator of Nuclear Factor κ B (RANK), RANK ligand (RANKL), 4-1BB (CD137), Inducible T-Cell Costimulator (ICOS), OX-40 (CD134), glucocorticoid-induced TNFR-related protein (GITR), CD27, IL6R, IL23R, IL17R, IL-6, IL-23, IL-17, CD39, CD40, CD40L, CD47, CD122, CD73, CCR4, CCR5, CXCR4, IL12R, CD4, IL-2R, CD25, CD3. In certain aspects, the targeting moiety polypeptide specifically binds one or more of the following: Epidermal growth factor receptor (EGFR, EGFR1, ErbB-1, HER1), ErbB-2 (HER2/neu), ErbB-3/HER3, ErbB-4/HER4, Epidermal growth factor (EGF), Transforming growth factor α (TGFα), Vascular endothelial growth factor (VEGF), Vascular endothelial growth factor receptor-1 (VEGFR-1), VEGFR-2 or VEGFR-3. In certain aspects, the targeting moiety polypeptide specifically binds the human immunodeficiency virus gp120 protein (HIV gp 120).

In various aspects, the immunomodulatory moiety comprises the extracellular ligand-binding sequence or ligand binding fragment thereof of transforming growth factor β receptor II (TGFβRII), programmed death 1 protein (PD-1), T cell immunoglobulin and mucin domain containing 3 (TIM-3), B- and T-lymphocyte attenuator (BTLA), CD160, CD226, T cell Ig and ITM domain (TIGIT), CD96, CD44, Colony stimulating factor 1 receptor (CSF1R), CCR4, Killer-cell immunoglobulin-like receptor (KIR), Vascular endothelial growth factor receptor (VEGFR), Receptor Activator of Nuclear Factor κ B (RANK), V-set and immunoglobulin domain containing 8 (VSIG8), LIGHT (TNFSF14), Leukocyte-associated immunoglobulin-like receptor 1 (LAIR1), or V domain Ig suppressor of T cell activation (VISTA).

In some aspects, the molecule comprises a linker and the linker may have the sequence of SEQ ID NO: 3. In a further aspect, the linker fuses the targeting moiety and the immunomodulatory moiety.

In some aspects, the immunomodulatory moiety is fused to the heavy chain of a targeting moiety comprising an antibody. In other aspects, the immunomodulatory moiety is fused to the light chain of a targeting moiety comprising an antibody. In an additional aspect, one immunomodulatory moiety (IM-1) is fused to the heavy chain of a targeting moiety comprising an antibody, and a second immunomodulatory moiety (IM-2) is fused to the light chain of the same targeting antibody.

In one aspect, the targeting moiety is fused to an immunomodulatory moiety that comprises the ECD or a ligand binding fragment thereof of TIM-3. In a specific aspect, the ligand binding fragment comprises the CEACAM-1 binding fragment of TIM-3. In a specific aspect, the immunomodulatory moiety comprises a ligand-binding sequence of the TIM-3 extracellular domain (TIM-3 ectodomain; TIM-3ecd) corresponding to SEQ ID NO: 2.

In one aspect, the targeting moiety comprises a polypeptide that binds to PD-1, PD-L1, CTLA4, LAG3, VISTA, TIGIT, BTLA, CCR4, 41BB, OX40, GITR, VEGF, RANKL, TGF-β, IL6R, EGFR, HER2/neu, or gp120, and the immunomodulatory moiety is the ECD or a ligand binding fragment thereof of TIM-3. In a specific aspect, the immunomodulatory moiety comprises the CEACAM-1 binding fragment of TIM-3. In a specific aspect, the immunomodulatory moiety comprises a ligand-binding sequence of the TIM-3ecd corresponding to SEQ ID NO: 2 .

In certain aspects, the targeting moiety comprises a polypeptide that binds PD-1 or PD-L1 and the immunomodulatory moiety comprises the ECD or ligand binding fragment thereof of TIM-3. In specific aspects, the molecule comprises the sequence of SEQ ID NO: 51, 52, 53 or 192. In specific aspects, the molecule comprises the sequence of SEQ ID NO: 282, 288, 274, 278. In specific aspects, the molecule comprises the sequences corresponding to SEQ ID NO: 51 and SEQ ID NO: 185. In specific aspects, the molecule comprises the sequences corresponding to SEQ ID NO: 52 and SEQ ID NO: 186. In specific aspects, the molecule comprises the sequences corresponding to SEQ ID NO: 53 and SEQ ID NO: 187. In specific aspects the molecule comprises the sequences corresponding to SEQ ID NO: 192 and SEQ ID NO: 193.

In one aspect, the targeting moiety comprises a polypeptide that binds CTLA-4 and the immunomodulatory moiety comprises the ECD or ligand binding fragment thereof of TIM-3. In a specific aspect, the molecule comprises the sequence of SEQ ID NO: 50. In specific aspects, the molecule comprises the sequence of SEQ ID NO: 204. In specific aspects, the molecule comprises the sequences corresponding to SEQ ID NO: 50 and SEQ ID NO: 184.

In another aspect, the targeting moiety comprises a polypeptide that binds LAG-3 and the immunomodulatory moiety comprises the ECD or ligand binding fragment thereof of TIM-3. In one aspect, the molecule comprises the sequence of SEQ ID NO: 26. In specific aspects, the molecule comprises the sequences corresponding to SEQ ID NO: 26 and SEQ ID NO: 161.

In an additional aspect, the targeting moiety comprises a polypeptide that binds VISTA and the immunomodulatory moiety comprises the ECD or ligand binding fragment thereof of TIM-3. In a further aspect, the molecule comprises the sequence of SEQ ID NO: 12. In specific aspects the molecule comprises the sequences corresponding to SEQ ID NO: 12 and SEQ ID NO: 147.

In one aspect, the targeting moiety comprises a polypeptide that binds TIGIT and the immunomodulatory moiety comprises the ECD or ligand binding fragment thereof of TIM-3. In a specific aspect, the molecule comprises the sequence of SEQ ID NO: 17. In specific aspects, the molecule comprises the sequences corresponding to SEQ ID NO: 17 and SEQ ID NO: 152.

In another aspect, the targeting moiety comprises a polypeptide that binds BTLA and the immunomodulatory moiety comprises the ECD or ligand binding fragment thereof of TIM-3. In an additional, aspect, the molecule comprises the sequence of SEQ ID NO: 22. In specific aspects, the molecule comprises the sequences corresponding to SEQ ID NO: 22 and SEQ ID NO: 157.

In an aspect, the targeting moiety comprises a polypeptide that binds CCR-4 and the immunomodulatory moiety comprises the ECD or ligand binding fragment thereof of TIM-3. In certain aspects, the molecule comprises the sequence of SEQ ID NO: 31. In specific aspects, the molecule comprises the sequences corresponding to SEQ ID NO: 31 and SEQ ID NO: 166.

In a further aspect, the targeting moiety comprises a polypeptide that binds 4-1BB and the immunomodulatory moiety comprises the ECD or ligand binding fragment thereof of TIM-3. In a specific aspect, the molecule comprises the sequence of SEQ ID NO: 116. In specific aspects, the molecule comprises the sequences corresponding to SEQ ID NO: 116 and SEQ ID NO: 117.

In one aspect, the targeting moiety comprises a polypeptide that binds VEGF and the immunomodulatory moiety comprises the ECD or ligand binding fragment thereof of TIM-3. In certain aspects, the molecule comprises the sequence of SEQ ID NO: 88. In specific aspects, the molecule comprises the sequence of SEQ ID NO: 252. In specific aspects, the molecule comprises the sequences corresponding to SEQ ID NO: 88 and SEQ ID NO: 89.

In one aspect, the targeting moiety comprises a polypeptide that binds RANKL and the immunomodulatory moiety comprises the ECD or ligand binding fragment thereof of TIM-3.

In one aspect, the targeting moiety comprises a polypeptide that binds IL6-R and the immunomodulatory moiety comprises the ECD or ligand binding fragment thereof of TIM-3. In certain aspects, the molecule comprises the sequence of SEQ ID NO: 219. In specific aspects, the molecule comprises the sequence of SEQ ID NO: 216. In specific aspects, the molecule comprises the sequences corresponding to SEQ ID NO: 219 and 61.

In one aspect, the targeting moiety comprises a polypeptide that binds EGFR and the immunomodulatory moiety comprises the ECD or ligand binding fragment thereof of TIM-3. In certain aspects, the molecule comprises the sequence of SEQ ID NO: 231. In specific aspects, the molecule comprises the sequence of SEQ ID NO: 228. In specific aspects the molecule comprises the sequences corresponding to SEQ ID NO: 231 and SEQ ID NO: 75.

In one aspect, the targeting moiety comprises a polypeptide that binds HER2/Neu and the immunomodulatory moiety comprises the ECD or ligand binding fragment thereof of TIM-3. In certain aspects, the molecule comprises the sequence of SEQ ID NO: 102. In specific aspects, the molecule comprises the sequence of SEQ ID NO: 240. In specific aspects, the molecule comprises the sequences corresponding to SEQ ID NO: 102 and SEQ ID NO: 103.

In one aspect, the targeting moiety comprises a polypeptide that binds HIV gp120 and the immunomodulatory moiety comprises the ECD or ligand binding fragment thereof of TIM-3. In certain aspects, the molecule comprises the sequence of SEQ ID NO: 267. In specific aspects, the molecule comprises the sequence of SEQ ID NO: 264. In specific aspects, the molecule comprises the sequences corresponding to SEQ ID NO: 267 and light chain of gp120 antibody.

In another aspect, the targeting moiety comprises fused to an immunomodulatory moiety that comprises the ECD or a ligand binding fragment thereof of PD-1. In certain aspects, this ligand binding fragment comprises a PD-L1 or PD-L2 binding fragment. In a specific aspect, the immunomodulatory moiety comprises a ligand-binding sequence of the PD-1 extracellular domain (PD-1ecd) corresponding to SEQ ID NO: 194. In a further aspect, the targeting moiety comprises a polypeptide that binds to TIM-3, CEACAM-1, BTLA, CCR4, CTLA4, LAG3, TIGIT, VISTA, 41BB, OX40, GITR, RANKL, TGF-β or VEGF and the immunomodulatory moiety comprises the ECD or a ligand binding fragment thereof of PD-1.

In another aspect, the targeting moiety comprises fused to an immunomodulatory moiety that comprises the ECD or a ligand binding fragment thereof of TGFβ receptor. In certain aspects, this ligand binding fragment comprises a TGFβ binding fragment of TGFβRII. In a specific aspect, the immunomodulatory moiety comprises a ligand-binding sequence of the TGFβRII extracellular domain (TGFβRII ecd) corresponding to SEQ ID NO: 6. In certain aspects, the targeting moiety comprises a polypeptide that binds to TIM-3, CEACAM-1, BTLA, CCR4, CTLA4, LAG3, PD-1, PD-L1, TIGIT, VISTA, 41BB, OX40, GITR, RANKL, or VEGF and the immunomodulatory moiety comprises the ECD or a ligand binding fragment thereof of TGFβRII.

In an additional aspect, the targeting moiety comprises fused to an immunomodulatory moiety that comprises the ECD or ligand binding fragment thereof of BTLA, CD160, or LIGHT. In certain aspects, the ligand binding fragment comprises an Herpesvirus entry mediator (HVEM, CD 270) binding fragment. In a specific aspect, the immunomodulatory moiety comprises a ligand-binding sequence of the BTLA extracellular domain (BTLA ecd) corresponding to SEQ ID NO: 8. In a specific aspect, the immunomodulatory moiety comprises a ligand-binding sequence of the LIGHT extracellular domain (LIGHT ecd) corresponding to SEQ ID NO: 302. In certain aspects, the targeting moiety comprises a polypeptide that binds to TIM-3, CEACAM-1, CCR4, CTLA4, LAG3, PD-1, PD-L1, TIGIT, VISTA, 41BB, OX40, GITR, RANKL, or VEGF and the immunomodulatory moiety comprises the ECD or a ligand binding fragment thereof of BTLA, CD160, or LIGHT.

In an additional aspect, the targeting moiety is fused to an immunomodulatory moiety that comprises the ECD or ligand binding fragment thereof of TIGIT or CD96. In one aspect, this ligand binding fragment comprises a CD155 or CD112 binding fragment. In certain aspects, the immunomodulatory moiety comprises the ECD or a ligand binding fragment thereof of TIGIT. In a specific aspect, the immunomodulatory moiety comprises a ligand-binding sequence of the TIGIT extracellular domain (TIGIT ECD) corresponding to SEQ ID NO: 289 . In a specific aspect, the immunomodulatory moiety comprises a ligand-binding sequence of the CD96 extracellular domain (CD96 ECD) corresponding to SEQ ID NO: 301. In another aspect, the targeting moiety comprises a polypeptide that binds to TIM-3, CEACAM-1, BTLA, CCR4, CTLA4, LAG3, PD-1, PD-L1, VISTA, 41BB, OX40, GITR, RANKL, TGF-β or VEGF and the immunomodulatory moiety comprises the ECD or a ligand binding fragment thereof of TIGIT.

In another aspect, the targeting moiety is fused to an immunomodulatory moiety that comprises the ECD or a ligand binding fragment thereof of CD226. In certain aspects, this ligand binding fragment comprises a CD155 or CD112 binding fragment. In a specific aspect, the immunodulatory moiety comprises a ligand-binding sequence of the CD226 extracellular domain (CD226 ECD) corresponding to SEQ ID NO: 9 . In another aspect, the targeting moiety comprises a polypeptide that binds to TIM-3, CEACAM-1, BTLA, CCR4, CTLA4, LAG3, PD-1, PD-L1, TIGIT, VISTA, 41BB, OX40, GITR, RANKL, TGF-β or VEGF and the immunomodulatory moiety comprises the ECD or a ligand binding fragment thereof of CD226.

In another aspect, the targeting moiety is fused to an immunomodulatory moiety that comprises the ECD or a ligand binding fragment thereof of VSIG8. In certain aspects, this ligand binding fragment comprises a VISTA binding fragment. In a specific aspect, the immunomodulatory moiety comprises a ligand-binding sequence of the VSIG8 extracellular domain (VSIG8 ecd) corresponding to SEQ ID NO: 55. In another aspect, the targeting moiety comprises a polypeptide that binds to TIM-3, CEACAM-1, BTLA, CCR4, CTLA4, LAG3, PD-1, PD-L1, TIGIT, 41BB, OX40, GITR, RANKL, TGF-β or VEGF and the immunomodulatory moiety comprises the ECD or a ligand binding fragment thereof of VSIG8.

In an additional aspect, the targeting moiety comprises a polypeptide that binds to TIM-3, CEACAM-1, BTLA, CCR4, CTLA4, LAG3, PD-1, PD-L1, TIGIT, VISTA, 41BB, OX40, GITR, RANKL, TGF-β or VEGF and the immunomodulatory moiety comprises the ECD or a ligand binding fragment thereof of CD44. In a specific aspect, the immunomodulatory moiety is a ligand-binding sequence of CD44 extracellular domain (CD44 ecd) corresponding to SEQ ID NO: 36.

In a further aspect, the immunomodulatory moiety comprises the ECD or ligand binding fragment thereof of CCR4 and the ligand binding fragment comprises a CCL22 binding fragment.
In an aspect, the molecule comprises the sequences of SEQ ID NOs: 10 and 145; 11 and 146; 12 and 147; 13 and 148; 14 and 149; 15 and 150; 16 and 151; 17 and 152; 18 and 153; 19 and 154, 20 and 155; 21 and 156; 22 and 157; 23 and 158; 24 and 159; 25 and 160; 26 and 161; 27 and 162; 28 and 163; 29 and 164; 30 and 165; 31 and 166; 32 and 167; 33 and 168; 34 and 169; 35 and 170; 37 and 171; 38 and 172; 39 and 173; 40 and 174; 41 and 175; 42 and 176; 43 and 177; 44 and 178; 45 and 179; 46 and 180; 47 and 181; 48 and 182; 49 and 183; 50 and 184; 51 and 185; 52 and 186; 53 and 187; 58 and 188; 59 and 189; 60 and 61; 62 and 63; 64 and 65; 66 and 67; 68 and 69; 70 and 71; 72 and 73; 74 and 75; 76 and 77; 78 and 79; 80 and 81; 82 and 83; 84 and 85; 86 and 87; 88 and 89; 90 and 91; 92 and 93; 94 and 95;, 96 and 97; 98 and 99; 100 and 101; 102 and 103; 104 and 105; 106 and 107; 108 and 109; 110 and 111; 112 and 113; 114 and 115; 116 and 117; 118 and 119; 120 and 121; 122 and 123; 124 and 125; 126 and 127;, 128 and 129; 130 and 131; 132 and 133; 134 and 135; 136 and 137; 138 and 139; 140 and 141; 142 and 143; 192 and 193; 209 and 210; 211 and 212; 221 and 222; 223 and 224; 233 and 234; 235 and 236; 245 and 246; 247 and 248; 257 and 258; 259 and 260; 269 and 270; 271 and 272; 275 and 276; 279 and 280; 283 and 284; 287 and 288; 290 and 291; 292 and 293; 294 and 295; 296 and 297; or 298 and 299.

In an additional embodiment, the present invention provides molecules comprising a targeting moiety, a first immunomodulatory moiety (IM-1) and a second immunomodulatory moiety (IM-2), wherein the targeting moiety comprises a polypeptide that specifically binds Cytotoxic T lymphocyte associated antigen-4 (CTLA-4, CD152), Programmed Death-1 protein (PD-1), Programmed death ligand-1 (PD-L1), V domain Ig suppressor of T cell activation (VISTA), B and T lymphocyte attenuator (BTLA), T cell Ig and ITIM domain (TIGIT), Lymphocyte activation gene-3 (LAG-3), 4-1BB ligand (CD137), OX-40 (CD134), glucocorticoid-induced TNFR-related protein (GITR), transforming growth factor β (TGF-β), CD27, CD47, IL6R, IL23R, IL17R, IL-6, IL-23, IL-17, CD39, CD73, CD122, CCR4, CCR5, CXCR4, IL12R, CD4, CD25, CD3, Receptor activator of nuclear factor kappa-B ligand (RANKL), RANK, epidermal growth factor receptor (EGFR), human epidermal growth factor receptor 2 (HER2) or Vascular endothelial growth factor (VEGF), the first immunomodulatory moiety (IM-1) comprises the extracellular ligand-binding sequence or ligand binding fragment thereof of transforming growth factor β receptor II (TGFβRII), programmed death 1 protein (PD-1), T cell immunoglobulin and mucin domain containing 3 (TIM-3), B- and T-lymphocyte attenuator (BTLA), CD226, T cell Ig and ITM domain (TIGIT), CD44, Colony stimulating factor 1 receptor (CSF1R), CCR4, Killer-cell immunoglobulin-like receptor (KIR), Vascular endothelial growth factor receptor (VEGFR), Receptor Activator of Nuclear Factor κ B (RANK), V-set and immunoglobulin domain containing 8 (VSIG8), LIGHT (TNFSF14) or V domain Ig suppressor of T cell activation (VISTA); and the second immunomodulatory moiety (IM-2) comprises the extracellular ligand-binding sequence or ligand binding fragment thereof of transforming growth factor β receptor II (TGFβRII), programmed death 1 protein (PD-1), T cell immunoglobulin and mucin domain containing 3 (TIM-3), B- and T-lymphocyte attenuator (BTLA), CD226, T cell Ig and ITIM domain (TIGIT), CD44, Colony stimulating factor 1 receptor (CSF1R), CCR4, Killer-cell immunoglobulin-like receptor (KIR), Vascular endothelial growth factor receptor (VEGFR), Receptor Activator of Nuclear Factor κ B (RANK), V-set and immunoglobulin domain containing 8 (VSIG8), LIGHT (TNFSF14) or V domain Ig suppressor of T cell activation (VISTA).

In some aspects, the molecule comprises the sequence of SEQ ID NOs: 201 and 202; 203 and 204; 205 and 206; 207 and 208; 213 and 214; 215 and 216; 217 and 218; 219 and 220; 225 and 226; 227 and 228; 229 and 230; 231 and 232; 237 and 238; 239 and 240; 241 and 242 ; 243 and 244; 249 and 250; 251 and 252; 253 and 254; 255 and 256; 261 and 262; 263 and 264; 265 and 266; 267 and 268; 273 and 274; 277 and 278; 281 and 282; 285 and 286.

In one aspect, the targeting moiety polypeptide comprises an antigen-binding domain of an immunoglobulin, antibody, bispecific or multispecific antibody, antibody fragment, single chain variable fragment (scFv), bivalent or multivalent scFv, a ligand-binding sequence from the extracellular domain (ECD) of a receptor, or Fc-containing polypeptide. In certain aspects, the polypeptide is an antibody.

In one aspect, the first immunomodulatory moiety is attached to the heavy chain of the antibody and the second immunomodulatory moiety is attached to the light chain of the antibody. In an additional aspect, the first immunomodulatory moiety is attached to the C-terminus of a heavy chain or a light chain of the antibody, the second immunomodulatory moiety is attached to the C-terminus of a heavy chain or a light chain of the antibody and/or the second immunomodulatory moiety is attached to the first immunomodulatory moiety.

In a further aspect, the first and second immunomodulatory moiety is attached to the heavy chain or the light chain of the antibody with a linker and/or the second immunomodulatory moiety is attached to the first immunomodulatory moiety with a linker. In certain aspects, the linker has the sequence of (GGGGS)n (SEQ ID NO: 3), wherein n can be 1,2,3,4,5,6,7,8,9, or 10. In certain aspects, the linker has the sequence of SEQ ID NO 3.In certain aspects, the first immunomodulatory moiety comprises the ECD or ligand binding fragment thereof of TIM3 and the second immunomodulatory moiety comprises the ECD or ligand binding fragment thereof of PD-1; the first immunomodulatory moiety comprises the ECD or ligand binding fragment thereof of TGFβRII and the second immunomodulatory moiety comprises the ECD or ligand binding fragment thereof of TIM-3; the first immunomodulatory moiety comprises the ECD or ligand binding fragment thereof of PD-1 and the second immunomodulatory moiety comprises the ECD or ligand binding fragment thereof of TIM-3; and/or the first immunomodulatory moiety comprises the ECD or ligand binding fragment thereof of TGFβRII and the second immunomodulatory moiety comprises the ECD or ligand binding fragment thereof of PD-1.

In a further aspect, the targeting moiety specifically binds IL6R, CTLA-4, PD-1, PD-L1, EGFR, HER2, VEGF, or gp120.

In one aspect, the targeting moiety comprises a polypeptide that binds CTLA-4, the first immunomodulatory moiety comprises the ECD or ligand binding fragment thereof of PD-1, and the second immunomodulatory moiety comprises the ECD or ligand binding fragment thereof of TGFβRII. In one aspect, the molecule comprises SEQ ID NO: 201 and SEQ ID NO: 202. In another aspect, the targeting moiety comprises a polypeptide that binds CTLA-4, the first immunomodulatory moiety comprises the ECD or ligand binding fragment thereof of PD-1, and the second immunomodulatory moiety comprises the ECD or ligand binding fragment thereof of TIM-3. In one aspect, the molecule comprises SEQ ID NO: 205 and SEQ ID NO: 206. In one aspect, the molecule comprises SEQ ID NO: 207 and SEQ ID NO: 208. In another aspect, the targeting moiety comprises a polypeptide that binds CTLA-4, the first immunomodulatory moiety comprises the ECD or ligand binding fragment thereof of TIM-3, and the second immunomodulatory moiety comprises the ECD or ligand binding fragment thereof of TGFβRII. In one aspect, the molecule comprises SEQ ID NO: 203 and SEQ ID NO: 204. In a further aspect, the targeting moiety polypeptide comprises ipilimumab. In a further aspect, this antibody is tremilimumab.

In one aspect, the targeting moiety comprises a polypeptide that binds PD-1 or PD-L1, the first immunomodulatory moiety comprises the ECD or ligand binding fragment thereof of TGFβRII, and the second immunomodulatory moiety comprises the ECD or ligand binding fragment thereof of TIM-3. In one aspect, the molecule comprises SEQ ID NO: 273 and SEQ ID NO: 274. In one aspect, the molecule comprises SEQ ID NO: 277 and SEQ ID NO: 278. In one aspect, the molecule comprises SEQ ID NO: 285 and SEQ ID NO: 286. In one aspect, the molecule comprises SEQ ID NO: 281 and SEQ ID NO: 282. In a further aspect, the targeting moiety polypeptide comprises Nivolumab or pembrolizumab. In a further aspect, this antibody is atezolizumab or avelumab or durvalumab.

In one aspect, the targeting moiety comprises a polypeptide that binds IL-6R, the first immunomodulatory moiety comprises the ECD or ligand binding fragment thereof of PD-1, and the second immunomodulatory moiety comprises the ECD or ligand binding fragment thereof of TGFβRII. In one aspect, the molecule comprises SEQ ID NO: 213 and SEQ ID NO: 214. In another aspect, the targeting moiety comprises a polypeptide that binds IL-6R, the first immunomodulatory moiety comprises the ECD or ligand binding fragment thereof of PD-1, and the second immunomodulatory moiety comprises the ECD or ligand binding fragment thereof of TIM-3. In one aspect, the molecule comprises SEQ ID NO: 217 and SEQ ID NO: 218. In one aspect, the molecule comprises SEQ ID NO: 219 and SEQ ID NO: 220. In another aspect, the targeting moiety comprises a polypeptide that binds IL-6R, the first immunomodulatory moiety comprises the ECD or ligand binding fragment thereof of TIM-3, and the second immunomodulatory moiety comprises the ECD or ligand binding fragment thereof of TGFβRII. In one aspect, the molecule comprises SEQ ID NO: 215 and SEQ ID NO: 216. In a further aspect, the targeting moiety polypeptide comprises an antibody that binds IL-6R. In a further aspect, this antibody comprises tocilizumab.

In one aspect, the targeting moiety comprises a polypeptide that binds VEGF, the first immunomodulatory moiety comprises the ECD or ligand binding fragment thereof of PD-1, and the second immunomodulatory moiety comprises the ECD or ligand binding fragment thereof of TGFβRII. In one aspect, the molecule comprises SEQ ID NO: 249 and SEQ ID NO: 250. In another aspect, the targeting moiety comprises a polypeptide that binds VEGF, the first immunomodulatory moiety comprises the ECD or ligand binding fragment thereof of PD-1, and the second immunomodulatory moiety comprises the ECD or ligand binding fragment thereof of TIM-3. In one aspect, the molecule comprises SEQ ID NO: 253 and SEQ ID NO: 254. In one aspect, the molecule comprises SEQ ID NO: 255 and SEQ ID NO: 256. In another aspect, the targeting moiety comprises a polypeptide that binds VEGF, the first immunomodulatory moiety comprises the ECD or ligand binding fragment thereof of TIM-3, and the second immunomodulatory moiety comprises the ECD or ligand binding fragment thereof of TGFβRII. In one aspect, the molecule comprises SEQ ID NO: 251 and SEQ ID NO: 252. In a further aspect, the targeting moiety polypeptide comprises Bevacizumab. In a further aspect, targeting moiety comprises a ligand binding ectodomain of VEGFR. In a further aspect, targeting moiety comprises aflibercept.

In one aspect, the targeting moiety comprises a polypeptide that binds EGFR, the first immunomodulatory moiety comprises the ECD or ligand binding fragment thereof of PD-1, and the second immunomodulatory moiety comprises the ECD or ligand binding fragment thereof of TGFβRII. In one aspect, the molecule comprises SEQ ID NO: 225 and SEQ ID NO: 226. In another aspect, the targeting moiety comprises a polypeptide that binds EGFR, the first immunomodulatory moiety comprises the ECD or ligand binding fragment thereof of PD-1, and the second immunomodulatory moiety comprises the ECD or ligand binding fragment thereof of TIM-3. In one aspect, the molecule comprises SEQ ID NO: 229 and SEQ ID NO: 230. In one aspect, the molecule comprises SEQ ID NO: 231 and SEQ ID NO: 232. In another aspect, the targeting moiety comprises a polypeptide that binds EGFR, the first immunomodulatory moiety comprises the ECD or ligand binding fragment thereof of TIM-3, and the second immunomodulatory moiety comprises the ECD or ligand binding fragment thereof of TGFβRII. In one aspect, the molecule comprises SEQ ID NO: 227 and SEQ ID NO: 228. In a further aspect, the targeting moiety polypeptide comprises Cetuximab or Panitumumab or Necitumumab.

In one aspect, the targeting moiety comprises a polypeptide that binds HER2/Neu, the first immunomodulatory moiety comprises the ECD or ligand binding fragment thereof of PD-1, and the second immunomodulatory moiety comprises the ECD or ligand binding fragment thereof of TGFβRII. In one aspect, the molecule comprises SEQ ID NO: 237 and SEQ ID NO: 238. In another aspect, the targeting moiety comprises a polypeptide that binds HER2, the first immunomodulatory moiety comprises the ECD or ligand binding fragment thereof of PD-1, and the second immunomodulatory moiety comprises the ECD or ligand binding fragment thereof of TIM-3. In one aspect, the molecule comprises SEQ ID NO: 243 and SEQ ID NO: 244. In one aspect, the molecule comprises SEQ ID NO: 241 and SEQ ID NO: 242. In another aspect, the targeting moiety comprises a polypeptide that binds HER2, the first immunomodulatory moiety comprises the ECD or ligand binding fragment thereof of TIM-3, and the second immunomodulatory moiety comprises the ECD or ligand binding fragment thereof of TGFβRII. In one aspect, the molecule comprises SEQ ID NO: 239 and SEQ ID NO: 240. In a further aspect, the targeting moiety polypeptide comprises Trastuzumab or Pertuzumab.

In one aspect, the targeting moiety comprises a polypeptide that binds HIV gp120, the first immunomodulatory moiety comprises the ECD or ligand binding fragment thereof of PD-1, and the second immunomodulatory moiety comprises the ECD or ligand binding fragment thereof of TGFβRII. In one aspect, the molecule comprises SEQ ID NO: 261 and SEQ ID NO: 262. In another aspect, the targeting moiety comprises a polypeptide that binds gp120, the first immunomodulatory moiety comprises the ECD or ligand binding fragment thereof of PD-1, and the second immunomodulatory moiety comprises the ECD or ligand binding fragment thereof of TIM-3. In one aspect, the molecule comprises SEQ ID NO: 265 and SEQ ID NO: 266. In one aspect, the molecule comprises SEQ ID NO: 267 and SEQ ID NO: 268. In another aspect, the targeting moiety comprises a polypeptide that binds gp120, the first immunomodulatory moiety comprises the ECD or ligand binding fragment thereof of TIM-3, and the second immunomodulatory moiety comprises the ECD or ligand binding fragment thereof of TGFβRII. In one aspect, the molecule comprises SEQ ID NO: 263 and SEQ ID NO: 264. In a further aspect, the targeting moiety polypeptide comprises Trastuzumab or Pertuzumab.

In a further embodiment, the present invention provides fusion proteins comprising the extracellular binding domain (ECD) or ligand binding fragment thereof of TIM-3 and the ECD or ligand binding fragment thereof of TGFRβII, PD-1, BTLA, LIGHT, CD226 or VSIG8. In one aspect, the fusion protein comprises the ECD or ligand binding fragment thereof of TIM-3 and the ECD or ligand binding fragment thereof of TGFβRII. In certain aspects, the fusion protein comprises the sequence of SEQ ID NO: 190. In another aspect, the fusion protein comprises the ECD or ligand binding fragment thereof of TIM-3 and the ECD or ligand binding fragment thereof of PD-1. In specific aspects, the fusion protein comprises the sequence of SEQ ID NO: 195, 196 or 197. In a further aspect, the fusion protein comprises an Fc. In a further aspect, the TIM-3 ECD and the other ECD are attached with a linker. In certain aspects, the linker has the sequence of SEQ ID NO 3, 4, or 5.

In one embodiment, the present invention provides for compositions comprising the previously described molecule or fusion protein and a pharmaceutical carrier.

In an additional embodiment, the present invention provides a method of treating a subject having cancer or an infectious disease comprising administering to the subject the previously described molecules, fusion proteins or compositions. In one aspect, the molecule, fusion protein or composition is administered by intracutaneous, subcutaneous, intravenous, intraperitoneal, intraarterial, intrathecal, intracapsular, intraorbital, intracardiac, intradermal, transdermal, transtracheal, subcuticular, intraarticulare, subcapsular, subarachnoid, intraspinal and intrasternal , oral, sublingual buccal, rectal, vaginal, nasal or ocular administrations, by infusion, inhalation, or nebulization or by parenteral administration.

In another aspect, the cancer is Lung cancer, Breast cancer, Colorectal cancer, Prostate cancer, Stomach cancer, Liver cancer, Cervical cancer, Esophageal cancer, Bladder cancer, Non-Hodgkin lymphoma, Leukemia, Pancreatic cancer, Kidney cancer, endometrial cancer, Head and Neck Cancer (HNSCC), Lip cancer, oral cancer, Thyroid cancer, Brain cancer, Ovary cancer, Melanoma, Gallbladder cancer, Laryngeal cancer, Multiple myeloma, Nasopharyngeal cancer, Hodgkin lymphoma, Testis cancer or Kaposi sarcoma.

In an additional aspect, the infectious pathogen or disease is HIV/AIDS, Herpes Simplex Virus/Herpes, Human Papilloma Virus, Hepatitis B or C Virus/Hepatitis, Epstein Barr Virus (EBV), Borrelia burgdorferi/Lyme disease, Influenza, Mycobacterium tuberculosis/Tuberculosis, Mycobacteria leprae,/Leprosy, Malaria, Syphilis, Gonorrhea, or Klebsiella Pneumoniae/Pneumonia.

In a further embodiment, a chemotherapeutic agent, immunosuppressive agent or antibiotic is administered simultaneously with, before or following the administration of the molecule, fusion protein or composition.

### BRIEF DESCRIPTION OF THE DRAWINGS

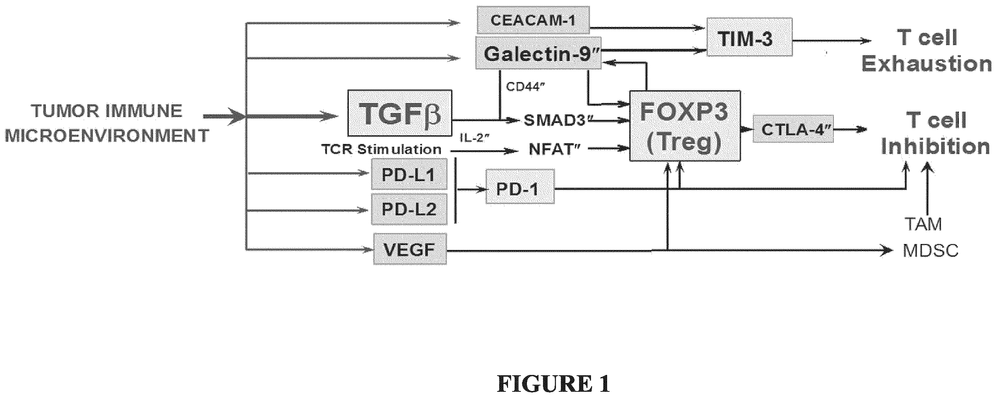

Figure 1. Schematic representation of autocrine/paracrine receptor-ligand interactions that inhibit T cell activation and induce immune tolerance and T cell exhaustion. Autocrine/paracrine TGFβ induces expression of FOXP3, the signature transcription factor of the Treg lineage. FOXP3 induces expression of CTLA-4, a T cell inhibitory receptor which restrains T cell costimulation by interfering with B7-CD28 interaction. Tregs express Galectin-9, a ligand that engages TIM-3 and triggers exhaustion or apoptosis of effector T cells by the TIM-3/CEACAM-1 co-inhibitory axis. Engagement of PD-1 by PD-L1 promotes TGFβ-induced expression of FOXP3 to induce and maintain Tregs, and cooperates with TIM-3/CEACAM-1 signaling to induce T cell exhaustion and death.

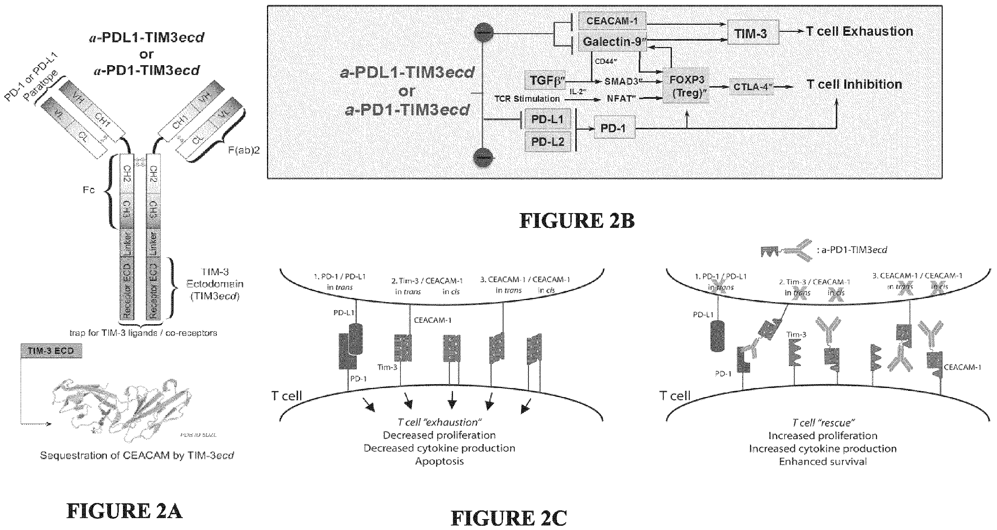

Figures 2A-C. *a*-PDL1-TIM3ecd and *a*-PD1-TIM3ecd counteract immune tolerance and T cell exhaustion by simultaneously interrupting PD-L1/PD-1 signaling and the TIM-3/CEACAM-1 co-inhibitory axis. (A) Schematic representation of the structure and targets of *a*-PDL1-TIM3*ecd* and *a*-PD1-TIM3*ecd*. a-PD1-TIM3*ecd* comprises: (i) Heavy chain of a human *a*-PD-1 antibody fused at the C-terminus to a ligand-binding IgV ectodomain sequence of TIM-3 via a flexible linker peptide, (GGGGS)₃ (SEQ ID NO: 53) and (ii) Light chain of a human *a*-PD-1 antibody (SEQ ID NO: 187). *a*-PDL1-TIM3*ecd* comprises: (i) Heavy chain of a human *a*-PD-L1 antibody fused at the C-terminus to a ligand-binding IgV ectodomain sequence of TIM-3 via a flexible linker peptide, (GGGGS)₃ (SEQ ID NO: 51 ) and (ii) Light chain of a human *a*-PD-L1 antibody (SEQ ID NO:185 ).(B) Schematic representation of the targets and mechanisms by which *a*-PDL1-TIM3*ecd* and *a*-PD1-TIM3*ecd* disrupt autocrine/paracrine receptor-ligand interactions that inhibit T cell activation and induce T cell exhaustion. Autocrine/paracrine TGFβ induces expression of FOXP3, the signature transcription factor of the Treg lineage. FOXP3 induces expression of CTLA-4, a T cell inhibitory receptor which restrains T cell costimulation by interfering with B7-CD28 interaction. Tregs express Galectin-9, a ligand that engages TIM-3 and triggers exhaustion or apoptosis of effector T cells by the TIM-3/CEACAM-1 co-inhibitory axis. Engagement of PD-1 by PD-L1 promotes TGFβ-induced expression of FOXP3 to induce and maintain Tregs, and cooperates with TIM-3/CEACAM-1 signaling to induce T cell exhaustion and death. *a*-PDL1-TIM3*ecd* and *a*-PD1-TIM3*ecd* disable PD-1/PD-L1 signaling and simultaneously disrupt *cis*/*trans* interactions involving the TIM-3 and CEACAM-1 co-inhibitory pathways.(C) Schematic representation of the mechanism by which *a*-PDL1-TIM3*ecd* and *a*-PD1-TIM3*ecd* simultaneously counteract multipronged suppression of T cells via the PD-L1/PD-1 and TIM-3/CEACAM-1 co-inhibitory axes. *a-*PDL1-TIM3ecd and *a*-PD1-TIM3ecd interrupt PD-L1/PD-1 interaction and simultaneously disrupt: (i) heterophilic TIM-3/CEACAM-1 interactions *(cis* CEACAM-1/TIM-3 heterodimerization; *trans* CEACAM1 heterodimerization with TIM-3); (ii) homophilic CEACAM-1/CEACAM-1 interactions *(cis* CEACAM-1 dimerization; *trans* CEACAM1-induced *cis* CEACAM-1 dimerization).

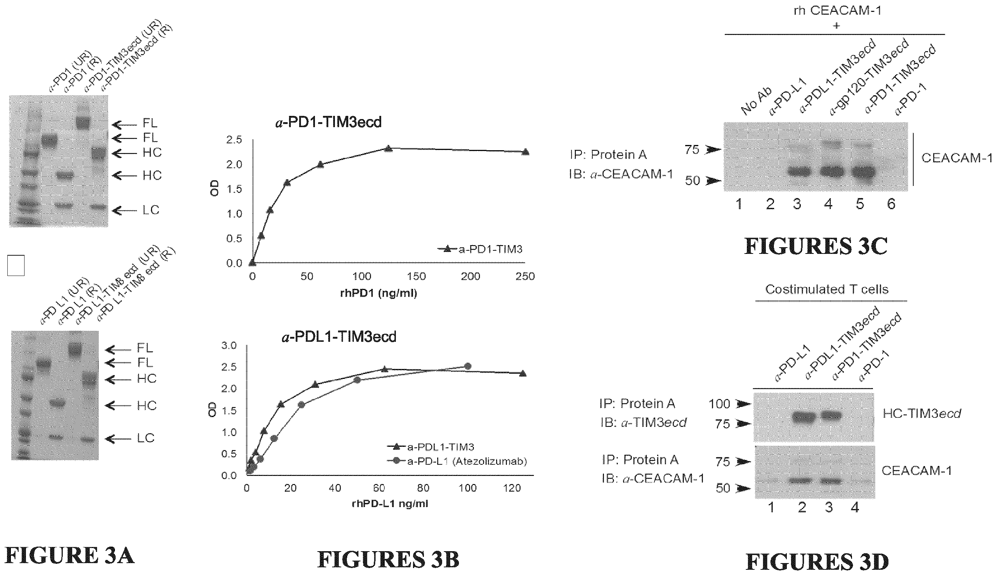

Figures 3A-D. Characterization and target-binding ability of *a*-PDL1-TIM3ecd and *a*-PD1-TIM3ecd. (A) SDS-PAGE under reducing (R) and non-reducing (NR) conditions was used to compare the full-length (FL), heavy chain (HC) and light chain (LC) of *a*-PD1-TIM3ecd and *a*-PD1 antibody (nivolumab). SDS-PAGE confirmed the expected higher molecular weight of the heavy chain of *a*-PD1-TIM3ecd compared to the heavy chain of *a*-PD1 antibody (*Top panel*). SDS-PAGE under reducing (R) and non-reducing (NR) conditions was used to compare the full-length (FL), heavy chain (HC) and light chain (LC) of *a*-PDL1-TIM3*ecd* and *a*-PDL1 antibody (atezolizumab). SDS-PAGE confirmed the expected higher molecular weight of the heavy chain of *a*-PDL1-TIM3*ecd* compared to the heavy chain of *a*-PDL1 antibody (*Bottom panel*). (B) ELISA showing binding of *a*-PD1-TIM3 to rhPD-1 (*Top panel*): Biotinylated rhPD-1 (0-250 ng/ml) was added to plates coated with *a*-PD1-TIM3ecd (1µg/ml). ELISA showing binding of *a*-PDL1-TIM3 to rhPD-L1 *(Bottom panel):* Biotinylated rhPD-L1 (0-250 ng/ml) was added to plates coated with either *a*-PDL1-TIM3 (1µg/ml) or *a*-PDL1 antibody (atezolizumab)(positive control). (C) *a*-PD1-TIM3ecd and *a*-PDL1-TIM3*ecd* bind recombinant human (rh) CEACAM-1 *in vitro.* rhCEACAM-1 (5 µg) was incubated (overnight, 4°C) in the presence or absence of *a*-PD1-TIM3ecd, *a*-PDL1-TIM3ecd, *a*-gp120-TIM3ecd, *a*-PD-L1 mAb (atezolizumab), or *a*-PD1 mAb (nivolumab)(10 µg each), followed by incubation with protein A-agarose beads (10 µl) for 6h, and then immunoprecipitated with protein A-agarose beads. The precipitated complexes were washed with immunoprecipitation buffer, re-suspended in NuPAGE sample buffer with reducing agent, boiled, resolved on a Bis-Tris Criterion XT Gel system and transferred to a PVDF membrane. Membranes were immunoblotted with primary antibodies [CEACAM1 (D1P4T); TIM3 (D5D5R^{™})] overnight, washed, incubated with secondary antibodies and visualized by Amersham ECL Western Blotting Detection Reagents. Immunoblot analyses detected rhCEACAM-1 in *a*-PD1-TIM3ecd, *a*-PDL1-TIM3ecd, and *a*-gp120-TIM3ecd protein A-pulled down immunoprecipitates, but not in immunoprecipitates of *a*-PD-L1 mAb (atezolizumab), or *a*-PD1 mAb (nivolumab). (D) *a-*PD1-TIM3ecd and *a*-PDL1-TIM3*ecd* bind CEACAM-1 on co-stimulated human T cells. Human PBMC were co-stimulated with anti-CD3/anti-CD28 beads in the presence of *a-*PD1-TIM3ecd, *a*-PDL1-TIM3ecd, *a*-PD-L1 mAb (atezolizumab), or *a*-PD1 mAb (nivolumab) for 6d. Cell lysates were immunoprecipitated with protein A-agarose beads (10 µl) for 6h. The precipitated complexes were washed with immunoprecipitation buffer, re-suspended in NuPAGE sample buffer with reducing agent, boiled, resolved on a Bis-Tris Criterion XT Gel system and transferred to a PVDF membrane. Membranes were immunoblotted with primary antibodies [CEACAM1 (D1P4T); TIM3 (D5D5R^{™})] overnight, washed, incubated with secondary antibodies and visualized by Amersham ECL Western Blotting Detection Reagents. Immunoblot analyses with the CEACAM-1 antibody detected CEACAM-1 in protein A-immunoprecipitates of *a*-PD1-TIM3ecd and *a*-PDL1-TIM3ecd treated cell lysates, but not in immunoprecipitates of *a*-PD-L1 mAb (atezolizumab), or *a*-PD1 mAb (nivolumab) treated cell lysates. Immunoblot analyses with the TIM-3 ecd antibody detected the heavy chain of *a*-PD1-TIM3ecd or *a*-PDL1-TIM3ecd in protein A-immunoprecipitates of *a*-PD1-TIM3ecd and *a*-PDL1-TIM3*ecd* treated cell lysates, but not in immunoprecipitates of *a*-PD-L1 mAb (atezolizumab), or *a*-PD1 mAb (nivolumab) treated cell lysates.

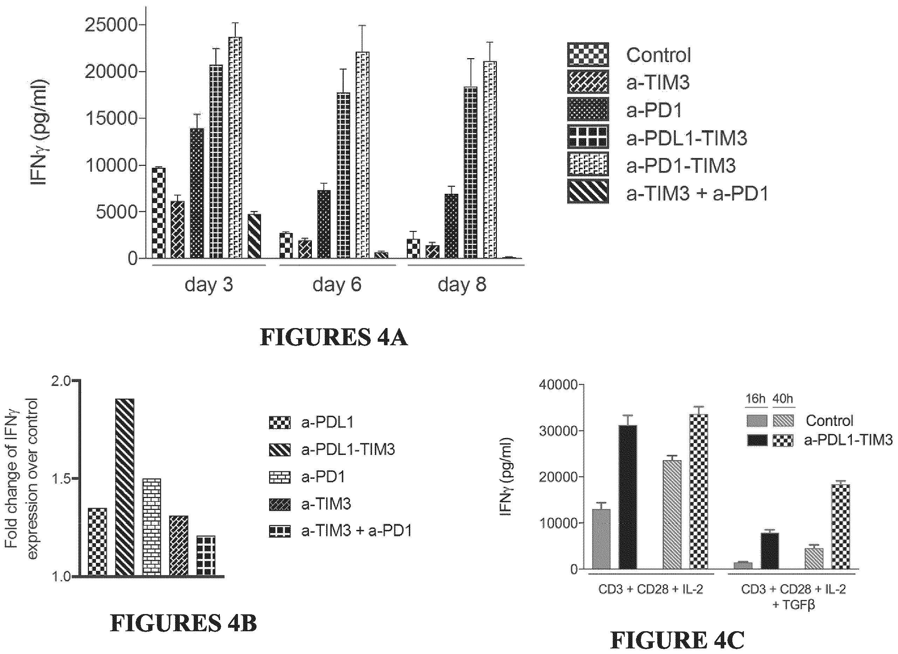

Figures 4A-C. *a*-PD1-TIM3*ecd* and *a*-PDL1-TIM3*ecd* are significantly more effective than *a*-PD-1 mAb or *a*-PD-L1 mAb in promoting IFNγ expression and counteracting exhaustion of co-stimulated T cells.
(A) Human PBMCs were stimulated with anti-CD3/anti-CD28 beads (5µl/ml) and rhIL-2, in the presence or absence of *a*-PD1-TIM3ecd, *a*-PDL1-TIM3ecd, *a*-PD-L1 mAb (atezolizumab), *a*-PD1 mAb (nivolumab), *a*-human TIM3 Ab (F38-2E2), or the combination of *a*-PD1 mAb and *a*-TIM3 Ab (5 µg/ml each) for 3-9d. Interferon-γ (IFN-γ) in cell supernatants at each indicated time (3d, 6d, and 9d) was quantified by ELISA (Data represent mean ± SEM of 3 *in vitro* replicates for each group). ELISA demonstrated that *a-*PDL1-TIM3*ecd* and *a*-PD1-TIM3*ecd* are significantly more effective in promoting IFNγ expression and counteracting exhaustion of co-stimulated T cells *in vitro* compared to either *a*-PDL1 (atezolizumab), *a*-PD-1 (pembrolizumab), *a*-TIM3 Ab, or even the combination of *a*-PD1 mAb and *a*-TIM3 Ab. (B) Human PBMCs were stimulated with anti-CD3/anti-CD28 beads (5µl/ml) and rhIL-2, in the presence or absence of *a*-PD1-TIM3ecd, *a*-PDL1-TIM3ecd, *a*-PD-L1 mAb (atezolizumab), *a*-PD1 mAb (nivolumab), *a*-human TIM3 Ab (F38-2E2), or the combination of *a*-PD1 mAb and *a*-TIM3 Ab (5 µg/ml each) for 16h or 40h, and IFN-γ in cell supernatants was quantified by ELISA (mean ± SEM of 3 *in vitro* replicates/group). *a*-PDL1-TIM3*ecd* is significantly more effective in promoting IFNγ expression by co-stimulated T cells *in vitro* compared to either *a*-PDL1 (atezolizumab), *a-*PD-1 (pembrolizumab), *a*-TIM3 Ab, or even the combination of *a*-PD1 mAb and *a*-TIM3 Ab [data represent the fold-change in IFNγ expression in each antibody-treated group compared to co-stimulation without antibody)]. (C) *a*-PD1-TIM3ecd counteracts TGFβ mediated suppression of IFNγ expression in co-stimulated T cells. Human PBMCs were stimulated with anti-CD3/anti-CD28 and rhIL-2 in the presence or absence of rhTGFβ1 (5 ng/ml) with or without either *a*-PDL1-TIM3 (5 µg/ml) for 16h or 40h, and Interferon-γ (IFN-γ) in cell supernatants was quantified by ELISA (Data represent mean ± SEM of 3 *in vitro* replicates for each group).

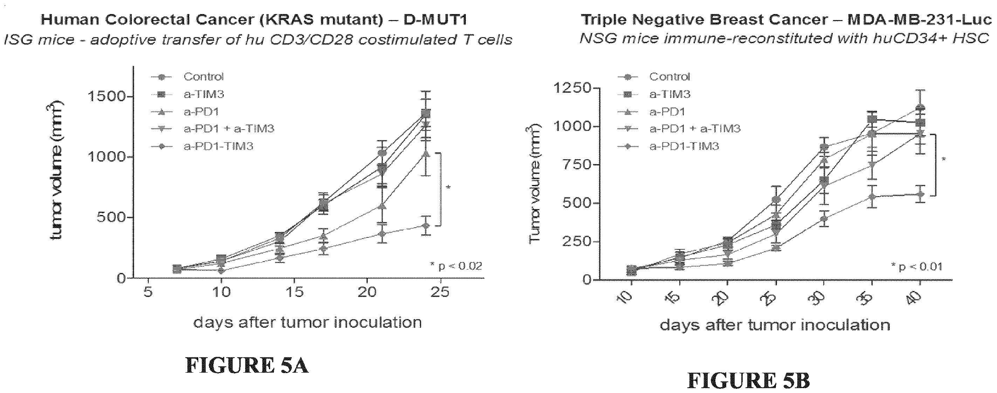

Figures 5A-B. a-PD1-TIM3ecd inhibits tumor growth more effectively than a-PD-1, a-TIM-3 or the combination of a-TIM-3 and a-PD1(A) NSG mice (NOD/Shi-scid IL-2rgnull)(4-8 weeks) were reconstituted with adoptive transfer of 3x106 human peripheral blood mononuclear cells (PBMCs) [that were costimulated with anti-CD3/anti-CD28 beads (5µl/ml) and rhIL-2 in the presence of the following specified treatments for 8 days (5 µg/ml each): *a*-PD1-TIM3ecd, a-PD1 mAb (nivolumab), a-human TIM3 Ab (F38-2E2), combination of a-PD1 mAb and a-TIM3 Ab, or vehicle alone. Reconstituted mice were injected subcutaneously with 2x106 D-MUT1 human colorectal cancer cells, and each group was treated in vivo (5 mg/kg i.p. every 5 days) with the same treatment used for in vitro treatment of PBMCs used for adoptive transfer, as indicated: a-PD1-TIM3ecd; a-PD1 (nivolumab); a-TIM-3 (F38-2E2); combination of a-PD1 and a-TIM-3; vehicle alone (untreated control) [≥5 mice/group]. Tumor size was measured blinded to the treatment group and tumor volume was calculated using the formula (length × width × height). In vivo tumor growth curves (mean + SEM) are shown. p values were derived using unpaired, two-sided t-test. The p value (p<0.02, Student's unpaired t-test) denotes significant difference between a-PD1-TIM3ecd and each other treatment group. (B) Immune deficient NSG mice (NOD/Shi-scid IL-2rgnull)(4-8 weeks) were irradiated at 200 cGy, and immune reconstituted by adoptive transfer of HLA A2-matched human CD34+.hematopoietic cells. Reconstituted humanized mice were injected subcutaneously with human Triple Negative Breast Cancer (TNBC) HLA A2+ tumor cells (MDA-MB-231-Luc)(1x106 in matrigel; mammary fat pad). At 3d following tumor inoculation, mice were randomized and treated with either vehicle alone (untreated control) or the following antibodies (5 mg/kg i.p. weekly): a-PD1-TIM3ecd, a-PD1 mAb (nivolumab), a-human TIM3 Ab (F38-2E2), combination of a-PD1 mAb and a-TIM3 Ab [>_5 mice/group]. Tumor size was measured blinded to the treatment group and tumor volume was calculated using the formula (length × width × height). In vivo tumor growth curves (mean ± SEM) are shown. p values were derived using unpaired, two-sided t-test. The p value (p<0.02, Student's unpaired t-test) denotes significant difference between *a*-PD1-TIM3ecd and each other treatment group.

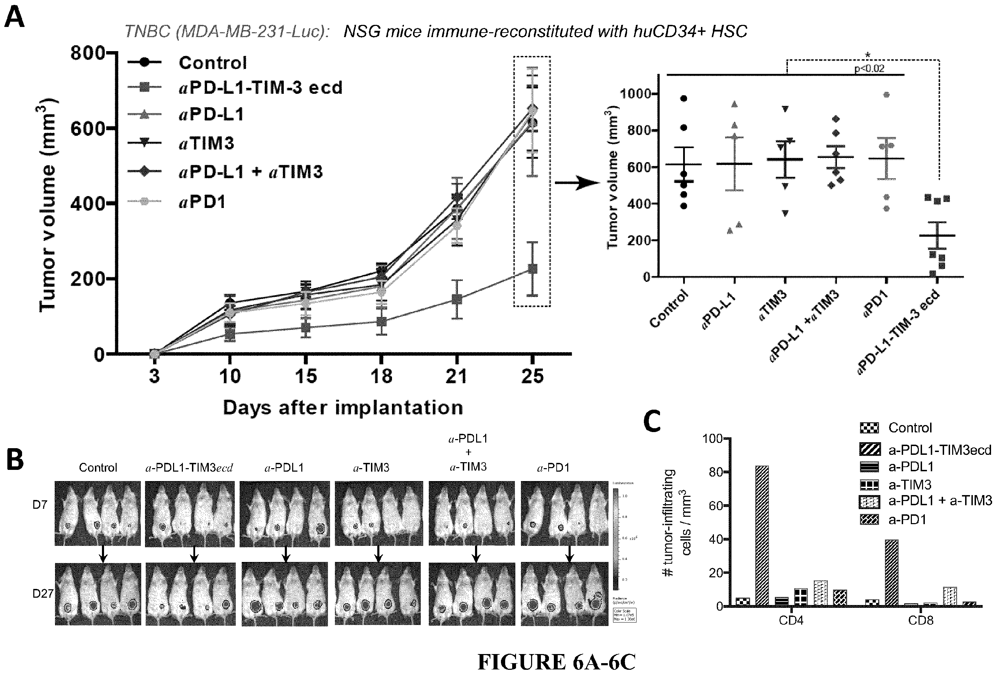

Figures 6A-C. *a*-PDL1-TIM3*ecd* inhibits tumor growth more effectively than a-PD-L1, a-PD-1, a-TIM-3 or the combination of a-PD-L1 and a-TIM-3.Immune deficient NSG mice (NOD/Shi-*scid IL-2rg^{null}*)(4-8 weeks) were irradiated at 200 cGy, and immune reconstituted by adoptive transfer of HLA A2 human CD34⁺.hematopoietic cells. Reconstituted humanized mice were injected subcutaneously with human Triple Negative Breast Cancer (TNBC) HLA A2+ tumor cells (MDA-MB-231-Luc)(1×10⁶ in matrigel; mammary fat pad). At 3d following tumor inoculation, mice were randomized and treated with either vehicle alone (untreated control) or the following antibodies (5 mg/kg i.p. weekly): *a*-PDL1-TIM3ecd, *a*-PD1 mAb (atezolizumab), *a*-PD1 mAb (pembrolizumab), *a-*human TIM3 Ab (F38-2E2), combination of *a*-PD-L1 mAb and *a*-TIM3 Ab [>_5 mice/group]. **(A)** Tumor size was measured blinded to the treatment group and tumor volume was calculated using the formula (length × width × height). *In vivo* tumor growth curves (mean ± SEM) are shown. *p* values were derived using unpaired, two-sided t-test. The p value (p<0.02, Student's unpaired t-test) denotes significant difference between *a-*PDL1-TIM3ecd and each other treatment group. **(B)** Bioluminescence assay of primary tumors in untreated controls or the indicated treatment groups at 7 and 27 d after tumor cell inoculation. **(C)** Quantification of T cell density in tumors: Tumor volume and flow cytometry was used to assess the absolute number of CD4+ and CD8+ T cells per mm³ of tumor.

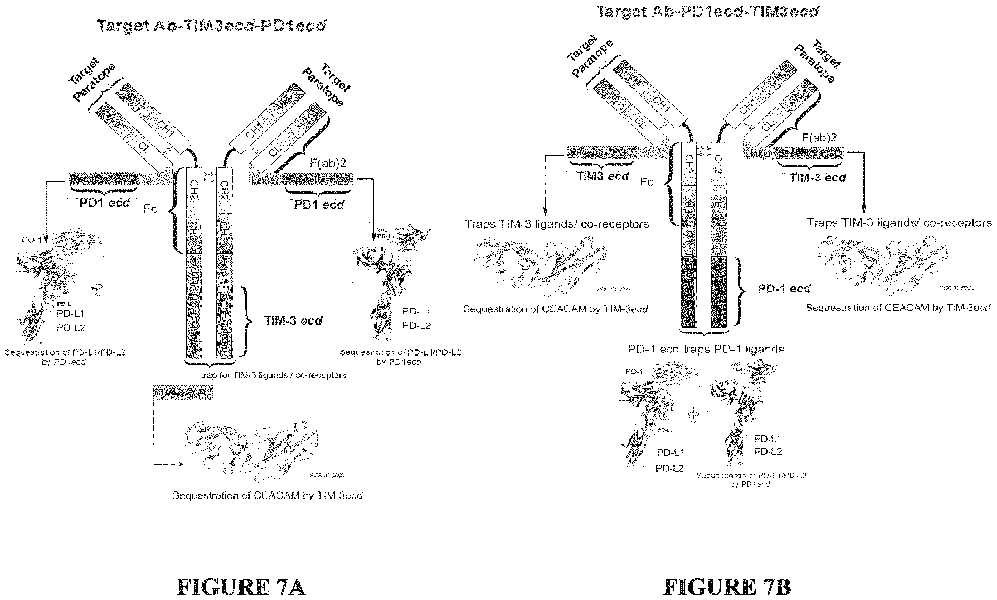

Figures 7A-B. Schematic design of multifunctional antibody-ligand traps of this invention comprising a targeting antibody that binds a target epitope, ligand-binding IgV ectodomain sequence of TIM-3 (TIM-3ecd), and ligand-binding ectodomain sequence of PD-1 (PD-1ecd). The multifunctional antibody-ligand traps can bind a target epitope (via the antigen-binding CDR), bind PD-1 ligands, PD-L1 and PD-L2 (via the PD-1ecd) and TIM-3 ligands (CEACAM-1)(via the TIM-3ecd). **(A)** Schematic design of multifunctional antibody-ligand traps of this invention comprising a targeting antibody, wherein the heavy chain of the antibody is fused at the C-terminus to a ligand-binding IgV ectodomain sequence of TIM-3 (TIM-3ecd: SEQ ID NO: 2) via a linker, and the light chain of the antibody is fused at the C-terminus to a ligand-binding ectodomain sequence of PD-1 (PD-1ecd: SEQ ID NO: 7 ) via a linker. In one aspect, the linker is a peptide, (GGGGS)ₙ (SEQ ID NO: 3).**(B)** Schematic design of multifunctional antibody-ligand traps of this invention comprising a targeting antibody, wherein the heavy chain of the antibody is fused at the C-terminus to a ligand-binding ectodomain sequence of PD-1 (PD-1ecd: SEQ ID NO: 7) via a linker peptide, and the light chain of the antibody is fused at the C-terminus to a ligand-binding ectodomain sequence of TIM-3 (TIM-3ecd: SEQ ID NO: 2 ) via a linker peptide. In one aspect, the linker is a peptide, (GGGGS)ₙ (SEQ ID NO: 3).In various embodiments, the targeting antibody of the multifunctional antibody-ligand traps of this invention binds one of the following molecules: Cytotoxic T lymphocyte associated antigen-4 (CTLA-4, CD152), B7-H3 (CD276), V domain Ig suppressor of T cell activation (VISTA), VSIG8, B and T lymphocyte attenuator (BTLA), Herpesvirus Entry Mediator (HVEM), CD160, T cell Ig and ITM domain (TIGIT), CD226, CD96, Lymphocyte activation gene-3 (LAG-3), transforming growth factor β (TGF-β), transforming growth factor β receptor (TGFβR), 4-1BB (CD137), Inducible T-Cell Costimulator (ICOS), OX-40 (CD134), glucocorticoid-induced TNFR-related protein (GITR), IL6R, IL23R, IL17R, IL-6, IL-23, IL-17, CD39, CD73, CCR4, CCR5, CXCR4, IL12R, CD4, CD25, CD3, epidermal growth factor receptor (EGFR, EGFR1, ErbB-1, HER1), ErbB-2 (HER2/neu), ErbB-3/HER3, ErbB-4/HER4, EGFR ligand, Vascular endothelial growth factor (VEGF), VEGFR (VEGFR1, VEGFR2), RANK ligand (RANKL), RANK, gp120.

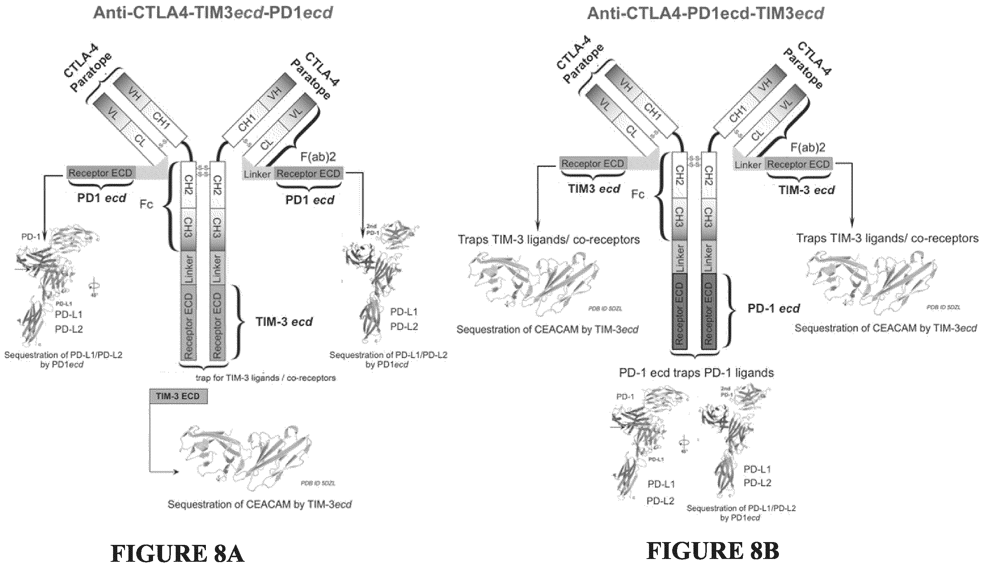

Figures 8A-B. Schematic design of multifunctional antibody-ligand traps of this invention comprising a targeting antibody that binds CTLA-4, ligand-binding IgV ectodomain sequence of TIM-3 (TIM-3ecd), and ligand-binding ectodomain sequence of PD-1 (PD-1ecd). The multifunctional antibody-ligand traps can bind CTLA-4 (via the antigen-binding CDR), bind PD-1 ligands, PD-L1 and PD-L2 (via the PD-1ecd) and TIM-3 ligands (CEACAM-1)(via the TIM-3ecd). **(A)** Schematic design of multifunctional antibody-ligand traps of this invention comprising a targeting antibody that binds CTLA-4, wherein the heavy chain of the antibody is fused at the C-terminus to a ligand-binding IgV ectodomain sequence of TIM-3 via a linker peptide (SEQ ID NO: 207), and the light chain of the antibody is fused at the C-terminus to a ligand-binding ectodomain sequence of PD-1 via a linker peptide (SEQ ID NO: 208). In one aspect, the linker is a peptide, (GGGGS)ₙ (SEQ ID NO: 3).**(B)** Schematic design of multifunctional antibody-ligand traps of this invention comprising a targeting antibody that binds CTLA-4, wherein the heavy chain of the antibody is fused at the C-terminus to a ligand-binding ectodomain sequence of PD-1 (SEQ ID NO: 205) via a linker peptide, and the light chain of the antibody is fused at the C-terminus to a ligand-binding ectodomain sequence of TIM-3 (SEQ ID NO: 206) via a linker peptide. In one aspect, the linker is a peptide, (GGGGS)ₙ (SEQ ID NO: 3).

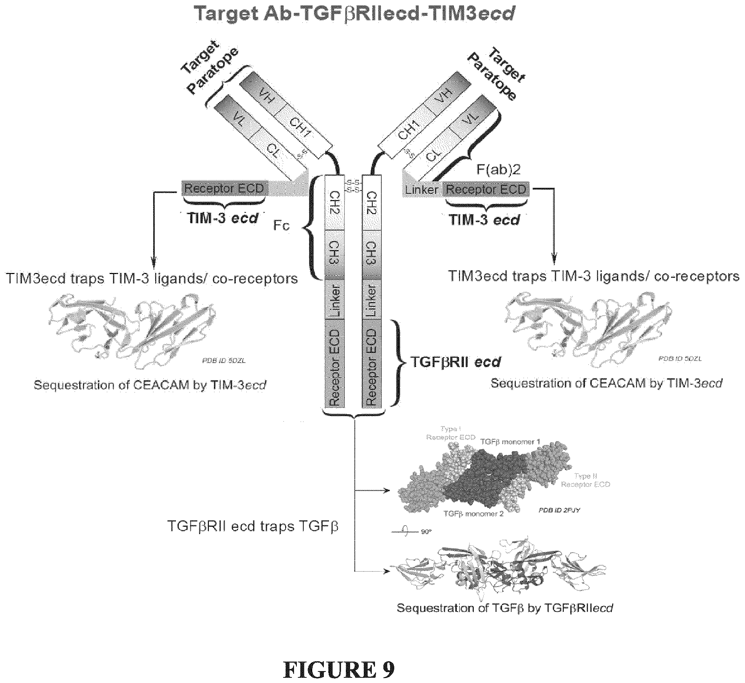

Figure 9. Schematic design of multifunctional antibody-ligand traps of this invention comprising a targeting antibody that binds a target epitope, ligand-binding IgV ectodomain sequence of TIM-3 (TIM-3ecd), and ligand-binding ectodomain sequence of TGFβRII (TGFβRIIecd). The multifunctional antibody-ligand traps can bind a target epitope (via the antigen-binding CDR), bind TGFβ (via the TGFβRIIecd) and TIM-3 ligands (CEACAM-1)(via the TIM-3ecd). In one embodiment, the multifunctional antibody-ligand traps of this invention comprise a targeting antibody, wherein the heavy chain of the antibody is fused at the C-terminus to a ligand-binding ectodomain sequence of TGFβRII (TGFβRIIecd) via a linker peptide (SEQ ID NO: 6), and the light chain of the antibody is fused at the C-terminus to a ligand-binding IgV ectodomain sequence of TIM-3 (TIM-3ecd: SEQ ID NO: 2) via a linker peptide. In one aspect, the linker is a peptide, (GGGGS)ₙ (SEQ ID NO: 3).In various embodiments, the targeting antibody of the multifunctional antibody-ligand traps of this invention binds one of the following molecules: Cytotoxic T lymphocyte associated antigen-4 (CTLA-4, CD 152), Programmed Death-1 protein (PD-1), Programmed death ligand (PD-L1), Programmed death ligand (PD-L2), B7-H3 (CD276) ,V domain Ig suppressor of T cell activation (VISTA), VSIG8, B and T lymphocyte attenuator (BTLA), Herpesvirus Entry Mediator (HVEM), CD160, T cell Ig and ITM domain (TIGIT), CD226, CD96, Lymphocyte activation gene-3 (LAG-3) ,4-1BB (CD137), Inducible T-Cell Costimulator (ICOS), OX-40 (CD134), glucocorticoid-induced TNFR-related protein (GITR), IL6R, IL23R, IL17R, IL-6, IL-23, IL-17, CD39, CD73, CCR4, CCR5, CXCR4, IL12R, CD4, CD25, CD3, epidermal growth factor receptor (EGFR, EGFR1, ErbB-1, HER1), ErbB-2 (HER2/neu), ErbB-3/HER3, ErbB-4/HER4, EGFR ligand, Vascular endothelial growth factor (VEGF), VEGFR (VEGFR1, VEGFR2), RANK ligand (RANKL), RANK.

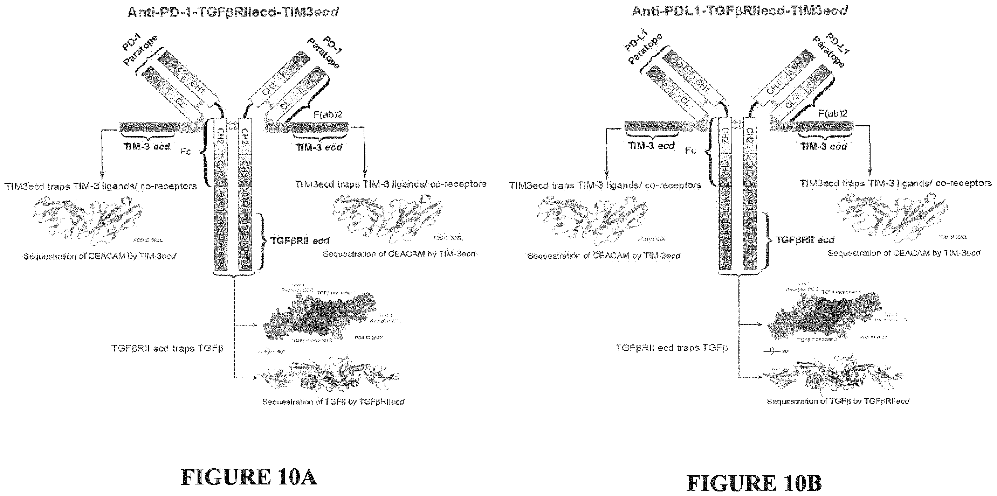

Figures 10A-C. Schematic design of multifunctional antibody-ligand traps of this invention comprising a targeting antibody that binds a T cell inhibitory receptor or ligand, ligand-binding IgV ectodomain sequence of TIM-3 (TIM-3ecd), and ligand-binding ectodomain sequence of TGFβRII (TGFβRIIecd).**(A)** Schematic design of multifunctional antibody-ligand traps of this invention comprising a targeting antibody that binds PD-1, ligand-binding IgV ectodomain sequence of TIM-3 (TIM-3ecd), and ligand-binding ectodomain sequence of TGFβRII (TGFβRIIecd). In one embodiment, the multifunctional antibody-ligand traps of this invention comprise a targeting antibody that binds PD-1, wherein the heavy chain of the antibody is fused at the C-terminus to a ligand-binding ectodomain sequence of TGFβRII (TGFβRIIecd)(SEQ ID NO: 6) via a linker peptide, and the light chain of the antibody is fused at the C-terminus to a ligand-binding IgV ectodomain sequence of TIM-3 (TIM-3ecd: SEQ ID NO: 2) via a linker peptide. In one aspect, the linker is a peptide, (GGGGS)ₙ (SEQ ID NO: 3). In one example, multifunctional antibody-ligand trap comprises a heavy chain corresponding to SEQ ID NO: 273 and a light chain corresponding to SEQ ID NO: 274 . In another example, multifunctional antibody-ligand trap comprises a heavy chain corresponding to SEQ ID NO: 277 and a light chain corresponding to SEQ ID NO: 278.**(B)** Schematic design of multifunctional antibody-ligand traps of this invention comprising a targeting antibody that binds PD-L1, ligand-binding IgV ectodomain sequence of TIM-3 (TIM-3ecd), and ligand-binding ectodomain sequence of TGFβRII (TGFβRIIecd). In one embodiment, the multifunctional antibody-ligand traps of this invention comprise a targeting antibody that binds PD-L1, wherein the heavy chain of the antibody is fused at the C-terminus to a ligand-binding ectodomain sequence of TGFβRII (TGFβRIIecd)(SEQ ID NO: 6) via a linker peptide, and the light chain of the antibody is fused at the C-terminus to a ligand-binding IgV ectodomain sequence of TIM-3 (TIM-3ecd: SEQ ID NO: 2 ) via a linker peptide. In one aspect, the linker is a peptide, (GGGGS)ₙ (SEQ ID NO: 3 ). In one example, multifunctional antibody-ligand trap comprises a heavy chain corresponding to SEQ ID NO: 285 and a light chain corresponding to SEQ ID NO: 285 . In another example, multifunctional antibody-ligand trap comprises a heavy chain corresponding to SEQ ID NO: 281 and a light chain corresponding to SEQ ID NO: 282. **(C)** Schematic design of multifunctional antibody-ligand traps of this invention comprising a targeting antibody that binds CTLA-4, ligand-binding IgV ectodomain sequence of TIM-3 (TIM-3ecd), and ligand-binding ectodomain sequence of TGFβRII (TGFβRIIecd). In one embodiment, the multifunctional antibody-ligand traps of this invention comprise a targeting antibody that binds CTLA-4, wherein the heavy chain of the antibody is fused at the C-terminus to a ligand-binding ectodomain sequence of TGFβRII (TGF□RIIecd)(SEQ ID NO: 6 ) via a linker peptide, and the light chain of the antibody is fused at the C-terminus to a ligand-binding IgV ectodomain sequence of TIM-3 (TIM-3ecd: SEQ ID NO: 2 ) via a linker peptide. In one aspect, the linker is a peptide, (GGGGS)ₙ (SEQ ID NO: 3). In one example, multifunctional antibody-ligand trap comprises a heavy chain corresponding to SEQ ID NO: 203 and a light chain corresponding to SEQ ID NO: 204.

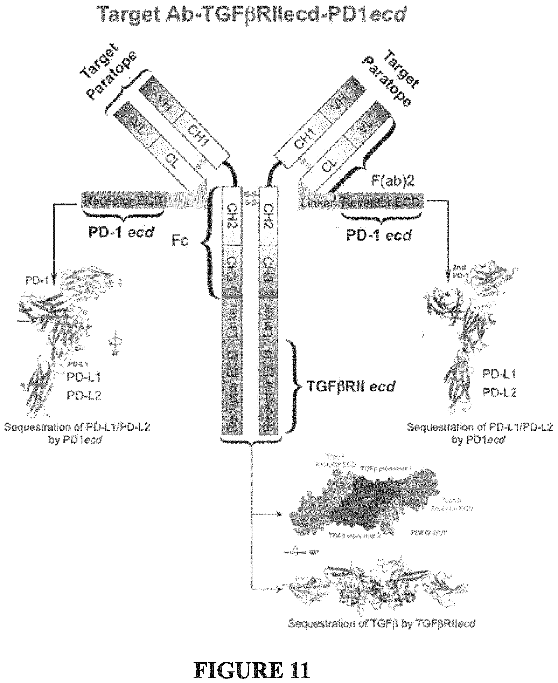

Figure 11. Schematic design of multifunctional antibody-ligand traps of this invention comprising a targeting antibody that binds a target epitope, ligand-binding ectodomain sequence of PD-1 (PD-1ecd), and ligand-binding ectodomain sequence of TGFβRII (TGFβRIIecd). The multifunctional antibody-ligand traps can bind a target epitope (via the antigen-binding CDR), bind TGFβ (via the TGFβRIIecd) and PD-1 ligands, PD-L1 and PD-L2 (via the PD-1ecd). In one embodiment, the multifunctional antibody-ligand traps of this invention comprise a targeting antibody, wherein the heavy chain of the antibody is fused at the C-terminus to a ligand-binding ectodomain sequence of TGFβRII (TGFβRIIecd)(SEQ ID NO: 6) via a linker peptide, and the light chain of the antibody is fused at the C-terminus to ligand-binding ectodomain sequence of PD-1 (PD1ecd: SEQ ID NO: 7) via a linker peptide. In one aspect, the linker is a peptide, (GGGGS)ₙ (SEQ ID NO: 3). In various embodiments, the targeting antibody of the multifunctional antibody-ligand traps of this invention binds one of the following molecules: Cytotoxic T lymphocyte associated antigen-4 (CTLA-4, CD152), T-cell immunoglobulin and mucin-domain containing-3 (TIM-3), CEACAM-1, Carcinoembryonic Antigen (CEA),V domain Ig suppressor of T cell activation (VISTA), VSIG8, B and T lymphocyte attenuator (BTLA), Herpesvirus Entry Mediator (HVEM), CD160, T cell Ig and ITM domain (TIGIT), CD226, CD96, Lymphocyte activation gene-3 (LAG-3), 4-1BB (CD137), Inducible T-Cell Costimulator (ICOS), OX-40 (CD134), glucocorticoid-induced TNFR-related protein (GITR), IL6R, IL23R, IL17R, IL-6, IL-23, IL-17, CD39, CD73, CD122, CCR4, CCR5, CXCR4, IL12R, CD4, CD25, CD3, epidermal growth factor receptor (EGFR, EGFR1, ErbB-1, HER1), ErbB-2 (HER2/neu), ErbB-3/HER3, ErbB-4/HER4, EGFR ligand, Vascular endothelial growth factor (VEGF), VEGFR, RANK ligand (RANKL), RANK.

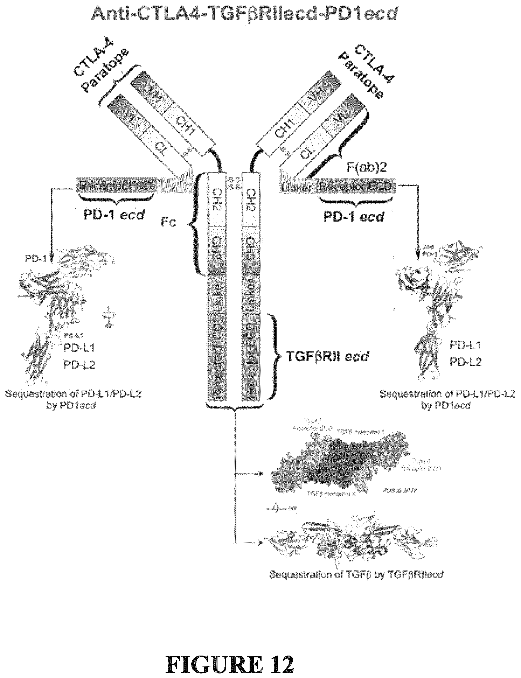

Figure 12. In various embodiments, multifunctional antibody-ligand traps of this invention comprises a targeting antibody that binds a T cell inhibitory receptor or ligand, ligand-binding ectodomain sequence of PD-1 (PD-1ecd), and ligand-binding ectodomain sequence of TGFβRII (TGFβRIIecd). In one embodiment, the multifunctional antibody-ligand traps of this invention comprise a targeting antibody that binds CTLA-4, wherein the heavy chain of the antibody is fused at the C-terminus to a ligand-binding ectodomain sequence of TGFβRII (TGFβRIIecd)(SEQ ID NO: 203) via a linker peptide, and the light chain of the antibody is fused at the C-terminus to a ligand-binding ectodomain sequence of PD-1 (PD-1ecd: SEQ ID NO: 202) via a linker peptide. In one aspect, the linker is a peptide, (GGGGS)ₙ (SEQ ID NO: 3). In one example, multifunctional antibody-ligand trap comprises a heavy chain corresponding to SEQ ID NO: 202 and a light chain corresponding to SEQ ID NO: 203.

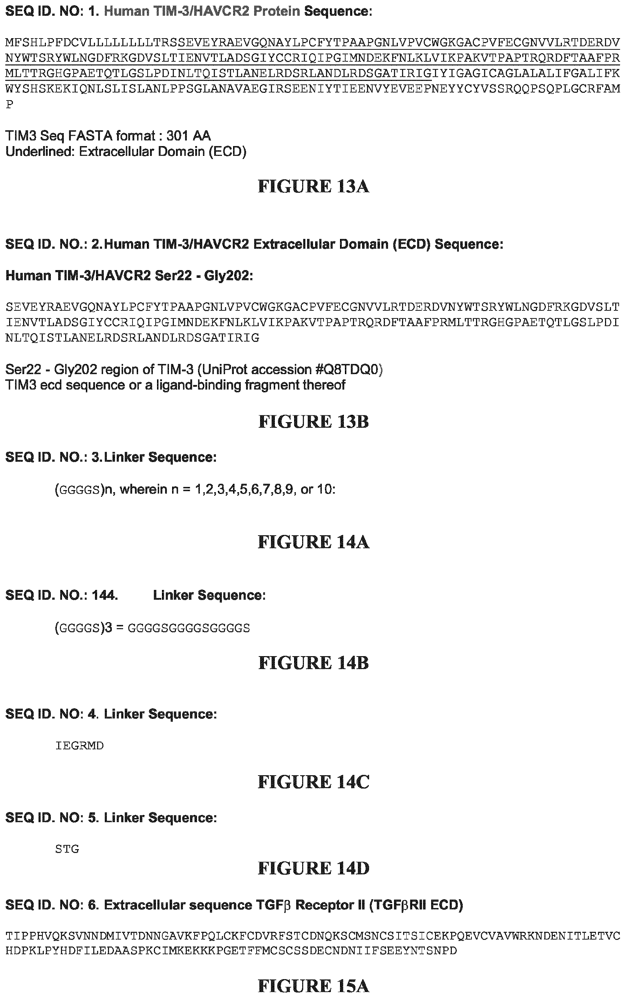

Figures 13 A-B are polypeptide sequences. A. Human TIM-3/HAVCR2 protein sequence, SEQ ID NO: 1. B. Human TIM-3/HAVCR2 Extracellular Domain (ECD) protein, sequence SEQ ID NO: 2.
Figures 14 A-D are polypeptide sequences. A. Linker sequence SEQ ID NO: 3. B. Linker sequence SEQ ID NO: 144. C. Linker sequence SEQ ID NO: 4. D. Linker sequence SEQ ID NO: 5.

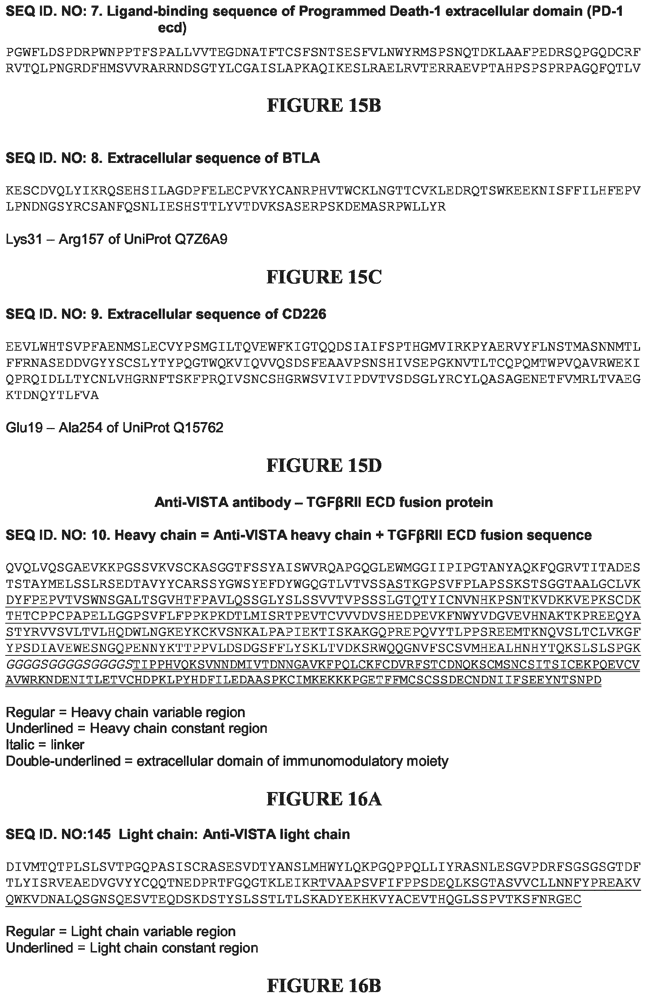

Figures 15 A-D are polypeptide sequences. A. TGFβ Receptor II extracellular domain protein sequence, SEQ ID NO: 6. B. PD-1 extracellular domain protein sequence, SEQ ID NO: 7. C. BTLA extracellular domain protein sequence, SEQ ID NO: 8. D. CD226 extracellular domain protein sequence, SEQ ID NO: 9.
Figure 16 A-B are polypeptide sequences. A. Anti-VISTA heavy chain + TGFβRII ECD fusion sequence, SEQ ID NO: 10. B. Anti-VISTA light chain protein sequence, SEQ ID NO: 145.

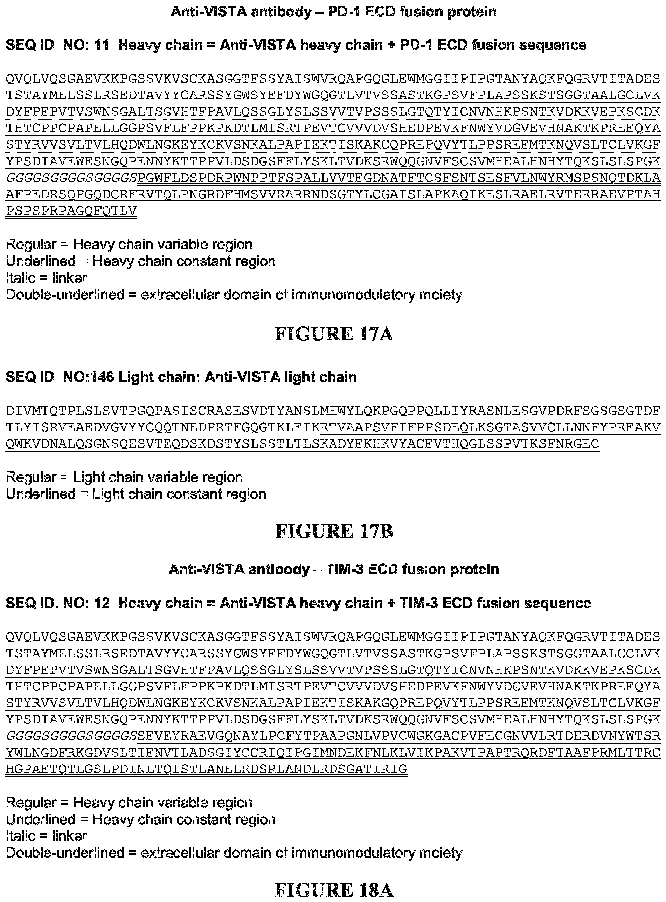

Figure 17 A- are polypeptide sequences. A. Anti-VISTA heavy chain + PD-1 ECD fusion sequence, SEQ ID NO: 11. B. Anti-VISTA light chain protein sequence, SEQ ID NO: 146.

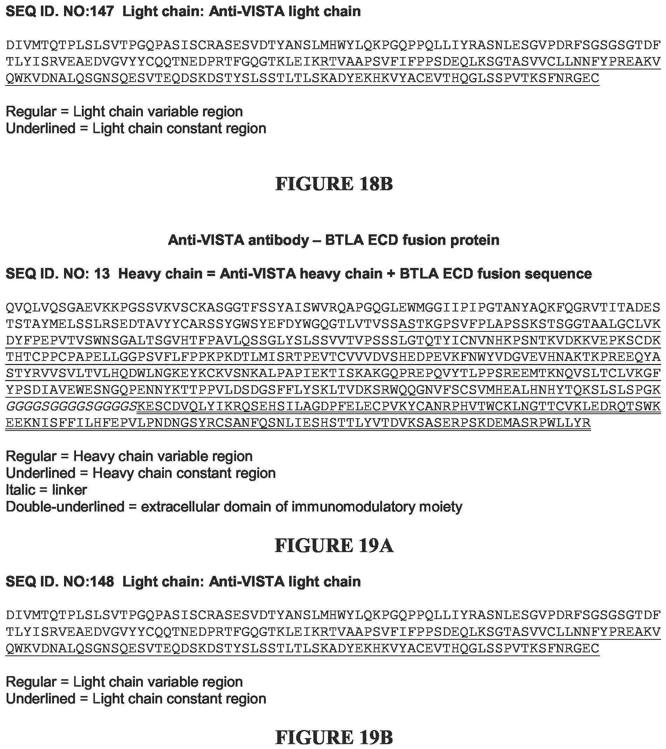

Figure 18 A-B are polypeptide sequences. A. Anti-VISTA heavy chain + TIM-3 ECD fusion sequence, SEQ ID NO: 12. B. Anti-VISTA light chain protein sequence, SEQ ID NO: 147.
Figure 19 A-B are polypeptide sequences. A. Anti-VISTA heavy chain + BTLA ECD fusion sequence, SEQ ID NO: 13. B. Anti-VISTA light chain protein sequence, SEQ ID NO: 148.

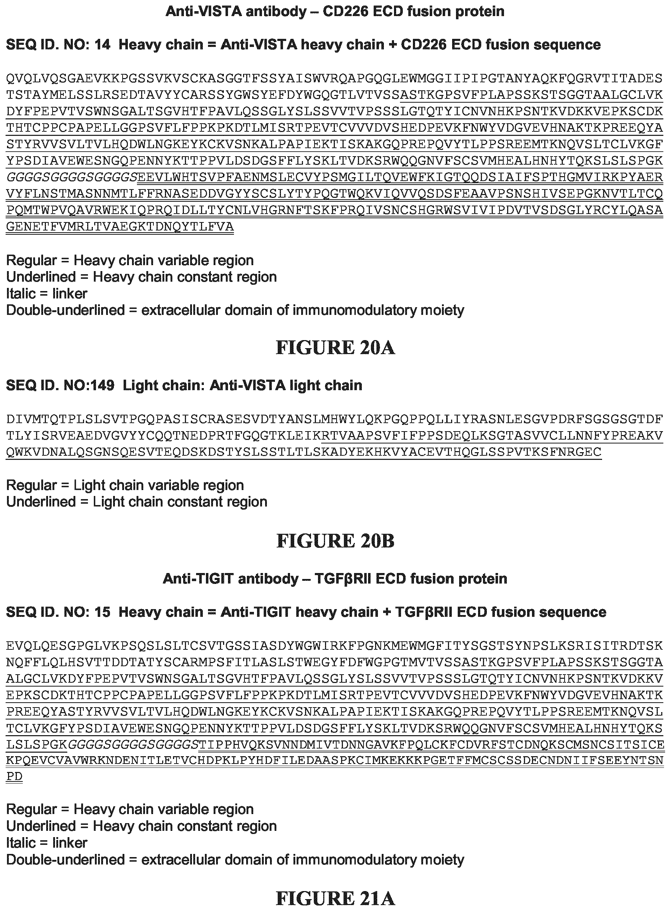

Figure 20 A- are polypeptide sequences. A. Anti-VISTA heavy chain + CD226 ECD fusion sequence, SEQ ID NO: 14. B. Anti-VISTA light chain protein sequence, SEQ ID NO: 149.

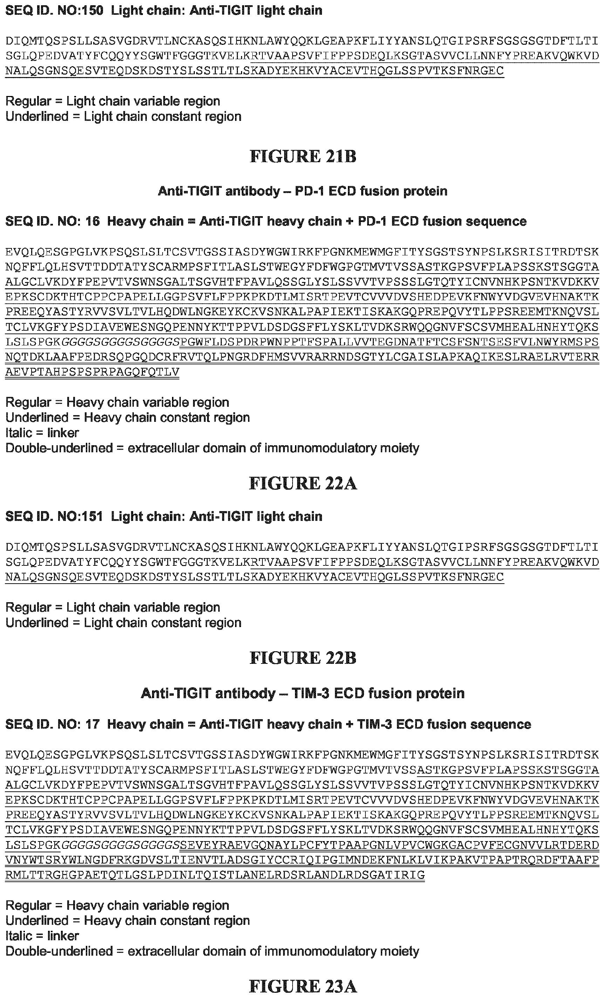

Figure 21 A-B are polypeptide sequences. A. Anti-TIGIT heavy chain + TGFβRII ECD fusion sequence, SEQ ID NO: 15. B. Anti-TIGIT light chain protein sequence, SEQ ID NO: 150.
Figure 22 A-B are polypeptide sequences. A. Anti-TIGIT heavy chain + PD-1 ECD fusion protein sequence, SEQ ID NO: 16. B. Anti- TIGIT light chain protein sequence, SEQ ID NO: 151.

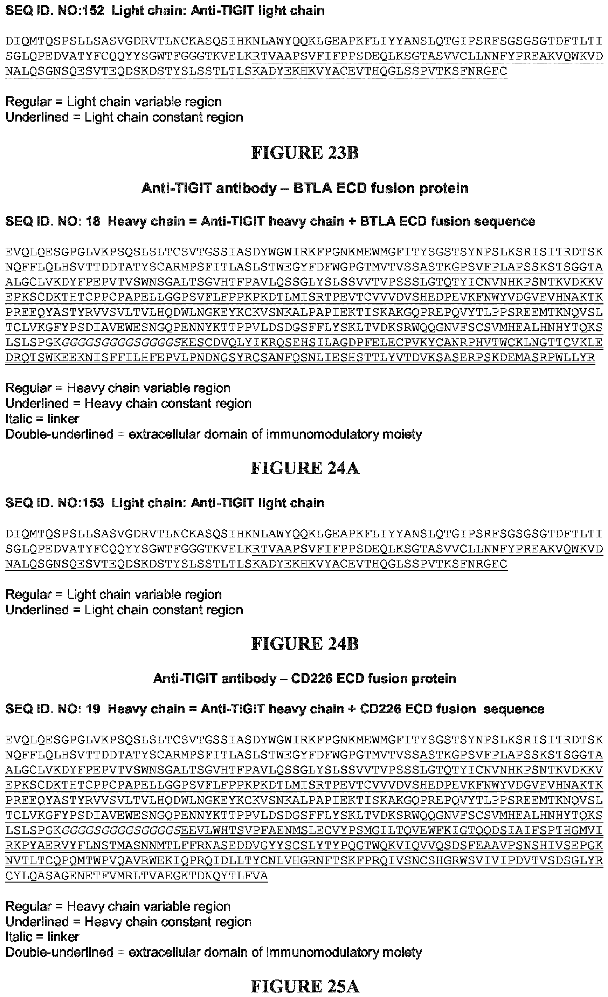

Figure 23 A-B are polypeptide sequences. A. Anti-TIGIT heavy chain + TIM-3 ECD fusion protein sequence, SEQ ID NO: 17. B. Anti- TIGIT light chain protein sequence, SEQ ID NO: 152.
Figure 24 A-B are polypeptide sequences. A. Anti-TIGIT heavy chain + BTLA ECD fusion protein sequence, SEQ ID NO: 18. B. Anti- TIGIT light chain protein sequence, SEQ ID NO: 153.

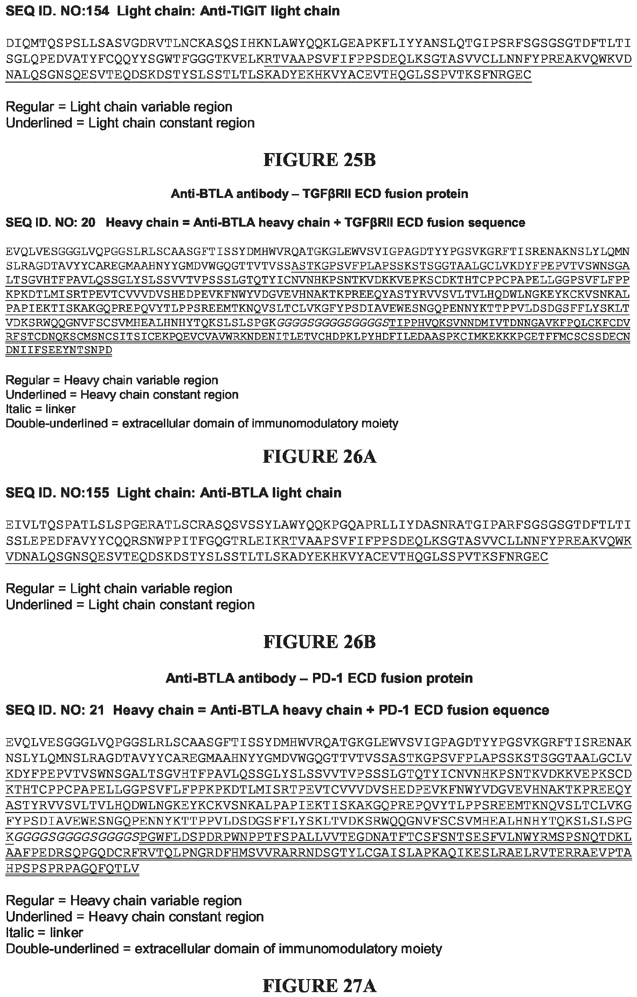

Figure 25 A-B are polypeptide sequences. A. Anti-TIGIT heavy chain + CD226 ECD fusion protein sequence, SEQ ID NO: 19. B. Anti- TIGIT light chain protein sequence, SEQ ID NO: 154.
Figure 26 A-B are polypeptide sequences A. Anti-BTLA heavy chain + TGFβRII ECD fusion protein sequence, SEQ ID NO: 20. B. Anti- BTLA light chain protein sequence, SEQ ID NO: 155.

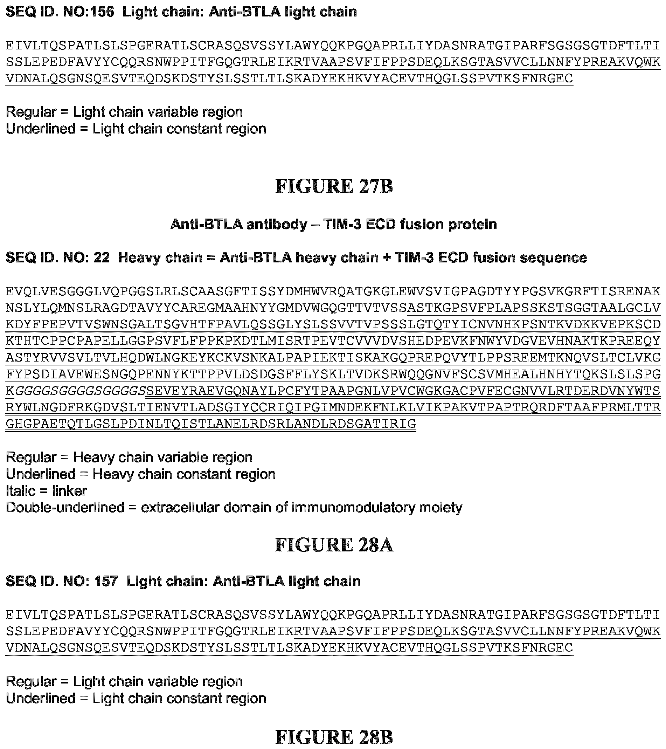

Figure 27 A-B are polypeptide sequences. A. Anti-BTLA heavy chain + PD-1 ECD fusion protein sequence, SEQ ID NOSEQ ID NO: 21. B. Anti- BTLA light chain protein sequence, SEQ ID NO: 156.
Figure 28 A-B are polypeptide sequences. A. Anti-BTLA heavy chain + TIM-3 ECD fusion protein sequence, SEQ ID NO: 22. B. Anti- BTLA light chain protein sequence, SEQ ID NO: 157.

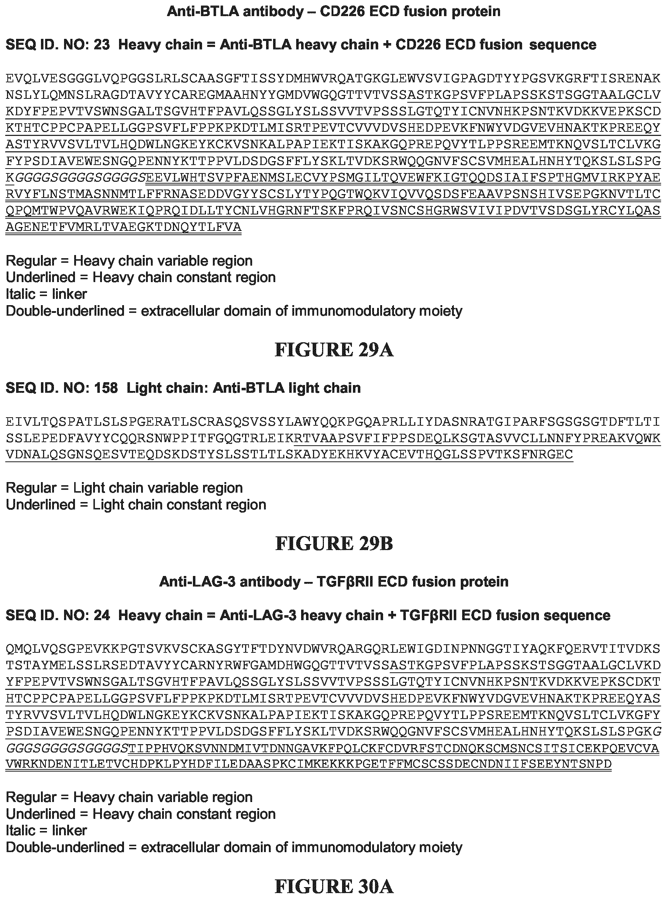

Figure 29 A- are polypeptide sequences. A. Anti-BTLA heavy chain + CD226 ECD fusion protein sequence, SEQ ID NO: 23. B. Anti- BTLA light chain protein sequence, SEQ ID NO: 158.

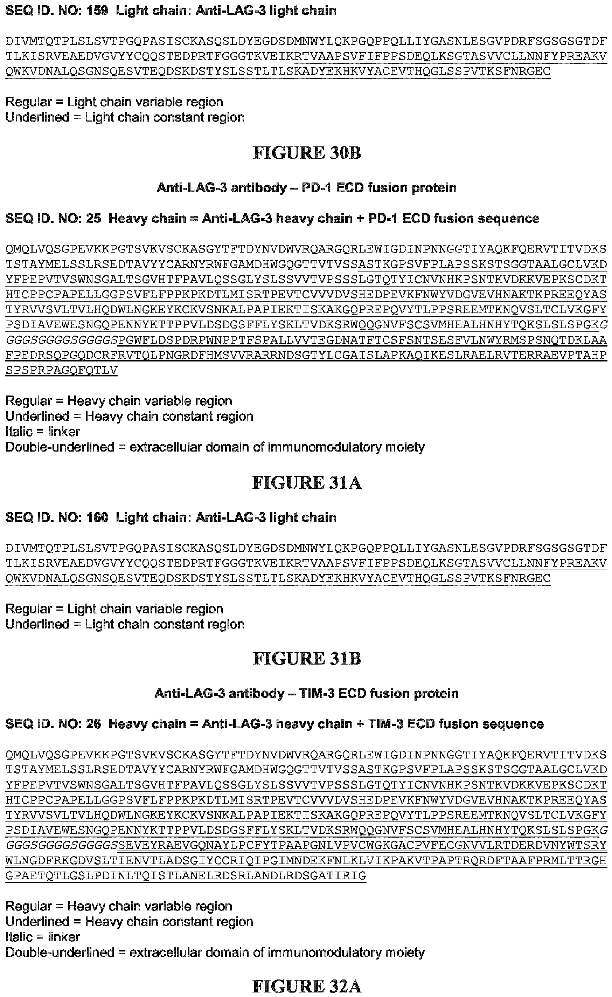

Figure 30 A-B are polypeptide sequences. A. Anti- LAG-3 heavy chain + TGFβRII ECD fusion protein sequence, SEQ ID NO: 24. B. Anti- LAG-3 light chain protein sequence, SEQ ID NO: 159.
Figure 31 A-B are polypeptide sequences. A. Anti- LAG-3 heavy chain + PD-1 ECD fusion protein sequence, SEQ ID NO: 25. B. Anti- LAG-3 light chain protein sequence, SEQ ID NO: 160.

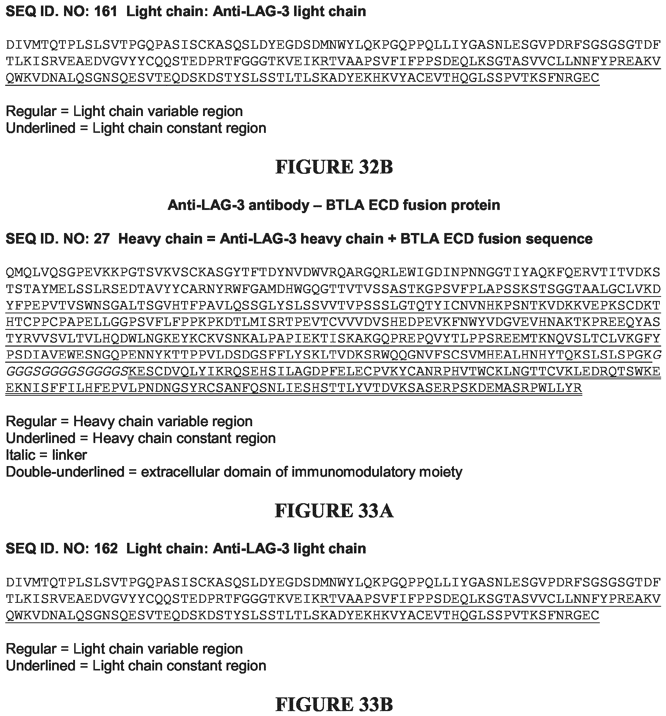

Figure 32 A-B are polypeptide sequences. A. Anti- LAG-3 heavy chain + TIM-3 ECD fusion protein sequence, SEQ ID NO: 26. B. Anti- LAG-3 light chain protein sequence, SEQ ID NO: 161.
Figure 33 A-B are polypeptide sequences. A. Anti- LAG-3 heavy chain + BTLA ECD fusion protein sequence, SEQ ID NO: 27. B. Anti- LAG-3 light chain protein sequence, SEQ ID NO: 162.

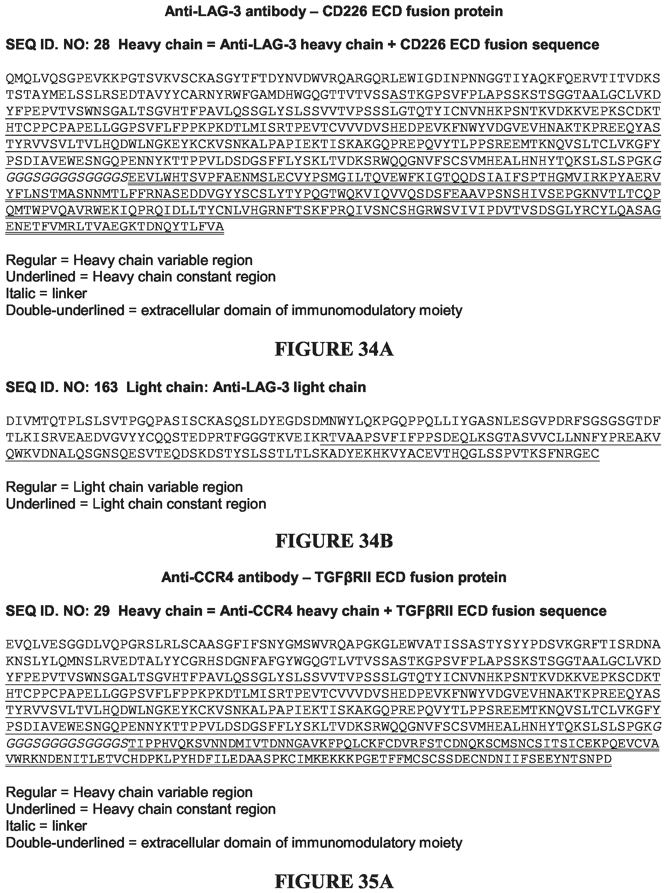

Figure 34 A-B are polypeptide sequences. A. Anti- LAG-3 heavy chain + CD226 ECD fusion protein sequence, SEQ ID NO: 28. B. Anti- LAG-3 light chain protein sequence, SEQ ID NO: 163.

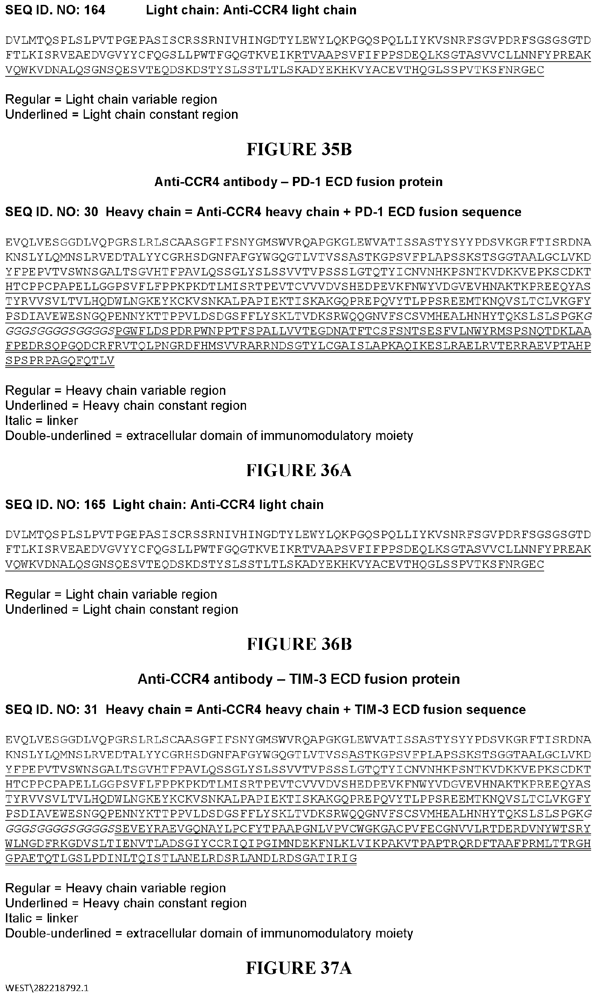

Figure 35 A-B are polypeptide sequences. A. Anti- CCR4 heavy chain + TGFβRII ECD fusion protein sequence, SEQ ID NO: 29. B. Anti- CCR4 light chain protein sequence, SEQ ID NO: 164.
Figure 36 A-B are polypeptide sequences. A. Anti- CCR4 heavy chain + PD-1 ECD fusion protein sequence, SEQ ID NO: 30. B. Anti- CCR4 light chain protein sequence, SEQ ID NO: 165.

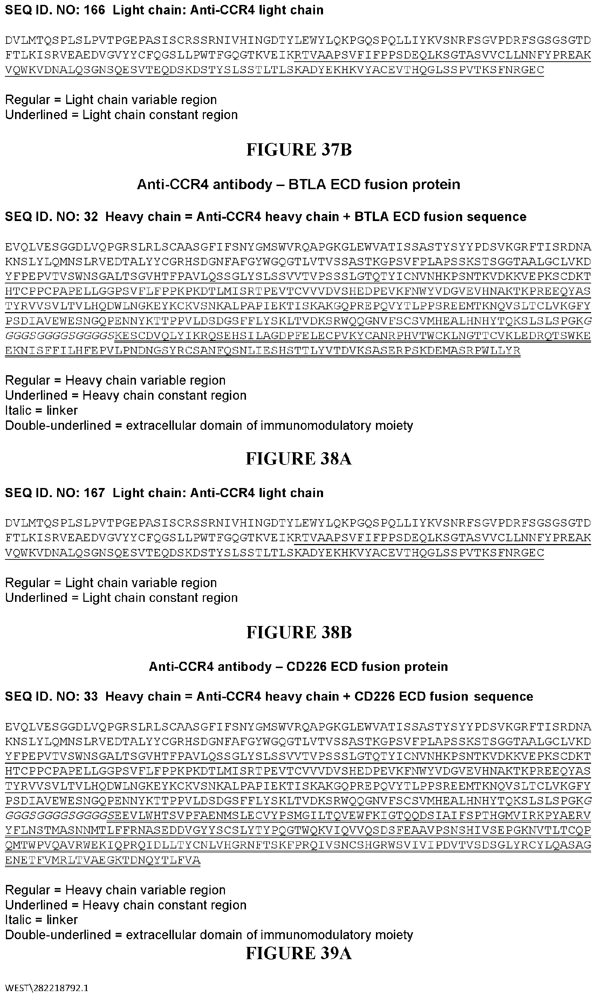

Figure 37 A-B are polypeptide sequences. A. Anti- CCR4 heavy chain + TIM-3 ECD fusion protein sequence, SEQ ID NO: 31. B. Anti- CCR4 light chain protein sequence, SEQ ID NO: 166.
Figure 38 A-B are polypeptide sequences. A. Anti- CCR4 heavy chain + BTLA ECD fusion protein sequence, SEQ ID NO: 32. B. Anti- CCR4 light chain protein sequence, SEQ ID NO: 167.

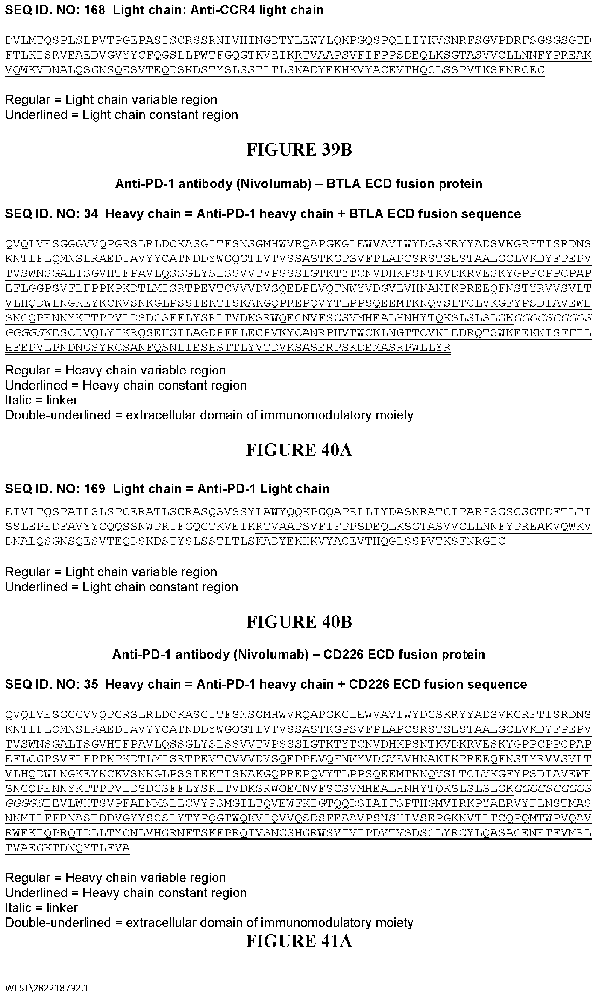

Figure 39 A-B are polypeptide sequences. A. Anti- CCR4 heavy chain + CD226 ECD fusion protein sequence, SEQ ID NO: 33. B. Anti- CCR4 light chain protein sequence, SEQ ID NO: 168.
Figure 40 A-B are polypeptide sequences. A. Anti- PD1 heavy chain + BTLA ECD fusion protein sequence, SEQ ID NO: 34. B. Anti- PD-1 light chain protein sequence, SEQ ID NO: 169.

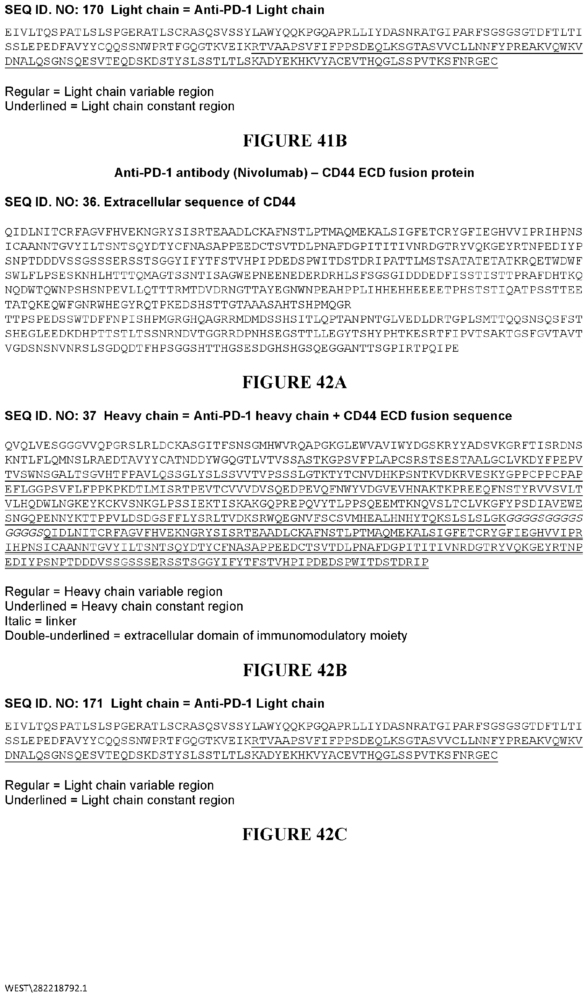

Figure 41 A-B are polypeptide sequences. A. Anti- PD1 heavy chain + CD226 ECD fusion protein sequence, SEQ ID NO: 35. B. Anti- PD-1 light chain protein sequence, SEQ ID NO: 170.
Figure 42 A-C are polypeptide sequences. A. Extracellular domain of CD44 protein sequence, SEQ ID NO: 36. B. Anti- PD1 heavy chain + CD44 ECD fusion protein sequence, SEQ ID NO: 37. C. Anti- PD-1 light chain protein sequence, SEQ ID NO: 171.

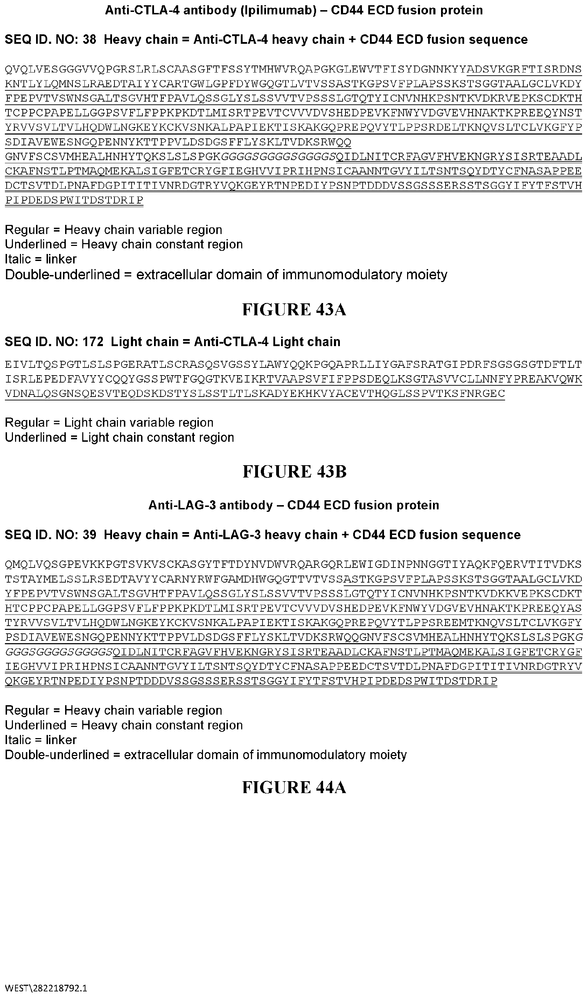

Figure 43 A-B are polypeptide sequences. A. Anti- CTLA-4 heavy chain + CD44 ECD fusion protein sequence, SEQ ID NO: 38. B. Anti- CTLA-4 light chain protein sequence, SEQ ID NO: 172.

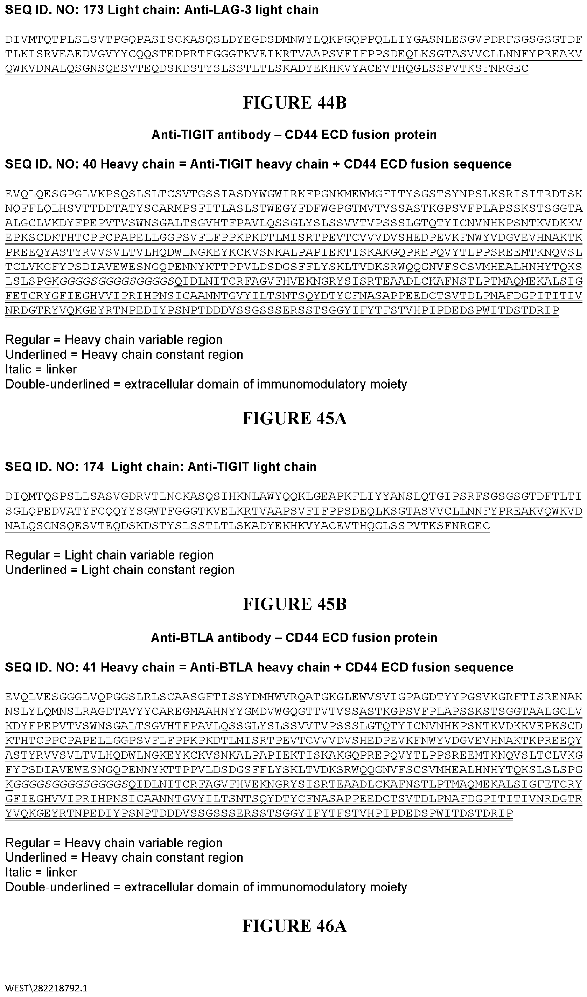

Figure 44 A-B are polypeptide sequences. A. Anti-LAG-3 heavy chain + CD44 ECD fusion protein sequence, SEQ ID NO: 39. B. Anti- LAG-3 light chain protein sequence, SEQ ID NO: 173.
Figure 45 A-B are polypeptide sequences. A. Anti-TIGIT heavy chain + CD44 ECD fusion protein sequence, SEQ ID NO: 40. B. Anti- TIGIT light chain protein sequence, SEQ ID NO: 174.

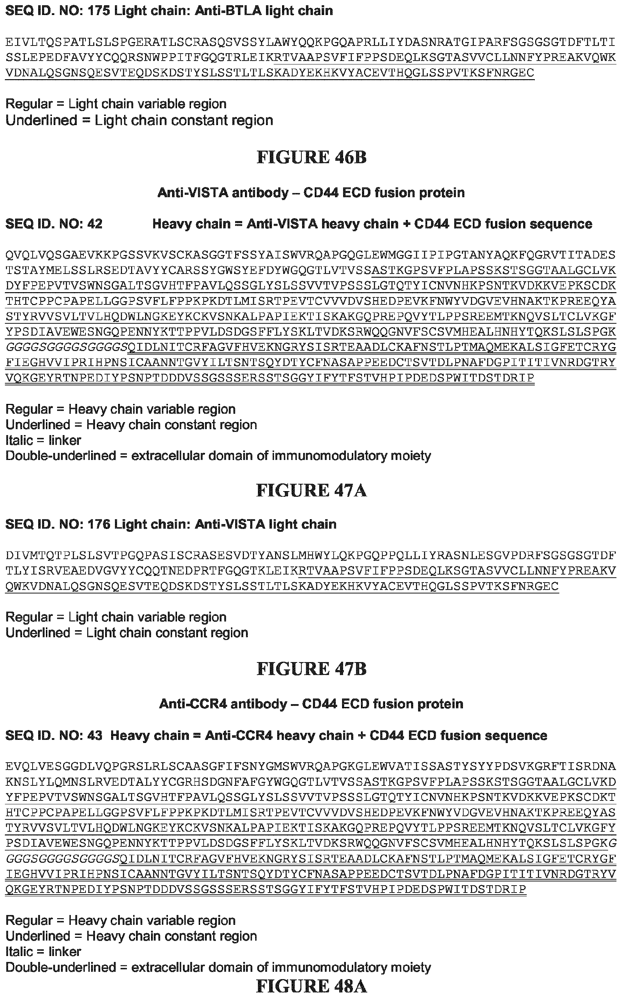

Figure 46 A-B are polypeptide sequences. A. Anti-BTLA heavy chain + CD44 ECD fusion protein sequence, SEQ ID NO: 41. B. Anti- BTLA light chain protein sequence, SEQ ID NO: 175.
Figure 47 A-B are polypeptide sequences. A. Anti- VISTA heavy chain + CD44 ECD fusion protein sequence, SEQ ID NO: 42. B. Anti- VISTA light chain protein sequence, SEQ ID NO: 176.

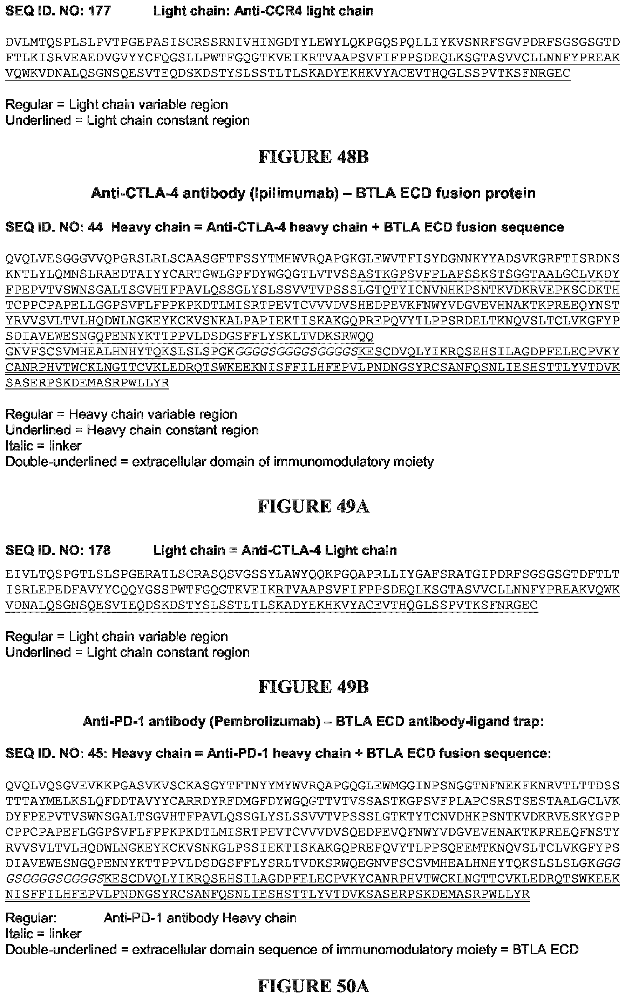

Figure 48 A-B are polypeptide sequences. A. Anti- CCR4 heavy chain + CD44 ECD fusion protein sequence, SEQ ID NO: 43. B. Anti- CCR4 light chain protein sequence, SEQ ID NO: 177.
Figure 49 A-B are polypeptide sequences. A. Anti- CTLA-4 heavy chain + BTLA ECD fusion protein sequence, SEQ ID NO: 44. B. Anti- CTLA-4 light chain protein sequence, SEQ ID NO: 178.

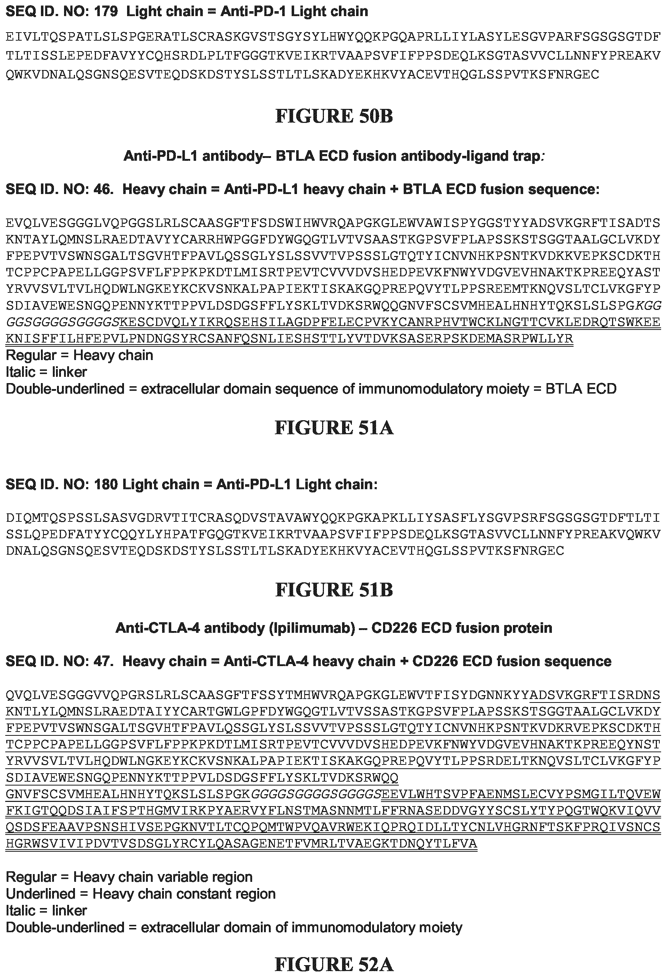

Figure 50 A-B are polypeptide sequences. A. Anti- PD-1 heavy chain + BTLA ECD fusion protein sequence, SEQ ID NO: 45. B. Anti- PD-1 light chain protein sequence, SEQ ID NO: 179.
Figure 51 A-B are polypeptide sequences. A. Anti- PD-L1 heavy chain + BTLA ECD fusion protein sequence, SEQ ID NO: 46. B. Anti- PD-L1 light chain protein sequence, SEQ ID NO: 180.

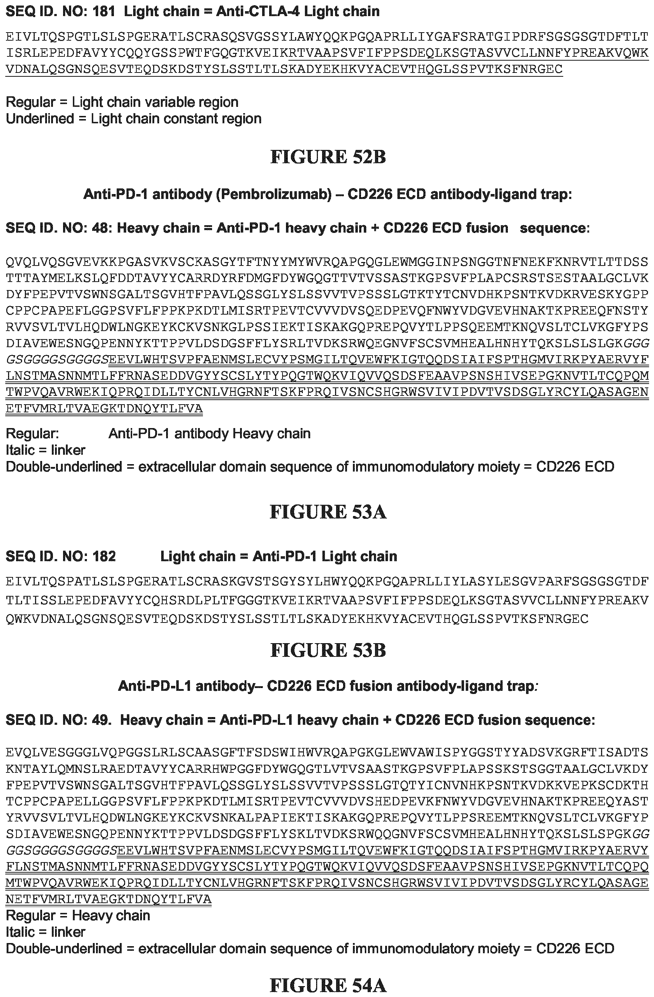

Figure 52 A-B are polypeptide sequences. A. Anti- CTLA-4 heavy chain + CD226 ECD fusion protein sequence, SEQ ID NO: 47. B. Anti- CTLA-4 light chain protein sequence, SEQ ID NO: 181.
Figure 53 A-B are polypeptide sequences. A. Anti- PD-1 heavy chain + CD226 ECD fusion protein sequence, SEQ ID NO: 48. B. Anti- PD-1 light chain protein sequence, SEQ ID NO: 182.

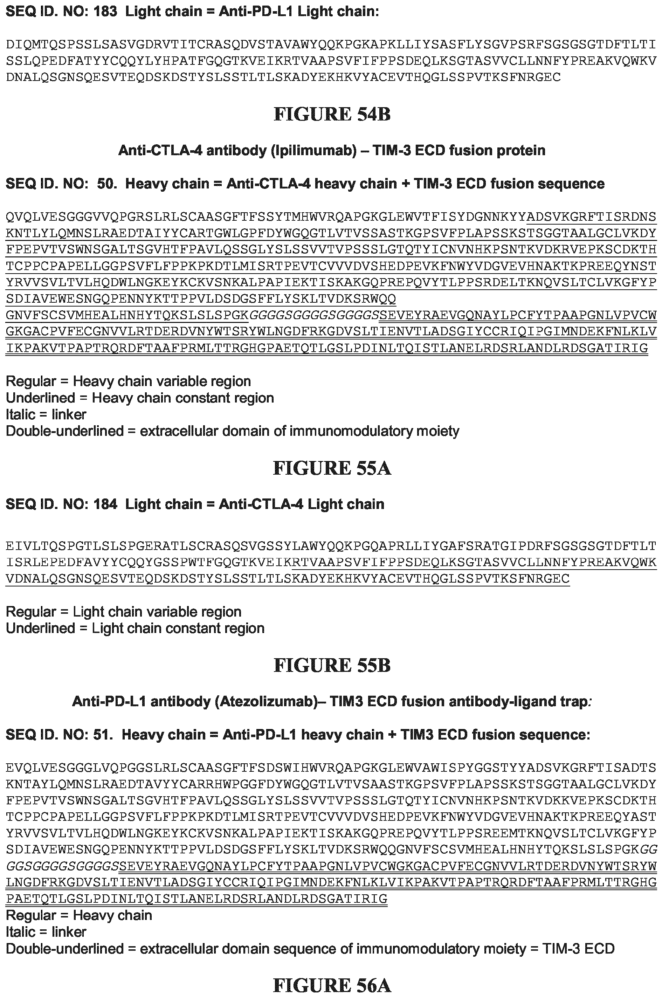

Figure 54 A-B are polypeptide sequences. A. Anti- PD-L1 heavy chain + CD226 ECD fusion protein sequence, SEQ ID NO: 49. B. Anti- PD-L1 light chain protein sequence, SEQ ID NO: 183.
Figure 55 A-B are polypeptide sequences. A. Anti- CTLA-4 heavy chain + TIM-3 ECD fusion protein sequence, SEQ ID NO: 50. B. Anti- CTLA-4 light chain protein sequence, SEQ ID NO: 184.

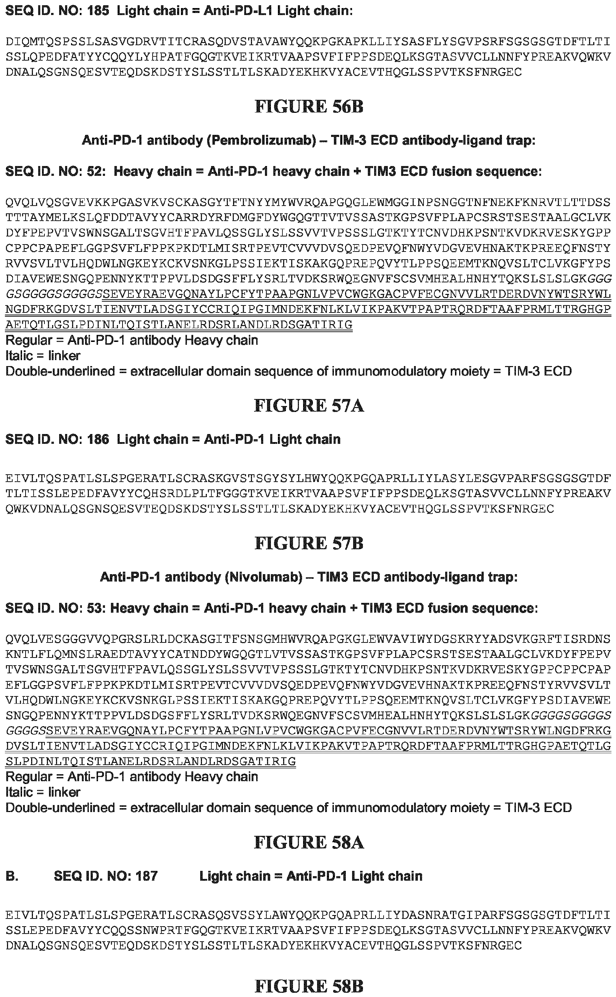

Figure 56 A-B are polypeptide sequences. A. Anti- PD-L1 heavy chain + TIM-3 ECD fusion protein sequence, SEQ ID NO: 51. B. Anti- PD-L1 light chain protein sequence, SEQ ID NO: 185.
Figure 57 A-B are polypeptide sequences. A. Anti- PD-1 heavy chain + TIM-3 ECD fusion protein sequence, SEQ ID NO: 52. B. Anti- PD-1 light chain protein sequence, SEQ ID NO: 186.
Figure 58 A-B are polypeptide sequences A. Anti- PD-1 heavy chain + TIM-3 ECD fusion protein sequence, SEQ ID NO: 53. B. Anti- PD-1 light chain protein sequence, SEQ ID NO: 187.

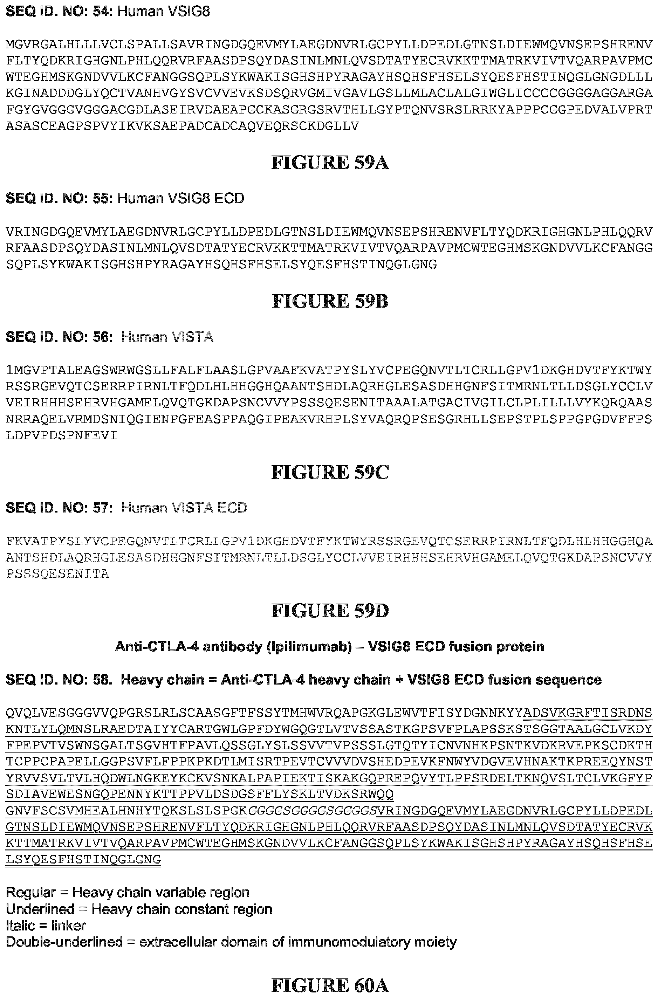

Figure 59 A-D are polypeptide sequences. B. Human VSIG8 ECD protein sequence, SEQ ID NO: 55. C. Human VISTA protein sequence, SEQ ID NO: 56. B. Human VISTA ECD protein sequence, SEQ ID NO: 57.

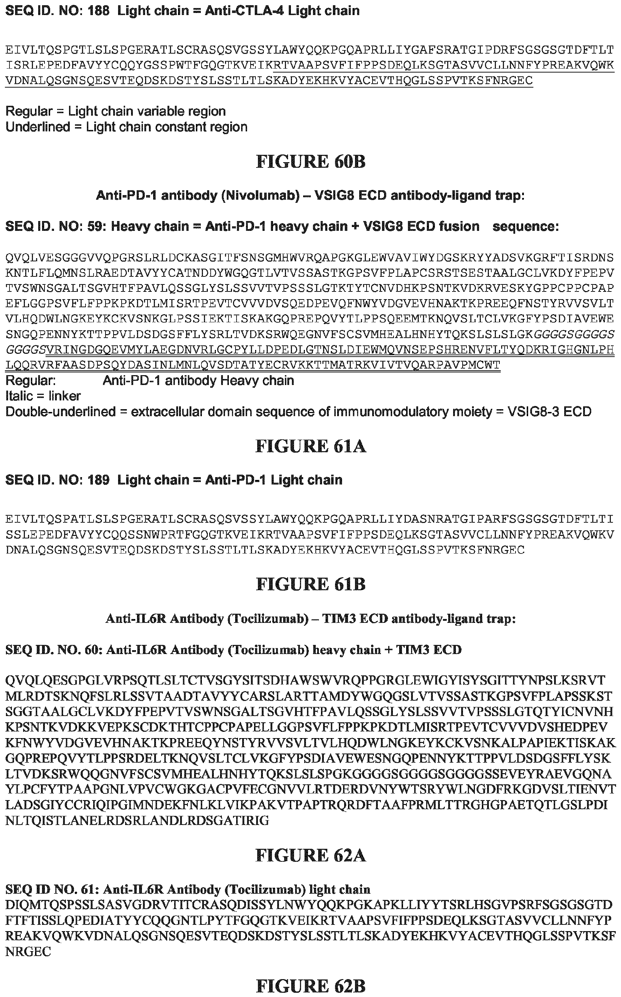

Figure 60 A-B are polypeptide sequences. A. Anti- CTLA-4 heavy chain + VSIG8 ECD fusion protein sequence, SEQ ID NO: 58. B. Anti- CTLA-4 light chain protein sequence, SEQ ID NO: 188.
Figure 61 A-B are polypeptide sequences. A. Anti- CTLA-4 heavy chain + VSIG8 ECD fusion protein sequence, SEQ ID NO: 59. B. Anti- CTLA-4 light chain protein sequence, SEQ ID NO: 189.
Figure 62 A-B are polypeptide sequences A. Anti- IL6R heavy chain + TIM3 ECD fusion protein sequence, SEQ ID NO: 60. B. Anti- IL6R light chain protein sequence, SEQ ID NO: 61.

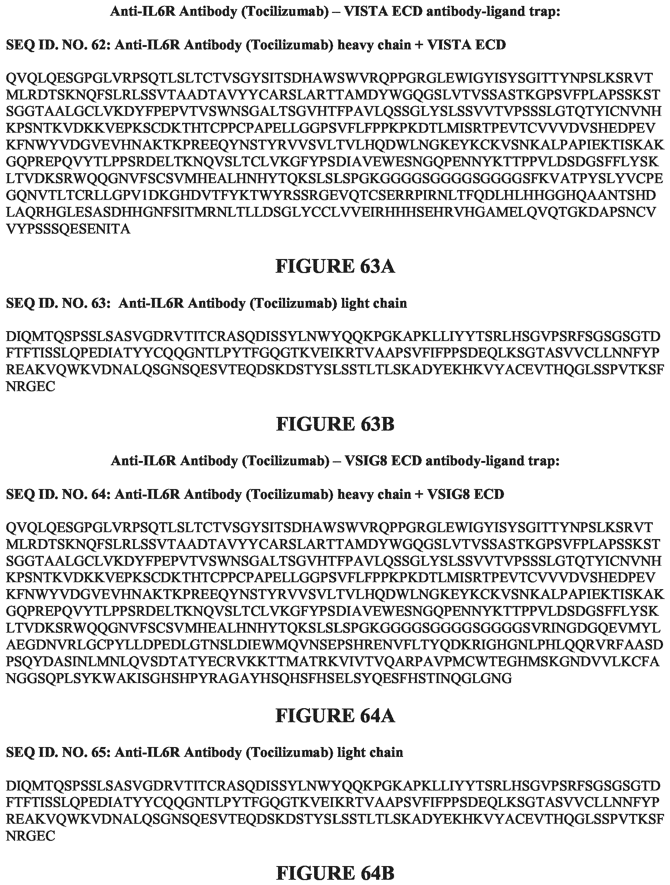

Figure 63 A- are polypeptide sequences. A. Anti- IL6R heavy chain + VISTA ECD fusion protein sequence, SEQ ID NO: 62. B. Anti- IL6R light chain protein sequence, SEQ ID NO: 63.
Figure 64 A-B are polypeptide sequences. A. Anti- IL6R heavy chain + VSIG8 ECD fusion protein sequence, SEQ ID NO: 64. B. Anti- IL6R light chain protein sequence, SEQ ID NO: 65.

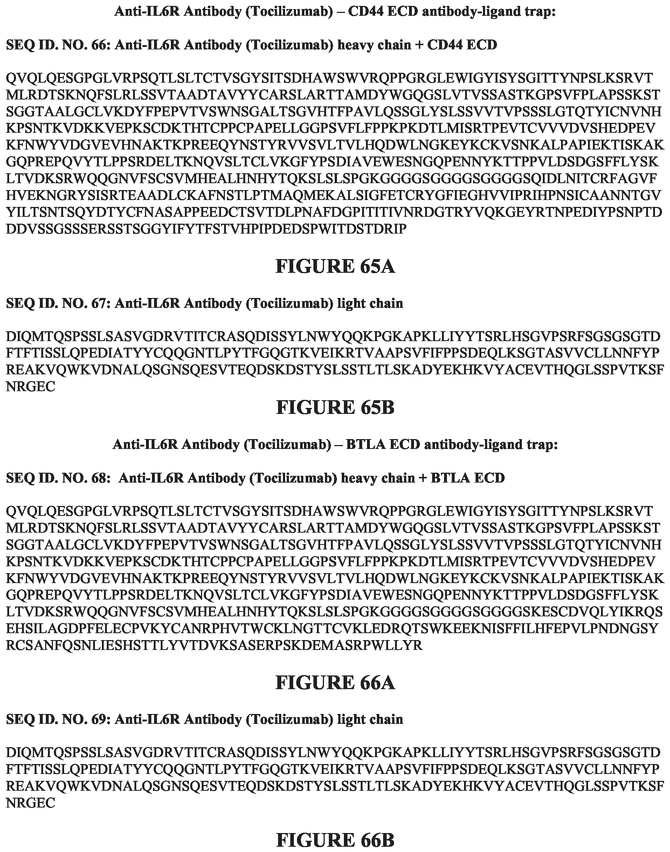

Figure 65 A-B are polypeptide sequences A. Anti- IL6R heavy chain + CD44 ECD fusion protein sequence, SEQ ID NO: 66. B. Anti- IL6R light chain protein sequence, SEQ ID NO: 67.
Figure 66 A-B are polypeptide sequences. A. Anti- IL6R heavy chain + BTLA ECD fusion protein sequence, SEQ ID NO: 68. B. Anti- IL6R light chain protein sequence, SEQ ID NO: 69.

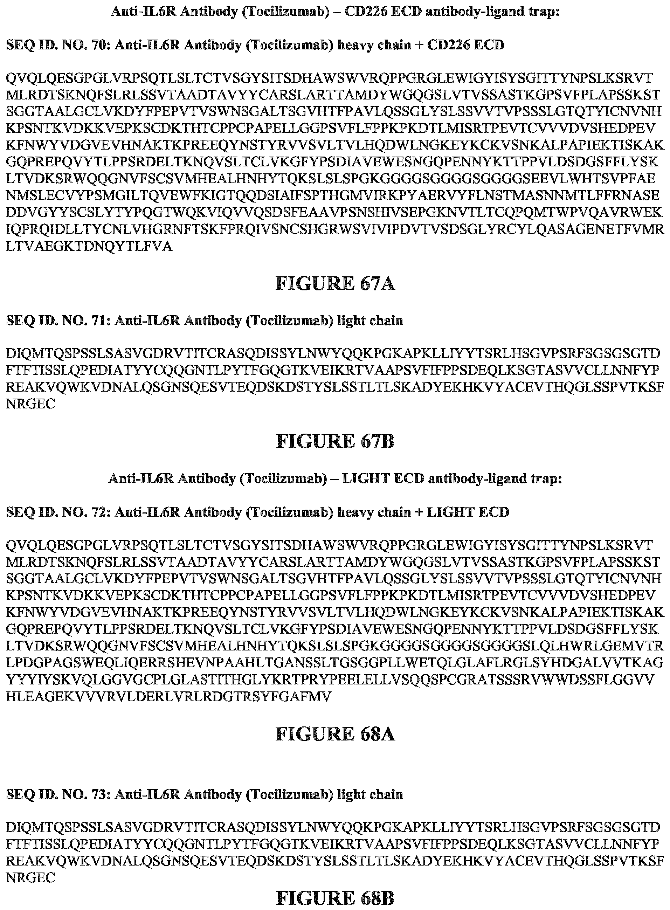

Figure 67 A-B are polypeptide sequences. A. Anti- IL6R heavy chain + CD226 ECD fusion protein sequence, SEQ ID NO: 70. B. Anti- IL6R light chain protein sequence, SEQ ID NO: 71.
Figure 68 A-B are polypeptide sequences A. Anti- IL6R heavy chain + LIGHT ECD fusion protein sequence, SEQ ID NO: 72. B. Anti- IL6R light chain protein sequence, SEQ ID NO: 73.

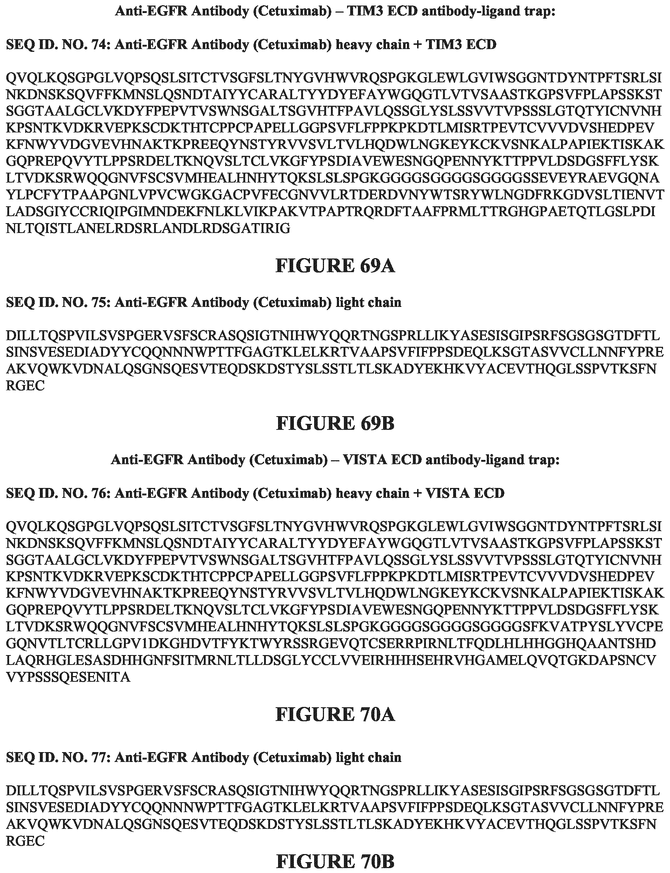

Figure 69 A-B are polypeptide sequences. A. Anti- EGFR heavy chain + TIM3 ECD fusion protein sequence, SEQ ID NO: 74. B. Anti- EGFR light chain protein sequence, SEQ ID NO: 75.
Figure 70 A-B are polypeptide sequences A. Anti- EGFR heavy chain + VISTA ECD fusion protein sequence, SEQ ID NO: 76. B. Anti- EGFR light chain protein sequence, SEQ ID NO: 77.

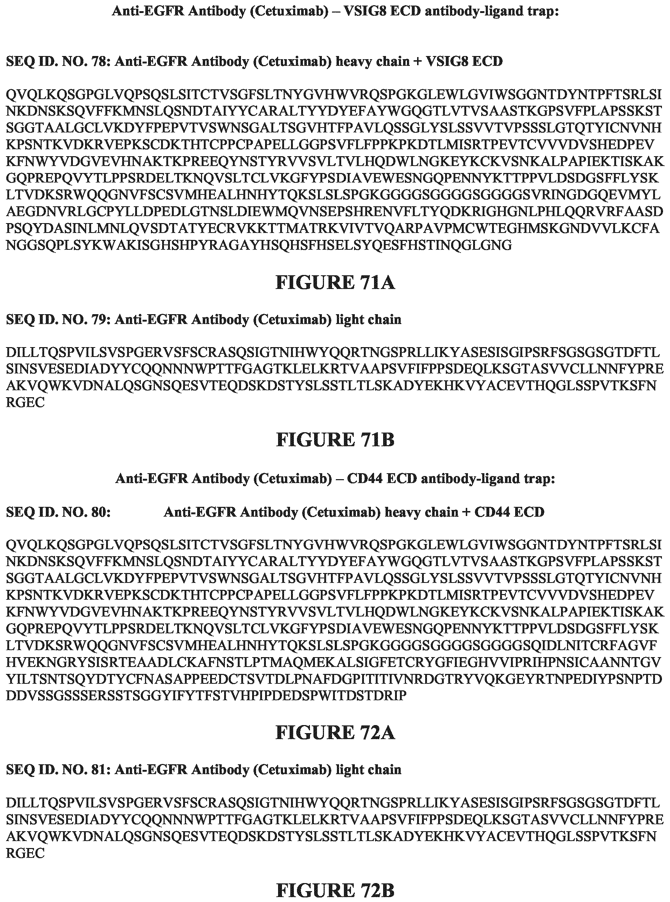

Figure 71 A-B are polypeptide sequences. A. Anti- EGFR heavy chain + VSIG8 ECD fusion protein sequence, SEQ ID NO: 78. B. Anti- EGFR light chain protein sequence, SEQ ID NO: 79.
Figure 72 A-B are polypeptide sequences. A. Anti- EGFR heavy chain + CD44 ECD fusion protein sequence, SEQ ID NO: 80. B. Anti- EGFR light chain protein sequence, SEQ ID NO: 81.

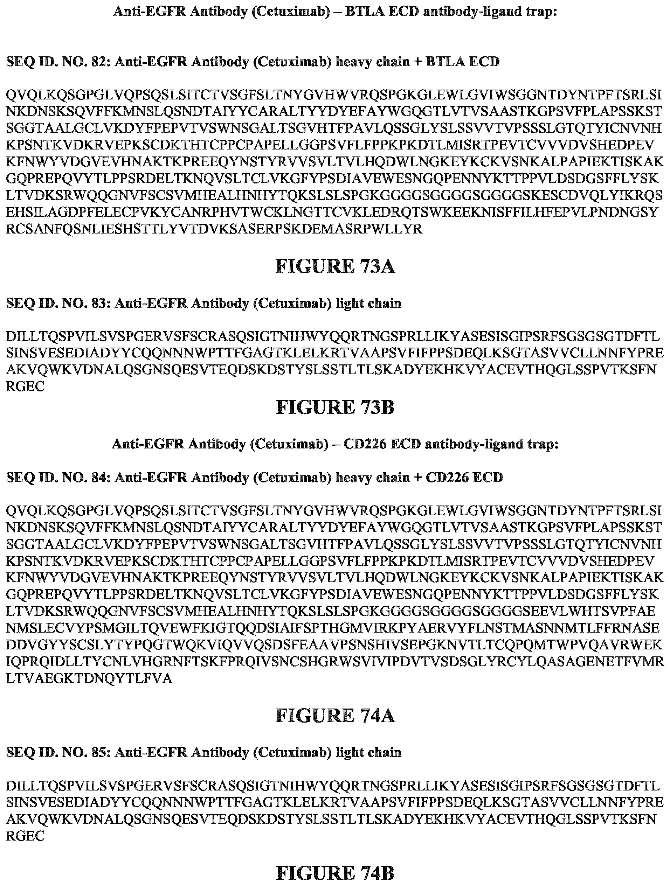

Figure 73 A-B are polypeptide sequences. A. Anti- EGFR heavy chain + BTLA ECD fusion protein sequence, SEQ ID NO: 82. B. Anti- EGFR light chain protein sequence, SEQ ID NO: 83.
Figure 74 A-B are polypeptide sequences A. Anti- EGFR heavy chain + CD226 ECD fusion protein sequence, SEQ ID NO: 84. B. Anti- EGFR light chain protein sequence, SEQ ID NO: 85.

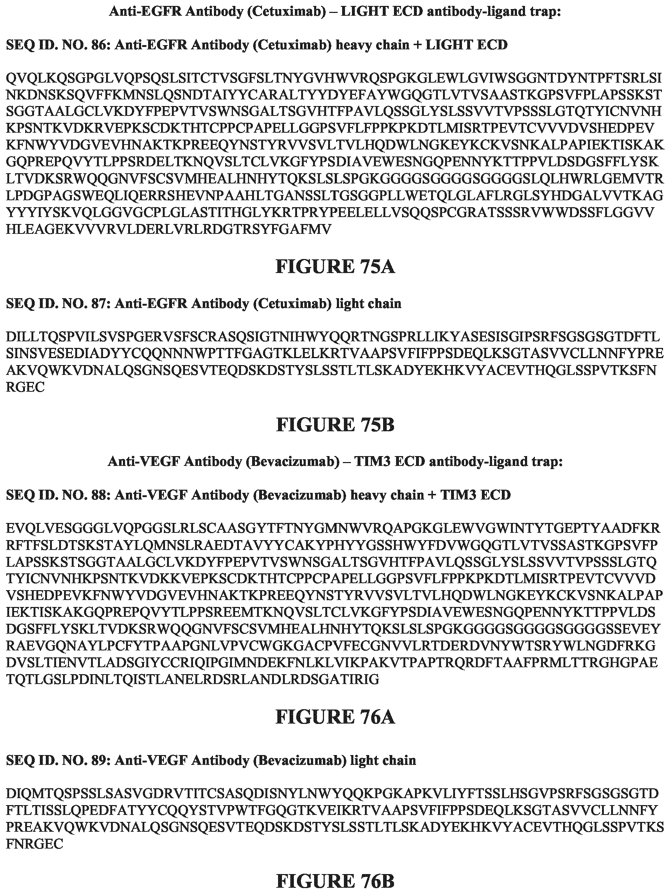

Figure 75 A-B are polypeptide sequences. A. Anti- EGFR heavy chain + LIGHT ECD fusion protein sequence, SEQ ID NO: 86. B. Anti- EGFR light chain protein sequence, SEQ ID NO: 87.
Figure 76 A-B are polypeptide sequences. A. Anti- VEGF heavy chain + TIM3 ECD fusion protein sequence, SEQ ID NO: 88. B. Anti- VEGF light chain protein sequence, SEQ ID NO: 89.

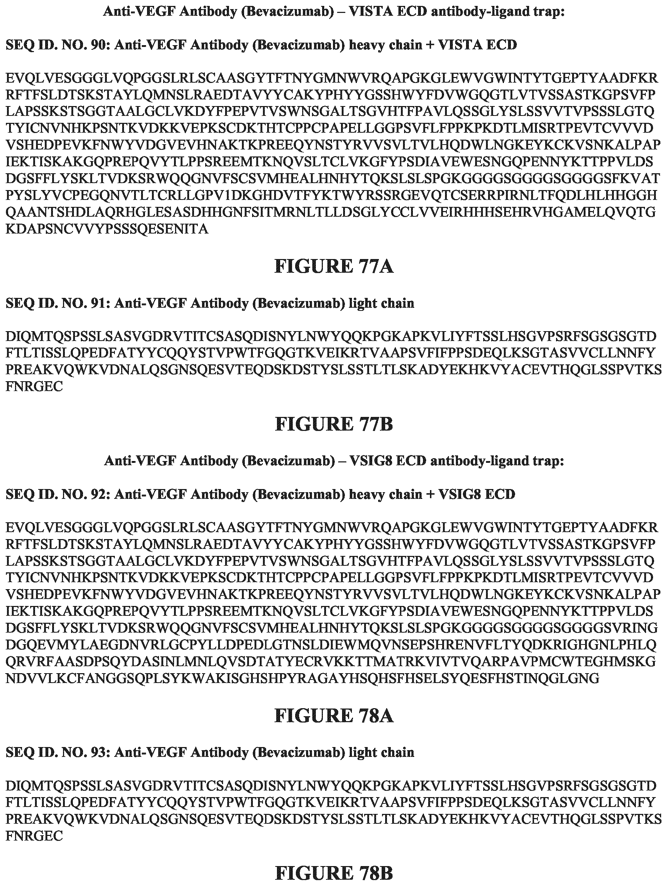

Figure 77 A-B are polypeptide sequences. A. Anti- VEGF heavy chain + VISTA ECD fusion protein sequence, SEQ ID NO: 90. B. Anti- VEGF light chain protein sequence, SEQ ID NO: 91.
Figure 78 A-B are polypeptide sequences. A. Anti- VEGF heavy chain + VSIG8 ECD fusion protein sequence, SEQ ID NO: 92. B. Anti- VEGF light chain protein sequence, SEQ ID NO: 93.

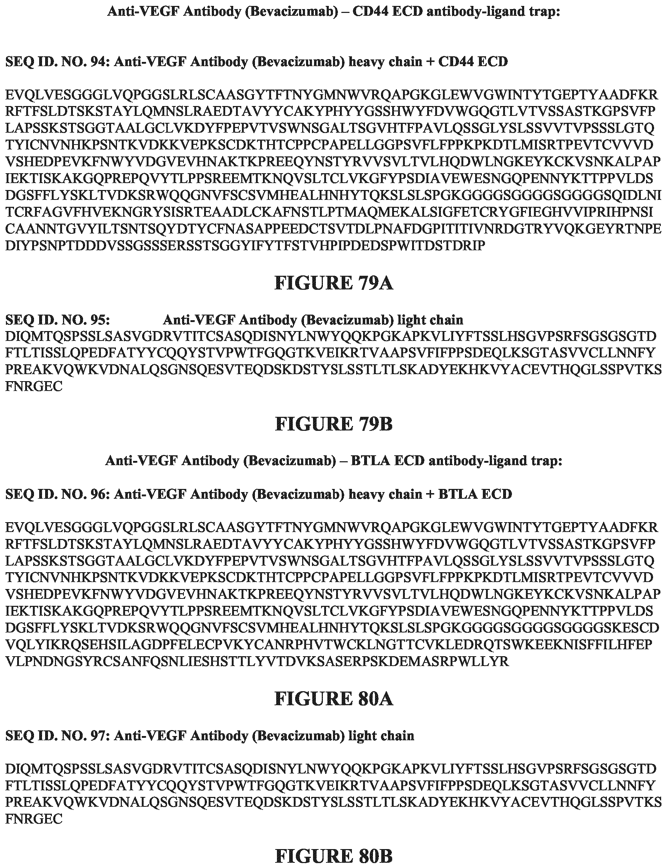

Figure 79 A-B are polypeptide sequences 5. A. Anti- VEGF heavy chain + CD44 ECD fusion protein sequence, SEQ ID NO: 94. B. Anti- VEGF light chain protein sequence, SEQ ID NO: 95.
Figure 80 A-B are polypeptide sequences A. Anti- VEGF heavy chain + BTLA ECD fusion protein sequence, SEQ ID NO: 96. B. Anti- VEGF light chain protein sequence, SEQ ID NO: 97.

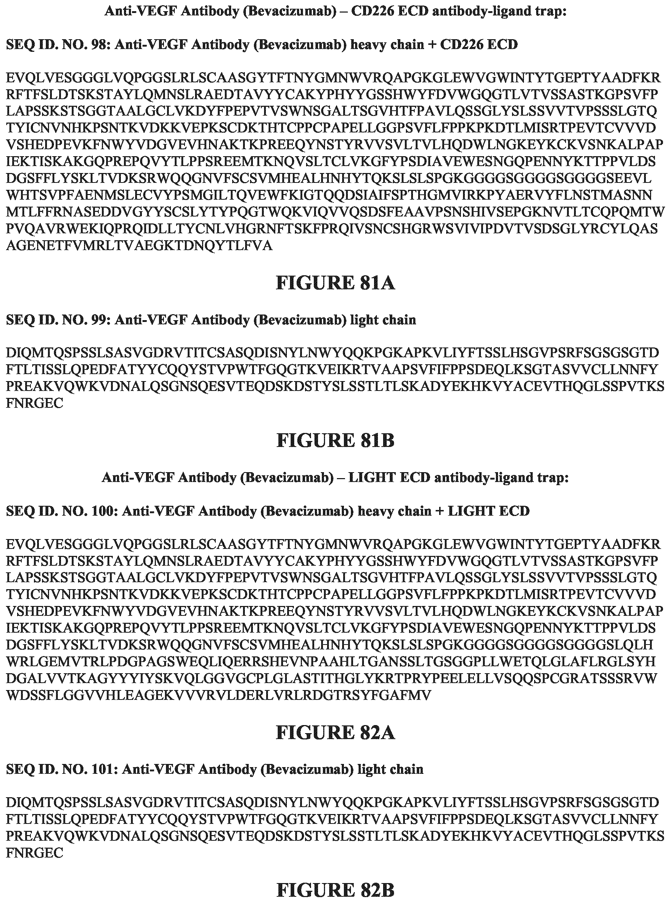

Figure 81 A-B are polypeptide sequences. A. Anti- VEGF heavy chain + CD226 ECD fusion protein sequence, SEQ ID NO: 98. B. Anti- VEGF light chain protein sequence, SEQ ID NO: 99.
Figure 82 A-B are polypeptide sequences. A. Anti- VEGF heavy chain + LIGHT ECD fusion protein sequence, SEQ ID NO: 100. B. Anti- VEGF light chain protein sequence, SEQ ID NO: 101.

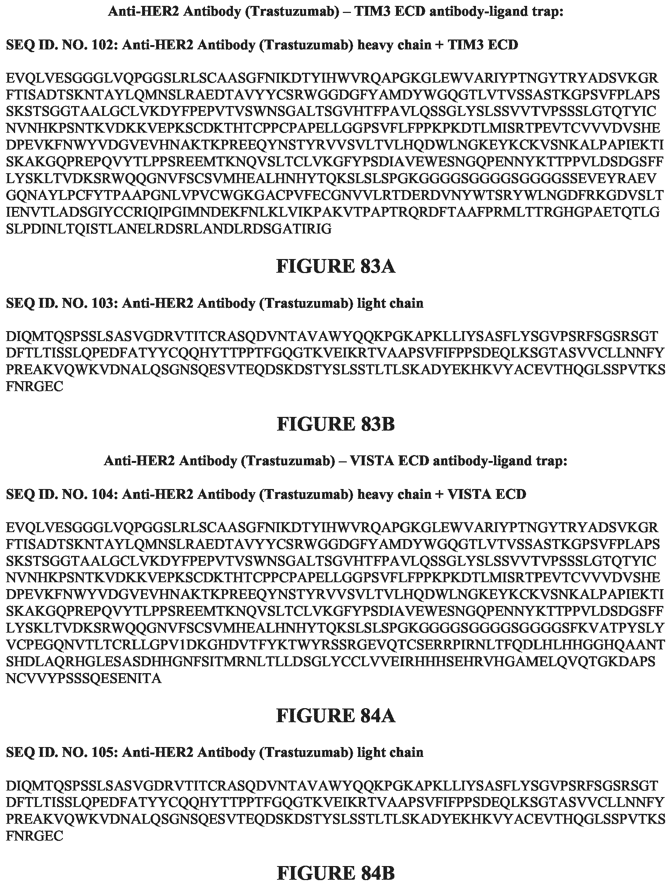

Figure 83 A-B are polypeptide sequences. A. Anti- HER2 heavy chain + TIM3 ECD fusion protein sequence, SEQ ID NO: 102. B. Anti- HER2 light chain protein sequence, SEQ ID NO: 103.
Figure 84 A-B are polypeptide sequences. A. Anti- HER2 heavy chain + VISTA ECD fusion protein sequence, SEQ ID NO: 104. B. Anti- HER2 light chain protein sequence, SEQ ID NO: 105.

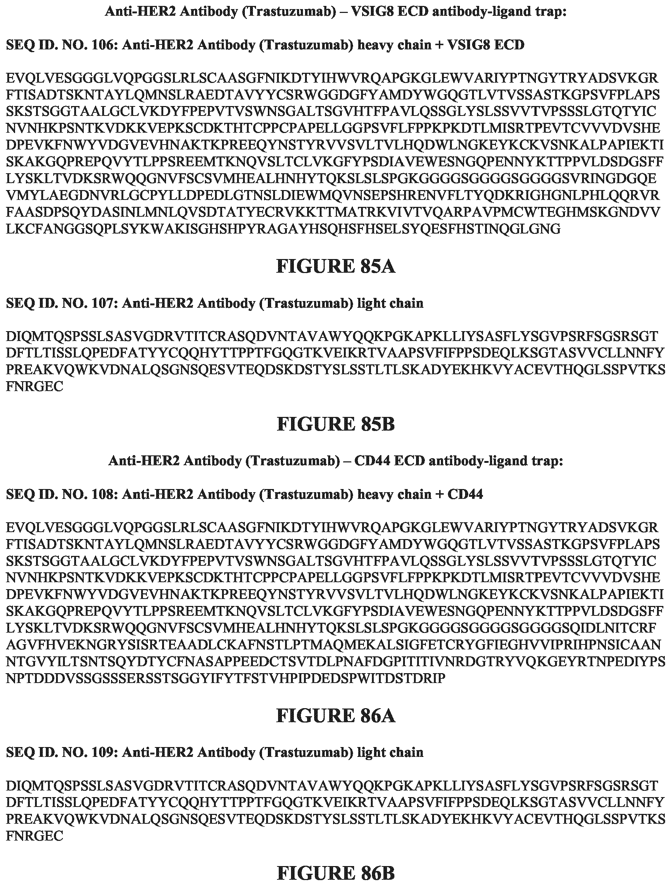

Figure 85 A-B are polypeptide sequences. A. Anti- HER2 heavy chain + VSIG8 ECD fusion protein sequence, SEQ ID NO: 106. B. Anti- HER2 light chain protein sequence, SEQ ID NO: 107.
Figure 86 A-B are polypeptide sequences. A. Anti- HER2 heavy chain + CD44 ECD fusion protein sequence, SEQ ID NO: 108. B. Anti- HER2 light chain protein sequence, SEQ ID NO: 109.

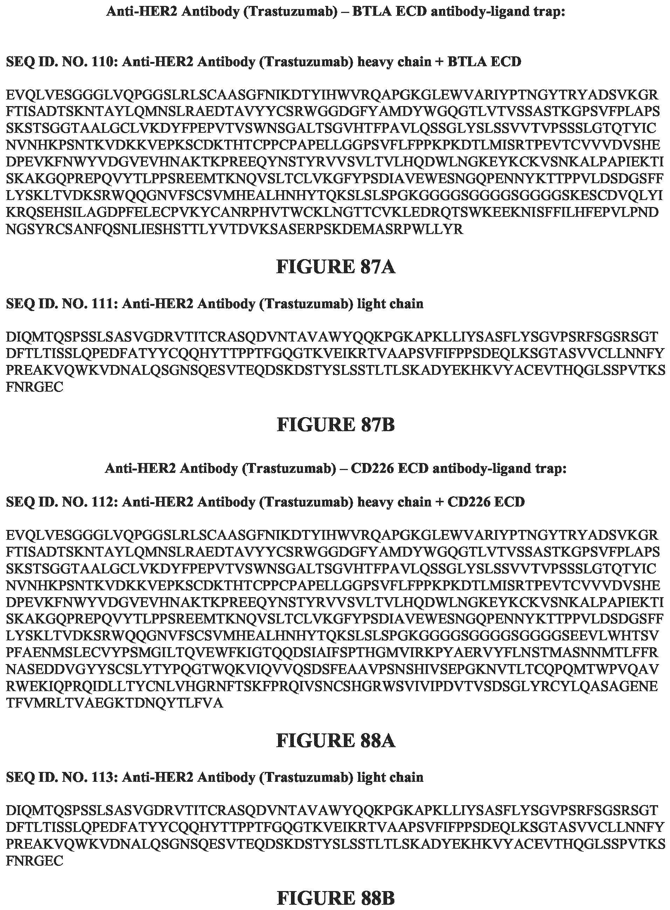

Figure 87 A-B are polypeptide sequences. A. Anti- HER2 heavy chain + BTLA ECD fusion protein sequence, SEQ ID NO: 110. B. Anti- HER2 light chain protein sequence, SEQ ID NO: 111.
Figure 88 A-B are polypeptide sequences A. Anti- HER2 heavy chain + CD226 ECD fusion protein sequence, SEQ ID NO: 112. B. Anti- HER2 light chain protein sequence, SEQ ID NO: 113.

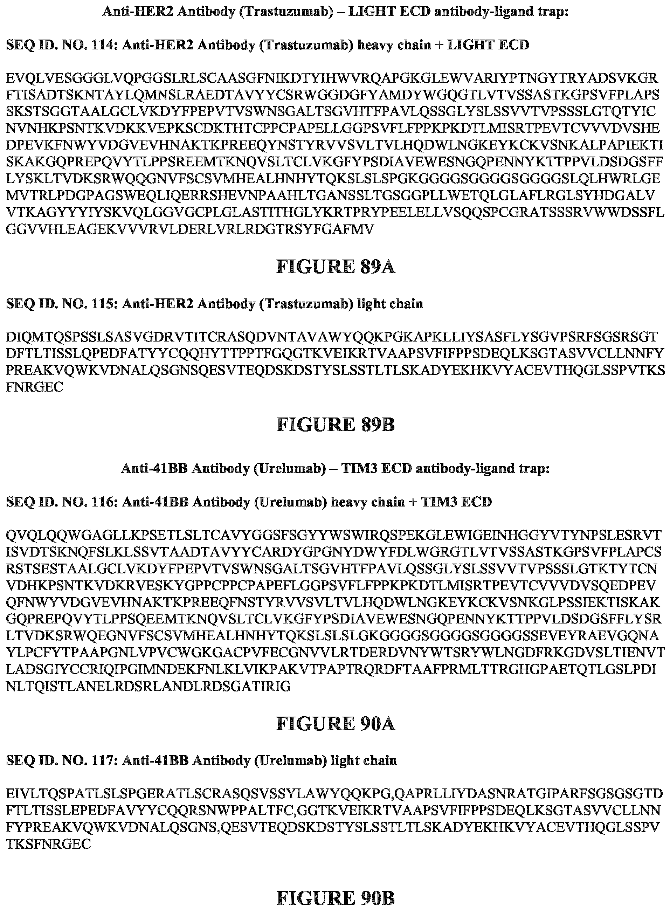

Figure 89 A-B are polypeptide sequences A. Anti- HER2 heavy chain + LIGHT ECD fusion protein sequence, SEQ ID NO: 114. B. Anti- HER2 light chain protein sequence, SEQ ID NO: 115.
Figure 90 A-B are polypeptide sequences. A. Anti- 41BB heavy chain + TIM3 ECD fusion protein sequence, SEQ ID NO: 116. B. Anti- 41BB light chain protein sequence, SEQ ID NO: 117.

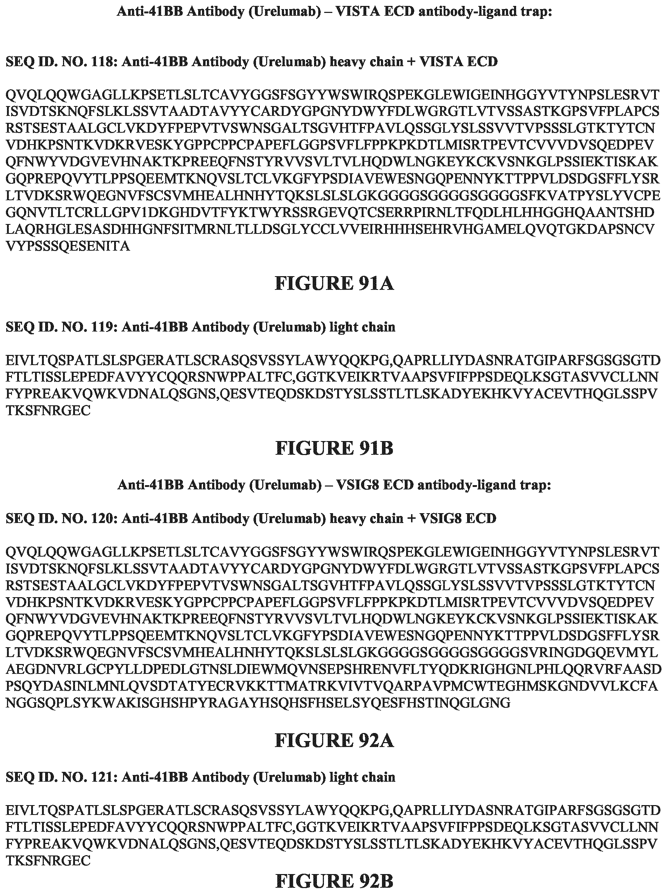

Figure 91 A-B are polypeptide sequences. A. Anti- 41BB heavy chain + VISTA ECD fusion protein sequence, SEQ ID NO: 118. B. Anti- 41BB light chain protein sequence, SEQ ID NO: 119.
Figure 92 A-B are polypeptide sequences. A. Anti- 41BB heavy chain + VSIG8 ECD fusion protein sequence, SEQ ID NO: 120. B. Anti- 41BB light chain protein sequence, SEQ ID NO: 121.

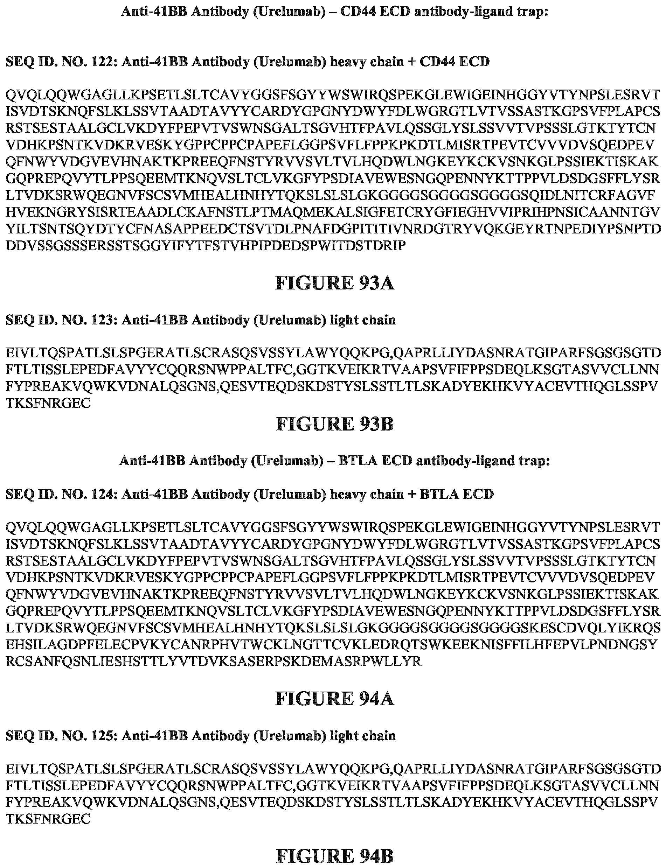

Figure 93 A-B are polypeptide sequences. A. Anti- 41BB heavy chain + CD44 ECD fusion protein sequence, SEQ ID NO: 122. B. Anti- 41BB light chain protein sequence, SEQ ID NO: 123.
Figure 94 A-B are polypeptide sequences 41BB light chain protein sequence, SEQ ID NO: 125.

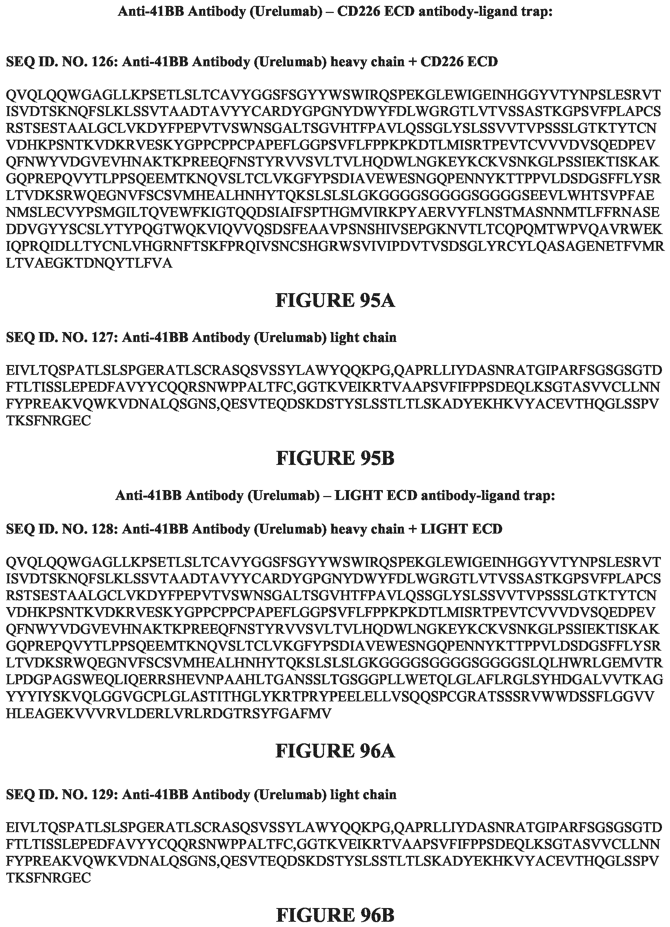

Figure 95 A-B are polypeptide sequences. A. Anti- 41BB heavy chain + CD226 ECD fusion protein sequence, SEQ ID NO: 126. B. Anti- 41BB light chain protein sequence, SEQ ID NO: 127.
Figure 96 A-B are polypeptide sequences. A. Anti- 41BB heavy chain + LIGHT ECD fusion protein sequence, SEQ ID NO: 128. B. Anti- 41BB light chain protein sequence, SEQ ID NO: 129.

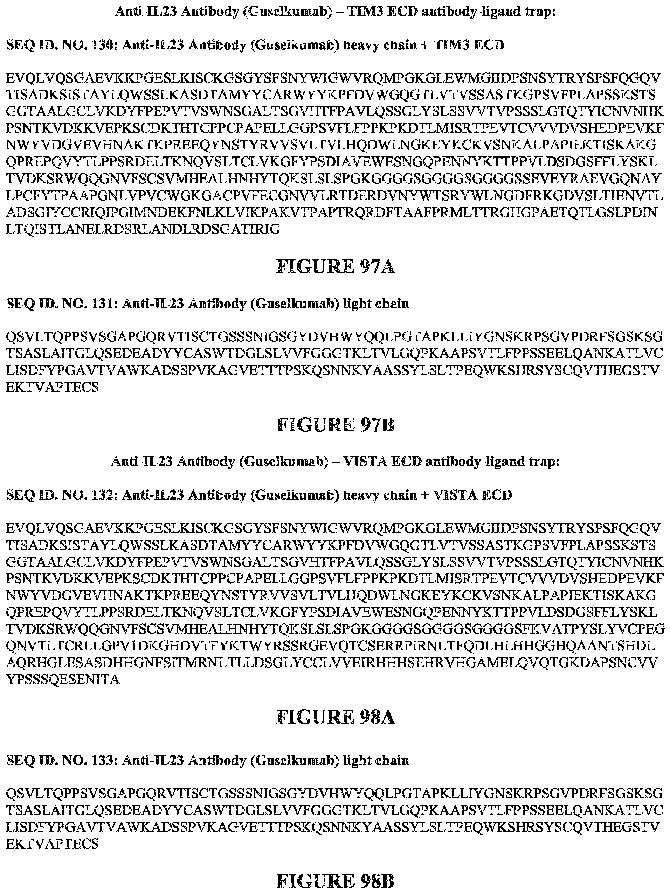

Figure 97 A-B are polypeptide sequences. A. Anti- IL23 heavy chain + TIM3 ECD fusion protein sequence, SEQ ID NO: 130. B. Anti- IL23 light chain protein sequence, SEQ ID NO: 131.
Figure 98 A-B are polypeptide sequences. A. Anti- IL23 heavy chain + VISTA ECD fusion protein sequence, SEQ ID NO: 132. B. Anti- IL23 light chain protein sequence, SEQ ID NO: 133.

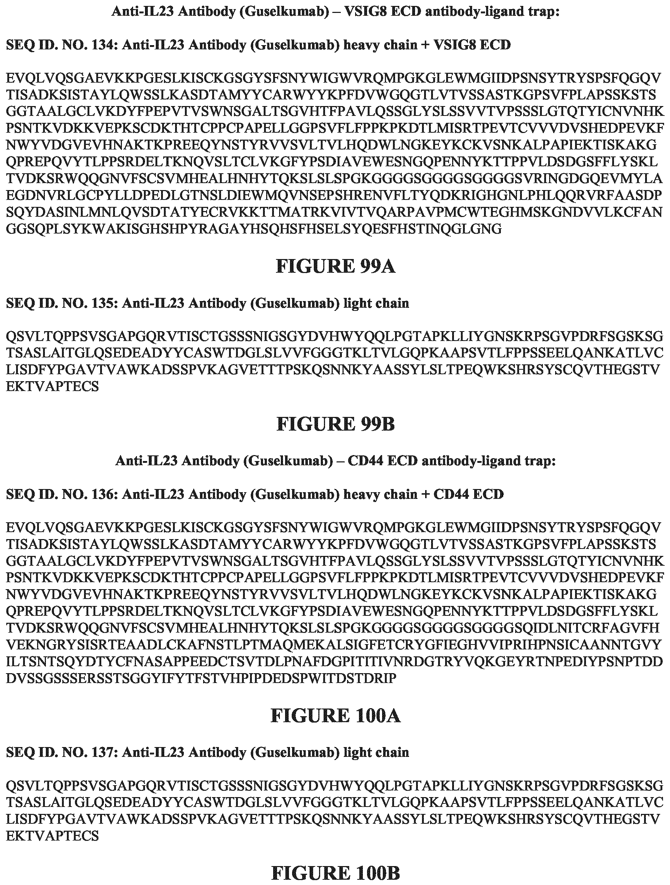

Figure 99 A-B are polypeptide sequences. A. Anti- IL23 heavy chain + VSIG8 ECD fusion protein sequence, SEQ ID NO: 134. B. Anti- IL23 light chain protein sequence, SEQ ID NO: 135.
Figure 100 A-B are polypeptide sequences. A. Anti- IL23 heavy chain + CD44 ECD fusion protein sequence, SEQ ID NO: 136. B. Anti- IL23 light chain protein sequence, SEQ ID NO: 137.

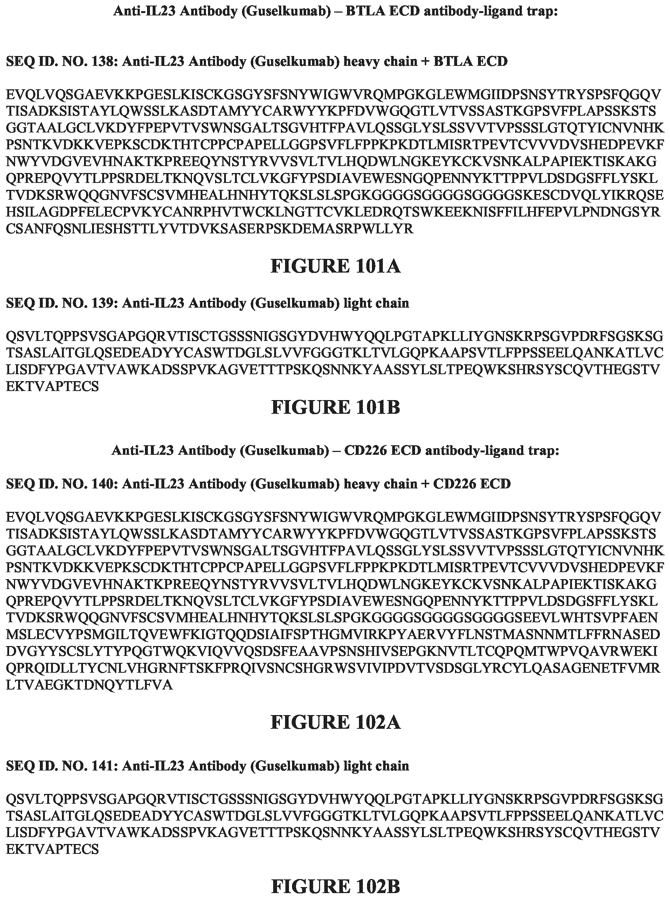

Figure 101 A-B are polypeptide sequences. A. Anti- IL23 heavy chain + BTLA ECD fusion protein sequence, SEQ ID NO: 138. B. Anti- IL23 light chain protein sequence, SEQ ID NO: 139.
Figure 102 A-B are polypeptide sequences. A. Anti- IL23 heavy chain + CD226 ECD fusion protein sequence, SEQ ID NO: 140. B. Anti- IL23 light chain protein sequence, SEQ ID NO: 141.

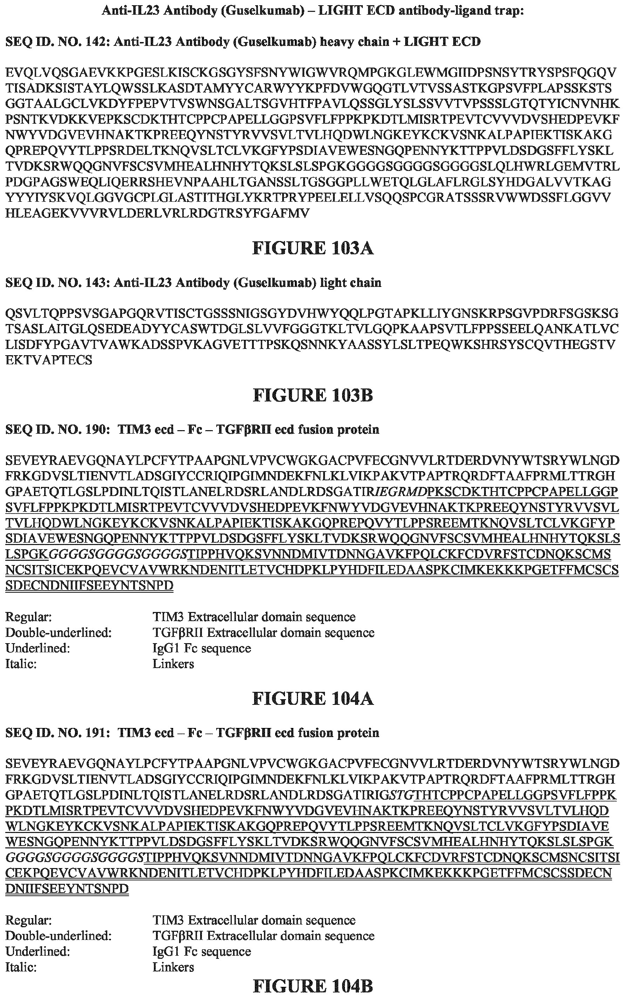

Figure 103 A-B are polypeptide sequences. A. Anti- IL23 heavy chain + LIGHT ECD fusion protein sequence, SEQ ID NO: 142. B. Anti- IL23 light chain protein sequence, SEQ ID NO: 143.
Figure 104 A-B are polypeptide sequences. TIM3 ecd - Fc - TGFβRII ecd fusion protein sequence, SEQ ID NO: 190. B. TIM3 ecd - Fc - TGFβRII ecd fusion protein sequence, SEQ ID NO: 191.

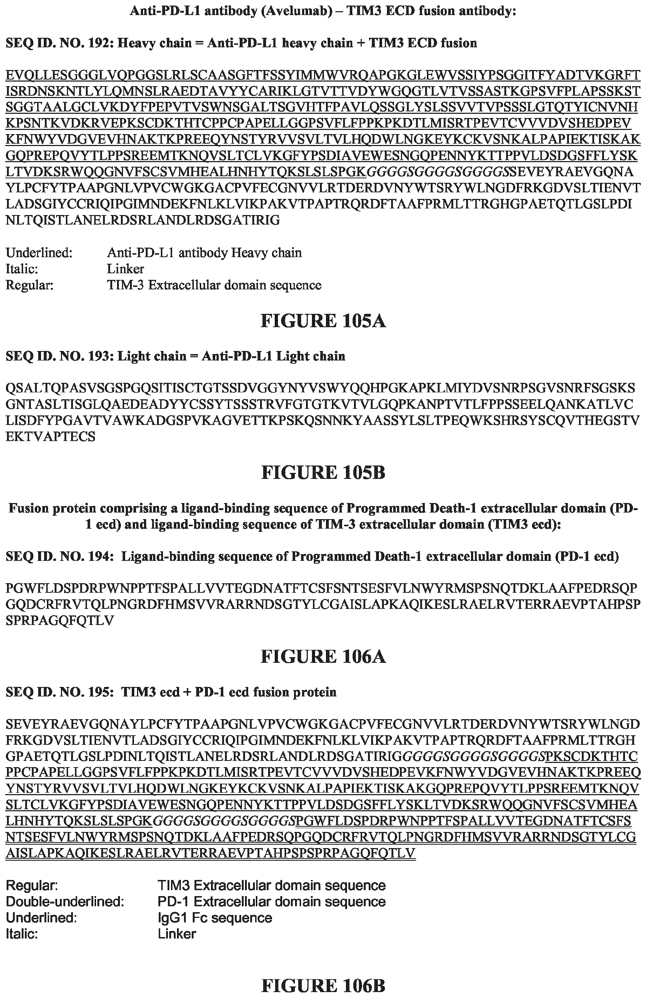

Figure 105 A-B are polypeptide sequences. A. Anti-PD-L1 heavy chain + TIM3 ECD fusion protein sequence, SEQ ID NO: 192. B. Anti-PD-L1 Light chain protein sequence, SEQ ID NO: 193.

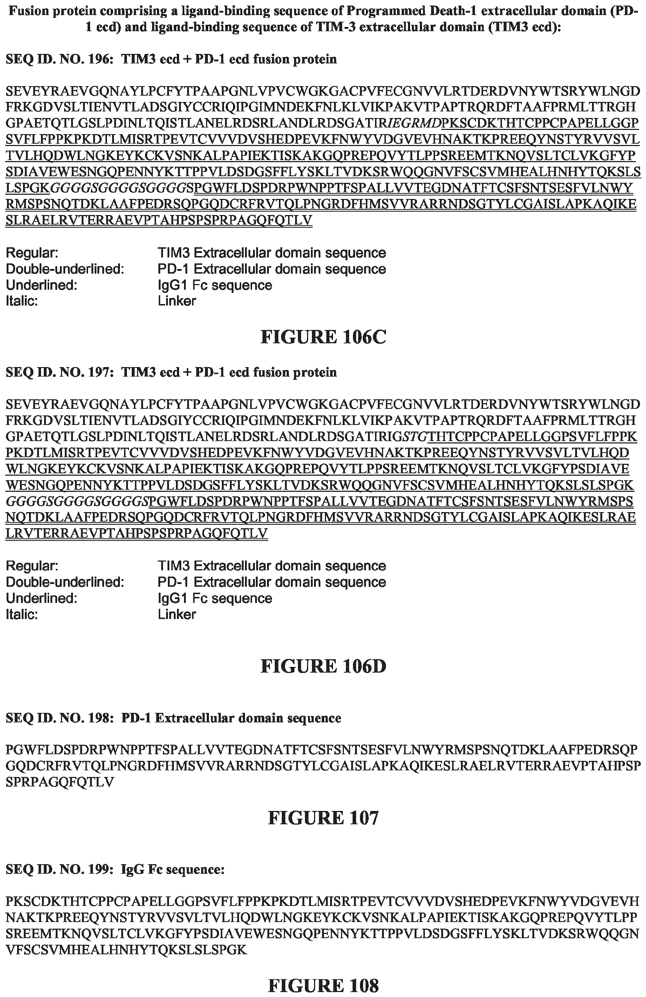

Figure 106 A-D are polypeptide sequences. A. Ligand-binding sequence of PD-1 ecd protein sequence, SEQ ID NO: 194. B. TIM3 ecd + PD-1 ecd fusion protein sequence, SEQ ID NO: 195. C TIM3 ecd + PD-1 ecd fusion protein sequence, SEQ ID NO: 196. D. TIM3 ecd + PD-1 ecd fusion protein sequence, SEQ ID NO: 197.
Figure 107 is PD-1 Extracellular domain protein sequence, SEQ ID NO: 198.
Figure 108 is IgG Fc protein sequence, SEQ ID NO: 199.

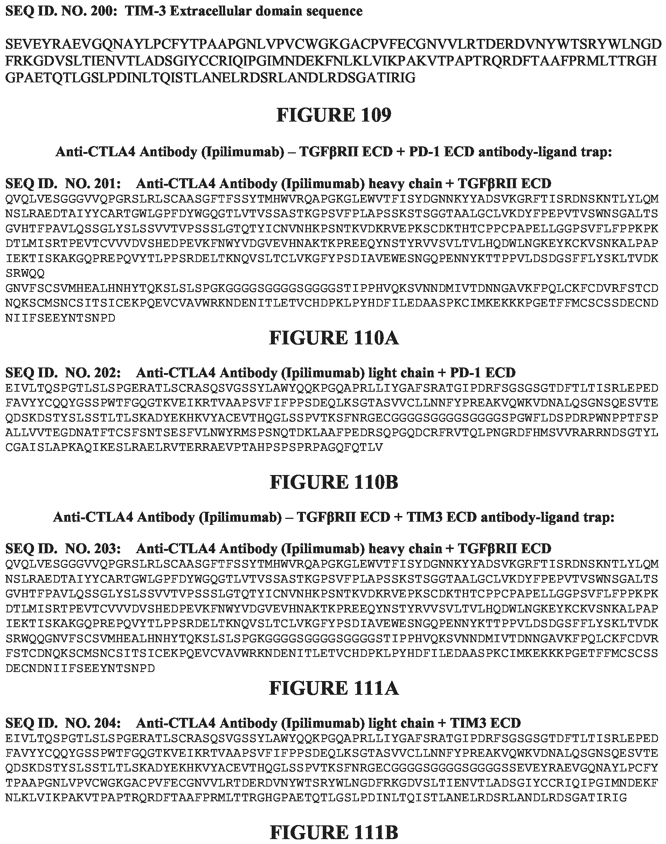

Figure 109 is TIM-3 Extracellular domain protein sequence, SEQ ID NO: 200.
Figures 110 A-B are polypeptide sequences. A. SEQ ID NO: 201: Anti-CTLA4 Antibody (Ipilimumab) heavy chain + TGFbRII ECD. B. SEQ ID NO: 202: Anti-CTLA4 Antibody (Ipilimumab) light chain + PD-1 ECD.
Figures 111 A-B are polypeptide sequences. A. SEQ ID NO: 203: Anti-CTLA4 Antibody (Ipilimumab) heavy chain + TGFbRII ECD. B. SEQ ID NO: 204: Anti-CTLA4 Antibody (Ipilimumab) light chain + TIM3 ECD.

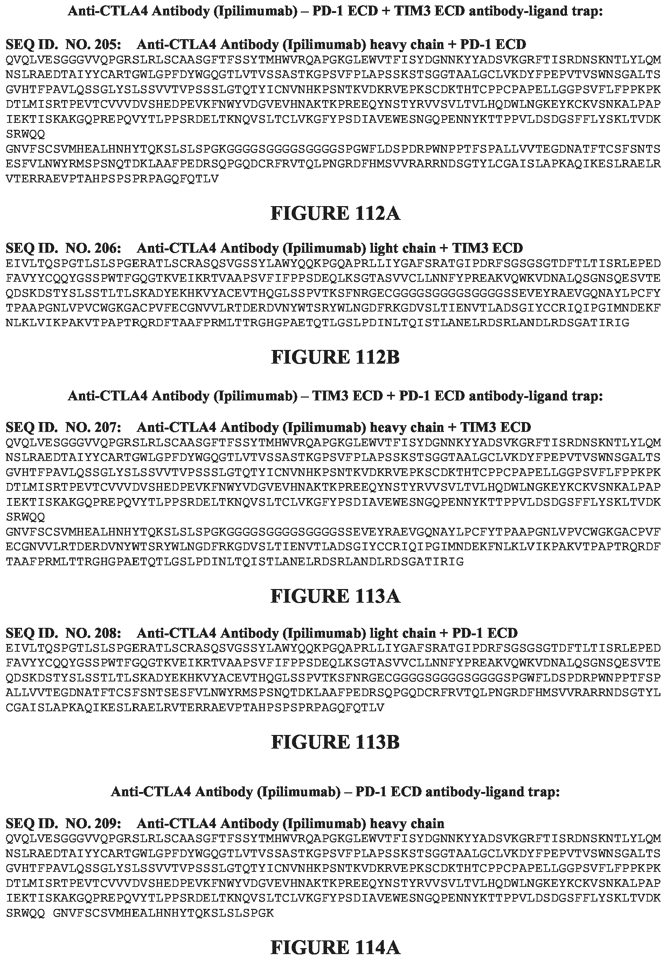

Figures 112 A-B are polypeptide sequences. A. SEQ ID NO: 205: Anti-CTLA4 Antibody (Ipilimumab) heavy chain + PD-1 ECD. B. SEQ ID NO: 206: Anti-CTLA4 Antibody (Ipilimumab) light chain + TIM3 ECD.
Figures 113 A-B are polypeptide sequences. A. SEQ ID NO: 207: Anti-CTLA4 Antibody (Ipilimumab) heavy chain + TIM3 ECD. B. SEQ ID NO: 208: Anti-CTLA4 Antibody (Ipilimumab) light chain + PD-1 ECD.

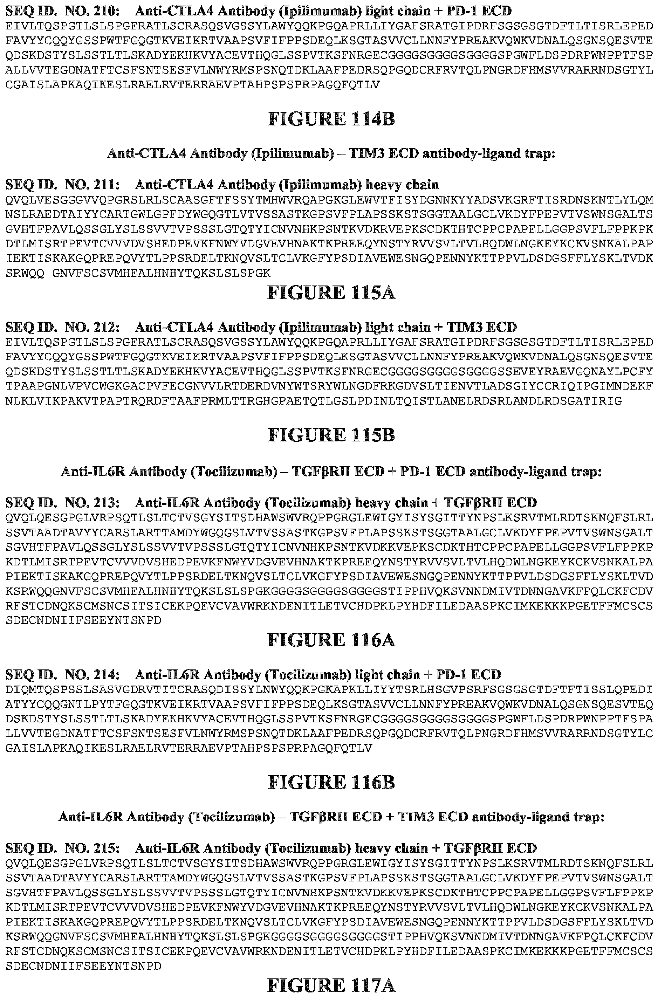

Figures 114 A-B are polypeptide sequences. A. SEQ ID NO: 209: Anti-CTLA4 Antibody (Ipilimumab) heavy chain. B. SEQ ID NO: 210: Anti-CTLA4 Antibody (Ipilimumab) light chain + PD-1 ECD.
Figures 115 A-B are polypeptide sequences. A. SEQ ID NO: 211: Anti-CTLA4 Antibody (Ipilimumab) heavy chain. B. SEQ ID NO: 212: Anti-CTLA4 Antibody (Ipilimumab) light chain + TIM3 ECD.
Figures 116 A-B are polypeptide sequences. A. SEQ ID NO: 213: Anti-IL6R Antibody (Tocilizumab) heavy chain + TGFbRII ECD. B. SEQ ID NO: 214: Anti-IL6R Antibody (Tocilizumab) light chain + PD-1 ECD.

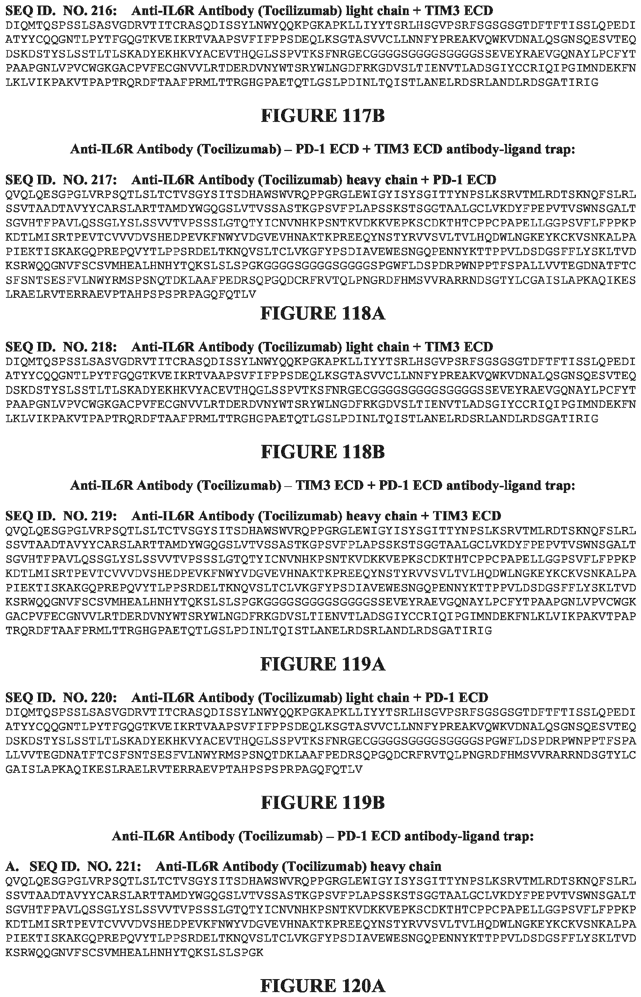

Figures 117 A-B are polypeptide sequences. A. SEQ ID NO: 215: Anti-IL6R Antibody (Tocilizumab) heavy chain + TGFbRII ECD. B. SEQ ID NO: 216: Anti-IL6R Antibody (Tocilizumab) light chain + TIM3 ECD.
Figures 118 A-B are polypeptide sequences. A. SEQ ID NO: 217: Anti-IL6R Antibody (Tocilizumab) heavy chain + PD-1 ECD.B. SEQ ID NO: 218: Anti-IL6R Antibody (Tocilizumab) light chain + TIM3 ECD.
Figures 119 A-B are polypeptide sequences. A. SEQ ID NO: 219: Anti-IL6R Antibody (Tocilizumab) heavy chain + TIM3 ECD. B. SEQ ID NO: 220: Anti-IL6R Antibody (Tocilizumab) light chain + PD-1 ECD.

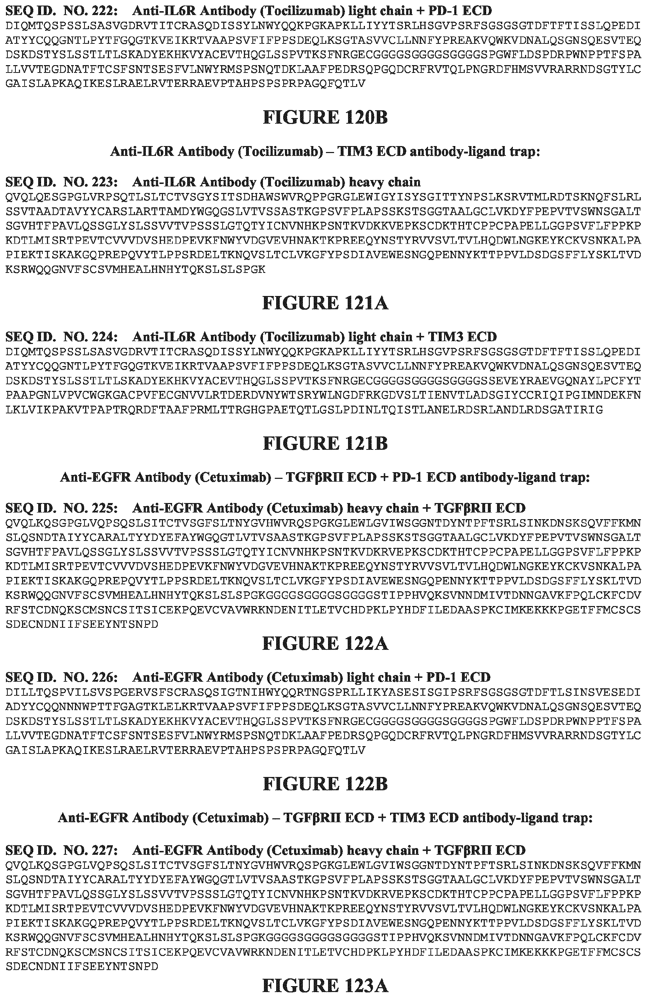

Figures 120 A-B are polypeptide sequences. A. SEQ ID NO: 221: Anti-IL6R Antibody (Tocilizumab) heavy chain. B. SEQ ID NO: 222: Anti-IL6R Antibody (Tocilizumab) light chain + PD-1 ECD.
Figures 121 A-B are polypeptide sequences. A. SEQ ID NO: 223: Anti-IL6R Antibody (Tocilizumab) heavy chain. B. SEQ ID NO: 224: Anti-IL6R Antibody (Tocilizumab) light chain + TIM3 ECD.
Figures 122 A-B are polypeptide sequences. A. SEQ ID NO: 225: Anti-EGFR Antibody (Cetuximab) heavy chain + TGFbRII ECD. B. SEQ ID NO: 226: Anti-EGFR Antibody (Cetuximab) light chain + PD-1 ECD.

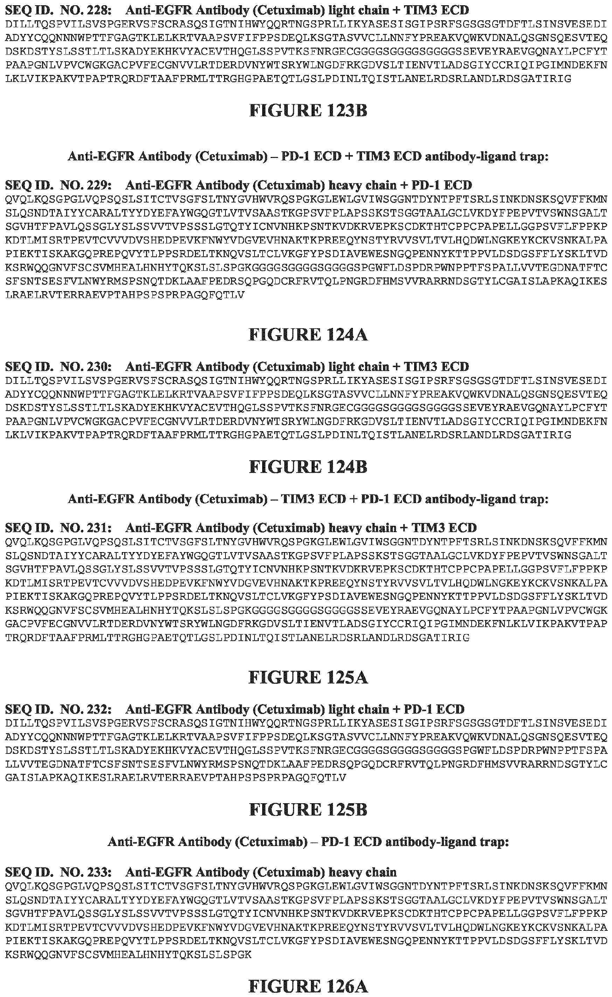

Figures 123 A-B are polypeptide sequences. A. SEQ ID NO: 227: Anti-EGFR Antibody (Cetuximab) heavy chain + TGFbRII ECD. B. SEQ ID NO: 228: Anti-EGFR Antibody (Cetuximab) light chain + TIM3 ECD.
Figures 124 A-B are polypeptide sequences. A. SEQ ID NO: 229: Anti-EGFR Antibody (Cetuximab) heavy chain + PD-1 ECD. B. SEQ ID NO: 230: Anti-EGFR Antibody (Cetuximab) light chain + TIM3 ECD.
Figures 125 A-B are polypeptide sequences. A. SEQ ID NO: 231: Anti-EGFR Antibody (Cetuximab) heavy chain + TIM3 ECD. B. SEQ ID NO: 232: Anti-EGFR Antibody (Cetuximab) light chain + PD-1 ECD.

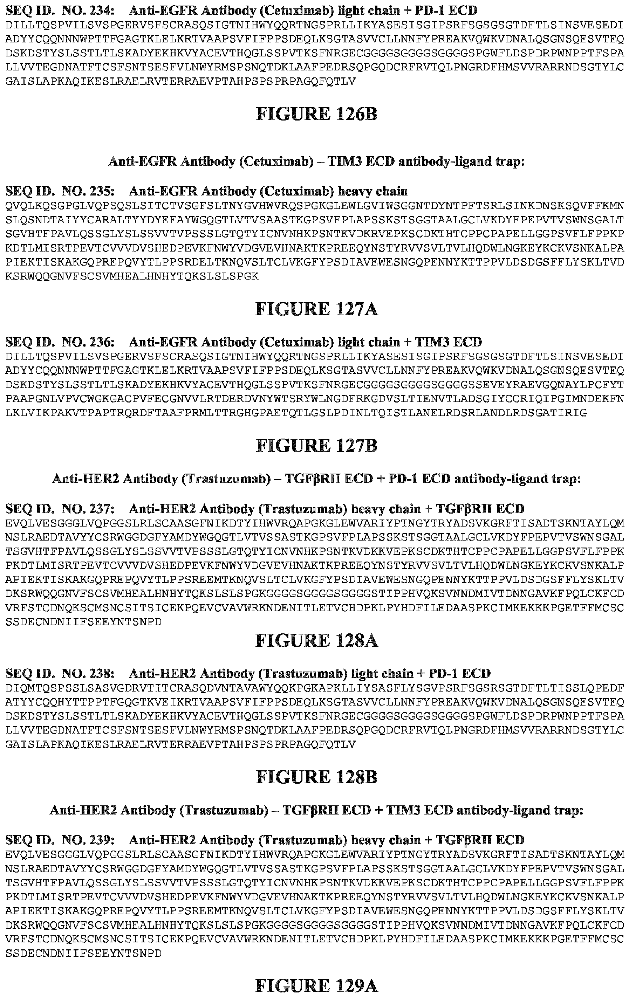

Figures 126 A-B are polypeptide sequences. A. SEQ ID NO: 233: Anti-EGFR Antibody (Cetuximab) heavy chain. B. SEQ ID NO: 234: Anti-EGFR Antibody (Cetuximab) light chain + PD-1 ECD.
Figures 127 A-B are polypeptide sequences. A. SEQ ID NO: 235: Anti-EGFR Antibody (Cetuximab) heavy chain. B. SEQ ID NO: 236: Anti-EGFR Antibody (Cetuximab) light chain + TIM3 ECD.
Figures 128 A-B are polypeptide sequences. A. SEQ ID NO: 237: Anti-HER2 Antibody (Trastuzumab) heavy chain + TGFbRII ECD. B. SEQ ID NO: 238: Anti-HER2 Antibody (Trastuzumab) light chain + PD-1 ECD.

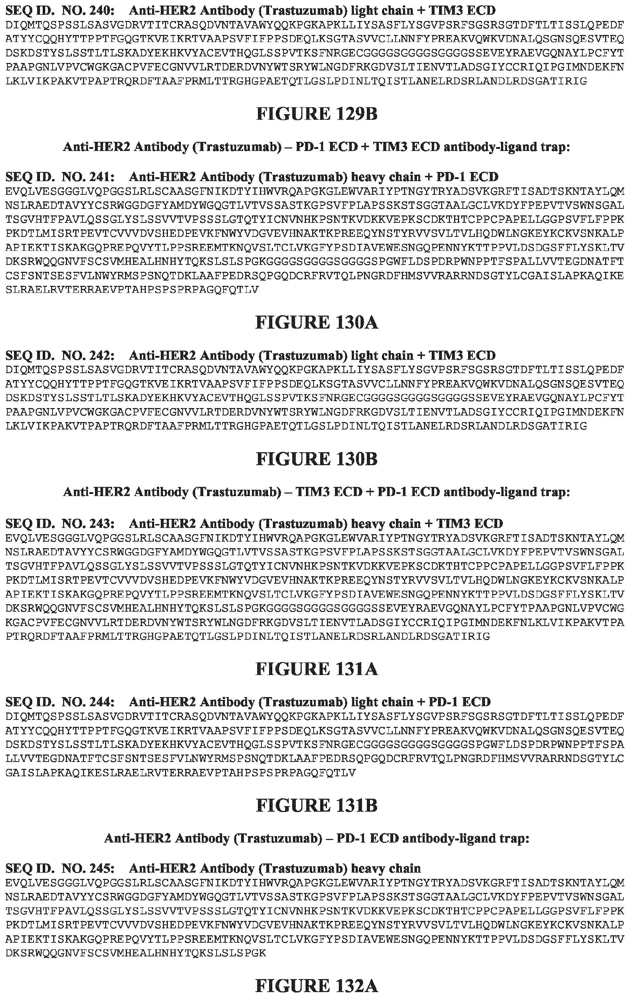

Figures 129 A-B are polypeptide sequences. A. SEQ ID NO: 239: Anti-HER2 Antibody (Trastuzumab) heavy chain + TGFbRII ECD. B. SEQ ID NO: 240: Anti-HER2 Antibody (Trastuzumab) light chain + TIM3 ECD.
Figures 130 A-B are polypeptide sequences. A. SEQ ID NO: 241: Anti-HER2 Antibody (Trastuzumab) heavy chain + PD-1 ECD. B. SEQ ID NO: 242: Anti-HER2 Antibody (Trastuzumab) light chain + TIM3 ECD.
Figures 131 A-B are polypeptide sequences. A. SEQ ID NO: 243: Anti-HER2 Antibody (Trastuzumab) heavy chain + TIM3 ECD. B. SEQ ID NO: 244: Anti-HER2 Antibody (Trastuzumab) light chain + PD-1 ECD.

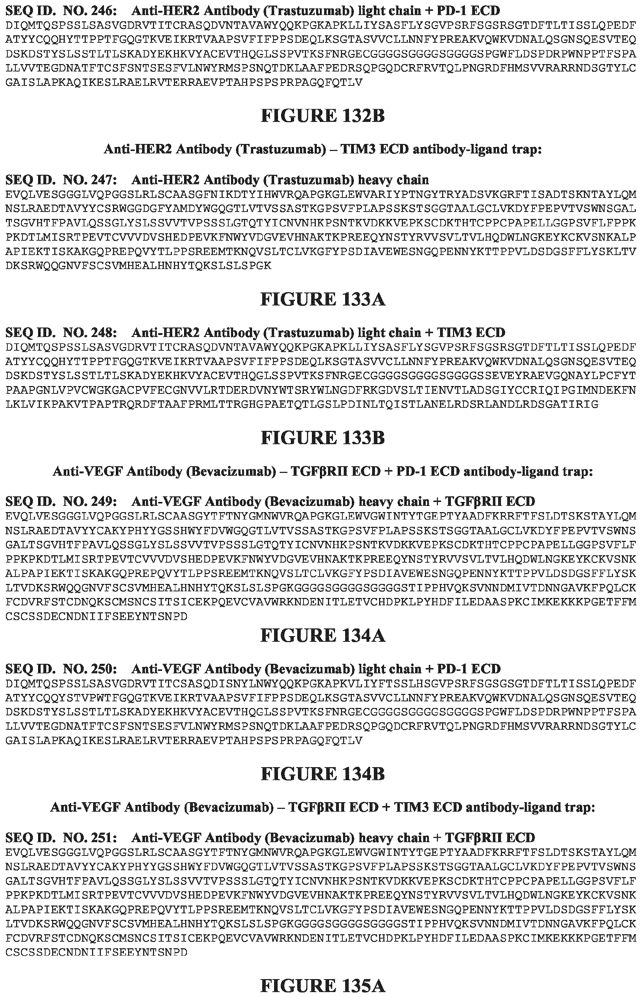

Figures 132 A-B are polypeptide sequences. A. SEQ ID NO: 245: Anti-HER2 Antibody (Trastuzumab) heavy chain. B. SEQ ID NO: 246: Anti-HER2 Antibody (Trastuzumab) light chain + PD-1 ECD.
Figures 133 A-B are polypeptide sequences. A. SEQ ID NO: 247: Anti-HER2 Antibody (Trastuzumab) heavy chain. B. SEQ ID NO: 248: Anti-HER2 Antibody (Trastuzumab) light chain + TIM3 ECD.
Figures 134 A-B are polypeptide sequences. A. SEQ ID NO: 249: Anti-VEGF Antibody (Bevacizumab) heavy chain + TGFbRII ECD. B. SEQ ID NO: 250: Anti-VEGF Antibody (Bevacizumab) light chain + PD-1 ECD.

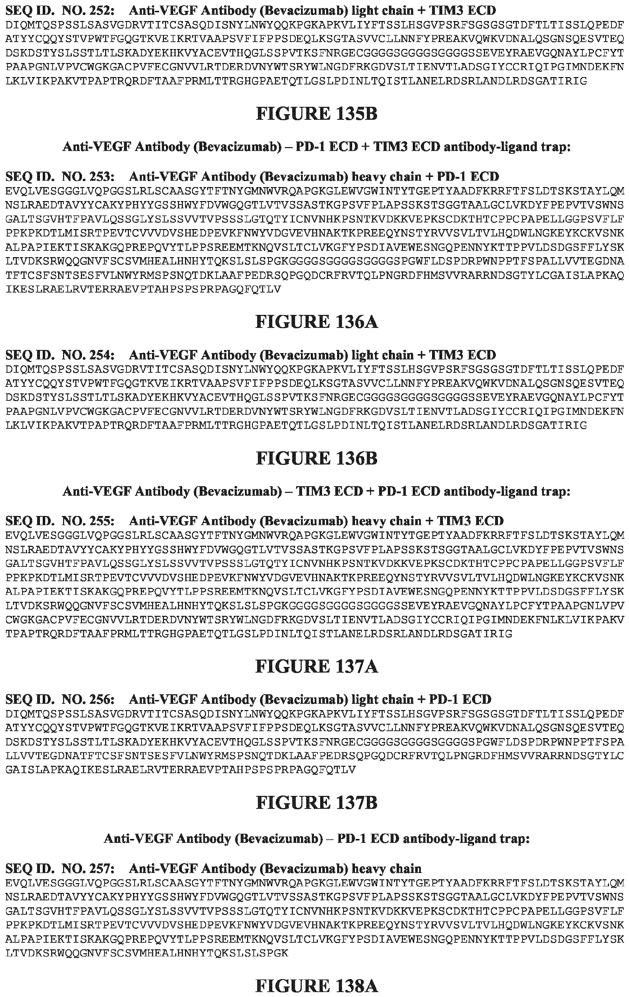

Figures 135 A-B are polypeptide sequences. A. SEQ ID NO: 251: Anti-VEGF Antibody (Bevacizumab) heavy chain + TGFbRII ECD. B. SEQ ID NO: 252: Anti-VEGF Antibody (Bevacizumab) light chain + TIM3 ECD.
Figures 136 A-B are polypeptide sequences. A. SEQ ID NO: 253: Anti-VEGF Antibody (Bevacizumab) heavy chain + PD-1 ECD. B. SEQ ID NO: 254: Anti-VEGF Antibody (Bevacizumab) light chain + TIM3 ECD.
Figures 137 A-B are polypeptide sequences. A. SEQ ID NO: 255: Anti-VEGF Antibody (Bevacizumab) heavy chain + TIM3 ECD. B. SEQ ID NO: 256: Anti-VEGF Antibody (Bevacizumab) light chain + PD-1 ECD.

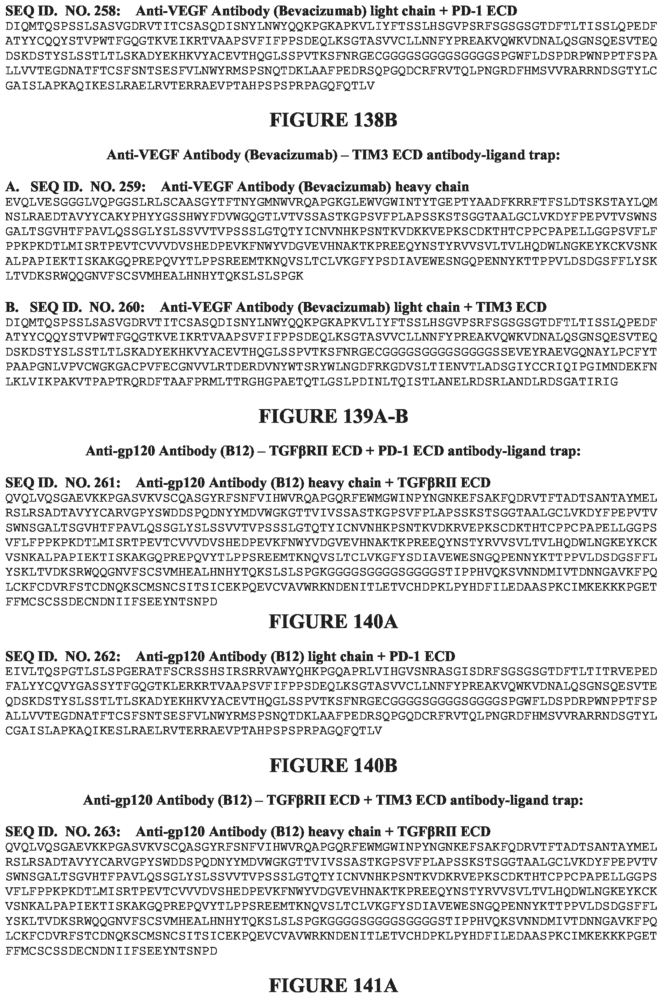

Figures 138 A-B are polypeptide sequences. A. SEQ ID NO: 257: Anti-VEGF Antibody (Bevacizumab) heavy chain. B. SEQ ID NO: 258: Anti-VEGF Antibody (Bevacizumab) light chain + PD-1 ECD.
Figures 139 A-B are polypeptide sequences. A. SEQ ID NO: 259: Anti-VEGF Antibody (Bevacizumab) heavy chain. B. SEQ ID NO: 260: Anti-VEGF Antibody (Bevacizumab) light chain + TIM3 ECD.
Figures 140 A-B are polypeptide sequences. A. SEQ ID NO: 261: Anti-gp120 Antibody (B12) heavy chain + TGFbRII ECD. B. SEQ ID NO: 262: Anti-gp120 Antibody (B12) light chain + PD-1 ECD.

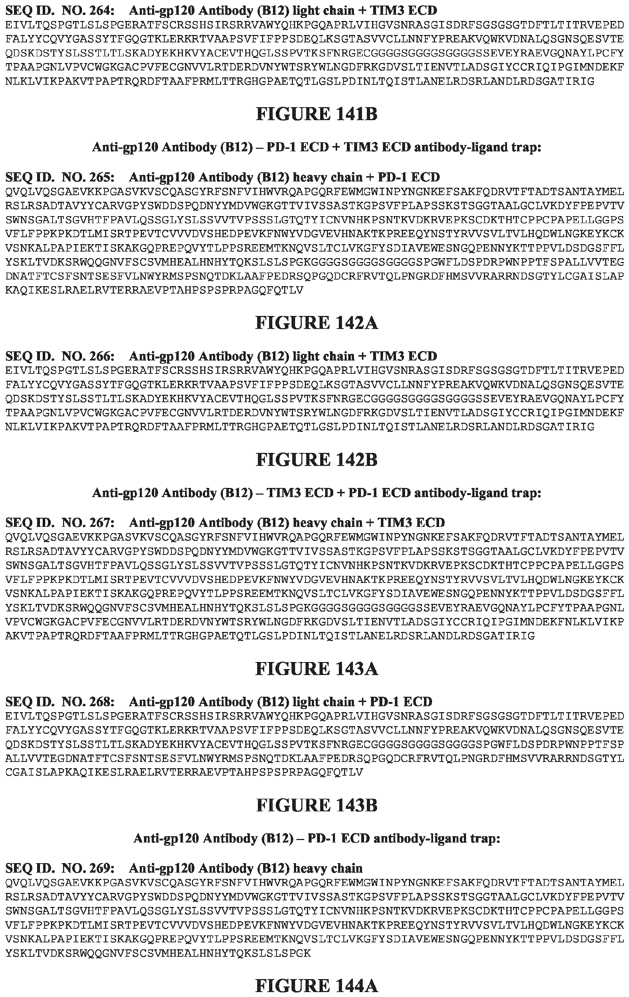

Figures 141 A-B are polypeptide sequences. A. SEQ ID NO: 263: Anti-gp120 Antibody (B12) heavy chain + TGFbRII ECD. B. SEQ ID NO: 264: Anti-gp120 Antibody (B12) light chain + TIM3 ECD.
Figures 142 A-B are polypeptide sequences. A. SEQ ID NO: 265: Anti-gp120 Antibody (B12) heavy chain + PD-1 ECD. B. SEQ ID NO: 266: Anti-gp120 Antibody (B12) light chain + TIM3 ECD.
Figures 143 A-B are polypeptide sequences. A. SEQ ID NO: 267: Anti-gp120 Antibody (B12) heavy chain + TIM3 ECD. B. SEQ ID NO: 268: Anti-gp120 Antibody (B12) light chain + PD-1 ECD.

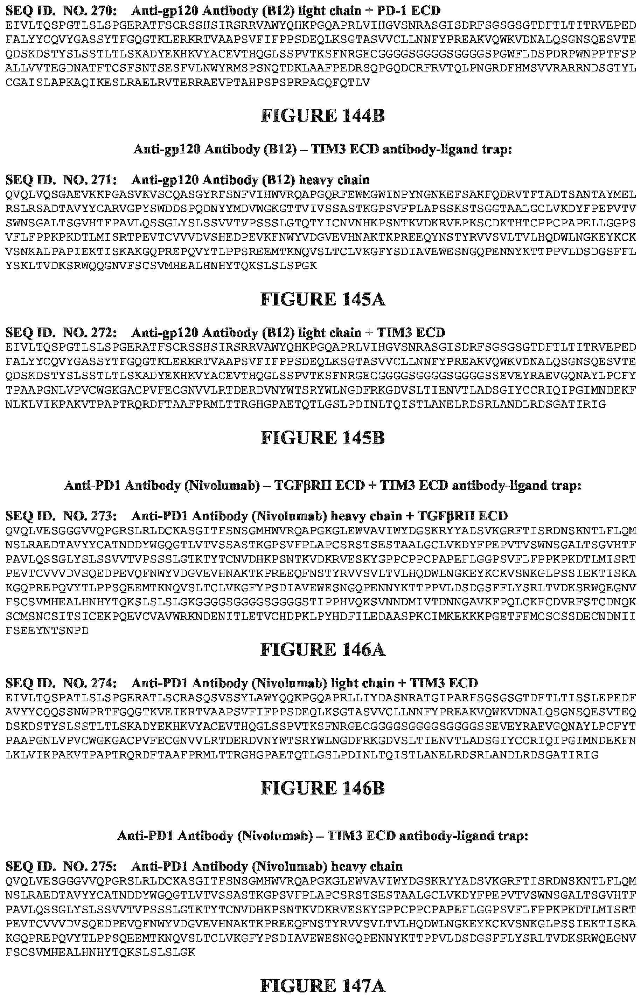

Figures 144 A-B are polypeptide sequences. A. SEQ ID NO: 269: Anti-gp120 Antibody (B12) heavy chain. B. SEQ ID NO: 270: Anti-gp120 Antibody (B12) light chain + PD-1 ECD.
Figures 145 A-B are polypeptide sequences. A. SEQ ID NO: 271: Anti-gp120 Antibody (B12) heavy chain. B. SEQ ID NO: 272: Anti-gp120 Antibody (B12) light chain + TIM3 ECD.
Figures 146 A-B are polypeptide sequences. A. SEQ ID NO: 273: Anti-PD1 Antibody (Nivolumab) heavy chain + TGFbRII ECD. B. SEQ ID NO: 274: Anti-PD1 Antibody (Nivolumab) light chain + TIM3 ECD.

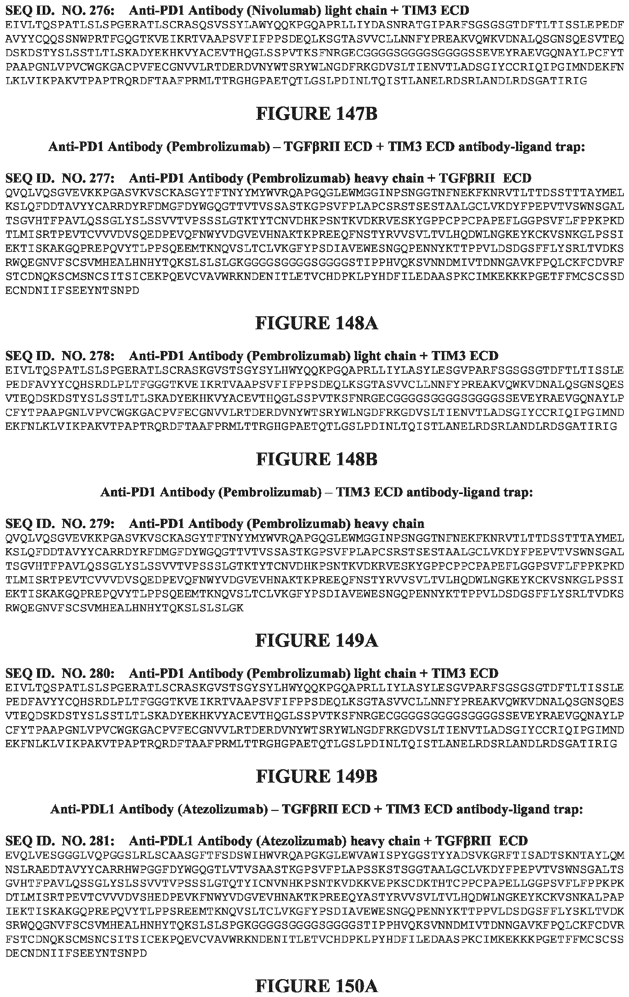

Figures 147 A-B are polypeptide sequences. A. SEQ ID NO: 275: Anti-PD1 Antibody (Nivolumab) heavy chain. B. SEQ ID NO: 276: Anti-PD1 Antibody (Nivolumab) light chain + TIM3 ECD.
Figures 148 A-B are polypeptide sequences. A. SEQ ID NO: 277: Anti-PD1 Antibody (Pembrolizumab) heavy chain + TGFbRII ECD. B. SEQ ID NO: 278: Anti-PD1 Antibody (Pembrolizumab) light chain + TIM3 ECD.
Figures 149 A-B are polypeptide sequences. A. SEQ ID NO: 279: Anti-PD1 Antibody (Pembrolizumab) heavy chain. B. SEQ ID NO: 280: Anti-PD1 Antibody (Pembrolizumab) light chain + TIM3 ECD.

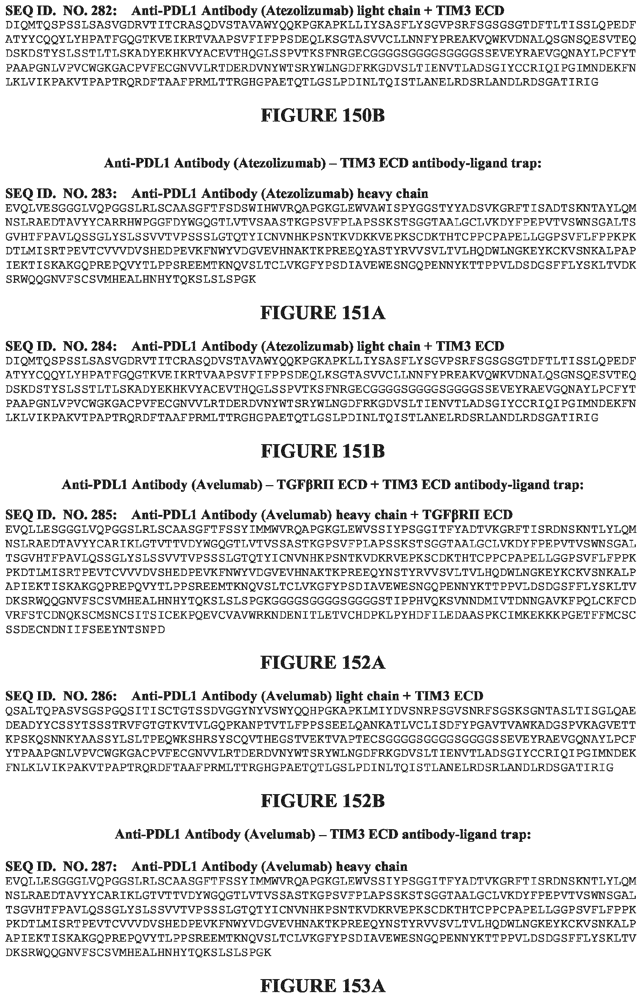

Figures 150 A-B are polypeptide sequences. A. SEQ ID NO: 281: Anti-PDL1 Antibody (Atezolizumab) heavy chain + TGFbRII ECD. B. SEQ ID NO: 282: Anti-PDL1 Antibody (Atezolizumab) light chain + TIM3 ECD.
Figures 151 A-B are polypeptide sequences. A. SEQ ID NO: 283: Anti-PDL1 Antibody (Atezolizumab) heavy chain. B. SEQ ID NO: 284: Anti-PDL1 Antibody (Atezolizumab) light chain + TIM3 ECD.
Figures 152 A-B are polypeptide sequences. A. SEQ ID NO: 285: Anti-PDL1 Antibody (Avelumab) heavy chain + TGFbRII ECD. B. SEQ ID NO: 286: Anti-PDL1 Antibody (Avelumab) light chain + TIM3 ECD.

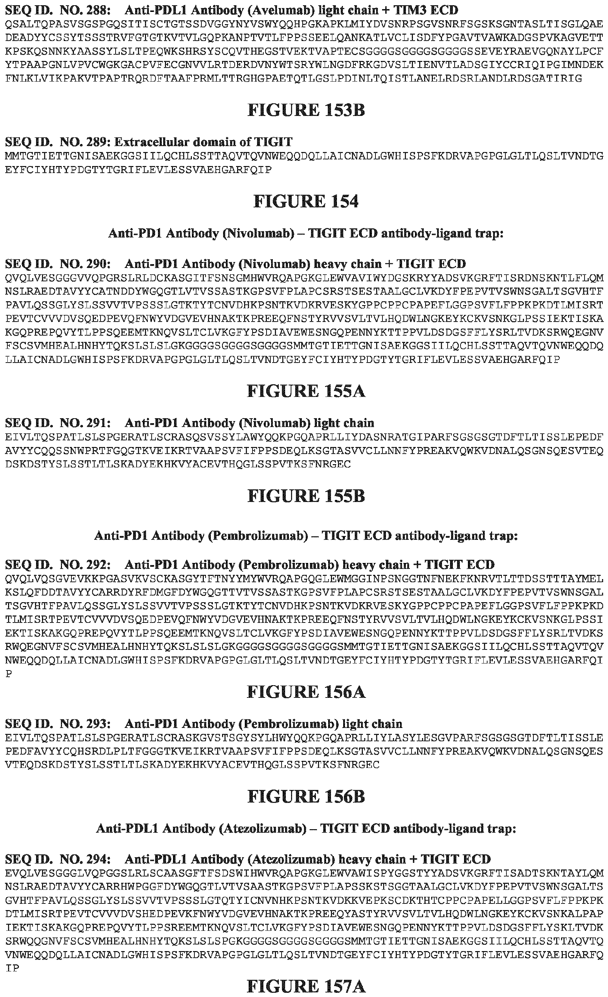

Figures 153 A-B are polypeptide sequences. A. SEQ ID NO: 287: Anti-PDL1 Antibody (Avelumab) heavy chain. B. SEQ ID NO: 288: Anti-PDL1 Antibody (Avelumab) light chain + TIM3 ECD.
Figure 154 is the polypeptide sequence of the extracellular domain of TIGIT, SEQ ID NO: 289.
Figures 155 A-B are polypeptide sequences. A. SEQ ID NO: 290: Anti-PD1 Antibody (Nivolumab) heavy chain + TIGIT ECD. B. SEQ ID NO: 291: Anti-PD1 Antibody (Nivolumab) light chain.
Figures 156 A-B are polypeptide sequences. A. SEQ ID NO: 292: Anti-PD1 Antibody (Pembrolizumab) heavy chain + TIGIT ECD. B. SEQ ID NO: 293: Anti-PD1 Antibody (Pembrolizumab) light chain.

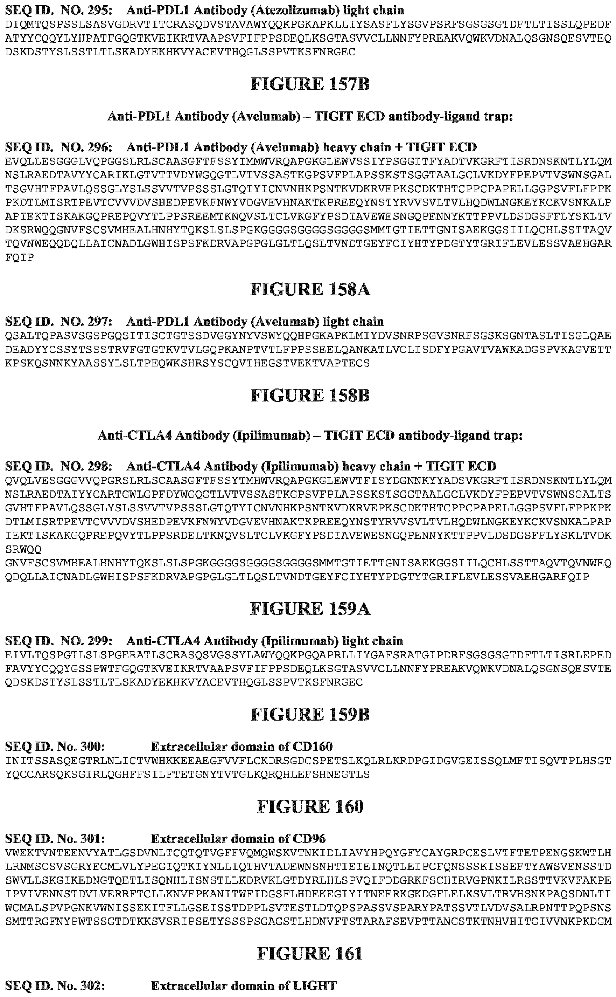

Figures 157 A-B are polypeptide sequences. A. SEQ ID NO: 294: Anti-PDL1 Antibody (Atezolizumab) heavy chain + TIGIT ECD. B. SEQ ID NO: 295: Anti-PDL1 Antibody (Atezolizumab) light chain.
Figures 158 A-B are polypeptide sequences. A. SEQ ID NO: 296: Anti-PDL1 Antibody (Avelumab) heavy chain + TIGIT ECD. B. SEQ ID NO: 297: Anti-PDL1 Antibody (Avelumab) light chain.
Figures 159 A-B are polypeptide sequences. A. SEQ ID NO: 298: Anti-CTLA4 Antibody (Ipilimumab) heavy chain + TIGIT ECD. B. SEQ ID NO: 299: Anti-CTLA4 Antibody (Ipilimumab) light chain.
Figure 160 is the polypeptide sequence of the extracellular domain of CD160, SEQ ID NO: 300.
Figure 161 is the polypeptide sequence of the extracellular domain of CD96, SEQ ID NO: 301.

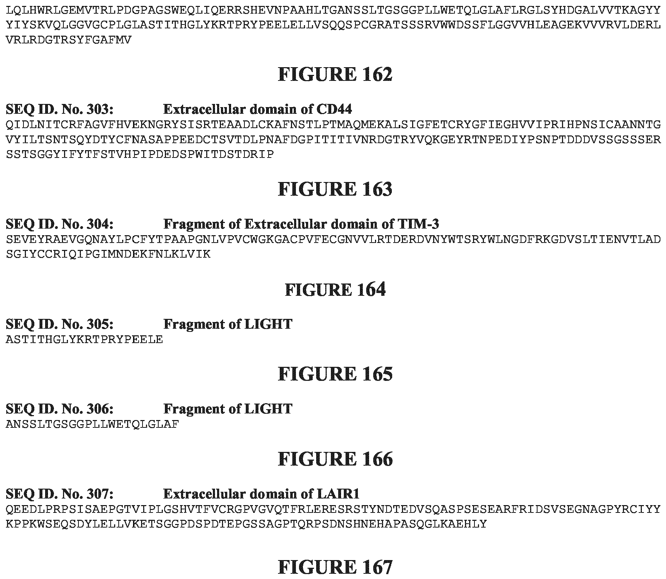

Figure 162 is the polypeptide sequence of the extracellular domain of LIGHT, SEQ ID NO: 302.
Figure 163 is the polypeptide sequence of the extracellular domain of CD44, SEQ ID NO: 303.
Figure 164 is the polypeptide sequence of a fragment of the extracellular domain of TIM-3, SEQ ID NO: 304.
Figure 165 is the polypeptide sequence of a fragment of LIGHT, SEQ ID NO: 305.
Figure 166 is the polypeptide sequence of a fragment of LIGHT, SEQ ID NO: 306.
Figure 167 is the polypeptide sequence of the extracellular domain of LAIR1, SEQ ID NO: 307.

### DETAILED DESCRIPTION OF THE INVENTION

The invention describes multifunctional antibody-ligand traps that are designed to counteract immune tolerance or T cell dysfunction in cancers and infectious disorders. The molecules of the invention are designed to efficiently interrupt the multiple autocrine/paracrine signaling loops involving diverse immunoreceptor-ligand interactions that cause immune dysfunction in the tumor or pathogen immune microenvironment. By counteracting the induction/function of regulatory T cells, immune tolerance, and/or T cell exhaustion, the molecules and compositions of this invention can restore and augment effective antitumor immunity for treatment of neoplastic diseases/cancer, or pathogen-directed immune responses for prevention or treatment of infectious diseases. The ability of this novel class of multifunctional antibody-ligand traps to unleash potent antitumor immune responses offers a promising immunotherapeutic strategy for many types of advanced and treatment-refractory cancers.

Before the present compositions and methods are described, it is to be understood that this invention is not limited to particular compositions, methods, and experimental conditions described, as such compositions, methods, and conditions may vary. It is also to be understood that the terminology used herein is for purposes of describing particular embodiments only, and is not intended to be limiting, since the scope of the present invention will be limited only in the appended claims.

As used in this specification and the appended claims, the singular forms "a", "an", and "the" include plural references unless the context clearly dictates otherwise. Thus, for example, references to "the method" includes one or more methods, and/or steps of the type described herein which will become apparent to those persons skilled in the art upon reading this disclosure and so forth.

All publications, patents, and patent applications mentioned in this specification are herein incorporated by reference to the same extent as if each individual publication, patent, or patent application was specifically and individually indicated to be incorporated by reference.

Unless defined otherwise, all technical and scientific terms used herein have the same meaning as commonly understood by one of ordinary skill in the art to which this invention belongs. Although any methods and materials similar or equivalent to those described herein can be used in the practice or testing of the invention, it will be understood that modifications and variations are encompassed within the spirit and scope of the instant disclosure. The preferred methods and materials are now described.

### Immune dysfunction in the tumor microenvironment - a hallmark of cancers

Genetic mutations accruing from the inherent genomic instability of tumor cells present neo-antigens that are recognized by the immune system. Cross-presentation of tumor antigens at the immune synapse between antigen-presenting dendritic cells and T lymphocytes can potentially activate an adaptive antitumor immune response that is mediated by CD4+ T helper cells (TH1) and CD8+ cytotoxic effector cells, and sustained by tumor-reactive central memory T cells. However, tumors continuously evolve to counteract and ultimately defeat such immune surveillance by co-opting and amplifying mechanisms of immune tolerance to evade elimination by the immune system. This prerequisite for tumor progression is enabled by the ability of cancers to produce immunomodulatory factors that create a dysfunctional tumor immune microenvironment. These factors include immunomodulatory ligands, cytokines, and chemokines that regulate the differentiation, activation, proliferation, survival, cytotoxic function, and recruitment of immune cells, such as T cells, NK cells, macrophages, and dendritic cells. The independent and cooperative actions of these factors in the tumor microenvironment results in a dysfunctional immunophenotype characterized by abnormal differentiation and function of tumor-infiltrating T cells: (i) Reduction in TH1 cells; (ii) Elevation of regulatory T cells (Tregs), a sub-population of CD4+ T cells that suppress effector T cells and mediate immune tolerance; (iii) Reduction in cytotoxic CD8+ T cells; (iv) Elevation of exhausted T cells which fail to proliferate and exert effector functions such as cytotoxicity and cytokine secretion in response to antigen stimulation. The functional orientation of tumor-infiltrating immune cells has a major impact on the outcome of patients with cancer. Whereas TH1 cells and cytotoxic CD8+ T cells are uniformly and strongly associated with a longer disease-free survival, the infiltration of tumors with Tregs and exhausted CD8+ T cells has been correlated with a poor prognosis.

### Molecular determinants of immune dysfunction in the tumor microenvironment

Cancers express immunosuppressive ligands that engage T cell inhibitory receptors (TCIR) and inhibit activation of T cells. These TCIR-TCIR ligand interactions serve as 'immune checkpoints' which counteract and restrain the activation of T cells by costimulatory receptors and ligands (Figure 1). The following TCIR and TCIR ligands mediate immune tolerance and T cell exhaustion in the tumor microenvironment:

Programmed death 1 (PD-1)/PD-1 ligands and cytotoxic T lymphocyte antigen-4 (CTLA-4):
The PD-1 receptor (CD279) and its ligands, PD-1 ligand 1 (PD-L1; B7-H1; CD274) and PD-1 ligand 2 (PD-L2; B7-DC; CD273) are members of the B7-CD28 family that play a key role in the maintenance of self-tolerance. Engagement of PD-1 by PD-1 ligand inhibits CD3/CD28-mediated activation of T cells, inhibits T cell effector function, induces T cell exhaustion, and promotes T cell apoptosis PD-1/PD-L1 signaling promotes the induction, function, and maintenance of regulatory T cells (Tregs), a sub-population of immunosuppressive CD4⁺ T cells that express the forkhead box P3 (FOXP3) transcription factor and cytotoxic T lymphocyte antigen-4 (CTLA-4), an co-inhibitory molecule that restrains activation of T cells.

The PD-1/PD-L1 pathway plays a key role in inducing immune tolerance and T cell exhaustion in cancer. PD-L1 is expressed at high levels in several different cancers and high expression of PD-L1 on tumors is strongly associated with poor prognosis.=Tumor cell expression of PD-L1 is upregulated by signaling pathways (RAS-MEK-ERK, PI3K/AKT and JAK/STAT) that are activated by intrinsic genetic aberrations (such as EGFR activation or overexpression, activating RAS mutation, amplification/mutation of PIK3CA, or loss of PTEN), or cytokines in the tumor microenvironment (such as Interferon, IL-6, TGFβ). PD-1 expressing tumor-infiltrating CD8+ T cells exhibit a dysfunctional exhausted phenotype in many cancers, and antibody-mediated blockade of PD-1/PD-L1 signaling has been shown to improve clinical outcome and restore functional T cell responses in several cancers.

TIM-3 and CEACAM-1-mediated immune tolerance and T cell exhaustion:
The T cell immunoglobulin and mucin-domain containing-3 (TIM-3) and CEACAM-1 (Carcinoembryonic antigen related cell adhesion molecule-1) co-inhibitory receptors are key determinants of immune tolerance and T cell exhaustion. TIM-3 is a type I trans-membrane protein expressed on many immune cell types, such as IFN-γ-producing CD4+ and CD8+ T cells, and plays a key role in regulating innate and adaptive immune responses. TIM-3 expression is induced by cytokines involved in effector T cell differentiation, and requires T-bet, the signature transcription factor of the TH1 phenotype. Chronically stimulated T cells and terminally differentiated effector TH1 cells exhibit stabilized, high-level expression of TIM-3. TIM-3 inhibits expression of IFN-γ, suppresses TH1 cells, and induces progressive loss of T cell function (T cell exhaustion). In addition to its key role in regulating T cell effector function, TIM-3 is expressed on FOXP3+ Tregs and enhances the suppressive function of FOXP3+ Tregs. TIM-3 is highly upregulated on both CD4+ and CD8+ tumor infiltrating lymphocytes (TILs), and elevated TIM-3 expression on T cells is associated with immune dysfunction, tumor progression, and poor prognosis in many human cancers. TIM-3 expression on CD8+ TILs is closely associated with that of PD-1, and TIM-3+ PD-1+ CD8+ T cells represent the most dysfunctional "deeply" exhausted T cell population. The increased frequency of PD-1+ and TIM-3+ CD8+ TILs in cancers is inversely correlated with response to therapy and clinical outcome. TIM-3 expression is also significantly upregulated on tumor-infiltrating Tregs, and a predominant TIM-3+ FoxP3+ Treg population in TILs is a common feature across multiple forms of cancer that correlates with poor clinico-pathological parameters, such as nodal metastases and advanced stage.

The immunosuppressive function of TIM-3 requires its heterodimeric interaction with CEACAM-1, another type 1 transmembrane protein that is co-expressed with TIM-3 on activated T cells to downregulate T cell activity and function. The extracellular N-terminal immunoglobulin (IgV) domains of TIM-3 and CEACAM-1 share structural similarities and interact in via a shared signature "cleft" structure along their FG-CC' interface. Heterophilic engagement with CEACAM1 facilitates the maturation and localization of TIM-3 to the T cell surface, and is instrumental for TIM-3-mediated induction of peripheral tolerance and suppression of antitumor immunity. Besides the heterophilic cis interaction described above, CEACAM1 can also ligate TIM-3 in trans suggesting that CEACAM1-expressing tumor cells may provide an inhibitory signal via TIM-3 on a T cell. The TIM-3 cytoplasmic tail is devoid of the classical immunoreceptor tyrosine-based inhibitory motifs (ITIMs) found in other inhibitory receptors, but contains conserved tyrosine residues (Tyr 265, 272) that are critical for binding SH2 domain-containing kinases of the T cell receptor (TCR) signaling pathway and HLA-B associated transcript 3 (Bat3). The interaction of CEACAM-1 with TIM-3 induces Y256/Y263 phosphorylation-mediated release of Bat3 and loss of Bat3-mediated TCR signaling, thereby enabling TIM-3 to cooperate with other binding partners to inhibit TCR signaling and induce T cell exhaustion. In addition to its ability to promote immunosuppression via its heterophilic interaction with TIM-3 (CEACAM1/Tim-3), CEACAM-1 can also suppress T cell activity via homodimerization of its extracellular domains in cis or trans (CEACAM1/CEACAM1). This homophilic interaction is required for phosphorylation of CEACAM-1 cytoplasmic domain ITIMs and recruitment of the SHP-1/SHP-2 tyrosine phosphatases which effect broad proximal suppression of TCR/CD3 complex signaling and inhibit a variety of effector functions, including T cell proliferation, TH1 cytokine production and cytotoxicity associated with T cell activation. These mechanistic insights indicate that the TIM-3/CEACAM1 axis exerts multipronged suppression of T cells via: (i) heterophilic TIM-3/CEACAM-1 interactions (cis CEACAM-1/TIM-3 heterodimerization; trans CEACAM1 heterodimerization with TIM-3); (ii) homophilic CEACAM-1/CEACAM-1 interactions (cis CEACAM-1 dimerization; trans CEACAM1-induced cis CEACAM-1 dimerization). Accordingly, tumor-infiltrating CD8+ T lymphocytes co-expressing TIM-3 and CEACAM1 exhibit the most exhausted phenotype in cancer, and elevated expression of CEACAM1 in several human cancers is correlated with metastasis and poor prognosis.

### V-domain Ig suppressor of T cell activation (VISTA)

V-domain Ig suppressor of T cell activation (VISTA) is an immune checkpoint molecule homologous to PD-L1 and PD-1 that is highly expressed on myeloid cells which causes suppression of T cell activation. VISTA is highly expressed on myeloid and granulocytic cells, and weakly on T cells. VISTA suppresses the activity of CD8+ and CD4+ T cells. VISTA-mediated T cell inhibition is long-lasting, even in the absence of VISTA (Lines et al., 2014). VISTA can act as a ligand, binding a receptor on T cells to inhibit proliferation and cytokine production. VISTA can also act as a receptor on T cells, where its ligation similarly induces inhibition of proliferation and cytokine production. VISTA additionally promotes Treg induction, and is highly expressed on tumor-infiltrating Tregs. VISTA and PD-1 non-redundantly suppress T cell activation.

### Lymphocyte-Activated Gene-3 (LAG-3)

Lymphocyte-Activated Gene-3 (LAG-3; also called CD223) is a negative checkpoint regulator expressed on activated CD4+ T cells, CD8+ T cells, Treg cells, Tr1 cells, and subsets of NK cells, B cells, and DCs. LAG-3 is structurally homologous to CD4, and similarly binds MHC class II molecules. LAG-3 expression on T cells directly inhibits T cell activation. In addition to binding MHC class II, LAG-3 also interacts with LSECtin and Galectin-3 (Gal-3), both of which are expressed in the tumor microenvironment. LAG-3 ligation by either LSECtin or Galectin-3 leads to a decrease in effector T cell IFNγ production and an increase in Treg IL-10 production. LAG-3 is co-expressed and synergizes with PD-1 and TIM-3 to induce and maintain an exhausted T cell phenotype.

### B and T lymphocyte attenuator (BTLA)

B and T lymphocyte attenuator (BTLA) is a negative checkpoint regulator with structural homology to CTLA-4 and PD-1. BTLA is expressed on T cells, mature DCs, macrophages, and most highly on B cells. BTLA binds to herpesvirus entry mediator (HVEM), which is expressed on T cells, B cells, NK cells, DCs, and myeloid cells. HVEM also interacts with CD160 (a negative checkpoint regulator expressed on T cells) and LIGHT (a costimulatory receptor). BTLA signaling on T cells, either through HVEM ligation or the administration of an α-BTLA agonist antibody, inhibits T cell proliferation. BTLA expression also restricts the expansion and activation of γδ T cells, via negative regulation of IL-17 and TNFα. BTLA is also capable of acting as a ligand, binding to HVEM expressed by T cells and promoting T cell survival. BTLA is co-expressed with PD-1 on CD8+ TILs, and appears to engage in synergistic and non-redundant immunosuppressive mechanisms.

### T cell Ig and ITIM Domain (TIGIT)

T cell Ig and ITIM Domain (TIGIT) is an immune checkpoint molecule expressed on CD8+ T cells, CD4+ T cells, and NK cells. TIGIT has been shown to specifically bind Poliovirus Receptor (PVR; also called NECL5 / CD155) and PVR-like protein PVRL2 (also called CD112). CD226 provides a positive co-stimulatory signal when it binds CD155/CD112, while TIGIT delivers an inhibitory signal. TIGIT is a higher-affinity binder for CD155 than it is for CD112. TIGIT forms a heterotetrameric complex with CD155, in which a core TIGIT homodimer binds a pair of CD155 molecules. CD226 on Th1/Th17 T helper cells provide positive co-stimulation when it binds CD155/CD112 on T cells. TIGIT also binds CD155/CD112 (and delivers a negative signal), and thus directly competes with CD226 for binding to CD155/CD112. In addition, TIGIT has been shown to directly bind to CD226 and interrupt its homodimerization, though it is not clear if this mechanism is operative in vivo. TIGIT binds CD155 on DCs to induce a tolerogenic phenotype. CD155 is highly expressed on DCs, and the TIGIT/CD155 interaction in the context leads to the development of tolerogenic DCs which induces IL-10 production, suppresses T-cell proliferation, and limits IFN-γ production. (Yu et al., 2009). CD155 and CD112 are highly expressed by tumor cells, which suggests alternative mechanisms for TIGIT-mediated immunosuppression, via direct effects on T cells. Indeed, TIGIT is uniquely enriched on tumor-infilitrating lymphocytes (TILs), and its function on FOXP3+ Tregs constitutes its most potent immunosuppressive capability.

TIGIT ligation additionally directly suppresses effector T cell function. TIGIT is found to be co-expressed with PD-1, TIM-3, and LAG-3 on CD8+ TILs. TIGIT has been shown to synergize with PD-1/PD-L1 and TIM-3 to impair anti-tumor immunity (Kurtulus et al., 2013). TIGIT ligation is capable of directly suppressing T cell proliferation and cytokine production on T and NK cells on which it is expressed. TIGIT+ CD8+ TILs are deficient in several effector functions (e.g., IL-2 and TNF-α production), and contribute to immunosuppression via enhanced IL-10 production. Amongst CD4+ TILs, TIGIT is almost exclusively expressed on a subset of FOXP3+ Tregs with high expression of Treg effector molecules. TIGIT+ Tregs appear to selectively suppress Th1 and Th17 responses, but not Th2 responses. TIGIT+ Tregs are associated with higher gene expression of Tgfb1, Foxp3, Havcr2, Lag3, and Ctla4. Furthermore, genes for Ccl4, Cxcr3, Ccr8, and Tbx21 are found downstream of TIGIT signaling in Tregs. This suggests an important causal role for TIGIT signaling in Treg migration and retention (Ccl4, Cxcr3, Ccr8) and stability (Tbx21; also called T-bet).

### CCR4

CCR4 is a chemokine receptor highly expressed on Tregs, which allows Tregs to be recruited to the tumor environment via CCL22 ligation. CCL22 is highly expressed by tumor cells to induce immunosuppression via Treg recruitment.

### LAIR1

LAIR1 is a transmembrane glycoprotein with an immunoglobulin-like domain and a cytoplasmic tail containing two immune receptor tyrosine-based inhibitory motifs. When activated, LAIR1 recruits SHP-1 and SHP-2 phosphatases, and can strongly inhibit NK cell-mediated cytotoxicity via cross-linking of the LAIR1 antigen on natural killer (NK) cells.

### Transforming growth factor-β (TGFβ)

Transforming growth factor-β (TGFβ) is a multifunctional cytokine that is overexpressed in a majority of cancers. The high-affinity binding of TGFβ to TGFβ receptor II (TGFβRII) recruits TGFβ receptor I into a heterotetrameric complex that phosphorylates SMAD2/3 and enables its interaction with SMAD4, leading to activation or repression of genes that control cell growth, differentiation, and migration. Tumor cells frequently become refractory to the growth-inhibitory effect of TGFβ and instead leverage its activity in the tumor microenvironment to facilitate immune tolerance and tumor progression. TGFβ plays a critical role in regulating the innate and adaptive immune system. TGF-β inhibits the activation and cytotoxic function of immune effector cells that mediate antibody dependent cellular cytotoxicity (ADCC). Autocrine/paracrine TGFβ-signaling in the localized microenvironment of tumor-infiltrating T cells attenuates the activity of CD8+ T cells, limits development of central memory cells, and skews the differentiation of CD4+ T helper cells (TH1) towards immunosuppressive regulatory T cells (Tregs) which facilitate tumor progression. TGFβ induces the expression of FOXP3, the signature transcription factor that determines and maintains the functional program of the Treg lineage. FOXP3, in turn, induces the expression of cytotoxic T lymphocyte antigen-4 (CTLA-4), an immune-inhibitory receptor that restrains co-stimulation of T cells, and Galectin-9 (GAL-9), a ligand that engages the T-cell immunoglobulin domain and mucin domain-3 (TIM-3) immune-inhibitory receptor and triggers exhaustion or apoptosis of effector T cells. GAL-9 further interacts with TGFβ receptors to drive FOXP3 expression in a positive-feed forward autocrine loop involving SMAD3 activation to induce and maintain Tregs. As such, the TGFβ-enriched tumor microenvironment promotes the development of Tregs that attenuate the activation of immune effectors. This ability of TGFβ to skew the differentiation of CD4+ T cells away from a TH1 phenotype toward a Treg lineage has significant clinical implications, since the functional orientation of tumor-infiltrating immune cells has a major impact on the outcome of patients with cancer. Whereas TH1 cells, cytotoxic CD8+ T cells, and central memory T cells are uniformly and strongly associated with a longer disease-free survival, the infiltration of tumors with Tregs has been correlated with a poor prognosis. Estimation of the relative abundance of T cell subtypes in TCGA-HNSCC dataset [utilizing T cell-type-specific reference gene expression profiles (RGEPs) from single-cell RNA sequencing] showed that the vast majority of cancers demonstrate dysfunctional immuno-inhibitory phenotypes characterized by abnormal skewing of CD4+ T cell differentiation away from TH1 cells toward Tregs and an increase in exhausted CD8+ T cells. The analysis of TCGA datasets showed that elevated TGFβ1/3 is strongly correlated with FOXP3 mRNA expression, and significantly associated with lower survival.

### Limitations of current immunotherapy of cancer:

Current clinical efforts to counteract tumor-induced immune tolerance are focused on monoclonal antibodies which target T cell inhibitory receptors or ligands that function as immune checkpoints, such as CTLA-4, PD-1, and PD-L1. One CTLA-4 antibody (ipilimumab), two PD-1 antagonists (Pembrolizumab, Nivolumab), and three PD-L1 inhibitors (Atezolizumab, Avelumab, Durvalumab) are currently approved in specific clinical indications for immunotherapy of cancers, such as melanoma, non-small cell lung cancer, head and neck cancer, or bladder cancer. Although a subset of patients with advanced cancers experience durable remissions and prolonged survival in response to CTLA-4, PD-1 or PD-L1 mAbs, the majority of patients do not respond to such therapy. Such tumors might use other immunomodulatory ligands or cytokines for inducing T cell dysfunction and inhibiting anti-tumor immunity. Because targeting the PD-1-PD-L pathway alone does not result in complete restoration of T cell function, and in some cancers targeting the PD-1-PD-L pathway does not restore T cell function at all, there is a need to identify and simultaneously counteract multiple molecules and inhibitory pathways that act in concert to execute immune tolerance and T cell exhaustion in the tumor microenvironment. Current immunotherapeutic strategies are limited by the following challenges posed by the dysfunctional immunophenotype of cancers:
(1) Multiple independent, cooperative, and redundant immunoreceptor-ligand interactions of immune tolerance and T cell exhaustion, each reinforced by feedback loops. Targeting individual molecular determinants are not sufficient to counteract multipronged mechanisms of immune dysfunction in cancers.
(2) The immuno-inhibitory signaling pathways which promote Tregs and induce T cell exhaustion involve autocrine and paracrine immunoreceptor-ligand interactions in cis and trans in the tumor microenvironment. Hyperactive immunosuppressive autocrine/paracrine ligand-receptor interactions in the tumor immune cell microenvironment are not effectively interrupted by current mAbs. Novel strategies are required to effectively compete with and physically disrupt such autocrine/paracrine signaling loops in the immediate microenvironment of the target tumor cell or tumor-infitrating T cell.
(3) De novo and adaptive mechanisms of tolerance: Besides exhibiting de novo mechanisms of immune dysfunction, tumors evolve to acquire additional mechanisms of immune tolerance over the course of treatment. As such, addressing one mechanism of might lead to the tumor exploiting an orthogonal pathway that achieves the equivalent immunosuppressive effect.
(4) The dysfunctional immunophenotype of cancers is characterized by skewed differentiation of CD4 cells away from TH1 phenotype toward a Treg or TH17 lineage in the tumor immune microenvironment. Antibodies that inhibit immune checkpoints (such as PD-1/PD-L1 or CTLA-4) or activate co-stimulatory receptors (such as 4-1BB or OX40) are focused on modulating the immune synapse to co-stimulate T cells, but fail to counteract the proximal ligands that divert CD4 cell differentiation from TH1 cells toward a Treg or TH17 lineage that promote immune tolerance and tumor-promoting inflammation.

### Targeted Multifunctional Immunomodulatory antibody-ligand traps

The invention describes targeted multifunctional immunomodulatory antibody-ligand traps that target a tumor cell or a tumor-infiltrating T cell and simultaneously sequester key ligands that contribute to immune dysfunction in the tumor microenvironment. Antibody-ligand traps comprise an antibody targeting a tumor cell or T cell immunoreceptor/ligand (targeting moiety) fused with a receptor extracellular domain (ECD) that simultaneously traps and disables one or more immunosuppressive ligand(s) in the immediate microenvironment of the targeted cell (Receptor ECD; ligand trap). The targeting moiety of the antibody-ligand trap exhibits its own specific function and simultaneously localizes the fused receptor ECD (trap) to the target tumor or immune cell microenvironment to effectively compete with the native receptor for ligands. The receptor ECD comprises a ligand-binding sequence of any immunomodulatory receptor that regulates the differentiation, proliferation, maintenance, survival, activity, function or recruitment of any immune cell, such as a T lymphocyte, myeloid cell, NK cell, or dendritic cell/antigen-presenting cell (DC/APC).

The targeting moiety of the invention comprises a polypeptide which specifically binds a component of a tumor cell, tumor microenvironment, tumor associated growth factor or receptor, tumor associated cytokine or receptor, tumor associated T lymphocyte, T cell co-stimulatory or inhibitory molecule, immune cell, pathogen, or pathogen-associated cell. In specific embodiments, the targeting moiety specifically binds an immunoreceptor, wherein said immunoreceptor is Cytotoxic T lymphocyte associated antigen-4 (CTLA-4, CD152), Programmed Death-1 (PD-1), Programmed death ligand (PD-L1), TIM-3, VISTA, B and T lymphocyte attenuator (BTLA), CD160, TIGIT, CD96, Lymphocyte activation gene-3 (LAG-3), B7-H3, CD39, CD73, adenosine A2a receptor, 4-1BB (CD137), OX-40 (CD134), GITR, CD27, HVEM, CD40L, CD40, CD47, CCR4, CCR5, CXCR4, Interleukin-12 receptor (IL-12R), Toll-like receptor (TLR1-10), CD4, CD25, CD3, ICOS, Killer cell immunoglobulin-like receptor (KIR), or T cell receptor (TCR).

The receptor ECD that is fused to the targeting moiety comprises the extracellular ligand-binding sequence of a T lymphocyte immunoreceptor, B lymphocyte receptor, DC receptor, NK cell receptor, cytokine receptor, growth factor receptor, chemokine receptor, or tumor cell receptor. In specific embodiments, the targeting moiety is fused to a ligand-binding sequence of one of the following the Receptor ECDs:

**Table 1**

| Receptor ECD | Cognate Ligands trapped by Receptor ECD |
|---|---|
| TGFβRII ecd | binds TGFβ |
| PD-1 ecd | binds PD-L1 and PD-L2 |
| TIM-3 ecd | binds CEACAM-1 |
| LIGHT ecd | binds HVEM |
| BTLA ecd | binds HVEM |
| CD226 ecd | binds CD155, CD112 |
| TIGIT ecd | binds CD155, CD112 |
| CD44 ecd | binds CD44 ligands |
| CSF1R ecd | binds CSF-1 |
| CCR4 ecd | binds CCL22 |
| KIR ecd | binds KIR ligands |
| VEGFR ecd | binds VEGF-A, VEGF-B, PIGF |
| RANK ecd | binds RANKL |
| VSIG8 ecd | binds VISTA |
| VISTA ecd | binds VSIG8 |
| LAIR1 ecd | Binds LAIR1 ligands |

The fused Receptor ECD sequence of the antibody-ligand trap optimally traps ligands in the target cell microenvironment:
a. Highly specific binding of all cognate ligands (and isoforms) that signal via the receptor with no risk of off-target effects
b. Predictable/reliable inhibition of all cognate ligands sequestered by the decoy receptor ECD; assured antagonist effects with no risk of paradoxical agonist function or redundant ligand-receptor interactions
c. Optimal avidity for the cognate ligands
d. Disrupts autocrine/paracrine signaling loops since the targeting moiety localizes the fused Receptor ECD to the target cell microenvironment, thereby enabling the decoy Receptor ECD to effectively compete with the native receptor for the targeted ligand(s).
e. Reduced risk of immunogenicity of natural receptor ECD

The Antibody-ligand traps of the invention can activate antitumor immunity by counteracting Tregs and adaptive T cell tolerance/exhaustion in the TME via multifunctional abilities:
a. Disable immune checkpoint receptor/ligand + counteract immunosuppressive ligand(s)
b. Activate a costimulatory receptor + counteract immunosuppressive ligand(s)/checkpoint
c. Target a tumor cell (e.g. GFR) + counteract immunosuppressive ligand(s)/checkpoint
d. Simultaneously counteract or limit the availability of multiple (T cell inhibitory receptor (TCIR) ligands or block multiple TCIRs/TCIR ligands.

The Antibody-ligand traps described in this invention can functionally block multiple TCIRs/TCIR ligands and serve as a strategy to broadly target T cell inhibitory pathways in the TME. Interfering with multiple tumor TCIR/TCIR ligand interactions and their autocrine/paracrine signaling in the tumor/immune cell microenvronment is more effective than multiple antibody-based immune checkpoint blockade. Their ability to cripple the broad multigenic resistance mechanisms of de novo and adaptive immune tolerance may address the challenges and limitations of combination immunotherapies. This is exemplified by antibody-ligand traps of this invention that simultaneously counteract PD-1/PD-L1 and TIM-3/CEACAM-1 mediated mechanisms of immune tolerance and T cell exhaustion in the tumor microenvironment.

Despite compelling antitumor activity of antibodies targeting the programmed death 1 (PD-1): programmed death ligand 1 (PD-L1) immune checkpoint, both *de novo* and adaptive resistance to these therapies is frequently observed. Targeting the PD-1/PD-L1 pathway may not be sufficient to break dysfunction in exhausted T cells, as complex cross-regulation exists between PD-1 and other checkpoint inhibitors to restrain anti-tumor T cell immunity, and in certain cases PD-1 blockade may be followed by development of adaptive resistance. The TIM-3/CEACAM1 axis is a key determinant of *de novo* and adaptive resistance to PD-1/PD-L1 therapy.

TIM-3/CEACAM1 exert multipronged suppression of T cells and NK cells via: (i) heterophilic TIM-3/CEACAM-1 interactions *(cis* CEACAM-1/TIM-3 heterodimerization; *trans* CEACAM1 heterodimerization with TIM-3); (ii) homophilic CEACAM-1/CEACAM-1 interactions *(cis* CEACAM-1 dimerization; *trans* CEACAM1-induced *cis* CEACAM-1 dimerization). Accordingly, tumor-infiltrating CD8⁺ T lymphocytes co-expressing TIM-3 and CEACAM1 exhibit the most exhausted phenotype in both mouse models and patients with cancer.

Since PD-1, TIM-3, and CEACAM-1 entrain independent and cooperative mechanisms of immune tolerance and T cell exhaustion in the tumor microenvironment, simultaneous blockade of these checkpoints is required to unleash potent antitumor innate and adaptive antitumor immune responses. Besides elevated co- expression of all three T cell inhibitory receptors (TCIRs: PD-1, TIM-3, CEACAM-1) on tumor-infiltrating immune cells, tumor cells also co-express multiple ligands that engage these TCIRs to suppress tumor- reactive T cells (PD-L1, CEACAM-1, Galectin-9). This poses the therapeutic challenge of disrupting multiple autocrine/paracrine *cis* and *trans* TCIR/ligand interactions in the TME that act in concert to induce immune tolerance (PD-1/PD-L1; Tim-3/CEACAM-1; CEACAM-1/CEACAM-1). This therapeutic challenge has been addressed by inventing novel bifunctional antibody-ligand traps (Y-traps): (i) *a*-PDL1-TIM3*ecd* comprising an antibody targeting PD-L1 fused to a TIM-3 IgV ectodomain sequence; (ii) *a*-PD1-TIM3*ecd* comprising an antibody targeting PD-1 fused to a TIM-3 IgV ectodomain sequence.

Mechanism of Action for *a*PDL1-TIM3*ecd* and *a*PD1-TIM3*ecd*: *a*PDL1-TIM3*ecd* and *a*PD1-TIM3*ecd* bind PD-1 or PD-L1 on activated T cells and tumor cells, and simultaneously decorate them with a *TIM-3ecd* decoy which effectively competes with native cellular TIM-3 for binding the FG-CC' cleft interface of CEACAM-1. As such these antibody-ligand traps not only disable PD-L1/PD-1 interactions, but simultaneously disrupt both *cis* and *trans* interactions involving Tim-3/CEACAM-1 as well as CEACAM-1/CEACAM-1 in the tumor microenvironment (TME). These studies demonstrate that *a-*PDL1-TIM3*ecd* and *a*-PD1-TIM3*ecd* are significantly more effective than either *a*-PDL1 (atezolizumab) or *a*-PD-1 (pembrolizumab), in promoting IFNγ expression and counteracting exhaustion of co-stimulated T cells *in vitro.* Most significantly, it was found that *in vivo* treatment of tumor-bearing humanized mice with these antibody-ligand traps results in a striking increase in the number of tumor-infiltrating CD4+ and CD8+ T cells, and is significantly more effective in inhibiting tumor progression compared to equivalent treatment with *a*-PDL1 (atezolizumab), *a*-PD-1 (pembrolizumab), *a*-Tim-3 antibody, or even a combination of *a*-PDL1 and *a*-Tim-3. These data demonstrate that heterophilic and homophilic Tim-3/CEACAM-1 interactions in the tumor microenvironment limit the antitumor efficacy of PD-1/PD-L1 inhibitors, and that *a*-PDL1-TIM3*ecd* and *a*-PD1-TIM3*ecd* provide a more effective immunotherapeutic strategy to counteract or reverse T cell exhaustion by simultaneously disabling the PD-1/PD-L1 checkpoint and counteracting both components of the Tim-3/CEACAM-1 axis.

In one embodiment, the present invention provides a molecule comprising a targeting moiety and an immunomodulatory moiety, wherein: the targeting moiety comprises a polypeptide which specifically binds a component of a tumor cell, tumor microenvironment, tumor associated growth factor or receptor, tumor associated cytokine or receptor, tumor associated T lymphocyte, T cell co-stimulatory or inhibitory molecule, immune cell, pathogen, or pathogen-associated cell, and the immunomodulatory moiety comprises the extracellular ligand-binding (ECD) sequence or ligand binding fragment thereof of a T lymphocyte immunoreceptor, B lymphocyte receptor, DC receptor, NK cell receptor, cytokine receptor, growth factor receptor, chemokine receptor, or tumor cell receptor.

As used herein, "targeting moiety" refers to a molecule that has the ability to localize and bind to a specific molecule or cellular component. The targeting moiety can be an antigen-binding domain of an immunoglobulin, antibody, bispecific or multispecific antibody, antibody fragment, scFv, Fc-containing polypeptide, polypeptide, peptide, or any combination thereof. In one embodiment, a targeting moiety can bind to a molecule present in a cell or tissue. In one aspect, the targeting moiety can bind a molecule in a diseased cell or tissue, such as a cancer cell or tumor. In, another aspect, the targeting molecule can bind a normal cell or tissue, such as an immune cell. In another aspect, the targeting moiety can bind a cellular or extracellular molecule that modulates the immune response. In another aspect, the targeting moiety binds a growth factor receptor, growth factor, cytokine receptor, cytokine, or cell surface molecule.

In another embodiment, the targeting moiety is a tumor-targeting moiety. The tumor-targeting moiety can bind a component of a tumor cell or bind in the vicinity of a tumor cell (e.g., tumor vasculature or tumor microenvironment). In one embodiment, the tumor targeting moiety binds to a component of a tumor cell, tumor microenvironment, tumor vasculature, tumor-associated lymphocyte, tumor antigen, tumor-associated antigen, tumor cell surface molecule, tumor antigenic determinant, tumor antigen-containing fusion protein, tumor-associated cell, tumor-associated immune cell, or tumor vaccine.

For example, in various embodiments, a targeting moiety is specific for or binds to a molecule or component, which includes but is not limited to, epidermal growth factor receptor (EGFR, EGFR1, ErbB-1, HER1), ErbB-2 (HER2/neu), ErbB-3/HER3, ErbB-4/HER4, EGFR ligand family; insulin-like growth factor receptor (IGFR) family, IGF-binding proteins (IGFBPs), IGFR ligand family (IGF-1R); platelet derived growth factor receptor (PDGFR) family, PDGFR ligand family; fibroblast growth factor receptor (FGFR) family, FGFR ligand family, vascular endothelial growth factor receptor (VEGFR) family, VEGF family; HGF receptor family: TRK receptor family; ephrin (EPH) receptor family; AXL receptor family; leukocyte tyrosine kinase (LTK) receptor family; TIE receptor family, angiopoietin 1, 2; receptor tyrosine kinase-like orphan receptor (ROR) receptor family; discoidin domain receptor (DDR) family; RET receptor family; KLG receptor family; RYK receptor family; MuSK receptor family; Transforming growth factor alpha (TGF-α), TGF-α receptor; Transforming growth factor-beta (TGF-β), TGF-β receptor; Interleukin 13 receptor alpha2 chain (lL13Ralpha2), Interleukin-6 (IL-6), 1L-6 receptor, Interleukin-4, IL-4 receptor, Cytokine receptors, Class I (hematopoietin family) and Class II (interferon/lL-10 family) receptors, tumor necrosis factor (TNF) family, TNF-α, tumor necrosis factor (TNF) receptor superfamily (TNTRSF), death receptor family, TRAIL-receptor; Chemokine family, Chemokine receptor family; cancer-testis (CT) antigens, lineage-specific antigens, differentiation antigens, alpha-actinin-4, ARTC1, breakpoint cluster region-Abelson (Bcr-abl) fusion products, B-RAF, caspase-5 (CASP-5), caspase-8 (CASP-8), beta-catenin (CTNNB1), cell division cycle 27 (CDC27), cyclin-dependent kinase 4 (CDK4), CDKN2A, COA-1, dek-can fusion protein, EFTUD-2, Elongation factor 2 (ELF2), Ets variant gene 6/acute myeloid leukemia 1 gene ETS (ETC6-AMI,1) fusion protein, fibronectin (FN), GPNMB, low density lipid receptor/GDP-L fucose: beta-Dgalactose 2-alpha-Lfucosyltraosferase (LDLR/FUT) fusion protein, HLA-A2, arginine to isoleucine exchange at residue 170 of the alpha-helix of the alpha2-domain in the HLA-A2 gene (HLA-A*201-R170I), MI,A-A11, heat shock protein 70-2 mutated (HSP70-2M), KIAA0205, MART2, melanoma ubiquitous mutated 1,2, 3 (MUM-1, 2,3), prostatic acid phosphatase (PAP), neo-PAP, Myosin class 1, NFYC, OGT, OS-9, pml-RARalpha fusion protein, PRDX5, PTPRK, K-ras (KRAS2), N-ras (NRAS), HRAS, RBAF600, SIRT2, SNRPD1, SYT-SSX1 or -SSX2 fusion protein, Triosephosphate Isomerase, BAGE, BAGE-1, BAGE-2,3,4,5, GAGE-1,2,3,4,5,6,7,8, GnT-V (aberrant N-acetyl giucosaminyl transferase V, MGAT5), HERV-K-MEL, KK-LC, KM-HN-1, LAGE, LAGE-1, CTL-recognixed antigen on melanoma (CAMEL), MAGE-Al (MAGE-1), MAGE-A2, MAGE-A3, MAGE-A4, MAGE-AS, MAGE-A6, MAGE-A8, MAGE-A9, MAGE-A10, MAGE-A11, MAGE-A12, MAGE-3, MAGE-B1, MAGE-B2, MAGE-B5, MAGE-B6, MAGE-C1, MAGE-C2, mucin 1 (MUCl), MART-1/Melan-A (MLANA), gp100, gp100/Pme117 (S1LV), tyrosinase (TYR), TRP-1, HAGE, NA-88, NY-ESO-1, NY-ESO-l/LAGE-2, SAGE, Sp17, SSX-1,2,3,4, TRP2-1NT2, carcino-embryonic antigen (CEA), Kallikfein 4, mammaglobm-A, OA1, prostate specific antigen (PSA), prostate specific membrane antigen, TRP-l/gp75, TRP-2, adipophilin, interferon inducible protein absent in nielanorna 2 (AIM-2), BING-4, CPSF, cyclin D1, epithelial cell adhesion molecule (Ep-CAM), EpbA3, fibroblast growth factor-5 (FGF-5), glycoprotein 250 (gp250intestinal carboxyl esterase (iCE), alpha-feto protein (AFP), M-CSF, mdm-2, MUCI, p53 (TP53), PBF, FRAME, PSMA, RAGE-1, RNF43, RU2AS, SOX10, STEAP1, survivin (BIRCS), human telomerase reverse transcriptase (hTERT), telomerase, Wilms' tumor gene (WT1), SYCP1, BRDT, SPANX, XAGE, ADAM2, PAGE-5, LIP1, CTAGE-1, CSAGE, MMA1, CAGE, BORIS, HOM-TES-85, AF15q14, HCA66I, LDHC, MORC, SGY-1, SPO11, TPX1, NY-SAR-35, FTHLI7, NXF2 TDRD1, TEX 15, FATE, TPTE, immunoglobulin idiotypes, Bence-Jones protein, estrogen receptors (ER), androgen receptors (AR), CD40, CD30, CD20, CD19, CD33, CD4, CD25, CD3, cancer antigen 72-4 (CA 72-4), cancer antigen 15-3 (CA 15-3), cancer antigen 27-29 (CA 27-29), cancer antigen 125 (CA 125), cancer antigen 19-9 (CA 19-9), beta-human chorionic gonadotropin, 1-2 microglobulin, squamous cell carcinoma antigen, neuron-specific enoJase, heat shock protein gp96, GM2, sargramostim, CTLA-4, 707 alanine proline (707-AP), adenocarcinoma antigen recognized by T cells 4 (ART-4), carcinoembryogenic antigen peptide-1 (CAP-1), calcium-activated chloride channel-2 (CLCA2), cyclophilin B (Cyp-B), human signet ring tumor-2 (HST-2), Human papilloma virus (HPV) proteins (HPV-E6, HPV-E7, major or minor capsid antigens, others), Epstein-Barr vims (EBV) proteins (EBV latent membrane proteins-LMP1, LMP2; others), Hepatitis B or C virus proteins, and HIV proteins, including gp120. A composition of the invention can further include the foregoing as a peptide/polypeptide and/or encoding the same.

In one aspect, the targeting moiety polypeptide specifically binds Cytotoxic T lymphocyte associated antigen-4 (CTLA-4, CD152), Programmed Death-1 protein (PD-1), Programmed death ligand-1 (PD-L1), Programmed death ligand (PD-L2),B7-H3 (CD276),T-cell immunoglobulin and mucin-domain containing-3 (TIM-3), Carcinoembryonic antigen-related cell adhesion molecule 1 (CEACAM-1), Carcinoembryonic Antigen (CEA), V domain Ig suppressor of T cell activation (VISTA), V-set and immunoglobulin domain containing 8 (VSIG8), B and T lymphocyte attenuator (BTLA), Herpesvirus Entry Mediator (HVEM), CD160,T cell Ig and ITM domain (TIGIT), CD226, CD96, CD122, Lymphocyte activation gene-3 (LAG-3), transforming growth factor β (TGF-β), transforming growth factor β receptor (TGFβR), 4-1BB (CD137), Inducible T-Cell Costimulator (ICOS), OX-40 (CD134), glucocorticoid-induced TNFR-related protein (GITR), IL6R, IL23R, IL17R, IL-6, IL-23, IL-17, CD39, CD47, CD73, CCR4, CCR5, CXCR4,IL12R,CD4, CD25, CD3, Receptor activator of nuclear factor kappa-B ligand (RANKL), epidermal growth factor receptor (EGFR, EGFR1, ErbB-1, HER1), ErbB-2 (HER2/neu), ErbB-3/HER3, ErbB-4/HER4, Epidermal growth factor (EGF), Transforming growth factor α (TGFα), Vascular endothelial growth factor (VEGF), Vascular endothelial growth factor receptor-1 (VEGFR-1), VEGFR-2, VEGFR-3.

In one embodiment, the present invention provides a molecule including a targeting moiety fused with an "immunomodulatory moiety". As used herein, "immunomodulatory moiety" refers to a polypeptide that comprises the extracellular domain (ECD) sequence or ligand binding fragment thereof of a T lymphocyte immunoreceptor, T cell inhibitory receptor (TCIR), T-cell co-inhibitory molecule, T cell co-stimulatory molecule, B lymphocyte receptor, DC receptor, NK cell receptor, cytokine receptor, growth factor receptor, chemokine receptor, or tumor cell receptor. In an additional aspect, the "immunomodulatory moiety" specifically binds a cytokine, cytokine receptor, co-stimulatory molecule, or co-inhibitory molecule that modulates the immune system. In one aspect, the immunomodulatory moiety is an agonist that increases the function of the bound molecule. In another aspect, the immunomodulatory moiety is an antagonist that inhibits the function of the targeted molecule.

In another aspect, the immunomodulatory moiety specifically comprises the extracellular domain or ligand binding fragment thereof of one of the following molecules: transforming growth factor β receptor II (TGFβRII), programmed death 1 protein (PD-1), T cell immunoglobulin and mucin domain containing 3 (TIM-3), B- and T-lymphocyte attenuator (BTLA), CD226, T cell Ig and ITM domain (TIGIT), CD44, CD96, CD160, Colony stimulating factor 1 receptor (CSF1R), CCR4, Killer-cell immunoglobulin-like receptor (KIR), Vascular endothelial growth factor receptor (VEGFR), Receptor Activator of Nuclear Factor κ B (RANK), V-set and immunoglobulin domain containing 8 (VSIG8), LIGHT (TNFSF14), leukocyte associated immunoglobulin like receptor 1 (LAIR1), or V domain Ig suppressor of T cell activation (VISTA).

In another aspect, the immunomodulatory moiety is fused to the C-terminus of the targeting moiety. In another aspect, the immunomodulatory moiety is fused to the N-terminus of the targeting moiety. In one aspect, the fusion molecule is represented by X-Fc-Y, wherein X is the targeting moiety, Fc is an immunoglobulin Fc region, and Y is the immunomodulatory moiety. In another aspect, the fusion molecule is represented by Y-Fc-X, wherein X is the targeting moiety, and Y is the immunomodulatory moiety.

As used herein, the terms "extracellular domain" or "ECD" refers to that part of the receptor that protrudes from the outer membrane of the cell organelles and cells into the extracellular space. The polypeptide sequence of the ECD includes any analog or fragment of a polypeptide or peptide whose amino acid residue sequence is shown herein.

The term "analog" includes any polypeptide or peptide or having an amino acid residue sequence substantially identical to a sequence specifically shown herein in which one or more residues have been conservatively substituted with a functionally similar residue and which displays the activity as described herein. Examples of conservative substitutions include the substitution of one non-polar (hydrophobic) residue such as isoleucine, valine, leucine or methionine for another, the substitution of one polar (hydrophilic) residue for another such as between arginine and lysine, between glutamine and asparagine, between glycine and serine, the substitution of one basic residue such as lysine, arginine or histidine for another, or the substitution of one acidic residue, such as aspartic acid or glutamic acid for another.

The term "fragment" refers to any subject polypeptide having an amino acid residue sequence shorter than that of a polypeptide whose amino acid residue sequence is disclosed herein.

As used herein, the term "ligand binding fragment" refers to a portion or fragment of the ECD that binds a ligand. In a situation where "ligand binding fragment" is used to refer to a portion or fragment of a molecule that is itself considered to be a "ligand," "ligand binding fragment" refers to a portion or fragment of the ECD of that molecule that binds its binding partner.

Antibodies: In one embodiment, the targeting moiety or fusion protein is an immunoglobulin. As used herein, the term "immunoglobulin" includes natural or artificial mono- or polyvalent antibodies including, but not limited to, polyclonal, monoclonal, multispecific, human, humanized or chimeric antibodies, single chain antibodies, Fab fragments. F(ab') fragments, fragments produced by a Fab expression library, anti-idiotypic (anti-Id) antibodies (including, e.g., anti-Id antibodies to antibodies of the invention), and epitope-binding fragments of any of the above. The term "antibody," as used herein, refers to immunoglobulin molecules and immunologically active portions of immunoglobulin molecules, i.e., molecules that contain an antigen binding site that immunospecifically binds an antigen. The immunoglobulin can be of any type (e.g., IgG, IgE, IgM, IgD, IgA, and IgY), class (e.g., IgGl, IgG2, IgG3, IgG4, IgAl, and IgA2) or subclass of immunoglobulin molecule.

An antibody as disclosed herein includes an antibody fragment, such as, but not limited to, Fab, Fab' and F(ab')2, Fd, single-chain Fvs (scFv), single-chain antibodies, disulfide-linked Fvs (sdfv) and fragments including either a VL or VH domain. In one embodiment, the targeting moiety is an antibody or scFv.

An antigen-binding antibody fragment, including single-chain antibody, may include the variable region(s) alone or in combination with the entirety or a portion of the following: hinge region, CH1, CH2, and CH3 domains. An antigen-binding fragment can also include any combination of variable region(s) with a hinge region, CHI, CH2, and CH3 domains. Also includes is a Fc fragment, antigen-Fc fusion proteins, and Fc-targeting moiety. The antibody may be from any animal origin including birds and mammals. In one aspect, the antibody is, or derived from, a human, murine (e.g., mouse and rat), donkey, sheep, rabbit, goat, guinea pig, camel, horse, or chicken. Further, such antibody may be a humanized version of an antibody. The antibody may be monospecific, bispecific, trispecific, or of greater multi specificity.

The antibody herein specifically include a "chimeric" antibody in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Pat. No. 4,816,567; and Morrison et al (1984) Proc. Natl. Acad. Sci. USA. 81:6851-6855). A chimeric antibody of interest herein includes "primatized" antibodies including variable domain antigen-binding sequences derived from a non-human primate (e.g., Old World Monkey, Ape etc) and human constant region sequences.

Various methods have been employed to produce antibodies. Hybridoma technology, which refers to a cloned cell line that produces a single type of antibody, uses the cells of various species, including mice (murine), hamsters, rats, and humans. Another method to prepare an antibody uses genetic engineering including recombinant DNA techniques. For example, antibodies made from these techniques include, among others, chimeric antibodies and humanized antibodies. A chimeric antibody combines DNA encoding regions from more than one type of species. For example, a chimeric antibody may derive the variable region from a mouse and the constant region from a human. A humanized antibody comes predominantly from a human, even though it contains nonhuman portions. Like a chimeric antibody, a humanized antibody may contain a completely human constant region. But unlike a chimeric antibody, the variable region may be partially derived from a human. The nonhuman, synthetic portions of a humanized antibody often come from CDRs in murine antibodies. In any event, these regions are crucial to allow the antibody to recognize and bind to a specific antigen.

In one embodiment, the molecules of this invention can be synthesisized using recombinant DNA methods well described in the art. The cDNA for the antibody heavy chain and the cDNA for the antibody light chain were gene synthesized and subsequently cloned into separate plasmids under the control of a mammalian promoter and polyadenylation signal. Plasmid DNA was amplified in E. coli and DNA purified using anion exchange kits for low endotoxin plasmid DNA preparation. DNA concentration was determined by measuring the absorption at a wavelength of 260 nm. Correctness of the sequences was verified with Sanger sequencing (with up to two sequencing reactions per plasmid depending on the size of the cDNA.) The plasmid DNAs for heavy and light chain were subsequently co-transfected into suspension-adapted CHO K1 cells (originally received from ATCC and adapted to serum-free growth in suspension culture at evitria). In one embodiment, a hybridoma can produce a targeted fusion protein comprising a targeting moiety and an immunomodulatory moiety.

In one embodiment, a targeting moiety comprising an antibody, antibody fragment, or polypeptide is linked or fused to an immunomodulatory moiety consisting of a polypeptide, with a linker or without a linker. The linker can be an amino acid linker. In one embodiment, a linker is (GGGGS)n (SEQ ID NO: 3) wherein n is 1,2,3,4,5,6,7, or 8. For example, GGGGSGGGGSGGGGS (SEQ ID NO: 4). In another embodiment, a linker is IEGRDMD (SEQ ID NO: 5). In various aspects, the length of the linker may be modified to optimize binding of the target moiety or the function of the immunomodulatory moiety.

In various aspects, the immunomodulatory moiety is a polypeptide that is fused to the C-terminus of the Fc region of the heavy chain of a targeting antibody or Fc-containing fusion protein. In another aspect, the immunomodulatory moiety is a polypeptide that is fused to the C-terminus of the light chain of a targeting antibody. In another aspect, the fusion protein comprises an X-Fc-Y sequence, wherein X is a targeting polypeptide and Y is an immunomodulatory polypeptide.

An antibody fragment can include a portion of an intact, antibody, e.g. including the antigen-binding or variable region thereof. Examples of antibody fragments include Fab, Fab', F(ab')2, and Fv fragments; Fc fragments or Fc-fusion products; diabodies; linear antibodies; single-chain antibody molecules; and multispecific antibodies formed from antibody fragment(s).

An intact antibody is one which includes an antigen-binding variable region as well as a light chain constant domain (CL) and heavy chain constant domains, CH1, CH2 and CH3. The constant domains may be native sequence constant domains (e.g., human native sequence constant domains) or amino acid sequence variant thereof or any other modified Fc (e.g. glycosylation or other engineered Fc).

The intact antibody may have one or more "effector functions" which refer to those biological activities attributable to the Fc region (a native sequence Fc region or amino acid sequence variant Fc region or any other modified Fc region) of an antibody. Examples of antibody effector functions include Clq binding; complement dependent cytotoxicity; Fc receptor binding; antibody-dependent cell-mediated cytotoxicity (ADCC); phagocytosis; down regulation of cell surface receptors (e.g., B cell receptor (BCR); and cross-presentation of antigens by antigen presenting cells or dendritic cells. In one embodiment, the targeting antibody or Fc-containing fusion protein facilitates focused or preferential delivery of a immunomodulatory moiety to a target cell. In another aspect, a targeting antibody can induce death of the targeted cell or sensitize it to immune cell-mediated cytotoxicity. In another aspect, the Fc-fusion protein or antibody can facilitate delivery of the immunomodulatory moiety or immunogenic apoptotic material from antibody-bound tumor targets, or both, to an antigen presenting cells (APC) via interactions between their Fc and Fc receptors (on APC).

Depending on the amino acid sequence of the constant domain of their heavy chains, intact antibodies can be assigned to different "classes." There are five major classes of intact antibodies: IgA, IgD, IgE, IgG, and IgM, and several of these may be further divided into "subclasses" (isotypes), e.g., IgG1, IgG2, IgC3, IgG4, IgA, and IgA2. The heavy-chain constant domains (hat correspond to the different classes of antibodies are called alpha (α), delta (δ), epsilon (ε), gamma (γ), and mu (µ) respectively. The subunit structures and three-dimensional configurations of different classes of immunoglobulins are well known.

In one aspect, the antibody specifically binds a T cell inhibitory receptor (TCIR), a T cell inhibitory receptor ligand (TCIR ligand), a T-cell co-inhibitory molecule, or a T cell co-stimulatory molecule.

T cell activation begins with the recognition of an antigenic peptide in the context of a major histocompatibility complex (MHC) on an antigen-presenting cell by the T cell receptor (TCR). The process of T cell activation is mediated by a number of signaling proteins through inducible phosphorylation, enzyme activation and protein-protein and protein-lipid interactions. T cells require two signals to become fully activated. A first signal, which is antigen-specific, is provided through the T cell receptor (TCR) which interacts with peptide-MHC molecules on the membrane of antigen presenting cells (APC). A second signal, the co-stimulatory signal, is antigen nonspecific and is provided by the interaction between co-stimulatory molecules expressed on the membrane of APC and the T cell. T cell co-stimulation is necessary for T cell proliferation, differentiation and survival. Activation of T cells without co-stimulation may lead to T cell anergy, T cell deletion or the development of immune tolerance.

The best characterized co-stimulatory molecules expressed by T cells is CD28 which interacts with CD80 (B7.1) and CD86 (B7.2) on the membrane of APC. Other costimulatory receptors expressed by T cells include 4-1BB (receptor for 4-1BB ligand), ICOS (Inducible Costimulator)(receptor for ICOS-L), OX40 (receptor for OX40 ligand), GITR (receptor for GITR ligand), CD27 (interacts with CD70), CD40L/CD40, HVEM (interacts with LIGHT), CD226 (interacts with CD155).

The activation signals are modulated by a family of receptors termed, co-inhibitory receptors that include CTLA-4, PD-1, LAG-3, TIM-3, CEACAM-1, TIGIT, CD96, BTLA, CD160, VISTA, VSIG8, LAIR. Co-inhibitory receptors modulate signaling by utilizing mechanisms such as ectodomain competition with counter receptors and by the use of intracellular mediators such as protein phosphatases. Co-inhibitory receptors can act as threshold-setters, modulators, checkpoints and feedback mechanisms that can fine tune the quality and magnitude of the T cell immune response.

Receptors that are inhibitory to T cell function are termed T cell co-inhibitory receptors. Inhibitory receptors attenuate and counterbalance activation signals initiated by stimulatory receptors. The subsequent outcomes on T cell function can range from temporary inhibition to permanent inactivation and cell death. TCR signaling can be controlled by various mechanisms that differ in their time of action and/or target molecule. Negative regulatory mechanisms are in place to act before T cell activation to maintain its quiescent state.

The majority of T cell co-inhibitory receptors belong to the immunoglobulin (Ig) superfamily. One mechanism involves the sequestration of the ligands for co-stimulatory receptors, depriving the T cell from receiving activation signals necessary for complete activation. The second mechanism involves the recruitment of intracellular phosphatases by an immunoreceptor tyrosine-based inhibition motif (ITIM) and/or an immunoreceptor tyrosine-based switch motif (ITSM) that make up the cytoplasmic tail of certain inhibitory receptors, which dephosphorylate signaling molecules downstream of the TCR and co-stimulatory pathways, leading to a quantitative reduction in activation-induced gene expression. The third mechanism involves the upregulation (or downregulation) of genes that code for proteins involved in the inhibition of immune functions . A co-inhibitory receptor could use a combination of the above and possibly other yet to be discovered mechanisms to regulate T cell signaling.

Co-inhibitory receptors are transmembrane glycoproteins that transmit dominant negative signals mainly via intracellular phosphatases that bind to phosphorylated tyrosine residues in the cytoplasmic domain. Co-inhibitory receptors can act as safety mechanisms and threshold setters to prevent uncontrolled detrimental extremes of reactivity by counteracting the stimulatory signals.

Examples of antibodies or fragments thereof which may be incorporated into molecules, fusion proteins, compositions and methods disclosed herein include, but are not limited, to antibodies such as trastuzumab (anti-HER2/neu antibody); Pertuzumab (anti-HER2 mAb); cetuximab (chimeric monoclonal antibody to epidermal growth factor receptor EGFR); panitumumab (anti-EGFR antibody); nimotuzumab (anti-EGFR antibody); Zalutumumab (anti-EGFR mAb); Necitumumab (anti-EGFR mAb); MDX-210 (humanized anti-HER-2 bispecific antibody); MDX-210 (humanized anti-HER-2 bispecific antibody); MDX-447 (humanized anti-EGF receptor bispecific antibody); Rituximab (chimeric murine/human anti-CD20 mAb); Obinutuzumab (anti-CD20 mAb); Ofatumumab (anti-CD20 mAb); Tositumumab-I131 (anti-CD20 mAb); Ibritumomab tiuxetan (anti-CD20 mAb); Bevacizumab (anti-VEGF mAb); Ramucirumab (anti-VEGFR2 mAb); Ranibizumab (anti-VEGF mAb); Aflibercept (extracellular domains of VEGFR1 and VEGFR2 fused to IgG1 Fc); AMG386 (angiopoietin-1 and -2 binding peptide fused to IgG1 Fc); Dalotuzumab (anti-IGF-1R mAb); Gemtuzumab ozogamicin (anti-CD33 mAb); Alemtuzumab (anti-Campath-1/CD52 mAb); Brentuximab vedotin (anti-CD30 mAb); Catumaxomab (bispecific mAb that targets epithelial cell adhesion molecule and CD3); Naptumomab (anti-5T4 mAb); Girentuximab (anti-Carbonic anhydrase ix); or Farletuzumab (anti-folate receptor). Other examples include antibodies such as Panorex^{™} (17-1A) (murine monoclonal antibody); Panorex (@ (17-1A) (chimeric murine monoclonal antibody); BEC2 (ami-idiotypic mAb, mimics the GD epitope) (with BCG); Oncolym (Lym-1 monoclonal antibody); SMART M195 Ab, humanized 13' 1 LYM-1 (Oncolym), Ovarex (B43.13, anti-idiotypic mouse mAb); 3622W94 mAb that binds to EGP40 (17-1A) pancarcinoma antigen on adenocarcinomas; Zenapax (SMART Anti-Tac (IL-2 receptor); SMART M195 Ab, humanized Ab, humanized); NovoMAb-G2 (pancarcinoma specific Ab); TNT (chimeric mAb to histone antigens); TNT (chimeric mAb to histone antigens); Gliomab-H (Monoclonals-Humanized Abs); GNI-250 Mab; EMD-72000 (chimeric-EGF antagonist); LymphoCide (humanized IL.L.2 antibody); and MDX-260 bispecific, targets GD-2, ANA Ab, SMART IDIO Ab, SMART ABL 364 Ab, ImmuRAIT-CEA, Zanulimumab (anti-CD4 mAb), Keliximab (anti-CD4 mAb); Ipilimumab (MDX-101; anti-CTLA-4 mAb); Tremilimumab (anti-CTLA-4 mAb); Daclizumab (anti-CD25/IL-2R mAb); Basiliximab (anti-CD25/IL-2R mAb); MDX-1106 (anti-PD1 mAb); antibody to GITR; GC1008 (anti-TGF-β antibody); metelimumab/CAT-192 (anti- TGF-β antibody); lerdelimumab/CAT-152 (anti- TGF-β antibody); ID11 (anti- TGF-β antibody); Denosumab (anti-RANKL mAb); BMS-663513 (humanized anti-4-1BB mAb); SGN-40 (humanized anti-CD40 mAb); CP870,893 (human anti-CD40 mAb); Infliximab (chimeric anti-TNF mAb; Adalimumab (human anti-TNF mAb); Certolizumab (humanized Fab anti-TNF); Golimumab (anti-TNF); Etanercept (Extracellular domain of TNFR fused to IgG1 Fc); Belatacept (Extracellular domain of CTLA-4 fused to Fc); Abatacept (Extracellular domain of CTLA-4 fused to Fc); Belimumab (anti-B Lymphocyte stimulator); Muromonab-CD3 (anti-CD3 mAb); Otelixizumab (anti-CD3 mAb); Teplizumab (anti-CD3 mAb); Tocilizumab (anti-IL6R mAb); REGN88 (anti-IL6R mAb); Ustekinumab (anti-IL-12/23 mAb); Briakinumab (anti-IL-12/23 mAb); Natalizumab (anti-α4 integrin); Vedolizumab (anti-α4 β7 integrin mAb); T1h (anti-CD6 mAb); Epratuzumab (anti-CD22 mAb); Efalizumab (anti-CD11a mAb); Urelumab, Utomilumab (anti -41BB mAbs: agonists); BMS 986178 (anti-OX40 mAb: agonist); Elotuzumab (anti-SLAM7/CS1 mAb); Daratumumab (anti-CD38 mAb); Guselkumab (anti-IL23); Nivolumab (anti-PD-1); Pembrolizumab (anti-PD-1); Atezolizumab (anti-PD-L1); Avelumab (anti-PD-L1); Durvalumab (anti-PD-L1); Relatlimab, BMS-986016 (anti-LAG3 mAb); NKTR-214 (CD-122 agonist mAb). Examples of antibodies include those disclosed in US5736167, US7060808, and US5821337.

Peptides: In some aspects of the invention the targeting moiety or immunomodulatory moiety is a peptide or polypeptide. A peptide includes any analog, fragment or chemical derivative of a peptide whose amino acid residue sequence is shown herein. Therefore, a present peptide can be subject to various changes, substitutions, insertions, and deletions where such changes provide for certain advantages in its use. In this regard, a peptide of this invention corresponds to, rather than is identical to, the sequence of a recited peptide where one or more changes are made and it retains the ability to function as the unmodified peptide in one or more of the assays.

As used herein "a tumor targeting peptide" includes polymers containing fewer than 100 amino acids, where the polymer specifically binds to a cellular component of a tumor cell, tumor vasculature, and/or a component of a tumor microenvironment.

A peptide of the present invention can be synthesized by any of the techniques that are known to those skilled in "the polypeptide art, including recombinant DNA techniques. Synthetic chemistry techniques, such as a solid-phase Merrifield-type synthesis, are preferred for reasons of purity, antigenic specificity, freedom from undesired side products, ease of production and the like. An excellent summary of the many techniques available can be found in Steward et al., "Solid Phase Peptide Synthesis"* W. H. Freeman Co., San Francisco, 1969; Bodanszky, et al., "Peptide Synthesis", John Wiley & Sons, Second Edition, 1976; J. Meienhofer, "Hormonal Proteins and Peptides". Vol. 2. p. 46, Academic Press (New York), 1983; Merrifield, Adv. Enzymol., 32:221-96, 1969; Fields et al.. Int. J. Peptide Protein Res., 35: 161-214, 1990; and U.S. Pat. No. 4,244,946 for solid phase peptide synthesis, and Schroder et al., "The Peptides", Vol. 1, Academic Press (New York), 1965 for classical solution synthesis. Appropriate protective groups usable in such synthesis are described in the above texts and in J. F. W. McOmie, "Protective Groups in Organic Chemistry", Plenum Press, New York, 1973.

In one aspect, the targeting moiety comprises a polypeptide that binds to BTLA, CCR4, CTLA4, LAG3, PD-1, PD-L1, TIGIT, VISTA, 41BB, OX40, GITR, RANKL, TGF-β or VEGF and the immunomodulatory moiety comprises the ECD or a ligand binding fragment thereof of TIM-3. In a specific aspect, the immunomodulatory moiety comprises the CEACAM 1 binding fragment of TIM-3. In another aspect, the targeting moiety comprises a polypeptide that binds to BTLA, CCR4, CTLA4, LAG3, PD-1, PD-L1, TIGIT, VISTA, 41BB, OX40, GITR, RANKL, TGF-β or VEGF and the immunomodulatory moiety comprises the ECD or a ligand binding fragment thereof of CD226. In certain aspects, the ligand binding fragment comprises a CD155 or CD112 binding fragment. In an additional aspect, the targeting moiety comprises a polypeptide that binds to BTLA, CCR4, CTLA4, LAG3, PD-1, PD-L1, TIGIT, VISTA, 41BB, OX40, GITR, TGF-β or VEGF and the immunomodulatory moiety comprises the ECD or a ligand binding fragment thereof of CD44. In a further aspect, the targeting moiety comprises a polypeptide that binds to BTLA, CCR4, CTLA4, LAG3, PD-1, PD-L1, TIGIT, VISTA, 41BB, OX40, GITR, TGF-β or VEGF and the immunomodulatory moiety comprises the ECD or a ligand binding fragment thereof of PD-1. In another aspect, the targeting moiety comprises a polypeptide that binds to BTLA, CCR4, CTLA4, LAG3, PD-1, PD-L1, TIGIT, VISTA, 41BB, OX40, GITR, TGF-β or VEGF and the immunomodulatory moiety comprises the ECD or a ligand binding fragment thereof of VSIG8. In an aspect, the ligand binding fragment comprises a VISTA binding fragment. In certain aspects, the targeting moiety comprises a polypeptide that binds to BTLA, CCR4, CTLA4, LAG3, PD-1, PD-L1, TIGIT, VISTA, 41BB, OX40, GITR, TGF-β or VEGF and the immunomodulatory moiety comprises the ECD or a ligand binding fragment thereof of TGFβRII.

In an aspect, the molecule comprises sequences corresponding to SEQ ID NOs: 10 and 145; 11 and 146; 12 and 147; 13 and 148; 14 and 149; 15 and 150; 16 and 151; 17 and 152; 18 and 153; 19 and 154, 20 and 155; 21 and 156; 22 and 157; 23 and 158; 24 and 159; 25 and 160; 26 and 161; 27 and 162; 28 and 163; 29 and 164; 30 and 165; 31 and 166; 32 and 167; 33 and 168; 34 and 169; 35 and 170; 37 and 171; 38 and 172; 39 and 173; 40 and 174; 41 and 175; 42 and 176; 43 and 177; 44 and 178; 45 and 179; 46 and 180; 47 and 181; 48 and 182; 49 and 183; 50 and 184; 51 and 185; 52 and 186; 53 and 187; 58 and 188; 59 and 189; 60 and 61; 62 and 63; 64 and 65; 66 and 67; 68 and 69; 70 and 71; 72 and 73; 74 and 75; 76 and 77; 78 and 79; 80 and 81; 82 and 83; 84 and 85; 86 and 87; 88 and 89; 90 and 91; 92 and 93; 94 and 95;, 96 and 97; 98 and 99; 100 and 101; 102 and 103; 104 and 105; 106 and 107; 108 and 109; 110 and 111; 112 and 113; 114 and 115; 116 and 117; 118 and 119; 120 and 121; 122 and 123; 124 and 125; 126 and 127;, 128 and 129; 130 and 131; 132 and 133; 134 and 135; 136 and 137; 138 and 139; 140 and 141; 142 and 143; 192 and 193; 209 and 210; 211 and 212; 221 and 222; 223 and 224; 233 and 234; 235 and 236; 245 and 246; 247 and 248; 257 and 258; 259 and 260; 269 and 270; 271 and 272; 275 and 276; 279 and 280; 283 and 284; 287 and 288; 290 and 291; 292 and 293; 294 and 295; 296 and 297 or 298 and 299..

In certain aspects, the targeting moiety comprises an antibody that binds PD-1 or PD-L1 and the immunomodulatory moiety comprises the ECD or ligand binding fragment thereof of Tim-3 and/or comprises the sequence of SEQ ID NO: 51, 52, 53 or 192. In one aspect, the targeting moiety comprises an antibody that binds CTLA-4 and the immunomodulatory moiety comprises the ECD or ligand binding fragment thereof of TIM-3. In a specific aspect, the molecule comprises the sequence of SEQ ID NO: 50. In another aspect, the targeting moiety comprises an antibody that binds LAG-3 and the immunomodulatory moiety is the ECD or ligand binding fragment thereof of TIM-3. In one aspect, the molecule comprises the sequence of SEQ ID NO: 26. In an additional aspect, the targeting moiety comprises an antibody that binds VISTA and the immunomodulatory moiety comprises the ECD or ligand binding fragment thereof of TIM-3. In a further aspect, the molecule comprises the sequence of SEQ ID NO: 12. In one aspect, the targeting moiety comprises an antibody that binds TIGIT and the immunomodulatory moiety comprises the ECD or ligand binding fragment thereof of TIM-3. In a specific aspect, the molecule comprises the sequence of SEQ ID NO: 17. In another aspect, the targeting moiety comprises an antibody that binds BTLA and the immunomodulatory moiety comprises the ECD or ligand binding fragment thereof of TIM-3. In an additional, aspect, the molecule comprises the sequence of SEQ ID NO: 22. In an aspect, the targeting moiety comprises s an antibody that binds CCR-4 and the immunomodulatory moiety comprises the ECD or ligand binding fragment thereof of TIM-3. In certain aspects, the molecule comprises the sequence of SEQ ID NO: 31. In a further aspect, the targeting moiety comprises is an antibody that binds 4 1BB and the immunomodulatory moiety comprises the ECD or ligand binding fragment thereof of TIM-3. In a specific aspect, the molecule comprises the sequence of SEQ ID NO: 116. In one aspect, the targeting moiety comprises an antibody that binds VEGF and the immunomodulatory moiety comprises the ECD or ligand binding fragment thereof of TIM-3. In certain aspects, the molecule comprises s the sequence of SEQ ID NO: 88.

In an additional aspect, the immunomodulatory moiety comprises the ECD or ligand binding fragment thereof of BTLA or LIGHT and the ligand binding fragment comprises an Herpesvirus entry mediator (HVEM, CD 270) binding fragment. In a further aspect, the immunomodulatory moiety comprises the ECD or ligand binding fragment thereof of CCR4 and the ligand binding fragment comprises a CCL22 binding fragment.

In an additional aspect, the immunomodulatory moiety comprises the ECD or ligand binding fragment thereof of CD160. In an additional aspect, the immunomodulatory moiety comprises the sequence of SEQ ID NO: 300.

In an additional aspect, the immunomodulatory moiety comprises the ECD or ligand binding fragment thereof of LAIR1. In an additional aspect, the immunomodulatory moiety comprises the sequence of SEQ ID NO: 307.

In an additional embodiment, the present invention provides molecules comprising a targeting moiety, a first immunomodulatory moiety (IM-1) and a second immunomodulatory moiety (IM-2), wherein the targeting moiety comprises a polypeptide that specifically binds Cytotoxic T lymphocyte associated antigen-4 (CTLA-4, CD152), Programmed Death-1 protein (PD-1), Programmed death ligand-1 (PD-L1), V domain Ig suppressor of T cell activation (VISTA), B and T lymphocyte attenuator (BTLA), T cell Ig and ITM domain (TIGIT), Lymphocyte activation gene-3 (LAG-3), 4-1BB ligand (CD137), OX-40 (CD134), glucocorticoid-induced TNFR-related protein (GITR), Receptor activator of Nuclear factor kappa B ligand (RANKL), transforming growth factor β (TGF-β), IL6R, epidermal growth factor receptor (EGFR), human epidermal growth factor receptor 2 (HER2) or Vascular endothelial growth factor (VEGF), the first immunomodulatory moiety comprises the extracellular ligand-binding sequence or ligand binding fragment thereof of transforming growth factor β receptor II (TGFβRII), programmed death 1 protein (PD-1), T cell immunoglobulin and mucin domain containing 3 (TIM-3), B- and T-lymphocyte attenuator (BTLA), CD226, T cell Ig and ITM domain (TIGIT), CD44, Colony stimulating factor 1 receptor (CSF1R), CCR4, Killer-cell immunoglobulin-like receptor (KIR), Vascular endothelial growth factor receptor (VEGFR), Receptor Activator of Nuclear Factor κ B (RANK), V-set and immunoglobulin domain containing 8 (VSIG8), LIGHT (TNFSF14) or V domain Ig suppressor of T cell activation (VISTA); and the second immunomodulatory moiety comprises the extracellular ligand-binding sequence or ligand binding fragment thereof of transforming growth factor β receptor II (TGFβRII), programmed death 1 protein (PD-1), T cell immunoglobulin and mucin domain containing 3 (TIM-3), B- and T-lymphocyte attenuator (BTLA), CD226, T cell Ig and ITM domain (TIGIT), CD44, Colony stimulating factor 1 receptor (CSF1R), CCR4, Killer-cell immunoglobulin-like receptor (KIR), Vascular endothelial growth factor receptor (VEGFR), Receptor Activator of Nuclear Factor κ B (RANK), V-set and immunoglobulin domain containing 8 (VSIG8), LIGHT (TNFSF14) or V domain Ig suppressor of T cell activation (VISTA).

In one aspect, the targeting moiety polypeptide comprises an antigen-binding domain of an immunoglobulin, antibody, bispecific or multispecific antibody, antibody fragment, single chain variable fragment (scFv), bivalent or multivalent scFv, a ligand-binding sequence from the extracellular domain (ECD) of a receptor, or Fc-containing polypeptide. In certain aspects, the polypeptide is an antibody.

In an additional aspect, the first immunomodulatory moiety is attached to the C-terminus of a heavy chain or a light chain of the antibody; the second immunomodulatory moiety is attached to the C-terminus of a heavy chain or a light chain of the antibody and/or the second immunomodulatory moiety is attached to the first immunomodulatory moiety.

In a further aspect, the first and second immunomodulatory moiety is attached to the heavy chain or the light chain of the antibody with a linker and/or second immunomodulatory moiety is attached to the first immunomodulatory moiety with a linker. In certain aspects, the linker has the sequence of SEQ ID NO 3.

In one aspect, the first immunomodulatory moiety is attached to the heavy chain of the antibody and the second immunomodulatory moiety is attached to the light chain of the antibody.

In certain aspects, the first immunomodulatory moiety comprises the ECD or ligand binding fragment thereof of TIM3 and the second immunomodulatory moiety comprise the ECD or ligand binding fragment thereof of PD-1; the first immunomodulatory moiety comprise the ECD or ligand binding fragment thereof of TGFβRII and the second immunomodulatory moiety comprise the ECD or ligand binding fragment thereof of TIM-3; the first immunomodulatory moiety comprise the ECD or ligand binding fragment thereof of PD-1 and the second immunomodulatory moiety comprise the ECD or ligand binding fragment thereof of TIM-3; and/or the first immunomodulatory moiety comprise the ECD or ligand binding fragment thereof of TGFβRII and the second immunomodulatory moiety comprise the ECD or ligand binding fragment thereof of PD-1. In a further aspect, the targeting moiety specifically binds IL6R, CTLA-4, PD-1, PD-L1, EGFR, HER2, VEGF, or gp 120.

In one aspect, the targeting moiety comprise a polypeptide that binds CTLA-4, the first immunomodulatory moiety comprise the ECD or ligand binding fragment thereof of PD-1, and the second immunomodulatory moiety comprise the ECD or ligand binding fragment thereof of TGFβRII. In one aspect, the molecule comprises SEQ ID NO: 201 and SEQ ID NO: 202. In another aspect, the targeting moiety comprise a polypeptide that binds CTLA-4, the first immunomodulatory moiety comprise the ECD or ligand binding fragment thereof of PD-1, and the second immunomodulatory moiety comprise the ECD or ligand binding fragment thereof of TIM-3. In one aspect, the molecule comprises SEQ ID NO: 205 and SEQ ID NO: 206. In one aspect, the molecule comprises SEQ ID NO: 207 and SEQ ID NO: 208. In another aspect, the targeting moiety comprise a polypeptide that binds CTLA-4, the first immunomodulatory moiety comprise the ECD or ligand binding fragment thereof of TIM-3, and the second immunomodulatory moiety comprise the ECD or ligand binding fragment thereof of TGFβRII. In one aspect, the molecule comprises SEQ ID NO: 203 and SEQ ID NO: 204. In a further aspect, the targeting moiety polypeptide comprise ipilimumab. In a further aspect, this antibody comprise tremilimumab.

In one aspect, the targeting moiety comprise a polypeptide that binds PD-1 or PD-L1, the first immunomodulatory moiety comprise the ECD or ligand binding fragment thereof of TGFβRII, and the second immunomodulatory moiety comprise the ECD or ligand binding fragment thereof of TIM-3. In one aspect, the molecule comprises SEQ ID NO: 273 and SEQ ID NO: 274. In one aspect, the molecule comprises SEQ ID NO: 277 and SEQ ID NO: 278. In one aspect, the molecule comprises SEQ ID NO: 285 and SEQ ID NO: 286. In one aspect, the molecule comprises SEQ ID NO: 281 and SEQ ID NO: 282. In a further aspect, the targeting moiety polypeptide comprise Nivolumab or pembrolizumab. In a further aspect, this antibody comprise atezolizumab or avelumab or durvalumab.

In one aspect, the targeting moiety comprise a polypeptide that binds IL-6R, the first immunomodulatory moiety comprise the ECD or ligand binding fragment thereof of PD-1, and the second immunomodulatory moiety comprise the ECD or ligand binding fragment thereof of TGFβRII. In one aspect, the molecule comprises SEQ ID NO: 213 and SEQ ID NO: 214. In another aspect, the targeting moiety comprise a polypeptide that binds IL-6R, the first immunomodulatory moiety comprise the ECD or ligand binding fragment thereof of PD-1, and the second immunomodulatory moiety comprise the ECD or ligand binding fragment thereof of TIM-3. In one aspect, the molecule comprises SEQ ID NO: 217 and SEQ ID NO: 218. In one aspect, the molecule comprises SEQ ID NO: 219 and SEQ ID NO: 220. In another aspect, the targeting moiety comprise a polypeptide that binds IL-6R, the first immunomodulatory moiety comprise the ECD or ligand binding fragment thereof of TIM-3, and the second immunomodulatory moiety comprise the ECD or ligand binding fragment thereof of TGFβRII. In one aspect, the molecule comprises SEQ ID NO: 215 and SEQ ID NO: 216. In a further aspect, the targeting moiety polypeptide comprises an antibody that binds IL-6R. In a further aspect, this antibody comprises tocilizumab.

In one aspect, the targeting moiety comprise a polypeptide that binds VEGF, the first immunomodulatory moiety comprise the ECD or ligand binding fragment thereof of PD-1, and the second immunomodulatory moiety comprise the ECD or ligand binding fragment thereof of TGFβRII. In one aspect, the molecule comprises SEQ ID NO: 249 and SEQ ID NO: 250. In another aspect, the targeting moiety comprise a polypeptide that binds VEGF, the first immunomodulatory moiety comprise the ECD or ligand binding fragment thereof of PD-1, and the second immunomodulatory moiety comprise the ECD or ligand binding fragment thereof of TIM-3. In one aspect, the molecule comprises SEQ ID NO: 253 and SEQ ID NO: 254. In one aspect, the molecule comprises SEQ ID NO: 255 and SEQ ID NO: 256. In another aspect, the targeting moiety comprise a polypeptide that binds VEGF, the first immunomodulatory moiety comprise the ECD or ligand binding fragment thereof of TIM-3, and the second immunomodulatory moiety comprise the ECD or ligand binding fragment thereof of TGFβRII. In one aspect, the molecule comprises SEQ ID NO: 251 and SEQ ID NO: 252. In a further aspect, the targeting moiety polypeptide comprise Bevacizumab. In a further aspect, targeting moiety comprises a ligand binding ectodomain of VEGFR. In a further aspect, targeting moiety comprise aflibercept.

In one aspect, the targeting moiety comprise a polypeptide that binds EGFR, the first immunomodulatory moiety comprise the ECD or ligand binding fragment thereof of PD-1, and the second immunomodulatory moiety comprise the ECD or ligand binding fragment thereof of TGFβRII. In one aspect, the molecule comprises SEQ ID NO: 225 and SEQ ID NO: 226. In another aspect, the targeting moiety comprise a polypeptide that binds EGFR, the first immunomodulatory moiety comprise the ECD or ligand binding fragment thereof of PD-1, and the second immunomodulatory moiety comprise the ECD or ligand binding fragment thereof of TIM-3. In one aspect, the molecule comprises SEQ ID NO: 229 and SEQ ID NO: 230. In one aspect, the molecule comprises SEQ ID NO: 231 and SEQ ID NO: 232. In another aspect, the targeting moiety comprises a polypeptide that binds EGFR, the first immunomodulatory moiety comprise the ECD or ligand binding fragment thereof of TIM-3, and the second immunomodulatory moiety comprise the ECD or ligand binding fragment thereof of TGFβRII. In one aspect, the molecule comprises SEQ ID NO: 227 and SEQ ID NO: 228. In a further aspect, the targeting moiety polypeptide comprise Cetuximab or Panitumumab or Necitumumab.

In one aspect, the targeting moiety comprise a polypeptide that binds HER2/Neu, the first immunomodulatory moiety comprise the ECD or ligand binding fragment thereof of PD-1, and the second immunomodulatory moiety comprise the ECD or ligand binding fragment thereof of TGFβRII. In one aspect, the molecule comprises SEQ ID NO: 237 and SEQ ID NO: 238. In another aspect, the targeting moiety comprise a polypeptide that binds HER2, the first immunomodulatory moiety comprise the ECD or ligand binding fragment thereof of PD-1, and the second immunomodulatory moiety comprise the ECD or ligand binding fragment thereof of TIM-3. In one aspect, the molecule comprises SEQ ID NO: 243 and SEQ ID NO: 244. In one aspect, the molecule comprises SEQ ID NO: 241 and SEQ ID NO: 242. In another aspect, the targeting moiety comprise a polypeptide that binds HER2, the first immunomodulatory moiety comprise the ECD or ligand binding fragment thereof of TIM-3, and the second immunomodulatory moiety comprise the ECD or ligand binding fragment thereof of TGFβRII. In one aspect, the molecule comprises SEQ ID NO: 239 and SEQ ID NO: 240. In a further aspect, the targeting moiety polypeptide comprise Trastuzumab or Pertuzumab.

In one aspect, the targeting moiety comprise a polypeptide that binds HIV gp120, the first immunomodulatory moiety comprise the ECD or ligand binding fragment thereof of PD-1, and the second immunomodulatory moiety comprise the ECD or ligand binding fragment thereof of TGFβRII. In one aspect, the molecule comprises SEQ ID NO: 261 and SEQ ID NO: 262. In another aspect, the targeting moiety comprise a polypeptide that binds gp120, the first immunomodulatory moiety comprise the ECD or ligand binding fragment thereof of PD-1, and the second immunomodulatory moiety comprise the ECD or ligand binding fragment thereof of TIM-3. In one aspect, the molecule comprises SEQ ID NO: 265 and SEQ ID NO: 266. In one aspect, the molecule comprises SEQ ID NO: 267 and SEQ ID NO: 268. In another aspect, the targeting moiety comprise a polypeptide that binds gp120, the first immunomodulatory moiety comprise the ECD or ligand binding fragment thereof of TIM-3, and the second immunomodulatory moiety comprise the ECD or ligand binding fragment thereof of TGFβRII. In one aspect, the molecule comprises SEQ ID NO: 263 and SEQ ID NO: 264. In a further aspect, the targeting moiety polypeptide comprise Trastuzumab or Pertuzumab.

In an aspect, the molecule comprises sequences corresponding to SEQ ID NOs: 201 and 202; 203 and 204; 205 and 206; 207 and 208; 213 and 214; 215 and 216; 217 and 218; 219 and 220; 225 and 226; 227 and 228; 229 and 230; 231 and 232; 237 and 238; 239 and 240; 241 and 242 ; 243 and 244; 249 and 250; 251 and 252; 253 and 254; 255 and 256; 261 and 262; 263 and 264; 265 and 266; 267 and 268; 273 and 274; 277 and 278; 281 and 282; 285 and 286.

In a further embodiment, the present invention provides fusion proteins comprising the extracellular binding domain (ECD) or ligand binding fragment thereof of TIM-3 and the ECD or ligand binding fragment thereof of TGFRβII, PD-1, BTLA, LIGHT, CD226 or VSIG8. In one aspect, the fusion protein comprises the ECD or ligand binding fragment thereof of TIM-3 and the ECD or ligand binding fragment thereof of TGFβRII. In certain aspects, the fusion protein has the sequence of SEQ ID NO: 190. In another aspect, the fusion protein comprises the ECD or ligand binding fragment thereof of TIM-3 and the ECD or ligand binding fragment thereof of PD-1. In specific aspects, the fusion protein comprises the sequence of SEQ ID NO: 195, 196 or 197. In a further aspect, the fusion protein comprises an Fc.

The terms "fusion molecule" and "fusion protein" are used interchangeably and are meant to refer to a biologically active polypeptide, with or without a further effector molecule, usually a protein or peptide sequence covalently linked (i.e. fused) by recombinant, chemical or other suitable method. If desired, the fusion molecule can include at one or several sites a peptide linker sequence. Alternatively, the peptide linker may be used to assist in construction of the fusion molecule. Specifically preferred fusion molecules are fusion proteins. Generally, fusion molecule also can include conjugate molecules.

In a further embodiment, the present invention provides fusion proteins comprising the extracellular binding domain (ECD) or ligand binding fragment thereof of TIM-3 and the ECD or ligand binding fragment thereof of TGFRβII, PD-1, BTLA, LIGHT, CD226 or VSIG8. In one aspect, the fusion protein comprises the ECD or ligand binding fragment thereof of TIM-3 and the ECD or ligand binding fragment thereof of TGFβRII. In certain aspects, the fusion protein comprises the sequence of SEQ ID NO: 190. In another aspect, the fusion protein comprises the ECD or ligand binding fragment thereof of TIM-3 and the ECD or ligand binding fragment thereof of PD-1. In specific aspects, the fusion protein comprises the sequence of SEQ ID NO: 195, 196 or 197. In a further aspect, the fusion protein comprises an Fc.

In another embodiment, the present invention provides a fusion protein of a Fc-containing polypeptide and the ECD or ligand binding fragment thereof of TIM-3, where the TIM-3 ECD is specifically fused to the C terminus of the Fc-containing polypeptide.

In a further embodiment, the present invention provides a method of treatment comprising a fusion protein of an immunoglobulin Fc and the ECD or ligand binding fragment thereof of TIM-3, and an antibody that binds PD-1 or PD-L1. In one aspect, the structure of said fusion protein is of the form (N terminus) Fc-TIM3 (C terminus). In another aspect, the structure of said fusion protein is of the form (N terminus) TIM3-Fc (C terminus).

In one embodiment, the composition of the present invention might for example be used in combination with antibody-ligand traps. For example, the antibody-ligand traps disclosed in US8993524, US9441044, and US9850306 may be used in combination with the composition of the present invention.

In one embodiment, the present invention provides for compositions comprising the previously described molecule or fusion protein and a pharmaceutically acceptable carrier.

By "pharmaceutically acceptable" it is meant the carrier, diluent or excipient must be compatible with the other ingredients of the formulation and not deleterious to the recipient thereof.

In an additional embodiment, the present invention provides a method of treating a subject having cancer or an infectious disease comprising administering to the subject the previously described molecules, fusion proteins or compositions.

The term "treatment" is used interchangeably herein with the term "therapeutic method" and refers to both 1) therapeutic treatments or measures that cure, slow down, lessen symptoms of, and/or halt progression of a diagnosed pathologic conditions or disorder, and 2) and prophylactic/ preventative measures. Those in need of treatment may include individuals already having a particular medical disorder as well as those who may ultimately acquire the disorder (i.e., those needing preventive measures).

The terms "administration of" and or "administering" should be understood to mean providing a pharmaceutical composition in a therapeutically effective amount to the subject in need of treatment. Administration routes can be enteral, topical or parenteral. As such, administration routes include but are not limited to intracutaneous, subcutaneous, intravenous, intraperitoneal, intraarterial, intrathecal, intracapsular, intraorbital, intracardiac, intradermal, transdermal, transtracheal, subcuticular, intraarticulare, subcapsular, subarachnoid, intraspinal and intrasternal , oral, sublingual buccal, rectal, vaginal, nasal ocular administrations, as well infusion, inhalation, and nebulization. The phrases "parenteral administration" and "administered parenterally" as used herein means modes of administration other than enteral and topical administration.

The terms "therapeutically effective amount", "effective dose," "therapeutically effective dose", "effective amount," or the like refer to that amount of the subject compound that will elicit the biological or medical response of a tissue, system, animal or human that is being sought by the researcher, veterinarian, medical doctor or other clinician. Generally, the response is either amelioration of symptoms in a patient or a desired biological outcome. The effective amount can be determined as described herein.

The term "subject" as used herein refers to any individual or patient to which the subject methods are performed. Generally the subject is human, although as will be appreciated by those in the art, the subject may be an animal. Thus other animals, including vertebrate such as rodents (including mice, rats, hamsters and guinea pigs), cats, dogs, rabbits, farm animals including cows, horses, goats, sheep, pigs, chickens, etc., and primates (including monkeys, chimpanzees, orangutans and gorillas) are included within the definition of subject.

The term "cancer" refers to a group diseases characterized by abnormal and uncontrolled cell proliferation starting at one site (primary site) with the potential to invade and to spread to others sites (secondary sites, metastases) which differentiate cancer (malignant tumor) from benign tumor. Virtually all the organs can be affected, leading to more than 100 types of cancer that can affect humans. Cancers can result from many causes including genetic predisposition, viral infection, exposure to ionizing radiation, exposure environmental pollutant, tobacco and or alcohol use, obesity, poor diet, lack of physical activity or any combination thereof.

As used herein, "neoplasm" or "tumor" including grammatical variations thereof, means new and abnormal growth of tissue, which may be benign or cancerous. In a related aspect, the neoplasm is indicative of a neoplastic disease or disorder, including but not limited, to various cancers. For example, such cancers can include prostate, pancreatic, biliary, colon, rectal, liver, kidney, lung, testicular, breast, ovarian, pancreatic, brain, and head and neck cancers, melanoma, sarcoma, multiple myeloma, leukemia, lymphoma, and the like.

Exemplary cancers described by the National Cancer Institute include: Acute Lymphoblastic Leukemia, Adult; Acute Lymphoblastic Leukemia, Childhood; Acute Myeloid Leukemia, Adult; Adrenocortical Carcinoma; Adrenocortical Carcinoma, Childhood; AIDS-Related Lymphoma; AIDS-Related Malignancies; Anal Cancer; Astrocytoma, Childhood Cerebellar; Astrocytoma, Childhood Cerebral; Bile Duct Cancer, Extrahepatic; Bladder Cancer; Bladder Cancer, Childhood; Bone Cancer, Osteosarcoma/Malignant Fibrous Histiocytoma; Brain Stem Glioma, Childhood; Brain Tumor, Adult; Brain Tumor, Brain Stem Glioma, Childhood; Brain Tumor, Cerebellar Astrocytoma, Childhood; Brain Tumor, Cerebral Astrocytoma/Malignant Glioma, Childhood; Brain Tumor, Ependymoma, Childhood; Brain Tumor, Medulloblastoma, Childhood; Brain Tumor, Supratentorial Primitive Neuroectodermal Tumors, Childhood; Brain Tumor, Visual Pathway and Hypothalamic Glioma, Childhood; Brain Tumor, Childhood (Other); Breast Cancer; Breast Cancer and Pregnancy; Breast Cancer, Childhood; Breast Cancer, Male; Bronchial Adenomas/Carcinoids, Childhood: Carcinoid Tumor, Childhood; Carcinoid Tumor, Gastrointestinal; Carcinoma, Adrenocortical; Carcinoma, Islet Cell; Carcinoma of Unknown Primary; Central Nervous System Lymphoma, Primary; Cerebellar Astrocytoma, Childhood; Cerebral Astrocytoma/Malignant Glioma, Childhood; Cervical Cancer; Childhood Cancers; Chronic Lymphocytic Leukemia; Chronic Myelogenous Leukemia; Chronic Myeloproliferative Disorders; Clear Cell Sarcoma of Tendon Sheaths; Colon Cancer; Colorectal Cancer, Childhood; Cutaneous T-Cell Lymphoma; Endometrial Cancer; Ependymoma, Childhood; Epithelial Cancer, Ovarian; Esophageal Cancer; Esophageal Cancer, Childhood; Ewing's Family of Tumors; Extracranial Germ Cell Tumor, Childhood; Extragonadal Germ Cell Tumor; Extrahepatic Bile Duct Cancer; Eye Cancer, Intraocular Melanoma; Eye Cancer, Retinoblastoma; Gallbladder Cancer; Gastric (Stomach) Cancer; Gastric (Stomach) Cancer, Childhood; Gastrointestinal Carcinoid Tumor; Germ Cell Tumor, Extracranial, Childhood; Germ Cell Tumor, Extragonadal; Germ Cell Tumor, Ovarian; Gestational Trophoblastic Tumor; Glioma. Childhood Brain Stem; Glioma. Childhood Visual Pathway and Hypothalamic; Hairy Cell Leukemia; Head and Neck Cancer; Hepatocellular (Liver) Cancer, Adult (Primary); Hepatocellular (Liver) Cancer, Childhood (Primary); Hodgkin's Lymphoma, Adult; Hodgkin's Lymphoma, Childhood; Hodgkin's Lymphoma During Pregnancy; Hypopharyngeal Cancer; Hypothalamic and Visual Pathway Glioma, Childhood; Intraocular Melanoma; Islet Cell Carcinoma (Endocrine Pancreas); Kaposi's Sarcoma; Kidney Cancer; Laryngeal Cancer; Laryngeal Cancer, Childhood; Leukemia, Acute Lymphoblastic, Adult; Leukemia, Acute Lymphoblastic, Childhood; Leukemia, Acute Myeloid, Adult; Leukemia, Acute Myeloid, Childhood; Leukemia, Chronic Lymphocytic; Leukemia, Chronic Myelogenous; Leukemia, Hairy Cell; Lip and Oral Cavity Cancer; Liver Cancer, Adult (Primary); Liver Cancer, Childhood (Primary); Lung Cancer, Non-Small Cell; Lung Cancer, Small Cell; Lymphoblastic Leukemia, Adult Acute; Lymphoblastic Leukemia, Childhood Acute; Lymphocytic Leukemia, Chronic; Lymphoma, AIDS-Related; Lymphoma, Central Nervous System (Primary); Lymphoma, Cutaneous T-Cell; Lymphoma, Hodgkin's, Adult; Lymphoma, Hodgkin's; Childhood; Lymphoma, Hodgkin's During Pregnancy; Lymphoma, Non-Hodgkin's, Adult; Lymphoma, Non-Hodgkin's, Childhood; Lymphoma, Non-Hodgkin's During Pregnancy; Lymphoma, Primary Central Nervous System; Macroglobulinemia, Waldenstrom's; Male Breast Cancer; Malignant Mesothelioma, Adult; Malignant Mesothelioma, Childhood; Malignant Thymoma; Medulloblastoma, Childhood; Melanoma; Melanoma, Intraocular; Merkel Cell Carcinoma; Mesothelioma, Malignant; Metastatic Squamous Neck Cancer with Occult Primary; Multiple Endocrine Neoplasia Syndrome, Childhood; Multiple Myeloma/Plasma Cell Neoplasm; Mycosis Fungoides; Myelodysplasia Syndromes; Myelogenous Leukemia, Chronic; Myeloid Leukemia, Childhood Acute; Myeloma, Multiple; Myeloproliferative Disorders, Chronic; Nasal Cavity and Paranasal Sinus Cancer; Nasopharyngeal Cancer; Nasopharyngeal Cancer, Childhood; Neuroblastoma; Non-Hodgkin's Lymphoma, Adult; Non-Hodgkin's Lymphoma, Childhood; Non-Hodgkin's Lymphoma During Pregnancy; Non-Small Cell Lung Cancer; Oral Cancer, Childhood; Oral Cavity and Lip Cancer; Oropharyngeal Cancer; Osteosarcoma/Malignant Fibrous Histiocytoma of Bone; Ovarian Cancer, Childhood; Ovarian Epithelial Cancer; Ovarian Germ Cell Tumor; Ovarian Low Malignant Potential Tumor; Pancreatic Cancer; Pancreatic Cancer, Childhood', Pancreatic Cancer, Islet Cell; Paranasal Sinus and Nasal Cavity Cancer; Parathyroid Cancer; Penile Cancer; Pheochromocytoma; Pineal and Supratentorial Primitive Neuroectodermal Tumors, Childhood; Pituitary Tumor; Plasma Cell Neoplasm/Multiple Myeloma; Pleuropulmonary Blastoma; Pregnancy and Breast Cancer; Pregnancy and Hodgkin's Lymphoma; Pregnancy and Non-Hodgkin's Lymphoma; Primary Central Nervous System Lymphoma; Primary Liver Cancer, Adult; Primary Liver Cancer, Childhood; Prostate Cancer; Rectal Cancer; Renal Cell (Kidney) Cancer; Renal Cell Cancer, Childhood; Renal Pelvis and Ureter, Transitional Cell Cancer; Retinoblastoma; Rhabdomyosarcoma, Childhood; Salivary Gland Cancer; Salivary Gland'Cancer, Childhood; Sarcoma, Ewing's Family of Tumors; Sarcoma, Kaposi's; Sarcoma (OsteosarcomaVMalignant Fibrous Histiocytoma of Bone; Sarcoma, Rhabdomyosarcoma, Childhood; Sarcoma, Soft Tissue, Adult; Sarcoma, Soft Tissue, Childhood; Sezary Syndrome; Skin Cancer; Skin Cancer, Childhood; Skin Cancer (Melanoma); Skin Carcinoma, Merkel Cell; Small Cell Lung Cancer; Small Intestine Cancer; Soft Tissue Sarcoma, Adult; Soft Tissue Sarcoma, Childhood; Squamous Neck Cancer with Occult Primary, Metastatic; Stomach (Gastric) Cancer; Stomach (Gastric) Cancer, Childhood; Supratentorial Primitive Neuroectodermal Tumors, Childhood; T-Cell Lymphoma, Cutaneous; Testicular Cancer; Thymoma, Childhood; Thymoma, Malignant; Thyroid Cancer; Thyroid Cancer, Childhood; Transitional Cell Cancer of the Renal Pelvis and Ureter; Trophoblastic Tumor, Gestational; Unknown Primary Site, Cancer of, Childhood; Unusual Cancers of Childhood; Ureter and Renal Pelvis, Transitional Cell Cancer; Urethral Cancer; Uterine Sarcoma; Vaginal Cancer; Visual Pathway and Hypothalamic Glioma, Childhood; Vulvar Cancer; Waldenstrom's Macro globulinemia; and Wilms' Tumor.

As used herein the term "infectious disease" refers to a disease resulting from an infection. An infection is the invasion of an organism's body tissues by disease-causing agents, their multiplication, and the reaction of host tissues to the infectious agents and the toxins they produce. Infectious agents include viruses, viroids, prions, bacteria, nematodes such as parasitic roundworms and pinworms, arthropods such as ticks, mites, fleas, and lice, fungi such as ringworm, and other macroparasites such as tapeworms and other helminths. A short-term infection is an acute infection, accordingly and as used herein "chronic infection" refers to a long-term infection or to a persistent infection.

Infectious diseases include: Acute Flaccid Myelitis (AFM); Anaplasmosis; Anthrax; Babesiosis; Botulism; Brucellosis; Burkholderia mallei (Glanders); Burkholderia pseudomallei (Melioidosis); Campylobacteriosis (Campylobacter); Carbapenem-resistant Infection (CRE/CRPA); Chancroid; Chikungunya Virus Infection (Chikungunya); Chlamydia; Ciguatera; Clostridium Difficile Infection; Clostridium Perfringens (Epsilon Toxin); Coccidioidomycosis fungal infection (Valley fever); Creutzfeldt-Jacob Disease, transmissible spongiform encephalopathy (CJD); Cryptosporidiosis (Crypto); Cyclosporiasis; Dengue , 1,2,3,4 (Dengue Fever); Diphtheria; E. coli infection (E.Coli); Eastern Equine Encephalitis (EEE); Ebola Hemorrhagic Fever (Ebola); Ehrlichiosis; Encephalitis; Arboviral or parainfectious; Enterovirus Infection , Non-Polio (Non-Polio Enterovirus); Enterovirus Infection; D68 (EV-D68); Giardiasis (Giardia); Gonococcal Infection (Gonorrhea); Granuloma inguinale; Haemophilus Influenza disease; Type B (Hib or H-flu); Hantavirus Pulmonary Syndrome (HPS); Hemolytic Uremic Syndrome (HUS); Hepatitis A (Hep A); Hepatitis B (Hep B); Hepatitis C (Hep C); Hepatitis D (Hep D); Hepatitis E (Hep E); Herpes; Herpes Zoster; zoster VZV (Shingles); Histoplasmosis infection (Histoplasmosis); Human Immunodeficiency Virus/AIDS (HIV/AIDS); Human Papillomarivus (HPV); Influenza (Flu); Lead Poisoning; Legionellosis (Legionnaires Disease); Leprosy (Hansens Disease); Leptospirosis; Listeriosis (Listeria); Lyme Disease; Lymphogranuloma venereum infection (LVG); Malaria; Measles; Meningitis; Viral (Meningitis, viral); Meningococcal Disease; Bacterial (Meningitis, bacterial); Middle East Respiratory Syndrome Coronavirus (MERS-CoV); Mumps; Norovirus; Paralytic Shellfish Poisoning (Paralytic Shellfish Poisoning, Ciguatera); Pediculosis (Lice, Head and Body Lice); Pelvic Inflammatory Disease (PID); Pertussis (Whooping Cough); Plague; Bubonic, Septicemic, Pneumonic (Plague); Pneumococcal Disease (Pneumonia); Poliomyelitis (Polio); Powassan; Psittacosis; Pthiriasis (Crabs; Pubic Lice Infestation); Pustular Rash diseases (Small pox, monkeypox, cowpox); Q-Fever; Rabies; Ricin Poisoning; Rickettsiosis (Rocky Mountain Spotted Fever); Rubella, Including congenital (German Measles); Salmonellosis gastroenteritis (Salmonella); Scabies Infestation (Scabies); Scombroid; Severe Acute Respiratory Syndrome (SARS); Shigellosis gastroenteritis (Shigella); Smallpox; Staphyloccal Infection, Methicillin-resistant (MRSA); Staphylococcal Food Poisoning; Enterotoxin - B Poisoning (Staph Food Poisoning); Staphylococcal Infection; Vancomycin Intermediate (VISA); Staphylococcal Infection; Vancomycin Resistant (VRSA); Streptococcal Disease; Group A (invasive) (Strep A); Streptococcal Disease; Group B (Strep-B); Streptococcal Toxic-Shock Syndrome; STSS; Toxic Shock (STSS, TSS); Syphilis, primary, secondary, early latent, late latent, congenital; Tetanus Infection; tetani (Lock Jaw); Trichonosis Infection (Trichinosis); Tuberculosis (TB); Tuberculosis (Latent) (LTBI); Tularemia (Rabbit fever); Typhoid Fever; Group D; Typhus; Vaginosis , bacterial (Yeast Infection); Varicella (Chickenpox); Vibrio cholerae (Cholera); Vibriosis (Vibrio); Viral Hemorrhagic Fever (Ebola, Lassa, Marburg);West Nile Virus; Yellow Fever; Yersenia (Yersinia); Zika Virus Infection (Zika); Acinetobacter infections; Actinomycosis; African sleeping sickness (African trypanosomiasis); Amebiasis Anaplasmosis; Anisakiasis; Arcanobacterium haemolyticum infection; Argentine Teagan fever; Ascariasis; Aspergillosis; Astrovirus infection; Bacillus cereus infection; Bacterial pneumonia; Bacterial vaginosis; Balantidiasis; Bartonellosis; Baylisascaris infection; BK virus infection; Black piedra; Blastocystosis; Bolivian hemorrhagic fever; Brazilian hemorrhagic fever; Bubonic plague; Buruli ulcer; Calicivirus infection; Campylobacteriosis; Candidiasis; Capillariasis Intestinal disease; Carrion's disease Bartonella bacilliformis; Cat-scratch disease Bartonella henselae; Cellulitis usually; Chagas Disease (American trypanosomiasis); Chancroid; Chickenpox; Chlamydophila pneumoniae infection; Chromoblastomycosis; Chytridiomycosis; Clonorchiasis; Colorado tick fever (CTF); Common cold; Crimean-Congo hemorrhagic fever (CCHF); Cryptosporidiosis; Cutaneous larva migrans (CLM); Cyclosporiasis; Cysticercosis; Cytomegalovirus infection; Desmodesmus infection ; Dientamoebiasis; Diphtheria; Diphyllobothriasis; Dracunculiasis; Echinococcosis; Ehrlichiosis; Enterobiasis (Pinworm infection); Enterococcus infection; Enterovirus infection; Epidemic typhus Rickettsia prowazekii ; Erythema infectiosum (Fifth disease); Exanthem subitum (Sixth disease); Fasciolasis; Fasciolopsiasis; Fatal familial insomnia (FFI) ; Filariasis; Free-living amebic infection multiple ; Fusobacterium infection; Gas gangrene (Clostridial myonecrosis); Gerstmann-Sträussler-Scheinker syndrome (GSS) ; Giardiasis; Glanders; Gnathostomiasis; Granuloma inguinale (Donovanosis); streptococcal infection; Haemophilus influenzae infection; Hand, foot and mouth disease (HFMD); Hantavirus Pulmonary Syndrome (HPS) Sin Nombre virus; Heartland virus disease Heartland virus; Helicobacter pylori infection; Hemolytic-uremic syndrome (HUS) E; Hemorrhagic fever with renal syndrome (HFRS); Histoplasmosis; Hookworm infection; Human bocavirus infection; Human ewingii; Human granulocytic anaplasmosis (HGA); Human metapneumovirus infection; Human monocytic ehrlichiosis; Human papillomavirus (HPV) infection; Human parainfluenza virus infection; Hymenolepiasis; Epstein-Barr virus infectious mononucleosis (Mono); Influenza (flu); Isosporiasis; Kawasaki disease; Keratitis multiple; Kingella kingae infection; Kuru PRNP; Lassa fever; Legionellosis (Pontiac fever); Leishmaniasis; Leprosy; Leptospirosis Leptospira species; Lymphatic filariasis (Elephantiasis); Lymphocytic choriomeningitis; Middle East respiratory syndrome (MERS); Melioidosis (Whitmore's disease); Meningitis multiple; Meningococcal disease; Metagonimiasis; Microsporidiosis; Molluscum contagiosum (MC); Monkeypox; Mumps; Murine typhus (Endemic typhus); Mycoplasma pneumonia; Mycetoma (disambiguation); Myiasis parasitic; Neonatal conjunctivitis (Ophthalmia neonatorum); Norovirus (children and babies) Norovirus; Nocardiosis; Onchocerciasis (River blindness); Opisthorchiasis; Paracoccidioidomycosis (South American blastomycosis); Paragonimiasis; Pasteurellosis; Pediculosis capitis (Head lice); Pediculosis corporis (Body lice); Pediculosis pubis (Pubic lice, Crab lice); Pelvic inflammatory disease (PID); Pertussis (Whooping cough); Plague; Pneumococcal infection; Pneumocystis pneumonia (PCP); Pneumonia multiple; Poliomyelitis; Prevotella infection; Primary amoebic meningoencephalitis (PAM); Progressive multifocal leukoencephalopathy; Psittacosis; Q fever; Rabies; Relapsing fever; Respiratory syncytial virus infection Respiratory syncytial virus (RSV); Rhinosporidiosis; Rhinovirus infection; Rickettsial infection; Rickettsialpox; Rift Valley fever (RVF); Rocky Mountain spotted fever (RMSF); Rotavirus infection; Rubella; Salmonellosis; SARS (Severe Acute Respiratory Syndrome); Scabies; Scarlet fever Group A S; Sepsis multiple; Shigellosis (Bacillary dysentery); Shingles (Herpes zoster); Smallpox (Variola); Sporotrichosis; Staphylococcal food poisoning; Staphylococcal infection; Strongyloidiasis; Syphilis; Taeniasis; Tetanus (Lockjaw); Tinea barbae (Barber's itch); Tinea capitis (Ringworm of the Scalp); Tinea corporis (Ringworm of the Body); Tinea cruris (Jock itch); Tinea manum (Ringworm of the Hand); Tinea nigra; Tinea pedis (Athlete's foot); Tinea unguium (Onychomycosis); Tinea versicolor (Pityriasis versicolor); Toxocariasis (Ocular Larva Migrans (OLM)); Toxocariasis (Visceral Larva Migrans (VLM)); Toxoplasmosis; Trachoma; Trichinosis; Trichomoniasis; Trichuriasis (Whipworm infection); Tuberculosis; Tularemia; Typhoid fever; Typhus fever; Ureaplasma urealyticum infection; Valley fever; Venezuelan equine encephalitis V; Venezuelan hemorrhagic fever; Vibrio vulnificus infection; Vibrio parahaemolyticus; Viral pneumonia; West Nile Fever; White piedra (Tinea blanca); Yersinia pseudotuberculosis infection; Yersiniosis; Zygomycosis.

In some aspects administration can be in combination with one or more additional therapeutic agents. The phrases "combination therapy", "combined with" and the like refer to the use of more than one medication or treatment simultaneously to increase the response. The composition of the present invention might for example be used in combination with other drugs or treatment such as immunotherapies or chemotherapies. Such therapies can be administered prior to, simultaneously with, or following administration of the composition of the present invention.

The term "chemotherapeutic agent" as used herein refers to any therapeutic agent used to treat cancer. Examples of chemotherapeutic agents include, but are not limited to, Actinomycin, Azacitidine, Azathioprine, Bleomycin, Bortezomib, Carboplatin, Capecitabine, Cisplatin, Chlorambucil, Cyclophosphamide, Cytarabine, Daunorubicin, Docetaxel, Doxifluridine, Doxorubicin, Epirubicin, Epothilone, Etoposide, Fiuorouracil, Gemcitabine, Hydroxyurea, Idarubicin, Imatinib, lrinotecan, Mechlorethamine, Mercaptopurine, Methotrexate, Mitoxantrone, Oxaliplatin, Paclitaxel, Pemetrexed, Teniposide, Tioguanine, Topotecan, Valrubicin, Vinblastine, Vincristine, Vindesine, Vinorelbine, panitumamab, Erbitux (cetuximab), matuzumab, IMC-IIF 8, TheraCIM hR3, denosumab, Avastin (bevacizumab), Humira (adalimumab), Herceptin (trastuzumab), Remicade (infliximab), rituximab, Synagis (palivizumab), Mylotarg (gemtuzumab oxogamicin), Raptiva (efalizumab), Tysabri (natalizumab), Zenapax (dacliximab), NeutroSpec (Technetium (99mTc) fanolesomab), tocilizumab, ProstaScint (Indium-Ill labeled Capromab Pendetide), Bexxar (tositumomab), Zevalin (ibritumomab tiuxetan (IDEC-Y2B8) conjugated to yttrium 90), Xolair (omalizumab), MabThera (Rituximab), ReoPro (abciximab), MabCampath (alemtuzumab), Simulect (basiliximab), LeukoScan (sulesomab), CEA-Scan (arcitumomab), Verluma (nofetumomab), Panorex (Edrecolomab), alemtuzumab, CDP 870, natalizumab Gilotrif (afatinib), Lynparza (olaparib), Perjeta (pertuzumab), Otdivo (nivolumab), Bosulif (bosutinib), Cabometyx (cabozantinib), Ogivri (trastuzumab-dkst), Sutent (sunitinib malate), Adcetris (brentuximab vedotin), Alecensa (alectinib), Calquence (acalabrutinib), Yescarta (ciloleucel), Verzenio (abemaciclib), Keytruda (pembrolizumab), Aliqopa (copanlisib), Nerlynx (neratinib), Imfinzi (durvalumab), Darzalex (daratumumab), Tecentriq (atezolizumab), and Tarceva (erlotinib). Examples of immunotherapeutic agent include, but are not limited to, interleukins (Il-2, Il-7, Il-12), cytokines (Interferons, G-CSF, imiquimod), chemokines (CCL3, CCl26, CXCL7), immunomodulatory imide drugs (thalidomide and its analogues).

Examples of antibiotics, include but are not limited to, Amikacin; Gentamicin; Kanamycin; Neomycin; Netilmicin; Tobramycin; Paromomycin; Streptomycin; Spectinomycin; Geldanamycin; Herbimycin; Rifaximin; Loracarbef; Ertapenem; Doripenem; Imipenem/Cilastatin; Meropenem; Cefadroxil; Cefazolin; Cefalexin; Cefaclor; Cefprozil; Cefuroxime; Cefixime; Cefdinir; Cefditoren; Cefoperazone; Cefotaxime; Cefpodoxime; Ceftazidime; Ceftibuten; Ceftriaxone; Cefepime; Ceftaroline fosamil; Ceftobiprole; Teicoplanin; Vancomycin; Telavancin; Dalbavancin; Oritavancin; Lincosamides; Clindamycin; Lincomycin; Lipopeptide; Daptomycin; Macrolides; Azithromycin; Clarithromycin; Erythromycin; Roxithromycin; Telithromycin; Spiramycin; Monobactams; Aztreonam; Nitrofurans; Furazolidone; Nitrofurantoin; Oxazolidinones; Linezolid; Posizolid; Radezolid; Torezolid; Penicillins; Amoxicillin; Ampicillin; Azlocillin; Dicloxacillin; Flucloxacillin; Mezlocillin; Methicillin; Nafcillin; Oxacillin; Penicillin G; Penicillin V; Piperacillin; Penicillin G; Temocillin; Ticarcillin; Penicillin combinations; Amoxicillin/clavulanate; Ampicillin/sulbactam; Piperacillin/tazobactam; Ticarcillin/clavulanate; Polypeptides; Bacitracin; Colistin; Polymyxin B; Ciprofloxacin; Enoxacin; Gatifloxacin; Gemifloxacin; Levofloxacin; Lomefloxacin; Moxifloxacin; Nadifloxacin; Nalidixic acid; Norfloxacin; Ofloxacin; Trovafloxacin; Grepafloxacin; Sparfloxacin; Temafloxacin; Sulfonamides; Mafenide; Sulfacetamide; Sulfadiazine; Silver sulfadiazine; Sulfadimethoxine; Sulfamethizole; Sulfamethoxazole; Sulfanilimide; Sulfasalazine; Sulfisoxazole; Trimethoprim-Sulfamethoxazole; Sulfonamidochrysoidine; Tetracyclines; Demeclocycline; Doxycycline; Metacycline; Minocycline; Oxytetracycline; Tetracycline; Clofazimine; Dapsone; Capreomycin; Cycloserine; Ethambutol; Ethionamide; Isoniazid; Pyrazinamide; Rifampicin; Rifabutin; Rifapentine; Streptomycin; Arsphenamine; Chloramphenicol;Fosfomycin; Fusidic acid; Metronidazole; Mupirocin; Platensimycin; Quinupristin/Dalfopristin; Thiamphenicol; Tigecycline; Tinidazole and Trimethoprim.

In another embodiment, the present invention provides a method of inducing or promoting immune tolerance. The method includes administering to a subject in need thereof one or more molecule of the invention.

The following examples are provided to further illustrate the embodiments of the present invention, but are not intended to limit the scope of the invention. While they are typical of those that might be used, other procedures, methodologies, or techniques known to those skilled in the art may alternatively be used.

### EXAMPLES

Despite compelling antitumor activity of antibodies targeting the programmed death 1 (PD-1): programmed death ligand 1 (PD-L1) immune checkpoint, both de novo and adaptive resistance to these therapies is frequently observed. Targeting the PD-1/PD-L1 pathway may not be sufficient to break dysfunction in exhausted T cells, as complex cross-regulation exists between PD-1 and other checkpoint inhibitors to restrain anti-tumor T cell immunity, and in certain cases PD-1 blockade may be followed by development of adaptive resistance. The TIM-3/CEACAM1 axis is a key determinant of de novo and adaptive resistance to PD-1/PD-L1 therapy.

TIM-3/CEACAM1 exert multipronged suppression of T cells and NK cells via: (i) heterophilic TIM-3/CEACAM-1 interactions (cis CEACAM-1/TIM-3 heterodimerization; trans CEACAM1 heterodimerization with TIM-3); (ii) homophilic CEACAM-1/CEACAM-1 interactions (cis CEACAM-1 dimerization; trans CEACAM1-induced cis CEACAM-1 dimerization). Accordingly, tumor-infiltrating CD8+ T lymphocytes co-expressing TIM-3 and CEACAM1 exhibit the most exhausted phenotype in both mouse models and patients with cancer.

Since PD-1, TIM-3, and CEACAM-1 entrain independent and cooperative mechanisms of immune tolerance and T cell exhaustion in the tumor microenvironment, simultaneous blockade of these checkpoints is required to unleash potent antitumor innate and adaptive antitumor immune responses. Besides elevated co- expression of all three T cell inhibitory receptors (TCIRs: PD-1, TIM-3, CEACAM-1) on tumor-infiltrating immune cells, tumor cells also co-express multiple ligands that engage these TCIRs to suppress tumor- reactive T cells (PD-L1, CEACAM-1, Galectin-9). This poses the therapeutic challenge of disrupting multiple autocrine/paracrine cis and trans TCIR/ligand interactions in the TME that act in concert to induce immune tolerance (PD-1/PD-L1; Tim-3/CEACAM-1; CEACAM-1/CEACAM-1). This therapeutic challenge was addressed by inventing novel bifunctional antibody-ligand traps (Y-traps): (i) a-PDL1-TIM3ecd comprising an antibody targeting PD-L1 fused to a TIM-3 IgV ectodomain sequence; (ii) a-PD1-TIM3ecd comprising an antibody targeting PD-1 fused to a TIM-3 IgV ectodomain sequence.

Mechanism of Action for aPDL1-TIM3ecd and aPD1-TIM3ecd: aPDL1-TIM3ecd and aPD1-TIM3ecd bind PD-1 or PD-L1 on activated T cells and tumor cells, and simultaneously decorate them with a TIM-3ecd decoy which effectively competes with native cellular TIM-3 for binding the FG-CC' cleft interface of CEACAM-1. As such these antibody-ligand traps not only disable PD-L1/PD-1 interactions, but simultaneously disrupt both cis and trans interactions involving Tim-3/CEACAM-1 as well as CEACAM-1/CEACAM-1 in the tumor microenvironment (TME). These studies demonstrate that a-PDL1-TIM3ecd and a-PD1-TIM3ecd are significantly more effective than either a-PDL1 (atezolizumab) or a-PD-1 (pembrolizumab), in promoting IFNγ expression and counteracting exhaustion of co-stimulated T cells in vitro. Most significantly, it was found that in vivo treatment of tumor-bearing humanized mice with these antibody-ligand traps results in a striking increase in the number of tumor-infiltrating CD4+ and CD8+ T cells, and is significantly more effective in inhibiting tumor progression compared to equivalent treatment with a-PDL1 (atezolizumab), a-PD-1 (pembrolizumab), a-Tim-3 antibody, or even a combination of a-PDL1 and a-Tim-3. These data demonstrate that heterophilic and homophilic Tim-3/CEACAM-1 interactions in the tumor microenvironment limit the antitumor efficacy of PD-1/PD-L1 inhibitors, and that a-PDL1-TIM3ecd and a-PD1-TIM3ecd provide a more effective immunotherapeutic strategy to counteract or reverse T cell exhaustion by simultaneously disabling the PD-1/PD-L1 checkpoint and counteracting both components of the Tim-3/CEACAM-1 axis.

Figure 1. Schematic representation of autocrine/paracrine receptor-ligand interactions that inhibit T cell activation and induce immune tolerance and T cell exhaustion. Autocrine/paracrine TGF□ induces expression of FOXP3, the signature transcription factor of the Treg lineage. FOXP3 induces expression of CTLA-4, a T cell inhibitory receptor which restrains T cell costimulation by interfering with B7-CD28 interaction. Tregs express Galectin-9, a ligand that engages TIM-3 and triggers exhaustion or apoptosis of effector T cells by the TIM-3/CEACAM-1 co-inhibitory axis. Engagement of PD-1 by PD-L1 promotes TGF□-induced expression of FOXP3 to induce and maintain Tregs, and cooperates with TIM-3/CEACAM-1 signaling to induce T cell exhaustion and death.

Figure 2. a-PDL1-TIM3ecd and a-PD1-TIM3ecd counteract immune tolerance and T cell exhaustion by simultaneously interrupting PD-L1/PD-1 signaling and the TIM-3/CEACAM-1 co-inhibitory axis.

(A) Schematic representation of the structure and targets of a-PDL1-TIM3ecd and a-PD1-TIM3ecd. a-PD1-TIM3ecd comprises: (i) Heavy chain of a human a-PD-1 antibody fused at the C-terminus to a ligand-binding IgV ectodomain sequence of TIM-3 via a flexible linker peptide, (GGGGS)3 (SEQ ID NO: 53) and (ii) Light chain of a human a-PD-1 antibody (SEQ ID NO: 187 ). a-PDL1-TIM3ecd comprises: (i) Heavy chain of a human a-PD-L1 antibody fused at the C-terminus to a ligand-binding IgV ectodomain sequence of TIM-3 via a flexible linker peptide, (GGGGS)3 (SEQ ID NO: 51 ) and (ii) Light chain of a human a-PD-L1 antibody (SEQ ID NO: 185). (B) Schematic representation of the targets and mechanisms by which a-PDL1-TIM3ecd and a-PD1-TIM3ecd disrupt autocrine/paracrine receptor-ligand interactions that inhibit T cell activation and induce T cell exhaustion. Autocrine/paracrine TGFβ induces expression of FOXP3, the signature transcription factor of the Treg lineage. FOXP3 induces expression of CTLA-4, a T cell inhibitory receptor which restrains T cell costimulation by interfering with B7-CD28 interaction. Tregs express Galectin-9, a ligand that engages TIM-3 and triggers exhaustion or apoptosis of effector T cells by the TIM-3/CEACAM-1 co-inhibitory axis. Engagement of PD-1 by PD-L1 promotes TGFβ-induced expression of FOXP3 to induce and maintain Tregs, and cooperates with TIM-3/CEACAM-1 signaling to induce T cell exhaustion and death. *a*-PDL1-TIM3*ecd* and *a*-PD1-TIM3*ecd* disable PD-1/PD-L1 signaling and simultaneously disrupt *cis*/*trans* interactions involving the TIM-3 and CEACAM-1 co-inhibitory pathways.(C) Schematic representation of the mechanism by which *a*-PDL1-TIM3*ecd* and *a*-PD1-TIM3*ecd* simultaneously counteract multipronged suppression of T cells via the PD-L1/PD-1 and TIM-3/CEACAM-1 co-inhibitory axes. *a-*PDL1-TIM3ecd and *a*-PD1-TIM3ecd interrupt PD-L1/PD-1 interaction and simultaneously disrupt: (i) heterophilic TIM-3/CEACAM-1 interactions (*cis* CEACAM-1/TIM-3 heterodimerization; *trans* CEACAM1 heterodimerization with TIM-3); (ii) homophilic CEACAM-1/CEACAM-1 interactions (*cis* CEACAM-1 dimerization; *trans* CEACAM1-induced cis CEACAM-1 dimerization).

Figure 3. Characterization and target-binding ability of a-PDL1-TIM3ecd and a-PD1-TIM3ecd. (A) SDS-PAGE under reducing (R) and non-reducing (NR) conditions was used to compare the full-length (FL), heavy chain (HC) and light chain (LC) of a-PD1-TIM3ecd and a-PD1 antibody (nivolumab). SDS-PAGE confirmed the expected higher molecular weight of the heavy chain of a-PD1-TIM3ecd compared to the heavy chain of a-PD1 antibody (Top panel). SDS-PAGE under reducing (R) and non-reducing (NR) conditions was used to compare the full-length (FL), heavy chain (HC) and light chain (LC) of a-PDL1-TIM3ecd and a-PDL1 antibody (atezolizumab). SDS-PAGE confirmed the expected higher molecular weight of the heavy chain of a-PDL1-TIM3ecd compared to the heavy chain of a-PDL1 antibody (Bottom panel). (B) ELISA showing binding of a-PD1-TIM3 to rhPD-1 (Top panel): Biotinylated rhPD-1 (0-250 ng/ml) was added to plates coated with a-PD1-TIM3ecd (1µg/ml). ELISA showing binding of a-PDL1-TIM3 to rhPD-L1 (Bottom panel): Biotinylated rhPD-L1 (0-250 ng/ml) was added to plates coated with either a-PDL1-TIM3 (1µg/ml) or a-PDL1 antibody (atezolizumab)(positive control). (C) a-PD1-TIM3ecd and a-PDL1-TIM3ecd bind recombinant human (rh) CEACAM-1 in vitro. rhCEACAM-1 (5 µg) was incubated (overnight, 4oC) in the presence or absence of a-PD1-TIM3ecd, a-PDL1-TIM3ecd, a-gp120-TIM3ecd, a-PD-L1 mAb (atezolizumab), or a-PD1 mAb (nivolumab)(10 µg each), followed by incubation with protein A-agarose beads (10 µl) for 6h, and then immunoprecipitated with protein A-agarose beads. The precipitated complexes were washed with immunoprecipitation buffer, re-suspended in NuPAGE sample buffer with reducing agent, boiled, resolved on a Bis-Tris Criterion XT Gel system and transferred to a PVDF membrane. Membranes were immunoblotted with primary antibodies [CEACAM1 (D1P4T); TIM3 (D5D5R^{™})] overnight, washed, incubated with secondary antibodies and visualized by Amersham ECL Western Blotting Detection Reagents. Immunoblot analyses detected rhCEACAM-1 in a-PD1-TIM3ecd, a-PDL1-TIM3ecd, and a-gp120-TIM3ecd protein A-pulled down immunoprecipitates, but not in immunoprecipitates of a-PD-L1 mAb (atezolizumab), or a-PD1 mAb (nivolumab). (D) a-PD1-TIM3ecd and a-PDL1-TIM3ecd bind CEACAM-1 on co-stimulated human T cells. Human PBMC were co-stimulated with anti-CD3/anti-CD28 beads in the presence of a-PD1-TIM3ecd, a-PDL1-TIM3ecd, a-PD-L1 mAb (atezolizumab), or a-PD1 mAb (nivolumab) for 6d. Cell lysates were immunoprecipitated with protein A-agarose beads (10 □l) for 6h. The precipitated complexes were washed with immunoprecipitation buffer, re-suspended in NuPAGE sample buffer with reducing agent, boiled, resolved on a Bis-Tris Criterion XT Gel system and transferred to a PVDF membrane. Membranes were immunoblotted with primary antibodies [CEACAM1 (D1P4T); TIM3 (D5D5R^{™})] overnight, washed, incubated with secondary antibodies and visualized by Amersham ECL Western Blotting Detection Reagents. Immunoblot analyses with the CEACAM-1 antibody detected CEACAM-1 in protein A-immunoprecipitates of a-PD1-TIM3ecd and a-PDL1-TIM3ecd treated cell lysates, but not in immunoprecipitates of a-PD-L1 mAb (atezolizumab), or a-PD1 mAb (nivolumab) treated cell lysates. Immunoblot analyses with the TIM-3ecd antibody detected the heavy chain of a-PD1-TIM3ecd or a-PDL1-TIM3ecd in protein A-immunoprecipitates of a-PD1-TIM3ecd and a-PDL1-TIM3ecd treated cell lysates, but not in immunoprecipitates of a-PD-L1 mAb (atezolizumab), or a-PD1 mAb (nivolumab) treated cell lysates.

Figure 4. a-PD1-TIM3ecd and a-PDL1-TIM3ecd are significantly more effective than a-PD-1 mAb or a-PD-L1 mAb in promoting IFN-γ expression and counteracting exhaustion of co-stimulated T cells. (A) Human PBMCs were stimulated with anti-CD3/anti-CD28 beads (5 µg/ml) and rhIL-2, in the presence or absence of a-PD1-TIM3ecd, a-PDL1-TIM3ecd, a-PD-L1 mAb (atezolizumab), a-PD1 mAb (nivolumab), a-human TIM3 Ab (F38-2E2), or the combination of a-PD1 mAb and a-TIM3 Ab (5 µg/ml each) for 3-9d. Interferon-γ (IFN-γ) in cell supernatants at each indicated time (3d, 6d, and 9d) was quantified by ELISA (Data represent mean ± SEM of 3 in vitro replicates for each group). ELISA demonstrated that a-PDL1-TIM3ecd and a-PD1-TIM3ecd are significantly more effective in promoting IFNγ expression and counteracting exhaustion of co-stimulated T cells in vitro compared to either a-PDL1 (atezolizumab), a-PD-1 (pembrolizumab), a-TIM3 Ab, or even the combination of a-PD1 mAb and a-TIM3 Ab. (B) Human PBMCs were stimulated with anti-CD3/anti-CD28 beads (5µl/ml) and rhIL-2, in the presence or absence of a-PD1-TIM3ecd, a-PDL1-TIM3ecd, a-PD-L1 mAb (atezolizumab), a-PD1 mAb (nivolumab), a-human TIM3 Ab (F38-2E2), or the combination of a-PD1 mAb and a-TIM3 Ab (5 µg/ml each) for 16h or 40h, and IFN-γ in cell supernatants was quantified by ELISA (mean ± SEM of 3 in vitro replicates/group). a-PDL1-TIM3ecd is significantly more effective in promoting IFNγ expression by co-stimulated T cells in vitro compared to either a-PDL1 (atezolizumab), a-PD-1 (pembrolizumab), a-TIM3 Ab, or even the combination of a-PD1 mAb and a-TIM3 Ab [data represent the fold-change in IFNγ expression in each antibody-treated group compared to co-stimulation without antibody)]. (C) a-PD1-TIM3ecd counteracts TGFβ mediated suppression of IFNγ expression in co-stimulated T cells. Human PBMCs were stimulated with anti-CD3/anti-CD28 and rhIL-2 in the presence or absence of rhTGFβ1 (5 ng/ml) with or without either a-PDL1-TIM3 (5 µg/ml) for 16h or 40h, and Interferon-γ (IFN-γ) in cell supernatants was quantified by ELISA (Data represent mean ± SEM of 3 in vitro replicates for each group).

Figure 5. a-PD1-TIM3ecd inhibits tumor growth more effectively than a-PD-1, a-TIM-3 or the combination of a-TIM-3 and a-PD1. (A) NSG mice (NOD/Shi-scid IL-2rgnull)(4-8 weeks) were reconstituted with adoptive transfer of 3x106 human peripheral blood mononuclear cells (PBMCs) [that were costimulated with anti-CD3/anti-CD28 beads (5□l/ml) and rhIL-2 in the presence of the following specified treatments for 8 days (5 □g/ml each): a-PD1-TIM3ecd, a-PD1 mAb (nivolumab), a-human TIM3 Ab (F38-2E2), combination of a-PD1 mAb and a-TIM3 Ab, or vehicle alone. Reconstituted mice were injected subcutaneously with 2x106 D-MUT1 human colorectal cancer cells, and each group was treated in vivo (5 mg/kg i.p. every 5 days) with the same treatment used for in vitro treatment of PBMCs used for adoptive transfer, as indicated: a-PD1-TIM3ecd; a-PD1 (nivolumab); a-TIM-3 (F38-2E2); combination of a-PD1 and a-TIM-3; vehicle alone (untreated control) [≥5 mice/group]. Tumor size was measured blinded to the treatment group and tumor volume was calculated using the formula (length × width × height). In vivo tumor growth curves (mean + SEM) are shown. p values were derived using unpaired, two-sided t-test. The p value (p<0.02, Student's unpaired t-test) denotes significant difference between a-PD1-TIM3ecd and each other treatment group. (B) Immune deficient NSG mice (NOD/Shi-scid IL-2rgnull)(4-8 weeks) were irradiated at 200 cGy, and immune reconstituted by adoptive transfer of HLA A2-matched human CD34+.hematopoietic cells. Reconstituted humanized mice were injected subcutaneously with human Triple Negative Breast Cancer (TNBC) HLA A2+ tumor cells (MDA-MB-231-Luc)(1×106 in matrigel; mammary fat pad). At 3d following tumor inoculation, mice were randomized and treated with either vehicle alone (untreated control) or the following antibodies (5 mg/kg i.p. weekly): a-PD1-TIM3ecd, a-PD1 mAb (nivolumab), a-human TIM3 Ab (F38-2E2), combination of a-PD1 mAb and a-TIM3 Ab [>_5 mice/group]. Tumor size was measured blinded to the treatment group and tumor volume was calculated using the formula (length × width × height). In vivo tumor growth curves (mean + SEM) are shown. p values were derived using unpaired, two-sided t-test. The p value (p<0.02, Student's unpaired t-test) denotes significant difference between a-PD1-TIM3ecd and each other treatment group.

Figure 6. a-PDL1-TIM3ecd inhibits tumor growth more effectively than a-PD-L1, a-PD-1, a-TIM-3 or the combination of a-PD-L1 and a-TIM-3. Immune deficient NSG mice (NOD/Shi-scid IL-2rgnull)(4-8 weeks) were irradiated at 200 cGy, and immune reconstituted by adoptive transfer of HLA A2 human CD34+.hematopoietic cells. Reconstituted humanized mice were injected subcutaneously with human Triple Negative Breast Cancer (TNBC) HLA A2+ tumor cells (MDA-MB-231-Luc)(1x106 in matrigel; mammary fat pad). At 3d following tumor inoculation, mice were randomized and treated with either vehicle alone (untreated control) or the following antibodies (5 mg/kg i.p. weekly): a-PDL1-TIM3ecd, a-PD1 mAb (atezolizumab), a-PD1 mAb (pembrolizumab), a-human TIM3 Ab (F38-2E2), combination of a-PD-L1 mAb and a-TIM3 Ab [>_5 mice/group]. (A) Tumor size was measured blinded to the treatment group and tumor volume was calculated using the formula (length × width × height). In vivo tumor growth curves (mean + SEM) are shown. p values were derived using unpaired, two-sided t-test. The p value (p<0.02, Student's unpaired t-test) denotes significant difference between a-PDL1-TIM3ecd and each other treatment group. (B) Bioluminescence assay of primary tumors in untreated controls or the indicated treatment groups at 7 and 27 d after tumor cell inoculation. (C) Quantification of T cell density in tumors: Tumor volume and flow cytometry was used to assess the absolute number of CD4+ and CD8+ T cells per mm3 of tumor.

Figure 7. Schematic design of multifunctional antibody-ligand traps of this invention comprising a targeting antibody that binds a target epitope, ligand-binding IgV ectodomain sequence of TIM-3 (TIM-3ecd), and ligand-binding ectodomain sequence of PD-1 (PD-1ecd). The multifunctional antibody-ligand traps can bind a target epitope (via the antigen-binding CDR), bind PD-1 ligands, PD-L1 and PD-L2 (via the PD-1ecd) and TIM-3 ligands (CEACAM-1)(via the TIM-3ecd). (A) Schematic design of multifunctional antibody-ligand traps of this invention comprising a targeting antibody, wherein the heavy chain of the antibody is fused at the C-terminus to a ligand-binding IgV ectodomain sequence of TIM-3 (TIM-3ecd: SEQ ID NO: 2) via a linker, and the light chain of the antibody is fused at the C-terminus to a ligand-binding ectodomain sequence of PD-1 (PD-1ecd: SEQ ID NO: 7) via a linker. In one aspect, the linker is a peptide, (GGGGS)n (SEQ ID NO: 3). (B) Schematic design of multifunctional antibody-ligand traps of this invention comprising a targeting antibody, wherein the heavy chain of the antibody is fused at the C-terminus to a ligand-binding ectodomain sequence of PD-1 (PD-1ecd: SEQ ID NO: 7) via a linker peptide, and the light chain of the antibody is fused at the C-terminus to a ligand-binding ectodomain sequence of TIM-3 (TIM-3ecd: SEQ ID NO: 2 ) via a linker peptide. In one aspect, the linker is a peptide, (GGGGS)n (SEQ ID NO: 3). In various embodiments, the targeting antibody of the multifunctional antibody-ligand traps of this invention binds one of the following molecules: Cytotoxic T lymphocyte associated antigen-4 (CTLA-4, CD152), B7-H3 (CD276), V domain Ig suppressor of T cell activation (VISTA), VSIG8, B and T lymphocyte attenuator (BTLA), Herpesvirus Entry Mediator (HVEM), CD160, T cell Ig and ITM domain (TIGIT), CD226, CD96, Lymphocyte activation gene-3 (LAG-3), transforming growth factor β (TGF-β), transforming growth factor β receptor (TGFβR), 4-1BB (CD137), Inducible T-Cell Costimulator (ICOS), OX-40 (CD134), glucocorticoid-induced TNFR-related protein (GITR), IL6R, IL23R, IL17R, IL-6, IL-23, IL-17, CD39, CD73, CCR4, CCR5, CXCR4, IL12R, CD4, CD25, CD3, epidermal growth factor receptor (EGFR, EGFR1, ErbB-1, HER1), ErbB-2 (HER2/neu), ErbB-3/HER3, ErbB-4/HER4, EGFR ligand, Vascular endothelial growth factor (VEGF), VEGFR (VEGFR1, VEGFR2), RANK ligand (RANKL), RANK, gp120.

Figure 8. Schematic design of multifunctional antibody-ligand traps of this invention comprising a targeting antibody that binds CTLA-4, ligand-binding IgV ectodomain sequence of TIM-3 (TIM-3ecd), and ligand-binding ectodomain sequence of PD-1 (PD-1ecd). The multifunctional antibody-ligand traps can bind CTLA-4 (via the antigen-binding CDR), bind PD-1 ligands, PD-L1 and PD-L2 (via the PD-1ecd) and TIM-3 ligands (CEACAM-1)(via the TIM-3ecd). (A) Schematic design of multifunctional antibody-ligand traps of this invention comprising a targeting antibody that binds CTLA-4, wherein the heavy chain of the antibody is fused at the C-terminus to a ligand-binding IgV ectodomain sequence of TIM-3 via a linker peptide (SEQ ID NO: 207), and the light chain of the antibody is fused at the C-terminus to a ligand-binding ectodomain sequence of PD-1 via a linker peptide (SEQ ID NO: 208). In one aspect, the linker is a peptide, (GGGGS)n (SEQ ID NO: 3).(B) Schematic design of multifunctional antibody-ligand traps of this invention comprising a targeting antibody that binds CTLA-4, wherein the heavy chain of the antibody is fused at the C-terminus to a ligand-binding ectodomain sequence of PD-1 SEQ ID NO:205) via a linker peptide, and the light chain of the antibody is fused at the C-terminus to a ligand-binding ectodomain sequence of TIM-3 (SEQ ID NO: 206) via a linker peptide. In one aspect, the linker is a peptide, (GGGGS)n (SEQ ID NO: 3).

Figure 9. Schematic design of multifunctional antibody-ligand traps of this invention comprising a targeting antibody that binds a target epitope, ligand-binding IgV ectodomain sequence of TIM-3 (TIM-3ecd), and ligand-binding ectodomain sequence of TGFβRII (TGFβRIIecd). The multifunctional antibody-ligand traps can bind a target epitope (via the antigen-binding CDR), bind TGFβ (via the TGFβRIIecd) and TIM-3 ligands (CEACAM-1)(via the TIM-3ecd). In one embodiment, the multifunctional antibody-ligand traps of this invention comprise a targeting antibody, wherein the heavy chain of the antibody is fused at the C-terminus to a ligand-binding ectodomain sequence of TGFβRII (TGF□RIIecd) via a linker peptide (SEQ ID NO: 6), and the light chain of the antibody is fused at the C-terminus to a ligand-binding IgV ectodomain sequence of TIM-3 (TIM-3ecd: SEQ ID NO: 2) via a linker peptide. In one aspect, the linker is a peptide, (GGGGS)n (SEQ ID NO: 3). In various embodiments, the targeting antibody of the multifunctional antibody-ligand traps of this invention binds one of the following molecules: Cytotoxic T lymphocyte associated antigen-4 (CTLA-4, CD152), Programmed Death-1 protein (PD-1), Programmed death ligand (PD-L1), Programmed death ligand (PD-L2), B7-H3 (CD276) ,V domain Ig suppressor of T cell activation (VISTA), VSIG8, B and T lymphocyte attenuator (BTLA), Herpesvirus Entry Mediator (HVEM), CD160, T cell Ig and ITM domain (TIGIT), CD226, CD96, Lymphocyte activation gene-3 (LAG-3) ,4-1BB (CD137), Inducible T-Cell Costimulator (ICOS), OX-40 (CD134), glucocorticoid-induced TNFR-related protein (GITR), IL6R, IL23R, IL17R, IL-6, IL-23, IL-17, CD39, CD73, CCR4, CCR5, CXCR4, IL12R, CD4, CD25, CD3, epidermal growth factor receptor (EGFR, EGFR1, ErbB-1, HER1), ErbB-2 (HER2/neu), ErbB-3/HER3, ErbB-4/HER4, EGFR ligand, Vascular endothelial growth factor (VEGF), VEGFR (VEGFR1, VEGFR2), RANK ligand (RANKL), RANK.

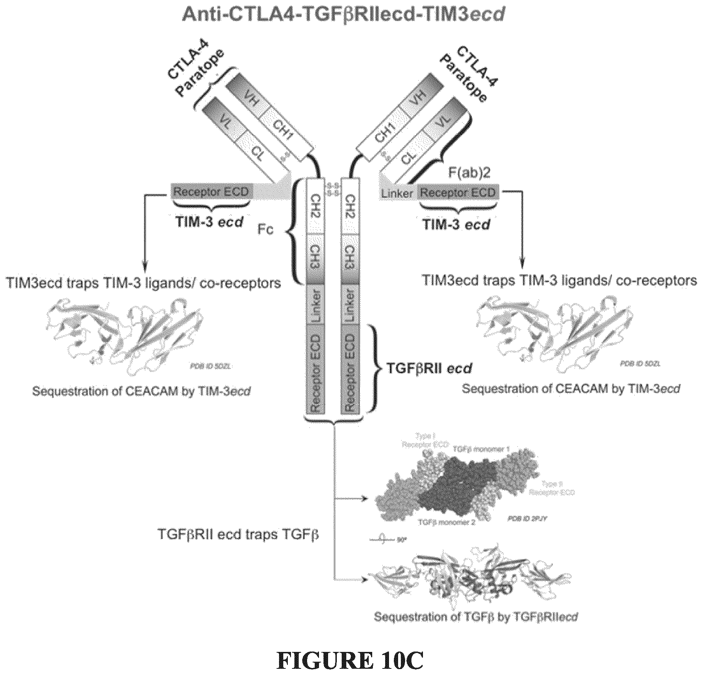

Figure 10. Schematic design of multifunctional antibody-ligand traps of this invention comprising a targeting antibody that binds a T cell inhibitory receptor or ligand, ligand-binding IgV ectodomain sequence of TIM-3 (TIM-3ecd), and ligand-binding ectodomain sequence of TGFβRII (TGFβRIIecd). (A) Schematic design of multifunctional antibody-ligand traps of this invention comprising a targeting antibody that binds PD-1, ligand-binding IgV ectodomain sequence of TIM-3 (TIM-3ecd), and ligand-binding ectodomain sequence of TGFβRII (TGFβRIIecd). In one embodiment, the multifunctional antibody-ligand traps of this invention comprise a targeting antibody that binds PD-1, wherein the heavy chain of the antibody is fused at the C-terminus to a ligand-binding ectodomain sequence of TGFβRII (TGFβRIIecd)(SEQ ID NO: 6) via a linker peptide, and the light chain of the antibody is fused at the C-terminus to a ligand-binding IgV ectodomain sequence of TIM-3 (TIM-3ecd: SEQ ID NO: 2) via a linker peptide. In one aspect, the linker is a peptide, (GGGGS)n (SEQ ID NO: 3). In one example, multifunctional antibody-ligand trap comprises a heavy chain corresponding to SEQ ID NO: 273 and a light chain corresponding to SEQ ID NO: 274. In another example, multifunctional antibody-ligand trap comprises a heavy chain corresponding to SEQ ID NO: 277 and a light chain corresponding to SEQ ID NO: 278. (B) Schematic design of multifunctional antibody-ligand traps of this invention comprising a targeting antibody that binds PD-L1, ligand-binding IgV ectodomain sequence of TIM-3 (TIM-3ecd), and ligand-binding ectodomain sequence of TGFβRII (TGFβRIIecd). In one embodiment, the multifunctional antibody-ligand traps of this invention comprise a targeting antibody that binds PD-L1, wherein the heavy chain of the antibody is fused at the C-terminus to a ligand-binding ectodomain sequence of TGFβRII (TGFβRIIecd)(SEQ ID NO: 6) via a linker peptide, and the light chain of the antibody is fused at the C-terminus to a ligand-binding IgV ectodomain sequence of TIM-3 (TIM-3ecd: SEQ ID NO: 2 ) via a linker peptide. In one aspect, the linker is a peptide, (GGGGS)n (SEQ ID NO: 3 ). In one example, multifunctional antibody-ligand trap comprises a heavy chain corresponding to SEQ ID NO: 285 and a light chain corresponding to SEQ ID NO: 285 . In another example, multifunctional antibody-ligand trap comprises a heavy chain corresponding to SEQ ID NO: 281 and a light chain corresponding to SEQ ID NO: 282. (C) Schematic design of multifunctional antibody-ligand traps of this invention comprising a targeting antibody that binds CTLA-4, ligand-binding IgV ectodomain sequence of TIM-3 (TIM-3ecd), and ligand-binding ectodomain sequence of TGFβRII (TGFβRIIecd). In one embodiment, the multifunctional antibody-ligand traps of this invention comprise a targeting antibody that binds CTLA-4, wherein the heavy chain of the antibody is fused at the C-terminus to a ligand-binding ectodomain sequence of TGFβRII (TGFβRIIecd)(SEQ ID NO: 6 ) via a linker peptide, and the light chain of the antibody is fused at the C-terminus to a ligand-binding IgV ectodomain sequence of TIM-3 (TIM-3ecd: SEQ ID NO: 2 ) via a linker peptide. In one aspect, the linker is a peptide, (GGGGS)n (SEQ ID NO: 3). In one example, multifunctional antibody-ligand trap comprises a heavy chain corresponding to SEQ ID NO: 203 and a light chain corresponding to SEQ ID NO: 204 .

Figure 11. Schematic design of multifunctional antibody-ligand traps of this invention comprising a targeting antibody that binds a target epitope, ligand-binding ectodomain sequence of PD-1 (PD-1ecd), and ligand-binding ectodomain sequence of TGFβRII (TGFβRIIecd). The multifunctional antibody-ligand traps can bind a target epitope (via the antigen-binding CDR), bind TGFβ (via the TGFβRIIecd) and PD-1 ligands, PD-L1 and PD-L2 (via the PD-1ecd). In one embodiment, the multifunctional antibody-ligand traps of this invention comprise a targeting antibody, wherein the heavy chain of the antibody is fused at the C-terminus to a ligand-binding ectodomain sequence of TGFβRII (TGFβRIIecd)(SEQ ID NO: 6) via a linker peptide, and the light chain of the antibody is fused at the C-terminus to ligand-binding ectodomain sequence of PD-1 (PD1ecd: SEQ ID NO: 7) via a linker peptide. In one aspect, the linker is a peptide, (GGGGS)n (SEQ ID NO: 3). In various embodiments, the targeting antibody of the multifunctional antibody-ligand traps of this invention binds one of the following molecules: Cytotoxic T lymphocyte associated antigen-4 (CTLA-4, CD152), T-cell immunoglobulin and mucin-domain containing-3 (TIM-3), CEACAM-1, Carcinoembryonic Antigen (CEA),V domain Ig suppressor of T cell activation (VISTA), VSIG8, B and T lymphocyte attenuator (BTLA), Herpesvirus Entry Mediator (HVEM), CD160, T cell Ig and ITM domain (TIGIT), CD226, CD96, Lymphocyte activation gene-3 (LAG-3), 4-1BB (CD137), Inducible T-Cell Costimulator (ICOS), OX-40 (CD134), glucocorticoid-induced TNFR-related protein (GITR), IL6R, IL23R, IL17R, IL-6, IL-23, IL-17, CD39, CD73, CCR4, CCR5, CXCR4, IL12R, CD4, CD25, CD3, epidermal growth factor receptor (EGFR, EGFR1, ErbB-1, HER1), ErbB-2 (HER2/neu), ErbB-3/HER3, ErbB-4/HER4, EGFR ligand, Vascular endothelial growth factor (VEGF), VEGFR, RANK ligand (RANKL), RANK.

Figure 12. In various embodiments, multifunctional antibody-ligand traps of this invention comprises a targeting antibody that binds a T cell inhibitory receptor or ligand, ligand-binding ectodomain sequence of PD-1 (PD-1ecd), and ligand-binding ectodomain sequence of TGFβRII (TGFβRIIecd). In one embodiment, the multifunctional antibody-ligand traps of this invention comprise a targeting antibody that binds CTLA-4, wherein the heavy chain of the antibody is fused at the C-terminus to a ligand-binding ectodomain sequence of TGFβRII (TGFβRIIecd)(SEQ ID NO: 203) via a linker peptide, and the light chain of the antibody is fused at the C-terminus to a ligand-binding ectodomain sequence of PD-1 (PD-1ecd: SEQ ID NO: 202) via a linker peptide. In one aspect, the linker is a peptide, (GGGGS)n (SEQ ID NO: 3). In one example, multifunctional antibody-ligand trap comprises a heavy chain corresponding to SEQ ID NO: 202 and a light chain corresponding to SEQ ID NO: 203.

### Design and characterization of antibody-ligand traps (Y-traps).

The cDNA for the antibody heavy chain and the cDNA for the antibody light chain were gene synthesized and subsequently cloned into separate plasmids (pEvi3; evitria AG, Switzerland) under the control of a mammalian promoter and polyadenylation signal. Plasmid DNA was amplified in E. coli and DNA was purified using anion exchange kits for low endotoxin plasmid DNA preparation. DNA concentration was determined by measuring the absorption at a wavelength of 260 nm. Correctness of the sequences was verified with Sanger sequencing (with up to two sequencing reactions per plasmid depending on the size of the cDNA.) The plasmid DNAs for heavy and light chain were subsequently co-transfected into suspension-adapted CHO K1 cells (originally received from ATCC and adapted to serum-free growth in suspension culture at evitria). The seed was grown in eviGrow medium, a chemically defined, animal-component free, serum-free medium. Cells were transfected with eviFect (evitria AG, Switzerland), and the CHO cells were cultured in eviMake2 (evitria AG, Switzerland), a serum-free, animal-component free medium. Production was terminated once viability reached 75%, which occurred at day 8 after transfection. Supernatant was harvested by centrifugation and subsequent filtration (0.2 um filter). The antibody was purified using MabSelectTM SureTM. Protein identification of the purified antibody from CHO-K1 cell supernatants was performed by liquid chromatography FTFT tandem mass spectrometry (LCMS/MS) to confirm the amino acid sequence of the heavy chain (HC) and light chain (LC)(e.g. Figure 2A. a-PDL1-TIM3ecd and a-PD1-TIM3ecd). SDS-PAGE under reducing and non-reducing conditions was used to compare the full-length, heavy chain and light chain of antibody-ligand traps and their corresponding parent antibodies [e.g. Figure 3A. a-PDL1-TIM3ecd, a-PD1-TIM3ecd, a-PDL1 mAb (atezolizumab) and a-PD1 mAb (nivolumab).

Target binding ability of antibody-ligand traps.
(i) Binding to PD-1 ligand (ELISA): Biotinylated rhPD-L1 (0-250 ng/ml) was added to ELISA plates coated with either antibody-ligand trap (1µg/ml) or a-PDL1 antibody (atezolizumab)(positive control)(1µg/ml), and detected by HRP-Avidin (R&D systems). Each experiment was performed twice, with triplicate wells for each indicated condition. ELISA showing the ability of a-PDL1-TIM3 (1µg/ml) or *a*-PDL1 antibody (atezolizumab)(positive control) (1µg/ml) to bind rhPD-L1 is shown in Figure 3A. This assay is also used to evaluate the binding of any antibody-ligand traps comprising a ligand-binding sequence of PD-1 ectodomain (PD-1ecd) to biotinylated rhPD-L1 and rh PD-L2.
(ii) Binding to PD-1 (ELISA): Biotinylated rhPD-1 (0-250 ng/ml) was added to plates coated with either antibody-ligand trap (1µg/ml) or *a*-PD1 antibody (nivolumab or pembrolizumab)(positive control) (1µg/ml), and detected by HRP-Avidin (R&D systems). Each experiment was performed twice, with triplicate wells for each indicated condition. ELISA showing binding of *a*-PD1-TIM3ecd to rhPD-1 is shown in Figure 3A. This assay is also used to evaluate the binding of any antibody-ligand trap comprising a PD-1 targeting antibody.
(iii) Binding to TIM-3 ligand/co-inhibitory receptor (CEACAM-1) *in vitro.* rhCEACAM-1 (5 µg) was incubated (overnight, 4°C) in the presence or absence of antibody-ligand trap comprising a ligand-binding sequence of TIM-3 ectodomain (TIM3ecd)(antibody-TIM3ecd) or *a*-gp120-TIM3ecd (positive control)(10 µg each), followed by incubation with protein A-agarose beads (10 µl) for 6h, and then immunoprecipitated with protein A-agarose beads. The precipitated complexes were washed with immunoprecipitation buffer, re-suspended in NuPAGE sample buffer with reducing agent, boiled, resolved on a Bis-Tris Criterion XT Gel system and transferred to a PVDF membrane. Membranes were immunoblotted with primary antibodies [CEACAM1 (D1P4T); TIM3 (D5D5R^{™})] overnight, washed, incubated with secondary antibodies and visualized by Amersham ECL Western Blotting Detection Reagents. Binding of *a*-PD1-TIM3ecd and a-PDL1-TIM3ecd to recombinant human (rh) CEACAM-1 *in vitro* is shown in Figure 2C. Immunoblot analyses detected rhCEACAM-1 in a-PD1-TIM3ecd, *a*-PDL1-TIM3ecd, and *a*-gp120-TIM3ecd protein A-pulled down immunoprecipitates, but not in immunoprecipitates of a-PD-L1 mAb (atezolizumab), or a-PD1 mAb (nivolumab)(Figure 2C). This assay is also used to evaluate the binding of any antibody-ligand trap comprising a ligand-binding sequence of a ligand-binding sequence of TIM-3 ectodomain(TIM-3ecd) to rh CEACAM-1.
(iv) Binding to TIM-3 ligand/co-inhibitory receptor (CEACAM-1) on co-stimulated human T cells. Human PBMC were co-stimulated with anti-CD3/anti-CD28 beads in ImmunoCult medium (STEMCELL technologies) in the presence or absence of antibody-ligand trap comprising a ligand-binding sequence of TIM3ecd (antibody-TIM3ecd) or *a*-gp120-TIM3ecd (positive control)(10 µg each) for 6d. Cell lysates were immunoprecipitated with protein A-agarose beads (10 µl) for 6h. The precipitated complexes were washed with immunoprecipitation buffer, re-suspended in NuPAGE sample buffer with reducing agent, boiled, resolved on a Bis-Tris Criterion XT Gel system and transferred to a PVDF membrane. Membranes were immunoblotted with primary antibodies [CEACAM1 (D1P4T); TIM3 (D5D5R^{™})] overnight, washed, incubated with secondary antibodies and visualized by Amersham ECL Western Blotting Detection Reagents. Binding of *a*-PD1-TIM3ecd and *a*-PDL1-TIM3ecd to CEACAM-1 on co-stimulated human T cells is shown in Figure 2D. Immunoblot analyses with the CEACAM-1 antibody detected CEACAM-1 in protein A-immunoprecipitates of *a*-PD1-TIM3ecd and *a*-PDL1-TIM3ecd treated cell lysates, but not in immunoprecipitates of *a*-PD-L1 mAb (atezolizumab), or *a*-PD1 mAb (nivolumab) treated cell lysates. Immunoblot analyses with the TIM-3ecd antibody detected the heavy chain of *a*-PD1-TIM3ecd or *a*-PDL1-TIM3ecd in protein A-immunoprecipitates of *a*-PD1-TIM3ecd and *a*-PDL1-TIM3ecd treated cell lysates, but not in immunoprecipitates of *a*-PD-L1 mAb (atezolizumab), or *a*-PD1 mAb (nivolumab) treated cell lysates. This assay is also used to evaluate the binding of any antibody-ligand trap comprising a ligand-binding sequence of TIM-3 ectodomain (TIM-3ecd) to CEACAM-1 on co-stimulated human T cells.
(v) Binding to TGFβ (ELISA). The ability of antibody-ligand traps comprising a ligand-binding sequence of TGFβRII ectodomain (TGFβRII)(antibody-TGFβRIIecd) to bind TGFβwas evaluated by ELISA, wherein recombinant human TGFβ (rhTGFβ1; 0-2000 pg/ml) was added to plates coated with antibody-TGFβRII (1 βg/ml), which was detected by biotinylated anti-TGFβ1 and HRP-Avidin (R&D systems). Plates coated with non-specific IgG-TGFβRII and rhTGFβRII-Fc served as positive controls to analyze the binding ability of the test samples to TGFβ.

Standard ELISA is used to assess the multifunctional ability of any antibody-ligand trap to bind the primary antibody target and also capture the ligands targeted by the fused cognate receptor ectodomain sequence (Receptor ecd). In each case, ELISA plates will be coated with antibody-ligand trap (1 µg/ml), followed by addition of biotinylated-target molecule (0-1000 ng/ml), and detection with HRP-Avidin (R&D systems).

### Ability of antibody-ligand traps to promote IFNγ expression and counteract exhaustion of co-stimulated T cells:

(**A**) Human PBMCs were stimulated with anti-CD3/anti-CD28 beads (5µl/ml) and rhIL-2, in the presence or absence of *a*-PD1-TIM3ecd, *a*-PDL1-TIM3ecd, *a*-PD-L1 mAb (atezolizumab), *a*-PD1 mAb (nivolumab), *a*-human TIM3 Ab (F38-2E2), or the combination of *a*-PD1 mAb and *a*-TIM3 Ab (5 □g/ml each) for 3-9d. Interferon-γ (IFN-γ) in cell supernatants at each indicated time (3d, 6d, and 9d) was quantified by ELISA (Data represent mean ± SEM of 3 *in vitro* replicates for each group). ELISA demonstrated that *a-*PDL1-TIM3*ecd* and *a*-PD1-TIM3*ecd* are significantly more effective in promoting IFNγ expression and counteracting exhaustion of co-stimulated T cells *in vitro* compared to either *a*-PDL1 (atezolizumab), a-PD-1 (pembrolizumab), *a*-TIM3 Ab, or even the combination of *a*-PD1 mAb and a-TIM3 Ab.
(**B**) Human PBMCs were stimulated with anti-CD3/anti-CD28 beads (5µl/ml) and rhIL-2, in the presence or absence of *a*-PD1-TIM3ecd, *a*-PDL1-TIM3ecd, *a*-PD-L1 mAb (atezolizumab), *a*-PD1 mAb (nivolumab), *a*-human TIM3 Ab (F38-2E2), or the combination of *a*-PD1 mAb and *a*-TIM3 Ab (5 µg/ml each) for 16h or 40h, and IFN-γ in cell supernatants was quantified by ELISA (mean ± SEM of 3 *in vitro* replicates/group). *a-*PDL1-TIM3*ecd* is significantly more effective in promoting IFNγ expression by co-stimulated T cells *in vitro* compared to either *a*-PDL1 (atezolizumab), a-PD-1 (pembrolizumab), *a*-TIM3 Ab, or even the combination of *a*-PD1 mAb and *a*-TIM3 Ab [data represent the fold-change in IFNγ expression in each antibody-treated group compared to co-stimulation without antibody].
(**C**) *a*-PD1-TIM3ecd counteracts TGFβ mediated suppression of IFNγ expression in co-stimulated T cells. Human PBMCs were stimulated with anti-CD3/anti-CD28 and rhIL-2 in the presence or absence of rhTGFβ1 (5 ng/ml) with or without either *a*-PDL1-TIM3 (5 µg/ml) for 16h or 40h, and Interferon-γ (IFN-γ) in cell supernatants was quantified by ELISA (Data represent mean ± SEM of 3 *in vitro* replicates for each group).

*Treatment of humanized mice bearing human tumor-xenografts.* Immune deficient NSG mice (*NOD*/*Shi-scid IL-2rg^{null}*)(4-8 week old) were reconstituted with adoptive transfer of human peripheral blood mononuclear cells (PBMCs) or human bone marrow CD34⁺ cells from a normal donor. Humanized mice were inoculated with human tumor cells . Following tumor inoculation, mice were allocated to groups using blinded block randomization and treated (5 mg/kg i.p. every 5d) with either vehicle alone (untreated control) or the following antibodies: *a*-PDL1-TIM3ecd; *a*-PDL1-TIM3ecd, *a*-PDL1 (atezolizumab); *a*-TIM-3; *a*-PD-1 (pembrolizumab); combination of *a*-PDL1 and *a*-TIM-3; combination of *a*-PDL1 and *a*-TIM-3.

*Bioluminescent imaging of primary tumors.* Tumor burden in mice bearing MDA-MB-231-Luc was assessed by visualization of *in vivo* luciferase activity using a Xenogen IVIS Spectrum system. Images were acquired at 10 min post injection of 50 mg/kg i.p. dose of luciferin. To detect metastases, the lower portion of each animal was shielded before re-imaging to minimize bioluminescence from the primary tumor. Lungs were harvested and imaged *ex vivo* to confirm *in vivo* observations. Photon flux was used to quantify the differences in tumor burden between treatment groups.

*Evaluation of therapeutic efficacy:* Tumor size was measured weekly for 6 weeks [length, width, height], and tumor volume calculated. Tumor growth inhibition (%TGI) values was calculated using initial (i) and final (f) tumor volumes for the treatment (T) and untreated control (C) groups by the formula: %TGI = [1-(Tf Ti)/(Cf Ci)] x100.

### Immunophenotype analysis of T cells - Flow cytometry:

Tumors, draining lymph node (DLN), and bone marrow (BM) were harvested from mice in untreated control and treatment groups (at the end of therapy) for immunophenotype analysis of T cells, as follows: *(a) Regulatory T cells:* Tregs in tumor-infiltrating, DLN, or BM T cells isolated from mice in each group were measured by flow cytometry. Cells were stained extracellularly with anti-human CD4-PE, anti-human CD25-PE-CY^{™}5, and anti-human CD127-FITC (BD Biosciences). The cells were then be permeabilized and stained intracellularly with anti-human FOXP3-APC or the corresponding isotype control IgG1-APC (eBioscience), and then analyzed by flow cytometry to quantify Tregs (CD4⁺CD25⁺CD127^{low}FOXP3⁺ cells). *(b) Memory T cells:* CD8+ and CD4+ T cells with a central memory (CD45RO+ CD62L+) or effector memory (CD45RO+ CD62L-) immunophenotype were measured in BM, DLN, and tumor-infiltrating T cells isolated from mice in each group. Cells were stained with anti-human CD4-PE, anti-human CD8-PE-CY^{™}7, anti-human CD45RO-APC, and anti-human CD62L-FITC (BD Biosciences), and analyzed by flow cytometry to quantify T cells with a central memory phenotype (CD45RO^{high}CD62L^{high}). *(c) Tumor-reactive CD4*+ *and CD8*+ *T cells expressing IFNγ:* To evaluate tumor-specific IFN*γ* expression in T cells, DLN or BM cells were plated (2×10⁵ cells/ well) in the presence of tumor cell lysate, non-specific control peptide, or medium. Cells were cultured for 72h followed by incubation with Golgi stop for 4h. Cells were stained extracellularly with anti-CD4-FITC and anti-CD8-APC antibodies, permeabilized, stained intracellularly with anti-IFNystained extracellularly with anti-CD4-FITC and anti-CD8-APC antibodies, permea*(d) T cell density in primary tumors:* Tumor volume was calculated, and flow cytometry was used to calculate the absolute number of CD4+ and CD8+ T cells per mm³ of tumor.

### Evaluation of immuno-inhibitory receptors/ligands in the TME:

Tumors from mice in untreated control and treatment groups (at the end of treatment) were analyzed for expression of the following immunoreceptors/ligands that inhibit T cell activation or induce T cell exhaustion: (*a*) Expression of PD-1 and PD-L1 on tumor-infiltrating T cells, DLN T cells, and tumor cells: Cells were stained with anti-human CD4-APC, CD8-FITC, and either anti-human PD-1-PE or PD-L1-PE, and the mean percentage of T cells expressing PD-1 or PD-L1 and tumor cells expressing PD-L1 were quantified by flow cytometry. The level of PD-L1 expression in tumors was also assessed by IHC. (*b*) Expression of TIM-3 and TIM-3 ligands (Galectin-9 and CEACAM-1) on tumor-infiltrating T cells, DLN T cells and tumor cells: Cells were stained with anti-human CD4-APC, CD8-FITC, and either anti-human TIM-3-PE, Galectin-9-PE, or CEACAM-1-PE (BD Biosciences), and the mean percentage of T cells expressing TIM-3 and tumor cells expressing Galectin-9 or CEACAM-1 were quantified by flow cytometry. (*c*) TGFβ signaling: Immunoblot analyses of T cells - Smad-2/3/4 expression and activity (phosphorylation).

*Assessment of signatures of immune dysfunction in TILs (elevated Tregs and*/*or T cell exhaustion).* RNA is extracted from tumors (from all treatment and control groups) and RNA-Seq was performed (including quality check, library preparation, sequencing and data processing). Mathematical deconvolution is applied to bulk RNA-Seq data using the cell type-specific reference gene expression profiles (RGEPs) from Cancer single-cell RNA sequencing data. Deconvolution is performed using a collection of differentially expressed genes that facilitate annotation of the four T-cell subtypes as naive-like, regulatory, cytotoxic, and exhausted, and the estimated proportion of each subtype is calculated for each sample¹⁰⁵. This approach is used to evaluate a relative index of four distinct T cells subtypes (CD4⁺ T_{H}1 cells, CD4⁺ Tregs, CD8⁺ cytotoxic T cells, and CD8⁺ exhausted T cells) based on the differential expression of T cell markers. The following ratios serve as numerical surrogates of the relative infiltration of tumors with different subsets of CD4⁺ or CD8⁺ TILs: (i) Treg/T_{H}1 and (ii) exhausted CD8+ T cells/cytotoxic CD8+ T cells. Simultaneously, expression of specific markers that characterize T_{H}1 cells (TBET, IFNγ cells (TBET, IFNed CD8+ T cells/cytotoxic CD8+ T cells. Simultaneously, expression of specific are measured in parallel multiplex RT-PCR reactions (QuantiTect, Qiagen) to calculate the following ratios: (i) Treg/T_{H}1 = *FOXP3*/*TBET* or *CTLA-4*/*IFN*□; (ii) exhausted T cells/cytotoxic T cells = (TIM3 + PD-1)/GZMB. RNA-Seq data is also be used to assess expression of immuno-inhibitory ligands.

Although the invention has been described with reference to the above example, it will be understood that modifications and variations are encompassed within the spirit and scope of the invention. Accordingly, the invention is limited only by the following claims.

### Clauses

**1.** A molecule comprising a targeting moiety and one or more immunomodulatory moieties, wherein:
   (a) the targeting moiety comprises a polypeptide which specifically binds a component of a tumor cell, tumor microenvironment, tumor associated growth factor or receptor, tumor associated cytokine or receptor, tumor associated T lymphocyte, T cell co-stimulatory or inhibitory molecule, immune cell, pathogen, or pathogen-associated cell, and
   (b) the immunomodulatory moiety comprises the extracellular domain (ECD) sequence or ligand binding fragment thereof of a T lymphocyte immunoreceptor, T cell inhibitory receptor (TCIR), T-cell co-inhibitory molecule, T-cell co-stimulatory molecule, B lymphocyte receptor, DC receptor, NK cell receptor, cytokine receptor, growth factor receptor, chemokine receptor, or tumor cell receptor.
**2.** The molecule of clause 1, wherein the targeting moiety polypeptide comprises an antigen-binding domain of an immunoglobulin, antibody, bispecific or multispecific antibody, antibody fragment, single chain variable fragment (scFv), bivalent or multivalent scFv, or a Fc-containing polypeptide.
**3.** The molecule of clause 1, wherein the targeting moiety polypeptide is an antibody that specifically binds a T cell inhibitory receptor (TCIR), a T cell inhibitory receptor ligand (TCIR ligand), a T-cell co-inhibitory molecule, or a T cell co-stimulatory molecule; or comprises a ligand-binding sequence from the extracellular domain (ECD) of a receptor or ligand.
**4.** The molecule of clause 2, wherein the targeting moiety is an antibody and the immunomodulatory moiety is fused to the C terminus of the heavy chain of the antibody.
**5.** The molecule of clause 2, wherein the targeting moiety is an antibody and the immunomodulatory moiety is fused to the C terminus of the light chain of the antibody.
**6.** The molecule of clause 1, wherein the targeting moiety polypeptide specifically binds one or more of Cytotoxic T lymphocyte associated antigen-4 (CTLA-4, CD 152), Programmed Death-1 protein (PD-1), Programmed death ligand-1 (PD-L1), Programmed death ligand (PD-L2), B7-H3 (CD276), T-cell immunoglobulin and mucin-domain containing-3 (TIM-3), Carcinoembryonic antigen-related cell adhesion molecule 1 (CEACAM-1), Carcinoembryonic Antigen (CEA), V domain Ig suppressor of T cell activation (VISTA), V-set and immunoglobulin domain containing 8 (VSIG8), B and T lymphocyte attenuator (BTLA), Herpesvirus Entry Mediator (HVEM), CD160, T cell Ig and ITM domain (TIGIT), CD226, CD96, Lymphocyte activation gene-3 (LAG-3), transforming growth factor β (TGF-β), transforming growth factor β receptor (TGFβR), Receptor Activator of Nuclear Factor κ B (RANK), RANK ligand (RANKL), 4-1BB (CD137), Inducible T-Cell Costimulator (ICOS), OX-40 (CD134), glucocorticoid-induced TNFR-related protein (GITR), CD27, IL6R, IL23R, IL17R, IL-6, IL-23, IL-17,CD39, CD40, CD40L, CD47, CD73,CCR4, CCR5, CXCR4, IL12R, CD4, IL-2R, CD25, CD3, gp120, Epidermal growth factor receptor (EGFR, EGFR1, ErbB-1, HER1), ErbB-2 (HER2/neu), ErbB-3/HER3, ErbB-4/HER4, Epidermal growth factor (EGF), Transforming growth factor α (TGFα), Vascular endothelial growth factor (VEGF), Vascular endothelial growth factor receptor-1 (VEGFR-1), VEGFR-2 or VEGFR-3.
**7.** The molecule of clause 1, wherein the immunomodulatory moiety comprises the extracellular ligand-binding sequence or ligand binding fragment thereof of transforming growth factor β receptor II (TGFβRII), programmed death 1 protein (PD-1), T cell immunoglobulin and mucin domain containing 3 (TIM-3), B- and T-lymphocyte attenuator (BTLA), CD160, CD226, T cell Ig and ITM domain (TIGIT), CD96, CD44, Colony stimulating factor 1 receptor (CSF1R), CCR4, Killer-cell immunoglobulin-like receptor (KIR), Vascular endothelial growth factor receptor (VEGFR), Receptor Activator of Nuclear Factor κ B (RANK), V-set and immunoglobulin domain containing 8 (VSIG8), LIGHT (TNFSF14), Leukocyte Associated Immunoglobulin-like Receptor 1 (LAIR1), or V domain Ig suppressor of T cell activation (VISTA).
**8.** The molecule of clause 1, further comprising a linker.
**9.** The molecule of clause 8, wherein the linker fuses the targeting moiety and the immunomodulatory moiety.
**10.** The molecule of clause 8, wherein the linker comprises the sequence of SEQ ID NOs: 3, 4, or 5.
**11.** The molecule of clause 1, wherein the immunomodulatory moiety comprises the ECD or a ligand binding fragment thereof of TIM-3.
**12.** The molecule of clause 11, wherein the ligand binding fragment comprises the CEACAM-1 binding fragment of TIM-3.
**13.** The molecule of clause 11, wherein the immunomodulatory moiety comprises the sequence of SEQ ID NO: 2.
**14.** The molecule of clause 1 wherein the targeting moiety is a polypeptide that binds to PD-1, PD-L1, CTLA4, LAG3, VISTA, TIGIT, BTLA, CCR4, 41BB, OX40, GITR, VEGF, RANKL, TGF-β, IL6R, EGFR, HER2/neu, or gp120 and the immunomodulatory moiety is the ECD or a ligand binding fragment thereof of TIM-3.
**15.** The molecule of clause 1 wherein the immunomodulatory moiety comprises the ECD or a ligand binding fragment thereof of CD226.
**16.** The molecule of clause 15 where in the immunomodulatory moiety comprises the sequence of SEQ ID NO: 9.
**17.** The molecule of clause 15 wherein the targeting moiety comprises a polypeptide that binds to TIM-3, BTLA, CCR4, CTLA4, LAG3, PD-1, PD-L1, TIGIT, VISTA, 41BB, OX40, GITR, RANKL, TGF-β, IL-6R, EGFR, HER2/neu, VEGF, or gp120.
**18.** The molecule of clause 1 wherein the immunomodulatory moiety is the ECD or a ligand binding fragment thereof of CD44.
**19.** The molecule of clause 15 where in the immunomodulatory moiety comprises the sequence of SEQ ID NO: 36.
**20.** The molecule of clause 1 wherein the targeting moiety comprises a polypeptide that binds to TIM-3, BTLA, CCR4, CTLA4, LAG3, PD-1, PD-L1, TIGIT, VISTA, 41BB, OX40, GITR, RANKL, TGF-β, IL-6R, EGFR, HER2/neu, VEGF, or gp120 and the immunomodulatory moiety is the ECD or a ligand binding fragment thereof of CD44.
**21.** The molecule of clause 1 wherein the immunomodulatory moiety comprises the ECD or a ligand binding fragment thereof of VSIG8.
**22.** The molecule of clause 15 where in the immunomodulatory moiety comprises the sequence of SEQ ID NO: 55.
**23.** The molecule of clause 1 wherein the targeting moiety comprises a polypeptide that binds to TIM-3, BTLA, CCR4, CTLA4, LAG3, PD-1, PD-L1, TIGIT, VISTA, 41BB, OX40, GITR, RANKL, TGF-β, IL-6R, EGFR, HER2/neu, VEGF, or gp120 and the immunomodulatory moiety is the ECD or a ligand binding fragment thereof of VSIG8.
**24.** The molecule of clause 1, wherein the molecule comprises the sequence of SEQ ID NOs: 10 and 145; 11 and 146; 12 and 147; 13 and 148; 14 and 149; 15 and 150; 16 and 151; 17 and 152; 18 and 153; 19 and 154, 20 and 155; 21 and 156; 22 and 157; 23 and 158; 24 and 159; 25 and 160; 26 and 161; 27 and 162; 28 and 163; 29 and 164; 30 and 165; 31 and 166; 32 and 167; 33 and 168; 34 and 169; 35 and 170; 37 and 171; 38 and 172; 39 and 173; 40 and 174; 41 and 175; 42 and 176; 43 and 177; 44 and 178; 45 and 179; 46 and 180; 47 and 181; 48 and 182; 49 and 183; 50 and 184; 51 and 185; 52 and 186; 53 and 187; 58 and 188; 59 and 189; 60 and 61; 62 and 63; 64 and 65; 66 and 67; 68 and 69; 70 and 71; 72 and 73; 74 and 75; 76 and 77; 78 and 79; 80 and 81; 82 and 83; 84 and 85; 86 and 87; 88 and 89; 90 and 91; 92 and 93; 94 and 95; 96 and 97; 98 and 99; 100 and 101; 102 and 103; 104 and 105; 106 and 107; 108 and 109; 110 and 111; 112 and 113; 114 and 115; 116 and 117; 118 and 119; 120 and 121; 122 and 123; 124 and 125; 126 and 127; 128 and 129; 130 and 131; 132 and 133; 134 and 135; 136 and 137; 138 and 139; 140 and 141; 142 and 143; 192 and 193; 209 and 210; 211 and 212; 221 and 222; 223 and 224; 233 and 234; 235 and 236; 245 and 246; 247 and 248; 257 and 258; 259 and 260; 269 and 270; 271 and 272; 275 and 276; 279 and 280; 283 and 284; 287 and 288; 290 and 291; 292 and 293; 294 and 295; 296 and 297 or 298 and 299.
**25.** The molecule of clause 14, wherein the targeting moiety comprises a polypeptide that binds PD-1 or PD-L1 and the immunomodulatory moiety is the ECD or ligand binding fragment thereof of TIM-3.
**26.** The molecule of clause 25, wherein the targeting moiety comprises an antibody and the immunomodulatory moiety is fused to either the heavy chain or to the light chain of the targeting moiety.
**27.** The molecule of clause 26, wherein the molecule comprises the sequence of SEQ ID NOs: 51 and 185.
**28.** The molecule of clause 26, wherein the molecule comprises the sequence of SEQ ID NOs: 52 and 186.
**29.** The molecule of clause 26, wherein the molecule comprises the sequence of SEQ ID NOs: 53 and 187.
**30.** The molecule of clause 26, wherein the molecule comprises the sequence of SEQ ID NOs: 192 and 193.
**31.** The molecule of clause 14, wherein the targeting moiety comprises a polypeptide that binds CTLA-4 and the immunomodulatory moiety is the ECD or ligand binding fragment thereof of TIM-3.
**32.** The molecule of clause 31, wherein the molecule comprises the sequence of SEQ ID NOs: 50 and 184.
**33.** The molecule of clause 14, wherein the targeting moiety comprises a polypeptide that binds LAG-3 and the immunomodulatory moiety is the ECD or ligand binding fragment thereof of TIM-3.
**34.** The molecule of clause 33, wherein the molecule comprises the sequence of SEQ ID NOs: 26 and 161.
**35.** The molecule of clause 14, wherein the targeting moiety comprises a polypeptide that binds VISTA and the immunomodulatory moiety is the ECD or ligand binding fragment thereof of TIM-3.
**36.** The molecule of clause 35, wherein the molecule comprises the sequence of SEQ ID NOs: 12 and 147.
**37.** The molecule of clause 14, wherein the targeting moiety comprises a polypeptide that binds TIGIT and the immunomodulatory moiety is the ECD or ligand binding fragment thereof of TIM-3.
**38.** The molecule of clause 37, wherein the molecule comprises the sequence of SEQ ID NOs: 17 and 152.
**39.** The molecule of clause 14, wherein the targeting moiety comprises a polypeptide that binds BTLA and the immunomodulatory moiety is the ECD or ligand binding fragment thereof of TIM-3.
**40.** The molecule of clause 39, wherein the molecule comprises the sequence of SEQ ID NOs: 22 and 157.
**41.** The molecule of clause 14, wherein the targeting moiety comprises a polypeptide that binds CCR-4 and the immunomodulatory moiety is the ECD or ligand binding fragment thereof of TIM-3.
**42.** The molecule of clause 41, wherein the molecule comprises the sequence of SEQ ID NOs: 31 and 166.
**43.** The molecule of clause 14, wherein the targeting moiety comprises a polypeptide that binds 4-1BB and the immunomodulatory moiety is the ECD or ligand binding fragment thereof of TIM-3.
**44.** The molecule of clause 43, wherein the molecule comprises the sequence of SEQ ID NOs: 116 and 117.
**45.** The molecule of clause 14, wherein the targeting moiety comprises a polypeptide that binds VEGF and the immunomodulatory moiety is the ECD or ligand binding fragment thereof of TIM-3.
**46.** The molecule of clause 45, wherein the molecule comprises the sequence of SEQ ID NOs: 88 and 89.
**47.** The molecule of clause 1, wherein the immunomodulatory moiety comprises the ECD or ligand binding fragment thereof of BTLA or LIGHT.
**48.** The molecule of clause 47, wherein the ligand binding fragment comprises an Herpesvirus entry mediator (HVEM, CD 270) binding fragment.
**49.** The molecule of clause 48, wherein the immunomodulatory moiety comprises the sequence of SEQ ID NO: 8 or 302.
**50.** The molecule of clause 1, wherein the immunomodulatory moiety comprises the ECD or ligand binding fragment thereof of CD226.
**51.** The molecule of clause 50, wherein the ligand binding fragment comprises a CD155 or CD112 binding fragment.
**52.** The molecule of clause 51, wherein the ligand binding fragment comprises the sequence of SEQ ID NO: 9.
**53.** The molecule of clause 1, wherein the immunomodulatory moiety comprises the ECD or ligand binding fragment thereof of VSIG8.
**54.** The molecule of clause 53, wherein the ligand binding fragment comprises a VISTA binding fragment.
**55.** The molecule of clause 52, wherein the ligand binding fragment comprises the sequence of SEQ ID NO: 55.
**56.** The molecule of clause 1, wherein the immunomodulatory moiety comprises the ECD or ligand binding fragment thereof of CCR4.
**57.** The molecule of clause 56, wherein the ligand binding fragment comprises a CCL22 binding fragment.
**58.** A molecule comprising a targeting moiety, a first immunomodulatory moiety and a second immunomodulatory moiety, wherein:
   (a) the targeting moiety comprises a polypeptide that specifically binds one or more of Cytotoxic T lymphocyte associated antigen-4 (CTLA-4, CD 152), Programmed Death-1 protein (PD-1), Programmed death ligand-1 (PD-L1), Programmed death ligand (PD-L2), B7-H3 (CD276), T-cell immunoglobulin and mucin-domain containing-3 (TIM-3), Carcinoembryonic antigen-related cell adhesion molecule 1 (CEACAM-1), Carcinoembryonic Antigen (CEA), V domain Ig suppressor of T cell activation (VISTA), V-set and immunoglobulin domain containing 8 (VSIG8), B and T lymphocyte attenuator (BTLA), Herpesvirus Entry Mediator (HVEM), CD160, T cell Ig and ITM domain (TIGIT), CD226, CD96, Lymphocyte activation gene-3 (LAG-3), transforming growth factor β (TGF-β), transforming growth factor β receptor (TGFβR), Receptor Activator of Nuclear Factor κ B (RANK), RANK ligand (RANKL), 4-1BB (CD137), Inducible T-Cell Costimulator (ICOS), OX-40 (CD134), glucocorticoid-induced TNFR-related protein (GITR), CD27, IL6R, IL23R, IL17R, IL-6, IL-23, IL-17,CD39, CD40, CD40L, CD47, CD73,CCR4, CCR5, CXCR4, IL12R, CD4, IL-2R, CD25, CD3, gp120, Epidermal growth factor receptor (EGFR, EGFR1, ErbB-1, HER1), ErbB-2 (HER2/neu), ErbB-3/HER3, ErbB-4/HER4, Epidermal growth factor (EGF), Transforming growth factor α (TGFα), Vascular endothelial growth factor (VEGF), Vascular endothelial growth factor receptor-1 (VEGFR-1), VEGFR-2 or VEGFR-3.
   (b) the first immunomodulatory moiety comprises the extracellular ligand-binding sequence or ligand binding fragment thereof of transforming growth factor β receptor II (TGFβRII), programmed death 1 protein (PD-1), T cell immunoglobulin and mucin domain containing 3 (TIM-3), B- and T-lymphocyte attenuator (BTLA), CD226, T cell Ig and ITIM domain (TIGIT), CD44, Colony stimulating factor 1 receptor (CSF1R), CCR4, Killer-cell immunoglobulin-like receptor (KIR), Vascular endothelial growth factor receptor (VEGFR), Receptor Activator of Nuclear Factor κ B (RANK), V-set and immunoglobulin domain containing 8 (VSIG8), LIGHT (TNFSF14), Leukocyte Associated Immunoglobulin-like Receptor 1 (LAIR1), or V domain Ig suppressor of T cell activation (VISTA); and
   (c) the second immunomodulatory moiety comprises the extracellular ligand-binding sequence or ligand binding fragment thereof of transforming growth factor β receptor II (TGFβRII), programmed death 1 protein (PD-1), T cell immunoglobulin and mucin domain containing 3 (TIM-3), B- and T-lymphocyte attenuator (BTLA), CD226, T cell Ig and ITM domain (TIGIT), CD44, Colony stimulating factor 1 receptor (CSF1R), CCR4, Killer-cell immunoglobulin-like receptor (KIR), Vascular endothelial growth factor receptor (VEGFR), Receptor Activator of Nuclear Factor κ B (RANK), V-set and immunoglobulin domain containing 8 (VSIG8), LIGHT (TNFSF14), Leukocyte Associated Immunoglobulin-like Receptor 1 (LAIR1), or V domain Ig suppressor of T cell activation (VISTA).
**59.** The molecule of clause 58, wherein the molecule comprises the sequence of SEQ ID NOs: 201 and 202; 203 and 204; 205 and 206; 207 and 208; 213 and 214; 215 and 216; 217 and 218; 219 and 220; 225 and 226; 227 and 228; 229 and 230; 231 and 232; 237 and 238; 239 and 240; 241 and 242 ; 243 and 244; 249 and 250; 251 and 252; 253 and 254; 255 and 256; 261 and 262; 263 and 264; 265 and 266; 267 and 268; 273 and 274; 277 and 278; 281 and 282; 285 and 286.
**60.** The molecule of clause 58, wherein the targeting moiety polypeptide comprises an antigen-binding domain of an immunoglobulin, antibody, bispecific or multispecific antibody, antibody fragment, single chain variable fragment (scFv), bivalent or multivalent scFv, a ligand-binding sequence from the extracellular domain (ECD) of a receptor, or Fc-containing polypeptide.
**61.** The molecule of clause 58, wherein the targeting moiety polypeptide is an antibody.
**62.** The molecule of clause 61, wherein the first immunomodulatory moiety is attached to the C-terminus of a heavy chain or a light chain of the antibody.
**63.** The molecule of clause 61, wherein the second immunomodulatory moiety is attached to the C-terminus of a heavy chain or a light chain of the antibody.
**64.** The molecule of clause 58, wherein the second immunomodulatory moiety is attached to the first immunomodulatory moiety.
**65.** The molecule of any of clauses 62-63 wherein the first and second immunomodulatory moiety is attached to the heavy chain or the light chain of the antibody with a linker.
**66.** The molecule of clause 64, wherein the second immunomodulatory moiety is attached to the first immunomodulatory moiety with a linker.
**67.** The molecule of clause 65 or 66, wherein the linker comprises the sequence of SEQ ID NOs: 3, 4 or 5.
**68.** The molecule of clauses 62 and 63, wherein the first immunomodulatory moiety is attached to the heavy chain of the antibody and the second immunomodulatory moiety is attached to the light chain of the antibody.
**69.** The molecule of clause 68, wherein the first immunomodulatory moiety comprises the ECD or ligand binding fragment thereof of TIM3.
**70.** The molecule of clause 68, wherein the second immunomodulatory moiety comprises the ECD or ligand binding fragment thereof of PD-1.
**71.** The molecule of clause 65, wherein the first immunomodulatory moiety comprises the ECD or ligand binding fragment thereof of TGFβRII and the second immunomodulatory moiety comprises the ECD or ligand binding fragment thereof of TIM-3.
**72.** The molecule of clause 71, wherein the first immunomodulatory moiety comprises the ECD or ligand binding fragment thereof of PD-1 and the second immunomodulatory moiety comprises the ECD or ligand binding fragment thereof of TIM-3.
**73.** The molecule of clauses 62 and 63, wherein the first immunomodulatory moiety comprises the ECD or ligand binding fragment thereof of TGFβRII and the second immunomodulatory moiety comprises the ECD or ligand binding fragment thereof of PD-1.
**74.** The molecule of clause 61, wherein the antibody specifically binds IL6R, CTLA-4, EGFR, HER2, or VEGF.
**75.** The molecule of clause 65, wherein the targeting moiety is an antibody that binds CTLA-4, the first immunomodulatory moiety is the ECD or ligand-binding fragment thereof of TGFβRII, and the second immunomodulatory moiety is the ECD or ligand-binding fragment thereof of TIM-3.
**76.** The molecule of clause 75, wherein the molecule comprises the sequences of SEQ ID NOs: 203 and 204.
**77.** The molecule of clause 65, wherein the targeting moiety is an antibody that binds CTLA-4, the first immunomodulatory moiety is the ECD or ligand-binding fragment thereof of TGFβRII, and the second immunomodulatory moiety is the ECD or ligand-binding fragment thereof of PD-1.
**78.** The molecule of clause 77, wherein the molecule comprises the sequences of SEQ ID NOs: 201 and 202.
**79.** The molecule of clause 65, wherein the targeting moiety is an antibody that binds CTLA-4, the first immunomodulatory moiety is the ECD or ligand-binding fragment thereof of TIM-3, and the second immunomodulatory moiety is the ECD or ligand-binding fragment thereof of PD-1.
**80.** The molecule of clause 65, wherein the targeting moiety is an antibody that binds CTLA-4, the first immunomodulatory moiety is the ECD or ligand-binding fragment thereof of PD-1 and the second immunomodulatory moiety is the ECD or ligand-binding fragment thereof of TIM-3.
**81.** The molecule of clause 79 or 80, wherein the molecule comprises the sequences of SEQ ID NOs: 205 and 206 or 207 and 208.
**82.** The molecule of clause 65, wherein the targeting moiety is an antibody that binds PD-1 or PD-L1, the first immunomodulatory moiety is the ECD or ligand-binding fragment thereof of TGFβRII, and the second immunomodulatory moiety is the ECD or ligand-binding fragment thereof of TIM-3.
**83.** The molecule of clause 82, wherein the molecule comprises the sequences of SEQ ID NOs: 273 and 274; 277 and 278; 285 and 286; or 281 and 282.
**84.** The molecule of clause 65, wherein the targeting moiety is an antibody that binds IL-6R, the first immunomodulatory moiety is the ECD or ligand-binding fragment thereof of TGFβRII, and the second immunomodulatory moiety is the ECD or ligand-binding fragment thereof of TIM-3.
**85.** The molecule of clause 84, wherein the molecule comprises the sequences of SEQ ID NOs: 215 and 216.
**86.** A fusion protein comprising the extracellular binding domain (ECD) or ligand binding fragment thereof of TIM-3 and the ECD or ligand binding fragment thereof of TGFRβII, PD-1, BTLA, LIGHT, CD226, LAIR1, or VSIG8.
**87.** The fusion protein of clause 86, wherein the fusion protein comprises the ECD or ligand binding fragment thereof of TIM-3 and the ECD or ligand binding fragment thereof of TGFβRII.
**88.** The fusion protein of clause 87, wherein the fusion protein comprises the sequence of SEQ ID NO: 190.
**89.** The fusion protein of clause 86, wherein the fusion protein comprises the ECD or ligand binding fragment thereof of TIM-3 and the ECD or ligand binding fragment thereof of PD-1.
**90.** The fusion protein of clause 89, wherein the fusion protein comprises the sequence of SEQ ID NOs: 195, 196 or 197.
**91.** The fusion protein of any of clauses 86-90 further comprising an Fc.
**92.** A composition comprising the molecule or fusion protein of any of clauses 1-90 and a pharmaceutically acceptable carrier.
**93.** A method of treating a subject having cancer or an infectious disease comprising administering to the subject the molecule or fusion protein of any of clauses 1-90 or the composition of clause 91.
**94.** The method of clause 93, where in the molecule or composition is administered by intracutaneous, subcutaneous, intravenous, intraperitoneal, intraarterial, intrathecal, intracapsular, intraorbital, intracardiac, intradermal, transdermal, transtracheal, subcuticular, intraarticulare, subcapsular, subarachnoid, intraspinal and intrasternal , oral, sublingual buccal, rectal, vaginal, nasal or ocular administrations, by infusion, inhalation, or nebulization or by parenteral administration.
**95.** The method of clause 93, wherein the cancer is selected from the group consisting of Lung cancer, Breast cancer, Colorectal cancer, Prostate cancer, Stomach cancer, Liver cancer, cervical cancer, Esophageal cancer, Bladder cancer, Non-Hodgkin lymphoma, Leukemia, Pancreatic cancer, Kidney cancer, endometrial cancer, Head and neck cancer, Lip cancer, oral cancer, Thyroid cancer, Brain cancer, Ovary cancer, Melanoma, Gallbladder cancer, Laryngeal cancer, Multiple myeloma, Nasopharyngeal cancer, Hodgkin lymphoma, Testis cancer and Kaposi sarcoma.
**96.** The method of clause 93, wherein the infectious disease is selected from the group consisting of Influenza, Tuberculosis, HIV/AIDS, Malaria, Hepatitis, Herpes Simplex Virus (HSV), and Human Papilloma Virus (HPV).
**97.** The method of clause 93, wherein a chemotherapeutic agent, immunotherapeutic agent or antibiotic is administered simultaneously with, before or following the administration of the molecule, fusion protein or composition.

## Claims

1. A fusion protein comprising a targeting moiety fused to an immunomodulatory moiety for use in treatment of cancer in a subject, wherein:
(a) the targeting moiety comprises a polypeptide comprising an antigen-binding domain of an immunoglobulin, antibody, bispecific or multispecific antibody, antibody fragment, single chain variable fragment (scFv), bivalent or multivalent scFv, or a Fc-containing polypeptide which specifically binds Epidermal growth factor receptor (EGFR), and
(b) the immunomodulatory moiety comprises a ligand binding fragment of the extracellular domain (ECD) of transforming growth factor β receptor II (TGFβRIIecd) having the amino acid sequence of SEQ ID NO: 6;
wherein a chemotherapeutic agent or immunotherapy is administered prior to, simultaneously with, or following administration of the fusion protein, wherein the chemotherapeutic agent is pembrolizumab.

2. The fusion protein for use of claim 1, wherein the targeting moiety is an antibody and the immunomodulatory moiety is fused to the C terminus of the heavy chain of the antibody.

3. The fusion protein for use of claim 1, wherein the targeting moiety is an antibody and the immunomodulatory moiety is fused to the C terminus of the light chain of the antibody.

4. The fusion protein for use of claim 3, further comprising an amino acid linker, wherein the linker fuses the targeting moiety and the immunomodulatory moiety.

5. The fusion protein for use of claim 4, wherein the linker comprises the sequence of SEQ ID NO: 4.

6. The fusion protein for use of any one of the preceding claims, wherein the fusion protein is administered by intracutaneous, subcutaneous, intravenous, intraperitoneal, intraarterial, intrathecal, intracapsular, intraorbital, intracardiac, intradermal, transdermal, transtracheal, subcuticular, intraarticulare, subcapsular, subarachnoid, intraspinal and intrasternal , oral, sublingual buccal, rectal, vaginal, nasal or ocular administrations, by infusion, inhalation, or nebulization or by parenteral administration.

7. The fusion protein for use of any one of the preceding claims, wherein the chemotherapeutic agent or immunotherapy is administered by intracutaneous, subcutaneous, intravenous, intraperitoneal, intraarterial, intrathecal, intracapsular, intraorbital, intracardiac, intradermal, transdermal, transtracheal, subcuticular, intraarticulare, subcapsular, subarachnoid, intraspinal and intrasternal, oral, sublingual buccal, rectal, vaginal or nasal ocular administrations, infusion, inhalation, or nebulization

8. The fusion protein for use of any one of the preceding claims, wherein the cancer is selected from the group consisting of Lung cancer, Breast cancer, Colorectal cancer, Prostate cancer, Stomach cancer, Liver cancer, cervical cancer, Esophageal cancer, Bladder cancer, Non-Hodgkin lymphoma, Leukemia, Pancreatic cancer, Kidney cancer, endometrial cancer, Head and neck cancer, Lip cancer, oral cancer, Thyroid cancer, Brain cancer, Ovary cancer, Melanoma, Gallbladder cancer, Laryngeal cancer, Multiple myeloma, Nasopharyngeal cancer, Hodgkin lymphoma, Testis cancer and Kaposi sarcoma.

9. The fusion protein for use of any one of the preceding claims, wherein the targeting moiety polypeptide comprises cetuximab, panitumumab or necitumumab.

10. The fusion protein for use of any one of claims 1-8, wherein the fusion protein comprises cetuximab; panitumumab; nimotuzumab; Zalutumumab; or Necitumumab, or an antigen binding fragment thereof.

11. The fusion protein for use of claim 10, wherein the fusion protein comprises an antigen binding fragment of cetuximab.

12. The fusion protein for use of claim 11, wherein the antigen binding fragment of cetuximab includes the variable region(s) alone of cetuximab.
